# EUROPEAN PATENT SPECIFICATION

(11) **EP 1 001 930 B1**
(45) Date of publication and mention of the grant of the patent: **04.12.2002**
(21) Application number: 98936014.4
(22) Date of filing: 27.07.1998
(51) Int. Cl.: C07C 259/00

(54) **N-HYDROXYFORMAMIDE DERIVATIVES AS INHIBITORS OF MATRIX METALLOPROTEINASES**
N-HYDROXYFORMAMID-DERIVATE ALS INHIBITOREN VON METALLOPROTEINASEN
INHIBITEURS D'HYDROXAMATES INVERSES DE METALLOPROTEINASES MATRICIELLES

(30) Priority: 31.07.1997 US 903632
(43) Date of publication of application: 24.05.2000
(73) Proprietor: Abbott Laboratories, Abbott Park, Illinois 60064-3500 (US)
(72) Inventor: CURTIN, Michael, L., Kenosha, WI 53142 (US); DAVIDSEN, Steven, K., Libertyville, IL 60048 (US); DELLARIA, Joseph, F., Jr., Lindenhurst, IL 60046 (US); FLORJANCIC, Alan, S., Lafayette, CO 80026 (US); GIESLER, Jamie, Oak Creek, WI 53154 (US); GONG, Jianchun, Gurnce, IL 60031 (US); GUO, Yan, Gurnee, IL 60031 (US); HEYMAN, H., Robin, Chicago, IL 60641 (US); HOLMS, James, H., Gurnee, IL 60031 (US); MICHAELIDES, Michael, R., Libertyville, IL 60048 (US); STEINMAN, Douglas, H., Morton Grove, IL (US); WADA, Carol, K., Grayslake, IL 60030 (US); XU, Lianhong, San Mateo, CA 94402 (US)
(74) Representative: Modiano, Guido, Dr.-Ing.
(86) International application number: US9815486
(87) International publication number: WO99006361

(56) References cited:
- EP-A- 0 196 184
- WO-A-94/02448
- WO-A-95/33731
- WO-A-97/18188

## Description

### Technical Field

This invention relates to compounds having activity to inhibit matrix metalloproteinases, to pharmaceutical compositions comprising these compounds and to a medical method of treatment. More particularly, this invention concerns reverse hydroxamate-containing compounds which inhibit matrix metalloproteinases, pharmaceutical compositions comprising these compounds.

### Background of the Invention

The matrix metalloproteinases (MMP's) are a class of extracellular enzymes including collagenase, stromelysin and gelatinase which are believed to be involved in the tissue destruction which accompanies a large number of disease states varying from arthritis to cancer.

Typical connective tissue cells are embedded within an extracellular matrix of high molecular weight proteins and glycoproteins. In healthy tissue, there is a continual and delicately-balanced series of processes which include cell division, matrix synthesis and matrix degradation. In certain pathological conditions, an imbalance of these three processes can lead to improper tissue restructuring. In arthritis, for example, joint mobility can be lost when there is improper remodelling of load-bearing joint cartilage. With cancer, lack of coordination of cell division and the two processes of matrix synthesis and degradation may lead to conversion of transformed cells to invasive phenotypes in which increased matrix turnover permits tumor cells to penetrate basement membranes surrounding capillaries which, in turn, may lead to subsequent metastasis.

There has been hightened interest in discovering therapeutic agents which bind to and inhibit MMP's. For example, WO A 97 18188 discloses biphenyl hydroxamate compounds having activity to inhibit matrix metalloproteinases and TNFα-secretion. The discovery of new therapeutic agents possessing this activity will lead to new drugs having a novel mechanism of action for combating disease states involving tissue degenerative processes including, for example, rheumatoid arthritis, osteoarthritis, osteopenias such as osteoporosis, periodontitis, gingivitis, corneal, epidermal or gastric ulceration, and tumor growth and metastasis or invasion.

### Summary of the Invention

In its principle embodiment, the present invention provides a matrix metalloproteinase inhibitory compound of formula (I), 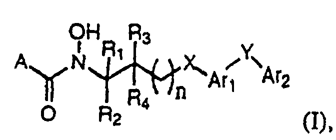 or a pharmaceutically acceptable salt or prodrug thereof, wherein
**A** is hydrogen;
**n** is zero;
**R**_{**1**} and **R**_{**3**} are independently selected from the group consisting of
(1) hydrogen and
(2) alkyl of one to 4 carbon atoms;
R₄ is hydrogen;
**R**_{**2**} is selected from the group consisting of
(1) hydrogen;
(2) alkyl of one to 4 carbon atoms;
(3) hydroxyalkyl, wherein the alkylene group is of one to 4 carbon atoms;
(4) (alkylene)-S(O)ₚ-alkyl, wherein the alkylene is of one to 4 carbon atoms, and the alkyl is is of one to 4 carbon atoms and p is 0 or 2;
(5) phenyl;
(6) phenylalkoxyalkyl, wherein the alkylene and alkyl groups are independently of one to 4 carbon atoms;
(7) phenylalkyl, wherein the alkylene group is of one to 4 carbon atoms;
(8) phenoxyalkyl, wherein the alkylene group is of one to 4 carbon atoms;
(9) (alkylene)-N(R₅)SO₂-phenyl, wherein the alkylene is of one to 4 carbon atoms, and wherein R₅ is alkyl of one to 2 carbon atoms;
(10) (heterocycle)oxyalkyl, wherein the alkylene group is of one to 4 carbon atoms and the heterocycle is 4-methyl-2-oxo-2H-1-benzopyranyl;
(11) -(alkylene)-heterocycle, wherein the alkylene group is of one to 4 carbon atoms; and the heterocycle is 1,6-dihydro-3-methyl-6-oxo-1-pyridazinyl;
(12) -(alkylene)-NR₆R₇, wherein the alkylene group is of one to 4 carbon atoms,
wherein for (5)-(9) the phenyl, and the phenyl parts of phenylalkoxyalkyl, phenylalkyl, -(alkylene)-N(R₅)SO₂-phenyl and phenoxyalkyl, are optionally substituted with one or two, substituents independently selected from the group consisting of
(a) alkyl of one to 4 carbon atoms;
(b) halo;
(c) -N(R₅)SO₂-alkyl,
(d) phenyl, wherein the phenyl is optionally substituted with one cyano substituent; and
R₆ and R₇, taken together with the nitrogen atom to which they are attached, define a group selected from the group consisting of
(1) phthalimidyl;
(2) succinimidyl;
(3) 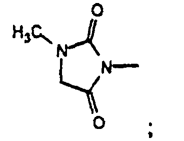
(4) 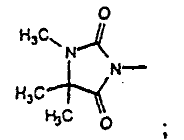
(5) 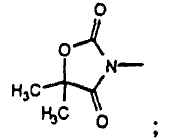
(6) 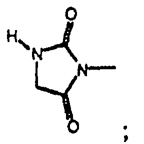
(7) 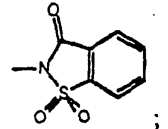
(8) 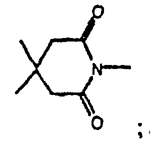 and
(9) 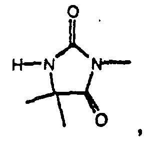
wherein option (2) is optionally substituted with one or two alkyl of 1 to 4 carbon atom substituents and option (9) is optionally substituted with one phenylmethyl substituent;
**X** is selected from the group consisting of
(1) -O- and
(2) -S(O)ₚ-; wherein p is zero or two;
**Ar**_{**1**} is phenyl which is optionally substituted with one nitro substituent
**Y** is selected from the group consisting of
(1) a covalent bond,
(2) -O-,
(3) alkenylene of two to four carbon atoms,
(4) piperidinyl, and
(5) -S(O)ₚ- and
**Ar**_{**2**} is an aryl group selected from the group consisting of
(1) phenyl;
(2) pyridyl;
(3) furyl; and
(4) thienyl;
   wherein the aryl group is optionally substituted with one, two, or three substituents independently selected from the group consisting of
   (a) alkyl of one to 4 carbon atoms;
   (b) alkoxy of one to 4 carbon atoms;
   (c) alkoxy of one to 4 carbon atoms substituted with alkoxy of one to 4 carbon atoms;
   (d) cyano;
   (e) cyanoalkyl of one to 4 carbon atoms;
   (f) halo;
   (g) haloalkyl of one to 4 carbon atoms;
   (h) thioalkoxy of one carbon atom;
   (i) perfluoroalkyl of one carbon atom;
   (j) perfluoroalkoxy of one carbon atom;
   (k) sulfinylalkyl, wherein the alkyl part is of one to 4 carbon atoms;
   (l) sulfonylalkyl, wherein the alkyl part is of one to 4 carbon atoms;
   (m) 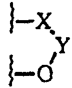 where X is -O-, and Y is -(C(R")₂)ᵥ-, where R" is hydrogen.

In another aspect, the present invention provides pharmaceutical compositions which comprise a therapeutically effective amount of compound of formula I in combination with a pharmaceutically acceptable carrier.

In yet another aspect, the present invention provides the use of these compounds for manufacturing a medicament for inhibiting matrix metalloproteinases in a host mammal in need of such treatment comprising administering to a mammal in need of such treatment a therapeutically effective amount of a compound of formula I.

### Detailed Description of the Invention

### Definition of Terms

As used throughout this specification and the appended claims, the following terms have the meanings specified:

The term "alkyl," as used herein, represents a monovalent group derived from a straight or branched chain saturated hydrocarbon by the removal of a single hydrogen atom and is exemplified by methyl, ethyl, n- and iso-propyl, n-, sec-, iso- and tert-butyl, neopentyl and the like.

The term "alkanoyl," as used herein, represents an alkyl group, as defined above, attached to the parent molecular group through a carbonyl group and is exemplified by formyl, acetyl, propionyl, butanoyl and the like.

The term "alkenyl," as used herein, represents monovalent straight or branched chain groups containing a carbon-carbon double bond derived from an alkene by the removal of one hydrogen atom and is exemplified by ethenyl, 1-propenyl, 2-propenyl, 2-methyl-1-propenyl, 1-butenyl, 2-butenyl and the like.

The term "alkoxy," as used herein, represents an alkyl group attached to the parent molecular group through an oxygen atom and is exemplified by methoxy, isopropoxy, tert-butoxy and the like.

The term "alkoxyalkyl" as used herein, represents an alkyl group to which is attached an alkoxy group.

The term "alkoxycarbonyl," as used herein, represents an ester group, i.e. an alkoxy group attached to the parent molecular group through a carbonyl group, and is exemplified by methoxycarbonyl, ethoxycarbonyl and the like.

The term "alkoxycarbonylalkyl," as used herein, represents an alkyl group, as defined above, substituted by a alkoxycarbonyl group.

The term "alkylene," as used herein, represents a saturated divalent hydrocarbon group derived from a straight or branched chain saturated hydrocarbon by the removal of two hydrogen atoms and is exemplified by methyene, ethylene, isopropylene and the like.

The term "alkynyl," as used herein, represents represents monovalent straight or branched chain groups of two to six carbon atoms containing a carbon-carbon triple bond derived from an alkyne by the removal of one hydrogen atom and is exemplified by ethynyl, 1-propynyl, and the like.

The term "benzyloxy," as used herein, represents phenyl-(CH₂)-O-.

The term "cyano," as used herein, represents a -CN group.

The term "cyanoalkyl," as used herein, represents a cyano group attached to the parent molecular moiety through an alkyl group.

The term "cycloalkyl," as used herein represents a monovalent saturated cyclic hydrocarbon group and is exemplified by cyclopropyl. cyclobutyl, cyclopentyl, cyclohexyl, cycloheptyl, bicyclo[2.2.1]heptyl and the like.

The term "cycloalkylalkyl," as used herein, represents a cycloalkyl group attached to the parent molecular group through an alkylene group.

The term "halo," as used herein, represents F, Cl, Br and I.

The term "haloalkyl," as used herein, represents an alkyl group substituted by one, two, three or four halogen atoms and is exemplified by chloromethyl, bromoethyl, chlorodifluoromethyl and the like.

The term "heterocycle," as used herein, represents a five-, six- or seven-membered ring containing one, two or three heteroatoms independently selected from the group consisting of nitrogen, oxygen and sulfur. The five-membered ring has zero to two double bonds and the six- and seven-membered rings have zero to three double bonds. Hererocycles include indolyl, quinolyl, isoquinolyl, tetrahydroquinolyl, benzofuryl, benzothienyl, pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, homopiperidinyl, pyrazinyl, piperazinyl, pyrimidinyl, pyridazinyl, oxazolyl, oxazolidinyl, isoxazolyl, isoxazolidinyl, morpholinyl, thiomorpholinyl, thiomorpholino sulfone, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl, thiazolidinyl, isothiazolyl, triazolyl, tetrazolyl, oxadiazolyl, thiadiazolyl, pyrimidyl, tetrahydrofuranyl, dihydrofuranyl, tetrahydrothienyl, dihydrothienyl, dihydroindolyl, tetrahydroquinolyl, tetrahydroisoquinolyl, pyranyl, dihydropyranyl, dithiazolyl, benzofuranyl, benzothienyl, succinimidyl, maleimidyl, glutarimidyl, phthalimidyl, naphthalimidyl and the like.

Heterocycles also include 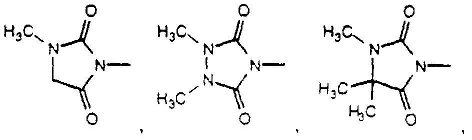 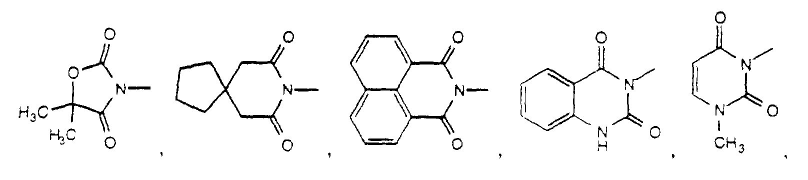 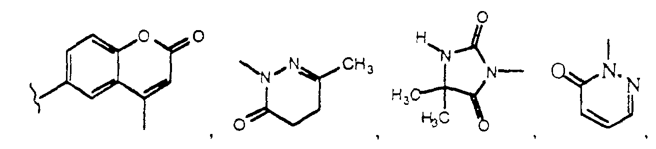 and the like.
The term "(heterocycle)oxy," as used herein, represents a heterocvcle group attached to the parent molecular moiety through oxygen.

The term "(heterocycle)oxyalkyl," as used herein, represents a (heterocycle)oxy group attached to the parent molecular group through an alkyl group.

The term "hydroxy" as used herein, represents an -OH group.

The term "hydroxyalkyl," as used herein, represents an alkyl group, as defined above, substituted by one to three hydroxy groups, with the proviso that no more than one hydroxy group may be attached to a single carbon atom of the alkyl group and is exemplified by hydroxymethyl, dihydroxypropyl and the like.

The term "nitro," as used herein, refers to -NO₂.

The term "perfluoroalkyl," as used herein, represents an alkyl group, as defined herein, wherein each hydrogen radical bound to the alkyl group has been replaced by a fluoride radical. Perfluoroalkyl groups are exemplified by trifluoromethyl, pentafluoroethyl, and the like.

The term "perfluoroalkoxy," as used herein, refers to a perfluoroalkyl group, as defined herein, attached to the parent molecular group through an oxygen atom.

The term "pharmaceutically acceptable salt," as use herein, represents those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well known in the art. For example, S. M Berge, et al. describe pharmaceutically acceptable salts in detail in J. Pharmaceutical Sciences, 1977, 66:1-19. The salts can be prepared in situ during the final isolation and purification of the compounds of the invention or separately by reacting the free base group with a suitable organic acid. Representative acid addition salts include acetate, adipate, alginate, ascorbate, aspartate, benzenesulfonate, benzoate, bisulfate, borate, butyrate, camphorate, camphersulfonate, citrate, cyclopentanepropionate, digluconate, dodecylsulfate, ethanesulfonate, fumarate. glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts and the like. Representative alkali or alkaline earth metal salts include sodium, lithium, potassium, calcium, magnesium and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine and the like.

The term "phenoxy," as used herein, represents a phenyl group attached to the parent molecular group through an oxygen atom.

The term "phenoxyalkyl," as used herein, represents a phenoxy group attached to the parent molecular group through an alkyl group.

The term "phenyl," as used herein, represents a 6-membered, monocyclic, aromatic carbocyclic ring.

The term "phenylalkyl," as used herein, represents an phenyl group attached to the parent molecular group through an alkylene group and is exemplified by benzyl, phenethyl and the like.

The term "phenylalkoxy," as used herein, represents a phenyl group attached to the parent molecular group through an alkoxy group.

The term "phenylalkoxyalkyl" as used herein, represents a phenylalkoxy group, as defined above, attached to the parent molecular group through an alkyl group.

The term "prodrug," as used herein, represents compounds which are rapidly transformed in vivo to the parent compound of the above formula, for example, by hydrolysis in blood. A thorough discussion is provided in T. Higuchi and V. Stella, Prodrugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference. Prodrugs of the compounds of the present invention are, within the scope of sound medical judgement, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention.

The term "spiroalkyl," as used herein, represents an alkylene diradical, both ends of which are bonded to the same carbon atom of the parent group to form a spirocyclic group.

The term "sulfinyl," as used herein, refers to an -S(O)- group.

The term "sulfinylalkyl," as used herein, refers to an alkyl group, as defined herein. attached to the parent mplecular group through a sulfinyl group.

The term "sulfonyl," as used herein, refers to an -SO₂- group.

The term "sulfonylalkyl," as used herein, refers to an alkyl group, as defined herein, attached to the parent mplecular group through a sulfonyl group.

The term "thioalkoxy," as used herein, represents represents an alkyl group attached to the parent molecular group through a sulfur atom.

Compounds of the present invention may exist as stereoisomers, wherein asymmetric or chiral centers are present. These compounds are designated by the symbols "R" or "S," depending on the configuration of subsitiuents around the chiral carbon atom. The present invention contemplates various stereoisomers and mixtures thereof. Stereoisomers include enantiomers and diastereomers, and mixtures of enantiomers or diastereomers are designated (±). Individual stereoisomers of compounds of the present invention may be prepared synthetically from commercially available starting materials which contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary or (2) direct separation of the mixture of optical enantiomers on chiral chromatographic columns.

### Preferred Embodiments

Preferred compounds of the present invention have formula (I), wherein
A is hydrogen;
R₁, R₃ and R₄ are H;
X is selected from the group consisting of
(1)-O-;
(2) -C(O)-;
(3) -S(O)ₚ-, wherein p is 2, and
(4) -NR₅SO₂-;
Ar₁ is phenyl;
Y is selected from the group consisting of a
(1) covalent bond and
(2) -O-; and
n is zero;
More preferred compounds of the present invention have formula (I), wherein
A is hydrogen
R₁, R₃ and R₄ are H;
X is -O-;
Ar₁ is phenyl;
Y is a covalent bond; and
n is zero.

Preferred compounds falling within the scope of formula (I) include but are not limited to:
(±)-N-[1-[[(4'-cyano-[1,1'-biphenyl]-4-yl)oxy]methyl]-2-phenoxyethyl]-N-hydroxyformamide,
(±)-N-[1-[[(4'-cyano-[1,1'-biphenyl]-4-yl)oxy]methyl]-2-(2,3-dihydro-1,3-dioxo-1H-isoindol-2-yl)ethyl]-N-hydroxyformamide,
(±)-N-[1-[[(4'-cyano-[1,1'-biphenyl]-4-yl)oxy]methyl]-2-(3,4,4-trimethyl-2,5-dioxo-1-imidazolidinyl)ethyl]-N-hydroxyformamide,
(±)-N-[1-[[(4'-cyano-[1,1'-biphenyl]-4-yl)oxy]methyl]-3-(3,4,4-trimethyl-2,5-dioxo-1-imidazolidinyl)propyl]-N-hydroxyformamide,
(±)-N-[1-[[[3'-(cyanomethyl]-[1,1'-biphenyl]-4-yl]oxy]methyl]pentyl]-N-hydroxyformamide,
(±)-N-[1-[[(4'-cyano-[1,1'-biphenyl]-4-yl)oxy]methyl]-3-methylbutyl]-N-hydroxyformamide,
(±)-N-[1-[[(4'-cyano-[1,1'-biphenyl]-4-yl)oxy]methyl]-2-methylbutyl]-N-hydroxyformamide,
(±)-N-[1-[[(4'-cyano-[1,1'-biphenyl]-4-yl)oxy]methyl]pentyl]-N-hydroxyformamide,
(±)-N-[1-[[(4'-cyano-[1,1'-biphenyl]-4-yl)oxy]methyl]-2-(4-methylphenyl)ethyl]-N-hydroxyformamide,
(±)-N-[2-[(4-cyano-[1,1'-biphenyl]-4-yl)oxy]-1-(4-fyuorophenyl)ethyl]-N-hydroxyformamide,
(±)-N-[1-[[(4'-cyano-[1,1'-biphenyl]4-yl)oxy]methyl]-2-(4-fluorophenyl)ethyl]-N-hydroxyformamide,
(±)-N-[1-[[(4'-cyano-[1,1'-biphenyl]-4-yl)oxy]methyl]ethyl]-N-hydroxyformamide,
N-[2-[(4'-cyano-[1,1'-biphenyl]-4-yl)oxy]ethyl]-N-hydroxyacetamide,
N-[2-[(4'-cyano-[1,1'-biphenyl)-4-yl)oxy]ethyl]-N-hydroxyformamide,
(±)-N-[1-[4-[(2E-phenylethenyl)phenoxy]methyl]-2-(3,4,4-trimethyl-2,5-dioxo-1-imidazolidinyl)ethyl]-N-hydroxyformamide,
(±)-N-[1-[[4-(2-furanyl)phenoxy]methyl]-2-(3,4,4-trimethyl-2,5-dioxo-1-imidazolidinyl)ethyl]-N-hydroxyformamide,
(±)-N-[1-[[(4'-butoxy[1,1'-biphenyl]-4-yl)oxy]methyl]-2-(3,4,4-trimethyl-2,5-dioxo-1-imidazolidinyl)ethyl]-N-hydroxyformamide,
(±)-N-[1-[[(4'-fluoro[1,1'-biphenyl]-4-yl)oxy]methyl]-2-(3,4,4-trimethyl-2,5-dioxo-1-imidazolidinyl)ethyl]-N-hydroxyformamide,
(±)-N-[1-[(3,4,4-trimethyl-2,5-dioxo-1-imidazolidinyl)methyl]-2-[[4'-(trifluoromethyl)[1,1'-biphenyl]-4-yl]oxy]ethyl]-N-hydroxyformamide,
(±)-N-[1-[[(4'-methoxy[1,1'-biphenyl]-4-yl)oxy]methyl]-2-(3,4,4-trimethyl-2,5-dioxo-1-imidazolidinyl)ethyl]-N-hydroxyformamide,
(±)-N-[1-[[(4'-methyl[1,1'-biphenyl]-4-yl)oxy]methyl]-2-(3,4,4-trimethyl-2,5-dioxo-1-imidazolidinyl)ethyl]-N-hydroxyformamide,
(±)-N-[1-[[(4'-butoxy[1,1'-biphenyl]-4-yl)oxy]methyl]-2-(4,4-dimethy-2,5-dioxo-1-imidazolidinyl)ethyl]-N-hydroxyformamide,
(±)-N-[1-[(4,4-dimethy-2,5-dioxo-1-imidazolidinyl)methyl]-2-[(4'-ethoxy[1,1'-biphenyl]-4-yl)oxy]ethyl]-N-hydroxyformamide,
(±)-N-[1-[[4-(1,3-benzodioxol-5-yl)phenoxy]methyl]-2-(4,4-dimethy-2,5-dioxo-1-imidazolidinyl)ethyl]-N-hydroxyformamide,
(±)-N-[1-[[(4'-butoxy[1,1'-biphenyl]-4-yl)oxy]methyl]-2-(3-methy-2,5-dioxo-1-imidazolidinyl)ethyl]-N-hydroxyformamide,
(±)-N-[1-[[4-(3-thienyl)phenoxy]methyl]-2-(3,4,4-trimethyl-2,5-dioxo-1-imidazolidinyl)ethyl]-N-hydroxyformamide,
(±)-N-[1-[[([1,1'-biphenyl]-4-yl)oxy]methyl]-2-(3,4,4-trimethyl-2,5-dioxo-1-imidazolidinyl)ethyl]-N-hydroxyformamide,
(±)-N-[1-[[(3'-chloro-4'-fluoro[1,1'-biphenyl]-4-yl)oxy]methyl]-2-(3,4,4-trimethyl-2,5-dioxo-1-imidazolidinyl)ethyl]-N-hydroxyformamide,
(±)-N-[1-[[(2'-methyl[1,1'-biphenyl]-4-yl)oxy]methyl]-2-(3,4,4-trimethy-2,5-dioxo-1-imidazolidinyl)ethyl]-N-hydroxyformamide,
(±)-N-[1-[[(4'-cyanol[1,1'-biphenyl]-4-yl)oxy]methyl]-2-(2,5-dioxo-1-imidazolidinyl)ethyl]-N-hydroxyformamide,
(±)-N-[1-[[(4'-cyanol[1,1'-biphenyl]-4-yl)oxy]methyl]-2-(1,1-dioxido-3-oxo-1,2-benzisothiazol-2(3H)-yl)ethyl]-N-hydroxyformamide,
(±)-N-[1-[(4,4-dimethy-2,5-dioxo- 1-imidazolidinyl)methyl]-2-[[4'-(trifluoromethoxy)[1,1'-biphenyl]-4-yl]oxy]ethyl]-N-hydroxyformamide,
(±)-N-[1-[[4-(4-phenyl-1-piperidinyl)phenoxy]methyl]-2-(3,4,4-trimethyl-2,5-dioxo-1-imidazolidinyl)ethyl]-N-hydroxyformamide,
(±)-N-[1-[(4,4-dimethy-2,5-dioxo- 1-imidazolidinyl)methyl]-2-[[4'-(trifluoromethyl)[1,1'-biphenyl]-4-yl]oxy]ethyl]-N-hydroxyformamide,
(±)-N-[1-[[(3'-cyano[1,1'-biphenyl]-4-yl)oxy]methyl]-2-[methyl[(4-methylphenyl)sulfonyl]amino]ethyl]-N-hydroxyformamide,
(±)-N-[1-[[(4'-cyano[1,1'-biphenyl]-4-yl)oxy]methyl]-2-[4,4-dimethy-2,5-dioxo-3-(3-pyridinylmethyl)-1-imidazolidinyl]ethyl]-N-hydroxyformamide,
(±)-N-[2-[(4'-cyano[1,1'-biphenyl]-4-yl)oxy]-1-methylpropyl]-N-hydroxyformamide,
(±)-N-[1-[[(3'-cyano[1,1'-bipheny]]-4-yl)oxy]methyl]-2-(3,4,4-trimethyl-2,5-dioxo-1-imidazolidinyl)ethyl]-N-hydroxyformamide,
(±)-N-[1-[[[4'-(methylthio)[1,1'-biphenyl)-4-yl]oxy]methyl]-2-(3,4,4-trimethyl-2,5-dioxo-1-imidazolidinyl)ethyl]-N-hydroxyformamide.
(±)-N-[1-[4-[[4-(trifluoromethyl)phenoxy]phenoxy]methyl]-2-(3,4,4-trimethyl-2,5-dioxo-1-imidazolidinyl)ethyl]-N-hydroxyformamide.
(±)-N-[1-[[[4'-(trifluoromethoxy)[1,1'-biphenyl]-4-yl]oxy|methyl]-2-(3,4,4-trimethy-2,5-dioxo-1-imidazolidinyl)ethyl]-2-N-hydroxyformamide,
(±)-N-[1-[[[4'-(methylsulfonyl)[1,1'-biphenyl]-4-yl]oxy]methyl]-2-(3,4,4-trimethy-2,5-dioxo-1-imidazolidinyl)ethyl]-N-hydroxyformamide,
(±)-N-[1-[[[3'-(cyanomethyll)-4'-methoxyl[1,1'-biphenyl]-4-yl]oxy]methyl]-2-(3,4,4-trimethy-2,5-dioxo-1-imidazolidinyl)ethyl]-N-hydroxyformamide,
(±)-N-[1-[[[3'-(cyanomethyl)(1,1'-biphenyl]-4-yl]oxy]methyl]-3-(4,4-dimethyl-2,5-dioxo-1-imidazolidinyl)propyl]]-N-hydroxyformamide,
(±)-N-[1-[[(4'-butoxy[1,1'-biphenyl]-4-yl)sulfonyl]methyl]-2-(4,4-dimethy-2,5-dioxo-1-imidazolidinyl)ethyl]-N-hydroxyformamide,
(±)-N-[1-[[(4'-cyano[1,1'-biphenyl]-4-yl)oxy]methyl]-3-(4,4-dimethyl-2,5-dioxo-1-imidazolidinyl)propyl]-N-hydroxyformamide,
(±)-N-[1-[[[4'-(methylsulfonyll)[1,1'-biphenyl]-4-yl)oxy]methyl]-2-(4,4-dimethyl-2,5-dioxo-1-imidazolidinyl)ethyl]-N-hydroxyformamide,
(±)-N-[1-[[(3'-cyano[1,1'-biphenyl]-4-yl)oxy)methyl]-2-(2,5-dioxo-1-pyrrolidinyl)ethyl]-N-hydroxyformamide,
(±)-N-[1-[[(4'-cyano[1,1'-biphenyl)-4-yl)oxy]methyl]-2-(4,4-dimethyl-2,6-dioxo-1-piperidinyl)ethyl]-N-hydroxyformamide,
N-[1S-[[(4'-cyano[1,1'-biphenyl]-4-yl)oxy]methyl]-2-(2,5-dioxo-1-pyrrolidinyl)ethyl]-N-hydroxyformamide,
N-[1R-[[(4'-cyano[1,1'-biphenyl]-4-yl)oxy]methyl]-2-(2,5-dioxo-1-pyrrolidinyl)ethyl]-N-hydroxyformamide,
(±)-N-[1-[[(4' -cyano[1,1'-biphenyl]-4-yl)oxy]methyl]-2-(3-ethyl-3-methyl-2,5-dioxo-1-pyrrolidinyl)ethyl]-N-hydroxyformamide,
(±)-N-[4-[4-[[(4-chlorophenoxy)phenyl]sulfonyl]methyl]tetrahydro-2H-pyran-4-yl]-N-hydroxyformamide,
(±)-N-[1-[[(4'-cyano[1,1'-biphenyl]-4-yl)oxy}methyl]-5-[(4-methyl-2-oxo-2H-1-benzopyran-6-yl)oxy]pentyl]-N-hydroxyformamide,
(±)-N-[1-[[(4'-cyano[1,1'-biphenyl]-4-yl)oxy]methyl]-4-[(4-methyl-2-oxo-2H-1-benzopyran-6-yl)oxy]butyl]-N-hydroxyformamide,
(±)-N-[1-[[(4'-cyano[1,1'-biphenyl]-4-yl)oxy]methyl]-4-[(4-methyl-2-oxo-2H-1-benzopyran-7-yl)oxy]butyl]-N-hydroxyformamide,
(±)-N-[1-[[(4'-cyano[1,1'-biphenyl]-4-yl)oxy]methyl]-5-[(4-methyl-2-oxo-2H-1-benzopyran-7-yl)oxy]pentyl]-N-hydroxyformamide,
(±)-N-[1-[[(4'-cyano[1,1'-biphenyl]-4-yl)oxy)methyl)-2-(5,5-dimethy-2,4-dioxo-3-oxazolidinyl)ethyl]-N-hydroxyformamide,
(±)-N-[1-[[(4'-cyano[1,1'-biphenyl]-4-yl)sulfonyl]methyl]-2-(3,4,4-trimethy-2,5-dioxo-1-imidazolidinyl)ethyl]-N-hydroxyformamide,
(±)-N-[1-[[(4'-cyano[1,1'-biphenyl]-4-yl)oxy]methyl]-2-(3-methyl-2,5-dioxo-1-imidazolidinyl)ethyl]-N-hydroxyformamide,
(±)-N-[1-[[(4' -cyano[1,1'-biphenyl]-4-yl)sulfonyl]methyl]-2-(4,4-dimethy-2,5-dioxo-1-imidazolidinyl)ethyl]-N-hydroxyformamide,
(±)-N-[1-[[(4'-chloro-[1,1'-biphenyl]-4-yl)oxy]methyl]-2-(3,4,4-trimethyl-2,5-dioxo-1-imidazolidinyl)ethyl]-N-hydroxyformamide,
(±)-N-[1-[[(3'-cyanomethyl-[1,1'-biphenyl]-4-y)oxy]methyl]-2-(3,5,5-trimethyl-2,4-dioxo-1-imidazolidinyl)propyl]-N-hydroxyformamide,
(±)-N-[1-[[(4'-cyano-[1,1'-biphenyl]-4-y)oxy]methyl]-2-isopropylthioethyl]-N-hydroxyformamide,
(±)-N-[1-[[(3'-cyanomethyl-[1,1'-biphenyl]-4-y)oxy]methyl]-2-(3,4,4-trimethyl-2,5-dioxo-1-imidazolidinyl)ethyl]-N-hydroxyformamide,
(±)-N-[1-[[(4'-cyano-[1,1'-biphenyl]-4-yl)oxy]methyl]-2-(3-ethyl-4,4-dimethyl-2,5-dioxo-1-imidazolidinyl)ethyl]-N-hydroxyformamide,
(±)-N-[1-[[(4'-cyano-[1,1'-biphenyl]4-yl)oxy]methyl]-2-(3-benzyl-4,4-dimethyl-2,5-dioxo-1-imidazolidinyl)ethyl]-N-hydroxyformamide,
(±)-N-[1-[[(4'-cyano-[1,1'-biphenyl]-4-y)oxy]methyl]-2-(3,5,5-trimethyl-2,4-dioxo-1-imidazolidinyl)ethyl]-N-hydroxyformamide,
(±)-N-[1-[[4'-methoxy[1,1'-biphenyl]-4-yl)sulfonyl]methyl]ethyl]-N-hydroxyformamide,
(±)-N-[1-[[(4'-chloro[1,1'-biphenyl]-4-yl)sulfonyl]methyl]ethyl]-N-hydroxyformamide,
(±)-N-[1-[[[4-(1,3-benzodioxol-5-yl)phenyl]sulfonyl]methyl]ethyl]-N-hydroxyformamide,
(±)-N-[1-[[[4-(4-chlorophenoxy)phenyl]sulfonyl]methyl]ethyl]-N-hydroxyformamide,
(±)-N-[1-[[(4-methoxy[1,1'-biphenyl]-4-yl)sulfonyl]methyl]propyl]-N-hydroxyformamide,
(±)-N-[1-[1,1-dimethyl-2-[(4'-(trifluoromethyl)[1,1'-biphenyl]-4-yl]sulfonyl]ethyl]-N-hydroxyformamide,
(±)-N-[1-[(phenylmethoxy)methyl]-2-[[4'-(trifluoromethyl)[1,1'-biphenyl]-4-yl]sulfonyl]ethyl]-N-hydroxyformamide,
(±)-N-[1-(hydroxymethyl)-2-[[(4'-(trifluoromethyl)[1,1'-biphenyl]-4-yl]sulfonyl]ethyl]-N-hydroxyformamide,
(±)-N-[1-[(4.4-dimethy-2,5-dioxo-1-imidazolidinyl)methyl]-2-[[4'-(trifluoromethyl)[1,1'-biphenyl]-4-yl]thio]ethyl]-N-hydroxyformamide.
(±)-N-[1-[(4,4-dimethy-2,5-dioxo-1-imidazolidinyl)methyl]-2-[[4'-(trifluoromethyl)[1,1'-biphenyl]-4-yl]sulfonyl]ethyl]-N-hydroxyformamide,
(±)-N-[1-[(2,5-dioxo-1-imidazolidinyl)methyl]-2-[[4'-(trifluoromethoxy)[1,1'-biphenyl]-4-yl]oxy]ethyl]-N-hydroxyformamide,
(±)-N-[1-[[[4'-(trifluoromethyl)[1,1'-biphenyl]-4-yl]sulfonyl]methyl]-2-(3,4,4-trimethyl-2,5-dioxo-1-imidazolidinyl)ethyl]-N-hydroxyformamide,
(±)-N-[1-[[(4'-butyl[1,1'-biphenyl]-4-yl)oxy]methyl]-2-(3-methy-2,5-dioxo-1-imidazolidinyl)ethyl]-N-hydroxyformamide,
(±)-N-[1-[(3-methy-2,5-dioxo-1-imidazolidinyl)methyl]-2-[[4'-(trifluoromethoxy)[1,1'-biphenyl]-4-yl]oxy)ethyl]-N-hydroxyformamide,
(±)-N-[4-[4-[(4'-chloro[1,1'-biphenyl]-4-yl)sulfonyl]methyl]tetrahydro-2H-pyran-4-yl]-N-hydroxyformamide,
(±)-N-[1-[[[4-(4-chlorophenoxy)phenyl]sulfonyl]methyl]-2-(4,4-dimethyl-2,5-dioxo-1-imidazolidinyl)ethyl]-N-hydroxyformamide,
(±)-N-[1-[[[4-(4-chlorophenoxy)phenyl]sulfonyl]methyl]-2-(3,4,4-trimethyl-2,5-dioxo-1-imidazolidinyl)ethyl]-N-hydroxyformamide,
(±)-N-[1-[[(4-butyl[1,1'-biphenyl]-4-yl)sulfonyl]methyl]-2-(3,4,4-trimethyl-2,5-dioxo-1-imidazolidinyl)ethyl]-N-hydroxyformamide,
(±)-N-[1-[[(4'-butyl[1,1'-biphenyl]-4-yl)oxy]methyl]-2-(4,4-dimethy-2,5-dioxo-1-imidazolidinyl)ethyl]-N-hydroxyformamide,
(±)-N-[1-[[[3'-(cyanomethyl)[1,1'-biphenyl]-4-yl]oxy]methyl]-2-(4,4-dimethy-2,5-dioxo-1-imidazolidinyl)ethyl]-N-hydroxyformamide,
(±)-N-[1-[4-(2-thienyl)phenoxy]methyl]-2-[1-(3,4,4-trimethyl-2,5-dioxo-1-imidazolidinyl)ethyl]-N-hydroxyformamide,
(±)-N-[1-[[(3-nitro[1,1'-biphenyl]-4-yl)oxy]methyl]-2-(3,4,4-trimethyl-2,5-dioxo-1-imidazolidinyl)ethyl]-N-hydroxyformamide,
(±)-N-[1-[[(4'-methyl[1,1'-biphenyl]-4-yl)oxy]methyl]-2-[[3-(methylsulfonyl)amino]phenyl]ethyl]-N-hydroxyformamide,
(±)-N-[1-[[[3-(diethylamino)carbonyl]phenyl]methyl]-2-[(4'-methyl[1,1'-biphenyl]-4-yl)oxy]ethyl]-N-hydroxyformamide,
N-[1-[[(4'-cyano[1,1'-biphenyl]-4-yl)oxy]methyl]-2-[(4'-cyano[1,1'-biphenyl]-4-yl)oxy]ethyl]-N-hydroxyformamide,
(±)-N-[1-[[(4'-cyano[1,1'-biphenyl]-4-yl)oxy]methyl]-2-(4,4-dimethy-2,5-dioxo-1-imidazolidinyl)ethyl]-N-hydroxyformamide,
(±)-N-[1-[(4,4-dimethyl-2,5-dioxo-1-imidazolidinyl)methyl]-2-[[4'-(2-methoxyethoxy)[1,1'-biphenyl]-4-yl]oxy]ethyl]-N-hydroxyformamide,
(±)-N-[1-[(4,4-dimethy-2,5-dioxo-1-imidazolidinyl)methyl]-2-[(4'-propoxy[1,1'-biphenyl]-4-yl)oxy]ethyl]-N-hydroxyformamide,
(±)-N-[1-[(4,4-dimethy-2,5-dioxo-1-imidazolidinyl)methyl]-2-[(4'-pentyloxy[1,1'-biphenyl]-4-yl)oxy]ethyl]-N-hydroxyformamide,
(±)-N-[1-[[[3'-(cyanomethyl)[1,1'-biphenyl]-4-yl]sulfonyl]methyl]-3-(4,4-dimethy-2,5-dioxo-1-imidazolidinyl)propyl]-N-hydroxyformamide,
(±)-N-[1-[[[4'-(trifluoromethoxy)[1,1'-biphenyl]-4-yl]sulfonyl]methyl]-2-(3,4,4-trimethy-2,5-dioxo-1-imidazolidinyl)ethyl]-N-hydroxyformamide,
(±)-N-[1-[[(4'-cyano[1,1'-biphenyl]-4-yl)sulfonyl]methyl]-2-(3-methy-2,5-dioxo-1-imidazolidinyl)ethyl]-N-hydroxyformamide,
(±)-N-[1-[[[3'-(cyanomethyl)[1,1'-biphenyl]-4-yl]sulfonyl]methyl]-2-(3,4,4-trimethyl-2,5-dioxo-1-imidazolidinyl)ethyl]-N-hydroxyformamide,
(±)-N-[1-[[(4'-cyano[1,1'-biphenyl]-4-yl)oxyl]methyl]-2-(1,6-dihydro-3-methyl-6-oxo-1-pyridazinyl)ethyl]-N-hydroxyformamide,
(±)-N-[1-[[(4'-cyano[1,1'-biphenyl]-4-yl)sulfonyl)methyl]-2-(4,4-dimethy-2,5-dioxo-1-imidazolidinyl)ethyl]-N-hydroxyformamide,
(±)-N-[1-[[[4-(4-fluorophenoxy)phenyl]sulfonyl]methyl]-2-(4,4-dimethyl-2,5-dioxo-1-imidazolidinyl)ethyl]-N-hydroxyformamide,
(±)-N-[1-[[4-(4-pyridinyl)phenoxy)methyl)-2-(3,4,4-trimethy-2,5-dioxo-1-imidazolidinyl)ethyl]-N-hydroxyformamide,
(S)-N-[1-[(4,4-dimethy-2,5-dioxo-1-imidazolidinyl)methyl]-2-[[4'-(trifluoromethoxy)[1,1'-biphenyl]-4-yl]oxy]ethyl]-N-hydroxyformamide,
(R)-N-[1-[(4,4-dimethy-2,5-dioxo-1-imidazolidinyl)methyl]-2-[[4'-(trifluoromethoxy)[1,1'-biphenyl]-4-yl]oxy]ethyl]-N-hydroxyformamide,
N-[1-[[[4'-(trifluoromethoxy))[1,1'-biphenyl]-4-yl]oxy]methyl]-3-(4,4-dimethyl-2,5-dioxo-1-imidazolidinyl)propyl]-N-hydroxyformamide;
N-[1-[4-[(4-pyridinylthio)phenoxy]methyl]-2-(4,4-dimethyl-2,5-dioxo-1-imidazolidinyl)ethyl]-N-hydroxyformamide;
N-[1-[[[(4-chlorophenoxy)phenyl]sulfonyl]methyl]-3-(4,4-dimethyl-2,5-dioxo-1-imidazolidinyl)propyl]-N-hydroxyformamide;
N-[1-[[(4'-cyano[1,1'-biphenyl]-4-yl)oxyl]methyl]-2-(1,6-dihydro-6-oxo-1-pyridazinyl)ethyl]-N-hydroxyformamide;
N-[1-[[[4'-(aminosulfonyl)[1,1'-biphenyl]-4-yl]oxy]methyl]-2-(3,4,4-trimethyl-2,5-dioxo-1-imidazolidinyl)ethyl]-N-hydroxyformamide;
N-[1-[[[4'-(trifluoromethoxy)[1,1'-biphenyl]-4-yl]sulfonyl]methyl]-2-(4,4-dimethyl-2,5-dioxo-1-imidazolidinyl)ethyl]-N-hydroxyformamide;
N-[1-[4-[(4-pyridinyloxy)phenyl]sulfonyl]]ethyl]-N-hydroxyformamide;
N-[1-[[[(4-cyanophenoxy)phenyl]sulfonyl]methyl]-2-(4,4-dimethyl-2,5-dioxo-1-imidazolidinyl)ethyl]-N-hydroxyformamide;
N-[1-[[4-[[4-(trifluoromethoxy)phenoxy]phenyl]sulfonyl]methyl]-3-(4,4-dimethyl-2,5-dioxo-1-imidazolidinyl)propyl]-N-hydroxyformamide; and
N-[1-[[4-[[4-(trifluoromethoxy)phenoxy]phenyl]sulfonyl]methyl]-2-(4,4-dimethyl-2,5-dioxo-1-imidazolidinyl)ethyl]-N-hydroxyformamide.

### Pharmaceutical Compositions

The present invention also provides pharmaceutical compositions which comprise compounds of the present invention formulated together with one or more non-toxic pharmaceutically acceptable carriers. The pharmaceutical compositions may be specially formulated for oral administration in solid or liquid form, for parenteral injection or for rectal administration.

The pharmaceutical compositions of this invention can be administered to humans and other animals orally, rectally, parenterally , intracistemally, intravaginally, intraperitoneally or topically (such as powders, ointments or drops), bucally or as an oral or nasal spray. The term "parenteral" administration, as used herein, refers to modes of administration which include intravenous, intramuscular, intraperitoneal, intrasternal, subcutaneous and intraarticular injection and infusion.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), and suitable mixtures thereof, vegetable oils (such as olive oil) and injectable organic esters such as ethyl oleate. Proper fluidity can be maintained, for example, by the use of coating materials such as lecithin, by the maintenance of the required particle size in the case of dispersions and by the use of surfactants.

These compositions may also contain adjuvants such as preservative, wetting agents, emulsifying agents and dispersing agents. Prevention of the action of microorganisms may be ensured by the inclusion of various antibacterial and antifungal agents, for example, paraben, chlorobutanol, phenol sorbic acid and the like. It may also be desirable to include isotonic agents such as sugars, sodium chloride and the like. Prolonged absorption of the injectable pharmaceutical form may be brought about by the inclusion of agents (such as aluminum monostearate and gelatin) which delay absorption.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.
The injectable formulations can be sterilized, for example, by filtration through a bacterial-retaining filter or by incorporating sterilizing agents in the form of sterile solid compositions which can be dissolved or dispersed in sterile water or other sterile injectable media just prior to use.

Solid dosage forms for oral administration include capsules, tablets, pills, powders and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate. solid polyethylene glycols, sodium lauryl sulfate and mixtures thereof. In the case of capsules, tablets and pills, the dosage form may also comprise buffering agents.
Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and may also be of a composition such that they release the active ingredient(s) only, or preferentially, in a certain part of the intestinal tract. optionally or in delayed fashion. Examples of embedding compositions which can be used include polymeric substances and waxes.

The active compounds may also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Suspensions, in addition to the active compounds, may contain suspending agents such as ethoxylated isostearyl alcohols, polyoxyethylene sorbitol and sorbitan esters, microcrystalline cellulose, aluminum metahydroxide, bentonite, agar-agar, and tragacanth and mixtures thereof.

Compositions for rectal or vaginal administration are preferably suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients and the like. The preferred lipids are the phospholipids and the phosphatidyl cholines (lecithins), both natural and synthetic. Methods to form liposomes are known in the art. See, for example, Prescott, Ed., Methods in Cell Biology, Volume XIV, Academic Press, New York, N.Y. (1976), p. 33 et seq.

Dosage forms for topical administration of a compound of this invention include powders, sprays, ointments and inhalants. The active compound is mixed under sterile conditions with a pharmaceutically acceptable carrier and any needed preservatives, buffers or propellants which may be required. Opthalmic formulations, eye ointments, powders and solutions are also contemplated as being within the scope of this invention.
Actual dosage levels of active ingredients in the pharmaceutical compositions of this invention may be varied so as to obtain an amount of the active compound(s) that is effective to achieve the desired therapeutic response for a particular patient, compositions, and mode of administration. The selected dosage level will depend upon the activity of the particular compound, the route of administration, the severity of the condition being treated and the condition and prior medical history of the patient being treated. However, it is within the skill of the art to start doses of the compound at levels lower than required for to achieve the desired therapeutic effect and to gradually increase the dosage until the desired effect is achieved.

Generally dosage levels of about 1 to about 50, more preferably of about 5 to about 20 mg, of active compound per kilogram of body weight per day when administered orally to a mammalian patient. If desired, the effective daily dose may be divided into multiple doses for purposes of administration, e.g. two to four separate doses per day.

### Determination of Stromelysin Inhibition

The efficacy of the compounds of this invention as matrix metalloproteinase inhibitors was determined by measuring the inhibition of stromelysin. The inhibition of stromelysin by the compounds of this invention was determined as follows: Recombinant truncated stromelysin (human sequence) produced in E. coli was prepared by expression and purification of the protein as described by Ye et al. (Biochemistry, 1992, 31, 11231-11235, which is incorporated herein by reference). The enzyme was assayed by its cleavage of the thiopeptide ester substrate Ac-Pro-Leu-Gly-[2-mercapto-4-methylpentanoyl]-Leu-Gly-OEt as described by Weingarten and Feder (Anal. Biochem., 1985, 147, 437-440 (1985), which is incorporated herein by reference) as a substrate of vertebrate collagenase. The reported conditions were modified to allow assays to be carried out in a microtiter plate. Upon hydrolysis of the thioester bond, the released thiol group reacted rapidly with 5,5'-dithio-bis(2-nitrobenzoic acid) (DTNB) to produce a yellow color which was measured by a microtiter plate reader set at 405 nm. The rates of cleavage of the substrate by stromelysin in the presence or absence of inhibitors were measured in a 30 minute assay at ambient temperature. Solutions of the compounds in DMSO were prepared, and these were diluted at various concentrations into the assay buffer (50 mM MES/NaOH pH 6.5 with 10 mM CaCl₂ and 0.2% Pluronic F-68), which was also used for dilution of the enzyme and substrate. The potency of the compounds [IC₅₀] was calculated from the inhibition/inhibitor concentration data. The compounds of this invention inhibited stromelysin as shown by the data for representative examples in Table 1 (The numbers in the left-hand column correspound to the examples whose syntheses are described later in the description.)

**Table 1**

| Example | IC₅₀(nM) |
|---|---|
| 1 | 130 |
| 2 | 36 |
| 3 | 21 |
| 4 | 9.1 |
| 5 | 17 |
| 6 | 30 |
| 7 | 120 |
| 8 | 170 |
| 9 | 100 |
| 10 | 1,500 |
| 11 | 300 |
| 12 | 180 |
| 13 | 310 |
| 14 | 4,000 |
| 15 | 620 |

### Preparation of Compounds of this Invention

The compounds and processes of the present invention will be better understood in connection with the following synthetic schemes. Representative procedures are outlined in the following Schemes 1-5.

### Abbreviations

Abbreviations which have been used in the descriptions of the schemes and the examples that follow are: THF for tetrahydrofuran and DMF for N,N-dimethylformamide.

As shown in Scheme 1, deprotonation of the phenolic moiety of 1 with a base, preferably sodium or potassium hydride; and alkylation of the resulting anion with an excess, preferably a two to four-fold excess. of an electrophile, preferably epibromohydrin or epichlorohydrin, provided the alkylated epoxide 2. An excess of a second nucleophile. preferably a two to four-fold excess, was deprotonated with a base such as sodium or potassium hydride and condensed with 2 to provide alcohol 3 which was treated with bis-Boc-hydroxylamine under Mitsunobu conditions to provide the bis-Boc protected hydroxylamine 6. Removal of the Boc-protecting groups with acid, preferably HCl in dioxane or trifluoroacetic acid in methylene chloride, and neutralization of the amine salt with a base, preferably sodium bicarbonate , provided an exposed hydroxylamine moiety which was treated with a formylating agent, preferably formicacetyl anhydride, in solvents such as THF or dichloromethane to provide hydroxamic acid 7.

Alternatively, 2 was converted to the corresponding iodoketone 4 by a two-step procedure which comprised (a) treatment of the epoxide with triphenylphosphine and an iodinating agent, preferably iodine, in an inert solvent such as dichloromethane to provide the corresponding iodoalcohol followed by (b) oxidation to the corresponding iodoketone 4 with a mild oxidizing agent, preferably Dess-Martin periodinane (Dess, D. B.; Martin, J. C., J. Am. Chem. Soc. 1991, 113, 7277-7287, which is incorporated herein by reference). Introduction of R₁ was accomplished by alkylation of the desired phenol or benzenenethiol derivative with 4 in the presence of base, preferably potassium carbonate, in a polar solvent such as DMF. The resulting ketone was converted to the corresponding oxime 5 by treatment with hydroxylamine hydrochloride in a hydroxylic solvent, preferably ethanol, with a catalytic amount of base, preferably pyridine. When R₁ contained sulfur, the alcohol was oxidized to the corresponding ketone using Dess-Martin periodinane in an inert solvent such as dichloromethane then converted to 5 as described above. Treatment of 5 with a reducing agent, preferably borane pyridine complex, in a hydroxylic solvent, preferably ethanol, and adding excess aqueous hydrochloric acid provided the corresponding hydroxylamine which was formylated as described above to provide 7. Depending on the nature of R₁ group, protection and subsequent deprotection of other reactive groups was required to successfully complete the described synthetic sequences. Commonly used protecting groups are disclosed in Greene, "Protective Groups In Organic Synthesis," (John Wiley & Sons, New York (1981)), which is incorporated herein by reference.

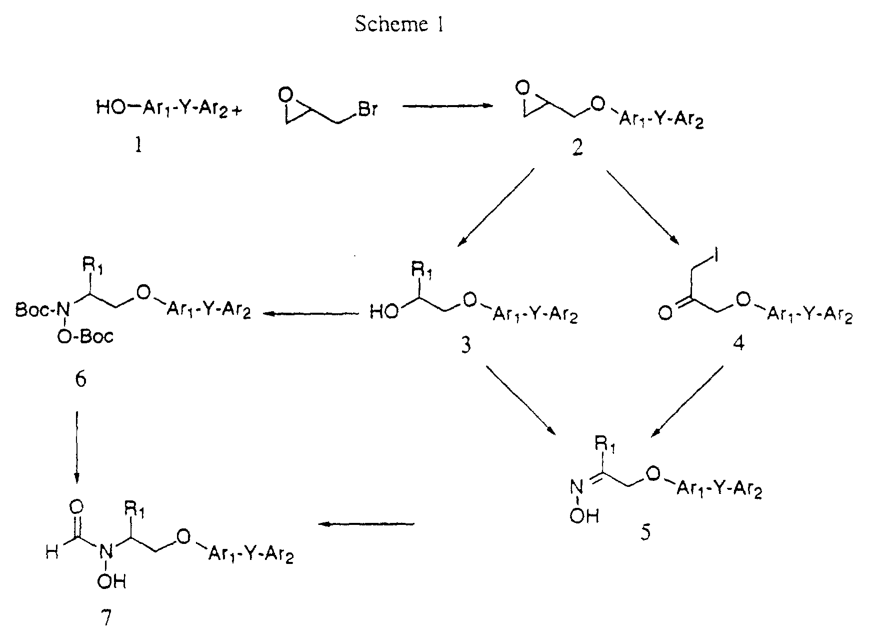

Scheme 2 shows an alternate preparation of intermediate 5. Alkylation of 1 with ethyl bromoacetate was accomplished in the presence of base, preferably potassium carbonate, in a polar solvent, preferably DMF, to provide 8, which was subsequently hydrolyzed to 9 by treatment with aqueous base, preferably lithium hydroxide in a solvent mixture, preferably water and dioxane. Amide 10 was prepared by coupling N,O-dimethylhydroxylamine hydrochloride to 9 with a coupling agent, preferably bis(2-oxo-3-oxazolidinyl)phosphinic chloride (BOP-Cl). Treatment of 10 with R₁-MgX, wherein X is Br or Cl. at reduced temperature, preferably -78 °C in an inert solvent, preferably THF, provided ketone 11, which was converted to 5, and finally to 7, as described in Scheme 1.

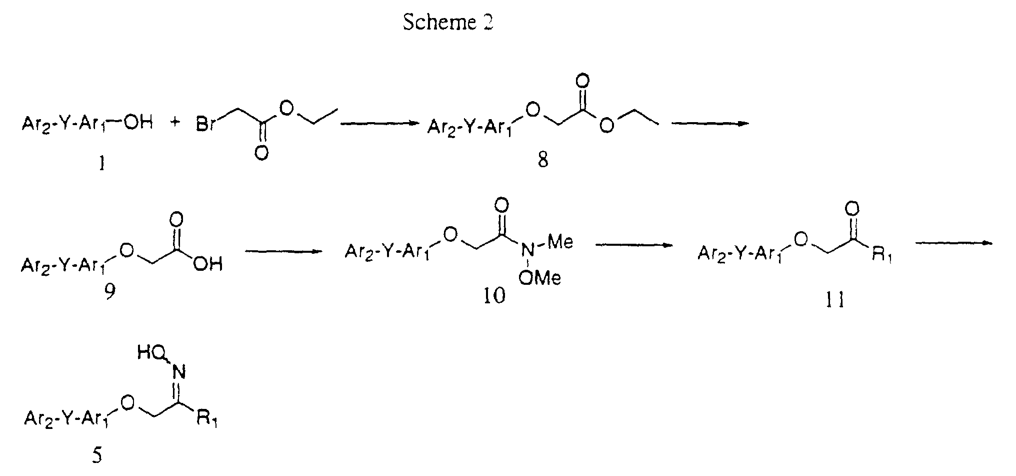

Scheme 3 shows the synthesis of compounds where the introduction of the phenolic and R₁ groups was reversed. This route intersects with the route described in Scheme 1 at epoxide 14, and the chemistry described in Scheme 1 may be utilized to convert 14 into the hydroxamic acid by employing HO-Ar₁-Y-Ar₂ in place of R₁-H. Heterocyclic derivatives of R₁-H, preferably those having appropriate pKa's, such as the hydantoin in this scheme, were condensed with the desired olefinic alcohol under Mitsunobu conditions to provide the corresponding N-alkenylheterocycle 12. Treatment of 12 with an alkylating agent, preferably methyl iodide, in the presence of base, preferably sodium hydride, provided N-methyl alkenylheterocycle 13, which was epoxidized with meta-chloroperbenzoic acid (MCPBA) in dichloromethane to provide 14. The reaction sequence described in Scheme 1 was then used to convert 14 to hydroxamic acid 5.

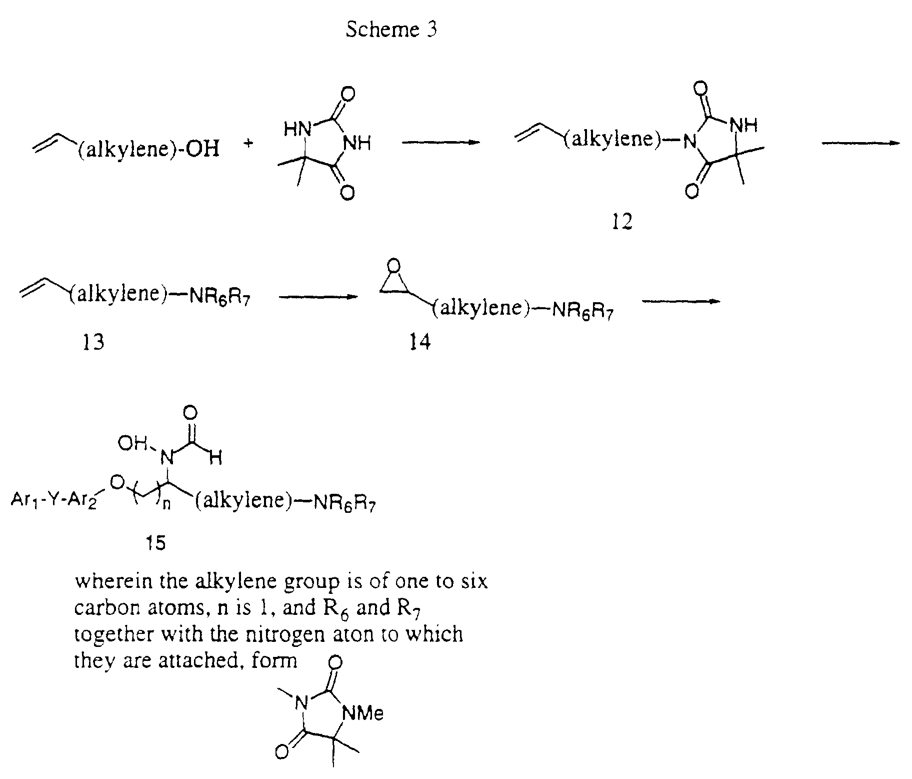

Scheme 4 shows an alternate synthesis of the hydantoin substituted compounds 22 and 23. Alkylation of 16 with a substituted hydantoin 17 in the presence of base, preferably potassium carbonate provides the enol ether 18. Treatment of 18 with a brominating agent such as NBS in acetone, gives the the bromoketone 19 which can then be alkylated with either aryl thiols (20, X =S) or substituted phenols (20,. X =O) to afford the ketones 21. Ketones 21, wherin Y is a covalent bond could also be prepared from 19 in a two step procedure, first alkylating with either bromo thiophenols (20a, X=S) or bromophenols (20a, H=O), then coupling the aryl bomides 10a with an appropriate aryl boronic acid following the Suzuki protocol, or an appropriate arul stannane. The reaction sequence described in scheme 1 can then be used to convert 21 into the hydroxamic acids 22. The compounds wherein X = S can be converted to the sulfones 23 via oxidation with an appropriate oxidant such as m-chloroperbenzoic acid or oxone.

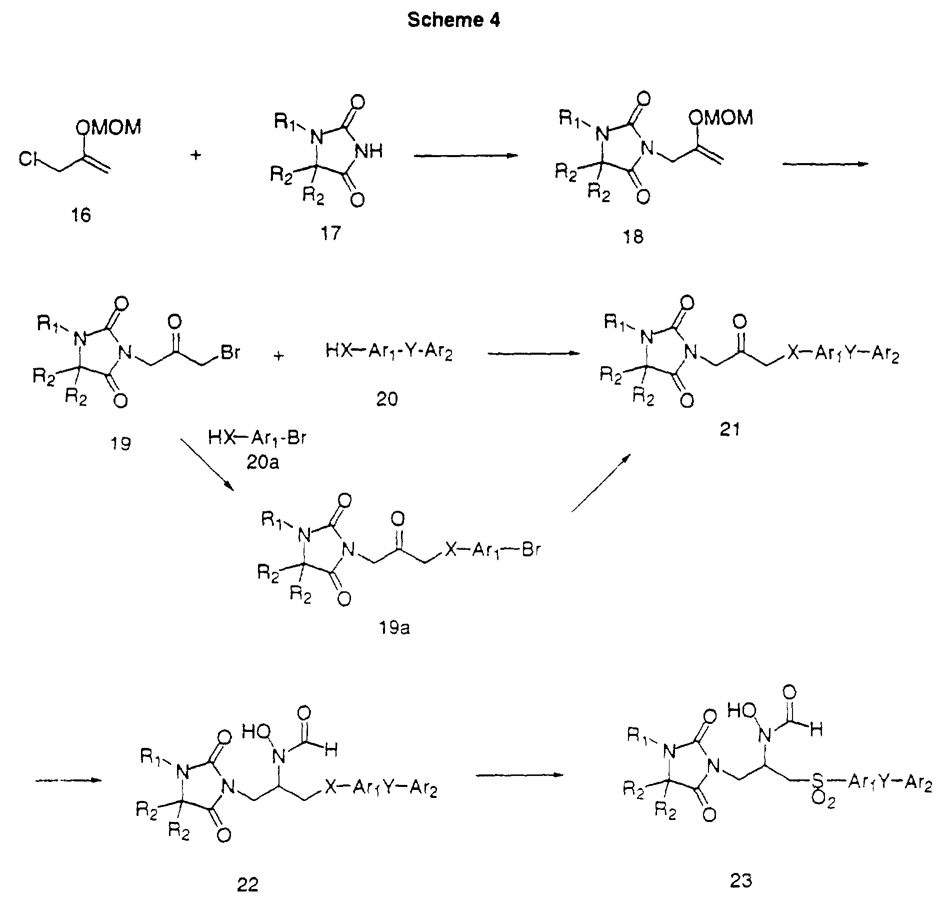

Scheme 5 shows an alternate synthesis of the sulfones 29. Deprotonation of the sulfone 25 with a base such as LDA followed by addition to a ketone or aldehyde 24 gives an alcohol which can be dehydrated either by reaction with acid, such as toluene sulfonic acid or by a stepwide 2 step procedure: first convering the alcohol into a leaving group, such as mesylate via treatment with mesyl chloride and triethyl amine, then eliminating with a base, preferably 1,8-diazabicyclo[5.4.0]undec-7-ene. Reaction of the olefin with an O-protected hydroxylamime preferably O-benzyl gives the adduct 28. Formylation as previously described in scheme 1 followed by removal of the protecting group, preferably under hydrogenation conditions for the compounds wherin P is benzyl affords the sulfone 29. The sulfone 28 can also be prepared directly via the deprotonation of sulfone 25, with a base such as n-BuLi and subsequent addition, preferably in the presence of boron trifluoride etherate, to a O-protected oxime 30.

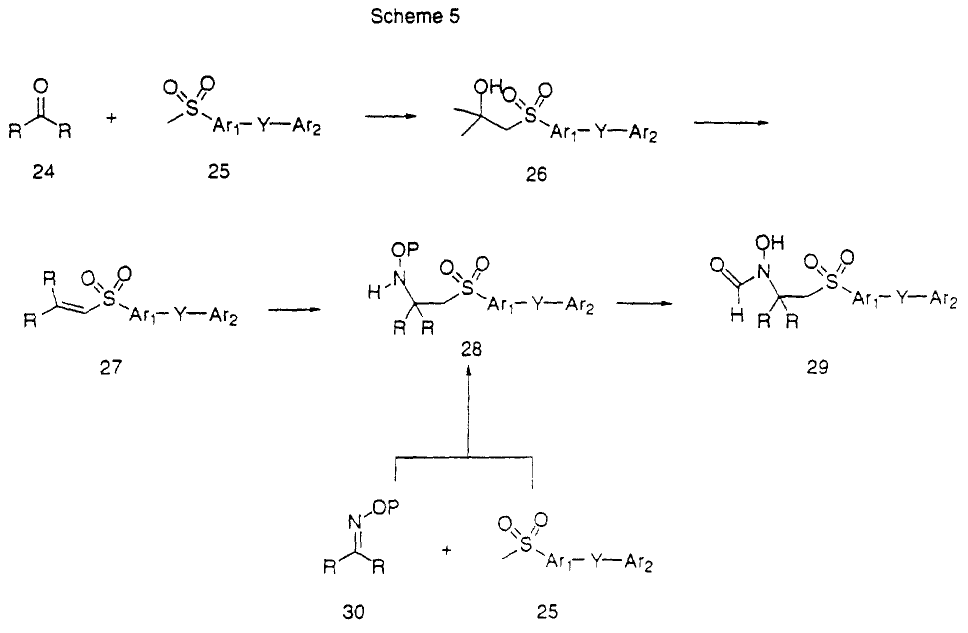

The foregoing may be better understood by reference to the following examples which illustrate the methods by which the compounds of the invention may be prepared and are not intended to limit the scope of the invention as defined in the appended claims.

### Example 1

### (±)-N-[1-[[(4'-cyano-[1,1'-biphenyl]-4-yl)oxy]methyl]-2-phenoxyethyl]-N-hydroxyformamide

### Example 1A

### (±)-3-Phenoxypropan-[1,2]oxirane

A suspension of sodium hydride (0.47 g, 11.7 mmol) in THF (20 mL) was treated sequentially with a solution of phenol (1.00 g, 10.6 mmol) in THF (20 mL) then epibromohydrin (2.73 mL, 31.8 mmol) in a single portion, refluxed for 2 hours, cooled, treated with 20% aqueous potassium hydrogen sulfate then partitioned between ethyl acetate and brine. The organic layer was washed sequentially with saturated aqueous sodium bicarbonate and brine, dried (Na₂SO₄) and concentrated to provide 1.65 g of a golden oil which was purified on silica gel with 10% ethyl acetate/hexanes (500 mL) and 20% ethyl acetate/hexanes to provide 1.19 g (75%) of the title compound.
MS (DCI/NH₃) m/e 168 (M+NH₄)⁺ and 185 (M+NH₄+NH₃)⁴⁺.

### Example 1B

### (±)-1-(4-(4'-Carbonitrilephenyl)phenoxy)-3-phenoxy-2-propanol

A suspension of sodium hydride (0.18 g, 4.39 mmol) in THF (4 mL) was treated sequentially with a solution of 4'-hydroxy-4-biphenylcarbonitrile (0.78 g, 3.99 mmol) in THF (4 mL), Example 1A (0.60 g, 3.99 mmol) in THF (2 mL) then DMF (6 mL), refluxed for 1 hour, cooled, treated with 20% aqueous potassium hydrogen sulfate and partitioned between ethyl acetate and brine. The organic layer was washed with saturated aqueous sodium bicarbonate, 15% aqueous sodium hydroxide and brine, dried (Na₂SO₄) and concentrated to provide 1.04 g of a yellow oil which was purified on silica gel with 25-30% ethyl acetate/hexanes to provide 0.42 g (22%) of the title compound.
MS (DCI/NH₃) m/e 363 (M+NH₄)⁺ and 380 (M+NH₄+NH₃)⁺.

### Example 1C

### (±)-N,O-bis(t-butlyoxycarbonyl)-1-(4-(4'-carbonitrilephenyl)phenoxy)-3-phenoxy-prop-2-yl-N-hydroxylamine

A solution of Example 1B (0.41 g, 1.19 mmol), triphenylphosphine (0.40 g, 1.54 mmol), and di-Boc-hydroxylamine (0.33 g, 1.42 mmol) in THF (5 mL) was treated dropwise with diethylazodicarboxylate (0.24 mL, 1.54 mmol), stirred at ambient temperature for 1 hour and concentrated. The resulting oil was redissolved in dichloromethane (30 mL) and concentrated under vacuum (2 cycles) to remove any excess THF then purified on silica gel with 15% ethyl acetate/hexanes to provide 0.50 g (75%) of the title compound as a colorless foam.
MS (DCI/NH₃) m/e 578 (M+NH₄)⁺.

### Example 1D

### (±)-1-(4-(4'-Carbonitrilephenyl)phenoxy)-3-phenoxy-prop-2-yl-N-hydroxylamine

A solution of Example 1C (0.45 g; 0.80 mmol) in dichloromethane (3 mL) was treated with trifluoroacetic acid (6 mL), stirred for 15 minutes at ambient temperature, poured into excess saturated aqueous sodium bicarbonate and extracted with ethyl acetate. The resulting organic extracts were washed with brine, dried (Na₂SO₄) and concentrated to provide 0.70 g of a brown oil which was purified on silica gel with 50% ethyl acetate/hexanes to provide 0.23 g (81%) of deprotected hydroxylamine as a light yellow foam.

### Example 1E

### (± )-N-[1-[[(4'-cyano-[1,1'-biphenyl]-4-yl)oxy]methyl]-2-phenoxyethyl]-N-hydroxyformamide

A solution of Example 1D (0.15 g, 0.41 mmol) in dichloromethane (2 mL) was cooled to -10 °C and treated with a solution of formicacetyl anhydride (38 mg, 0.43 mmol) in dichloromethane (1 mL), stirred for 15 minutes, diluted with ether and washed sequentially with saturated aqueous sodium bicarbonate, 10% aqueous hydrochloric acid, saturated aqueous sodium bicarbonate and brine, dried (Na₂SO₄) and concentrated to provide 0.17 g of a brown, glassy oil which was purified on silica gel with 97.5% (40% ethyl acetate/hexanes)/2.5% methanol to provide 67 mg (42%) of light brown foam which was recrystallized from ethyl acetate/hexanes/acetone to provide the title compound as light pink, clumpy crystals.
mp 133-135 °C;
¹H NMR (300 MHz, CDCl₃) δ 8.15 (br s; 1H), 8.07 (s; 1H), 7.69 (AB;1H; J=9 Hz), 7.62 (AB; 1H; J=9 Hz), 7.54 (d; 1H; J=9 Hz), 7.32 (dd; 1H; J=6.5,8.0 Hz), 6.97-7.06 (m; 3H), 6.92 (d; 2H; J=7.5 Hz), 4.24-4.47 (m; 5H);
MS (DCI/NH₃) m/e 345 (M+NH₄-HCONHOH)⁺;
Anal. calcd for C₂₃H₂₀N₂O₄: C, 71.12; H, 5.19; N, 7.21. Found: C, 71.04; H, 5.16; N, 7.01.

### Example 2

### (±)-N-[1-[[(4'-cyano-[1,1'-biphenyl]-4-yl)oxy]methyl]-2-(phenylthio)ethyl]-N-hydroxyformamide

### Example 2A

### (±)-3-(4-(4'-Carbonitrilephenyl)phenoxy)propan-[1,2]oxirane

The title compound was prepared following the procedure from Example 1A but using 4'-hydroxy-4-biphenylcarbonitrile (10.0 g, 51.2 mmol) in place of phenol. Purification by trituration with ether provided 9.13 g (71%)the title compound as a chalky solid.
mp 115-116°C;
MS (DCI/NH₃) m/e 269 (M+NH₄)⁺ and 286 (M+NH₄+NH₃)⁺.

### Example 2B

### (±)-1-(4-(4'-Carbonitrilephenyl)phenoxy)-3-throphenoxy-2-propanol

A solution of Example 2A (0.90 g), triethylamine (1.75 mL), and benzenethiol (1.10 mL) in absolute ethanol (14 mL) was heated at reflux for 1 hour, cooled and partitioned between ethyl acetate and 10% aqueous sodium hydroxide. The organic layer was washed sequentially with 10% aqueous hydrochloric acid, saturated aqueous sodium bicarbonate and brine, dried (Na₂SO₄) and concentrated to provide 1.27 g of a thick golden oil which was purified by recrystallization from ethyl acetate/hexanes/methanol to provide the title compound as colorless, clumpy crystals.
mp 105-106 °C;
MS (DCI/NH₃) m/e 379 (M+NH₄)⁺.

### Example 2C

### (±)-1-(4-(4'-Carbonitrilephenyl)phenoxy)-3-thiophenoxy-2-propanone

A suspension of the Dess-Martin periodinane in dichloromethane (25 mL) was treated with Example 2B (2.02 g) in dichloromethane (15 mL), stirred at ambient temperature for 0.5 hours and partitioned between ethyl acetate and saturated aqueous sodium bicarbonate. The organic layer was washed sequentially with saturated aqueous sodium thiosulfate, saturated aqueous sodium bicarbonate and bnne, dried (Na₂SO₄) and concentrated to provide 2.27 g of a clumpy, orange solid which was purified on silica gel with 30% ethyl acetate/hexanes to provide 1.90 g of the title compound as a chalky, light yellow solid.
MS (DCI/NH₃) m/e 377 (M+NH₄)⁺.

### Example 2D

### (±)-1-(4-(4'-Carbonitrilephenyl)phenoxy)-3-thiophenoxy-2-propanone oxime

A solution of Example 2C (2.02 g) in methanol (20 mL) and THF (10 mL) was treated sequentially with 10 drops of pyridine then hydroxylamine hydrochloride (0.78 g), heated at reflux for 1 hour, cooled and partitioned between ethyl acetate and saturated aqueous sodium bicarbonate. The organic layer was washed sequentially with water and brine, dried (Na₂SO₄) and concentrated to provide 1.90 g of the title compound as a chalky yellow solid which was used without further purification.
MS (DCI/NH₃) m/e 375 (M+H)⁺ and 392 (M+NH₄)⁺.

### Example 2E

### (± )-N-(4-(4'-carbonitrilephenyl)phenoxy)-3-thiophenoxyprop-2-yl)hydroxylamine

A solution of Example 2D (1.90 g) in THF (10 mL was treated sequentially with absolute ethanol (20 mL), borane pyridine (1.5 mL) then dropwise with 6N aqueous hydrochloric acid, stirred for 1 hour at ambient temperature, poured into excess saturated aqueous sodium bicarbonate and extracted with ethyl acetate. The combined organic extracts were washed with brine, dried (Na₂SO₄) and concentrated to provide 2.25 g of an orange oil which was purified on silica gel with 30% ethyl acetate/hexanes to provide 1.26 g of the title compound as a light gold oil.
MS (DCI/NH₃) m/e 377 (M+H)⁺ and 394 (M+NH₄)⁺.

### Example 2F

### (±)-N-[1-[[(4'-cyano-[1,1'-biphenyl]-4-yl)oxy]methyl]-2-(phenylthio)ethyl]-N-hydroxyformamide

A solution of compound 2E (1.24 g) in THF (10 mL) was cooled to -23 °C and treated with a solution of formicacetylanhydride (280 µL) in THF (2 mL), stirred for 15 minutes, diluted with ether and washed sequentially with saturated aqueous sodium bicarbonate, 10% aqueous hydrochloric acid, saturated aqueous sodium bicarbonate and brine, dried (Na₂SO₄) and concentrated to provide 1.27 g of a glassy orange oil which was purified on silica gel with 97.5% (40% ethyl acetate/hexanes)/2.5% methanol to provide 300 mg of the title compound as a light orange foam.
¹H NMR (300 MHz, CDCl₃) δ 7.95 (br s; 1H), 7.90 (s; 1H), 7.70 (AB;1H; J=7.5 Hz), 7.62 (AB; 1H; J=7.5 Hz), 7.51 (d; 1H; J=9 Hz), 7.20-7.43 (m; 5H), 6.95 (d; 2H; J=9 Hz), 4.33 (dd; 1H; J=8.5,10.5 Hz), 4.17 (dd; 1H; J=4.5,10.5 Hz), 4.0 (m; 1H), 3.36 (dd; 1H; J=8.5,14 Hz), 3.28 (dd; 1H; J=6,14 Hz);
MS (DCI/NH₃) m/e 422 (M+NH₄)⁺.
Anal. calcd for C₂₃H₂₀N₂O₃S: C, 68.30; H, 4.98; N, 6.73. Found: C, 68.19; H, 4.86; N, 6.73.

### Example 3

### (±)-N-[1-[[(4'-cyano-[1,1'-biphenyl]-4-yl)oxy]methyl]-2-(2,3-dihydro-1,3-dioxo-1H-isoindol-2-yl)ethyl]-N-hydroxyformamide

### Example 3A

### (±)-3-(4-(4'-Carbonitrilephenyl)phenoxy)-3-iodo-propanol

A solution of iodine (1.54 g, 6.0 mmol) in dichloromcthane (20 mL) was treated with triphenylphosphine (1.58 g.6.0 mmol). stirred for 5 minutes, treated with 3-(4'-carbonitrilephenyl)phenoxy)propan-(1,2) oxirane (1.0 g, 4.0 mmol) in a single portion, stirred at ambient temperature for 30 minutes, treated with water and partitioned between ethyl acetate and brine. The organic layer was dried (Na₂SO₄) and concentrated to provide 3 g of crude product which was purified on silica gel with 30% ethyl acetate/hexanes to provide 1.38 g
(91%) of the title compound.
MS (DCI/NH₃) m/e 397 (M+NH₄)⁺ and 414 (M+NH₄+NH₃)⁺.

### Example 3B

### 3-(4-(4'-Carbonitrilephenyl)phenoxy)-1-iodopropan-2-one

The title compound was prepared as in Example 2C but using Example 3A (1.0 g, 2.63 mmol) in place of 3-(4-(4'-carbonitrilephenyl)phenoxy)-1-thiophenoxypropan-2-ol. Purification on silica gel with 20% ethyl acetate/hexanes provided 0.65 g (66%) of the title compound.
MS (DCI/NH₃) m/e 395 (M+NH₄)⁺ and 412 (M+NH₄+NH₃)⁺.

### Example 3C

### 1-(4-(4'-Carbonitrilephenyl)phenoxy)-3-phthaloylpropan-2-one

A solution of Example 3B (1.38 g; 3.66 mmol) in DMF (20 mL) was treated with potassium phthalimide (1.02 g; 5.50 mmol), stirred at ambient temperature for 10 minutes, treated with water and partitioned between ethyl acetate and brine. The organic layer was dried (Na₂SO₄) and concentrated to provide 1.1 g of crude product which was purified on silica gel with ethyl acetate to provide 0.98 g (67%) of the title compound.
MS (DCI/NH₃) m/e 414 (M+NH₄)⁺.

### Example 3D

### (±)-N-[1-[[(4'-cyano-[1,1'-biphenyl]-4-yl]oxy]methyl]-2-(2,3-dihydro-1,3-dioxo-1H-isoindol-2-yl)ethyl]-N-hydroxyformamide

The title compound was prepared according to Example 2D but using Example 3C (0.56 g, 1.41 mmol) in place of 1-(4-(4'-carbonitrilephenyl)phenoxy)-3-thiophenoxypropan-2-one to provide the corresponding oxime which was reduced according to Example 2E using 1-(4-(4'-carbonitrilephenyl)phenoxy)-3-phthaloylpropan-2-one oxime in place of 1-(4-(4'-carbonitrilephenyl)phenoxy)-3-thiophenoxypropan-2-one oxime. The resulting hydroxylamine was formylated according to Example 2F but using 1-(4-(4'-carbonitrilephenyl)phenoxy)-3-phthaloyl-2-propylhydroxylamine in place of 1-(4-(4'-carbonitrilephenyl)phenoxy)-3-thiophenoxy-2-propylhydroxylamine. Purification on silica gel with 60% ethyl acetate/hexanes provided 0.185 g (30%) of the title compound. mp 199-202 °C;
¹H NMR (300 MHz, CDCl₃) δ 10.06 (s; 0.5H), 9.67 (s; 0.5H), 8.32 (s; 0.5H), 7.99 (s; 0.5H), 7.88 (m; 8H), 7.72 (m; 2H), 7.02 (m; 3H), 4.96 (m; 0.5H), 4.52 (m; 0.5H), 4.25 (m; 2H), 3.78-4.00 (m; 2H);
MS (DCI/NH₃) m/e 459 (M+NH₄)⁺;
Anal. calcd for C₂₅H₁₉N₃O₅: C, 67.96; H, 4.304; N, 9.51. Found: C, 67.43; H, 4.34; N, 9.04.

### Example 4

### (±)-N-[1-[[(4'-cyano-[1,1'-biphenyl]-4-yl)oxy]methyl]-2-(3,4,4-trimethyl-2,5-dioxoimidazolidiny-1-yl)ethyl]-N-hydroxyformamide

### Example 4A

### (±)-1-(4-(4'-Carbonitrilephenyl)phenoxy)-3-((5,5-dimethyl)hydantoin-3-yl)-2-propanol

A solution of 5,5-dimethylhydantoin (0.26 g, 1.99 mmol) in THF (20 mL) was treated with potassium tert-butoxide (1.99 mL, 1.99 mmol), stirred for 5 minutes, treated with 3-(4-carbonitrilephenyl)phenoxy)-(1,2) oxirane (0.50 g, 1.99 mmol) in a single portion, stirred at 70 °C for 6 hours, treated with excess saturated aqueous ammonium chloride and partitioned between ethyl acetate and brine. The organic layer was dried (Na₂SO₄) and concentrated to provide a yellow solid which was purified on silica gel with ethyl acetate to provide 0.70 g (93%) of the title compound.
MS (DCI/NH₃) m/e 397 (M+NH₄)⁺.

### Example 4B

### (±)-1-(4-(4'-Carbonitrilephenyl)phenoxy)-3-(3-(5,5-dimethyl)hydantoin)-2-(t-butyldimethylsilyloxy)propane

A solution of Example 4A (0.40 g, 1.06 mmol) in dichloromethane (20 mL) was treated with tert-butyldimethylsilyl chloride (0.24 g, 1.60 mmol) and imidazole (0.1 g, 1.6 mmol), stirred at ambient temperature for 30 minutes, treated with water and partitioned between ethyl acetate and brine. The organic layer was dried (Na₂SO₄) and concentrated to provide a solid which was purified on silica gel with 50% ethyl acetate/hexanes to provide 0.50 g (95%) of the title compound.
MS (DCI/NH₃) m/e 511 (M+NH₄)⁺.

### Example 4C

### (± )-1-(4'-Cyano-[1,1'-biphenyl]-4-yl)oxy)-3-(3,4,4-trimethyl-2,5-dioxo-1-imidazolidin-1-yl)-2-t-butlydimethylsilyloxypropane

A solution of 4B (0.60 g, 1.20 mmol) in THF (20 mL) was treated with sodium hydride (0.035g, 1.40 mmol) then iodomethane (0.26g, 1.8 mmol) in a single portion, stirred at 70 °C for 30 minutes, treated with saturated aqueous ammonium chloride and partitioned between ethyl acetate and brine. The organic layer was dried (Na₂SO₄) and concentrated to provide the title compound as white crystals.

### Example 4D

### (±)-1-(4'-Cyano-[1,1'-biphenyl]-4-yl)oxy)-3-(3,4,4-trimethyl-2,5-dioxo-1-imidazolidin-1-yl)-2-propanol

A solution of Example 4C in THF (30 mL) was treated with tetrabutylammonium fluoride (1M in THF, 2.0 mL, 2.0 mmol), stirred at ambient temperature for 30 minutes, treated with water and partioned between ethyl acetate and brine. The organic layer was dried (Na₂SO₄) and concentrated to provide crude product which was purified on silica gel with ethyl acetate to provide 0.47 g (100%) of the title compound.
MS (DCI/NH₃) m/e 411 (M+NH₄)⁺.

### Example 4E

### 1-(4'-Cyano-[1,1'-biphenyl]-4-yl)oxy)-3-(3,4,4-trimethyl-2,5-dioxoimidazolidin-1-yl)-2-propanone

Example 4D (0.59 g, 1.50 mmol) was processed according to the procedure in Example 2C. Purification of the crude product on silica gel with 50% ethyl acetate/hexanes provided 0.58 g (98%) of the title compound.
MS (DCI/NH₃) m/e 409 (M+NH₄)⁺.

### Example 4F

### (±)-[1-(4'-Cyano-[1,1'-biphenyl]-4-yl)oxyl-3-(3,4,4-trimethyl-2,5-dioxoimidazolidin-1-yl)-prop-2-yl]hydroxylamine

Example 4E (0.57 g, 1.46 mmol) was processed according to the procedures in Examples 2D and 2E. Purification of the crude product on silica gel with 60% ethyl acetate/hexanes provide 0.31 g (52%) of the title compound.
MS (DCI/NH₃) m/e 409 (M+H)⁺.

### Example 4G

### (±)-N-[1-[[(4'-cyano-[1,1'-biphenyl]-4-yl)oxy]methyl]-2-(3,4,4-trimethyl-2,5-dioxoimidazolidin-1-yl)ethyl]-N-hydroxyformamide

Example 4F was processed according to the procedure in Example 2F. Purification of the crude product on silica gel with 60% ethyl acetate/hexanes provided 0.19 g (60%) of the title compound.
mp 65-67 °C;
MS (DCI/NH₃) m/e 437 (M+H)⁺ and 454 (M+NH₄)⁺.
¹H NMR (300 MHz, CDCl₃) δ 9.90 (s; 0.5H), 9.58 (s; 0.5H), 8.32 (s; 0.5H), 7.92 (s; 0.5H), 7.85 (m; 4H), 7.72 (d; 2H; J=5.6 Hz), 7.02 (dd; 2H; J=5.5, 2.5 Hz), 4.86 (m; 0.5H), 4.42 (m; 0.5H), 4.08-4.02 (m; 2H), 3.82-3.70 (m; 1H), 3.55-4.05 (m; 1H), 2.8 (s; 1.5H), 2.78 (s; 1.5H), 1.5 (s; 3H), 1.48 (s; 3H);
Anal. calcd for C₂₃H₂₄N₄O₅: C, 63.23; H, 5.50; N, 12.83. Found: C, 62.96; H, 5.55; N, 12.45.

### Example 5

### (±)-N-[1-[[(4'-cyano-[1,1'-biphenyl]-4-yl)oxy]methyl-3-(3,4,4-trimethyl-2,5-dioxoimidazolidiny-1-yl)propyl]-N-hydroxyformamide

### Example 5A

### 1-(Prop-2-enyl)-4,4-dimethyl-2,5-dioxoimidazolidine

A solution of 3-buten-1-ol (1 g, 13.9 mmol), triphenylphosphine (4.73 g, 18 mmol) and 5,5-dimethylhydantoin (2.1 g, 16.7 mmol) in THF (50 mL) was treated dropwise with diethylazodicarboxylate (3.13 g, 18.0 mmol), stirred at ambient temperature for 1 hour, treated with water and partitioned between ethyl acetate and bnne. The organic layer was dried (Na₂SO₄) and concentrated to provide crude product which was purified on silica gel with 50% ethyl acetate/hexanes to provide 2.5 g (100%) of the title compound.

### Example 5B

### 1-(Prop-2-enyl)-3,4,4-trimethyl-2,5-dioxoimidazolidine

A solution of Example 5A ( 2.3 g, 12.6 mmol) in THF (50 mL) was treated with sodium hydride (0.45 g, 18.9 mmol) then iodomethane (2.7 g, 18.9 mmol) in a single portion, refluxed for 2 hours, cooled, treated with water, and partitioned between ethyl acetate and brine. The organic layer was dried (Na₂SO₄) and concentrated to provide 3.5 g of a yellow solid which was purified on silica gel with 50% ethyl acetate/hexanes to provide 2.4 g (98%) of the title compound.
MS (DCI/NH₃) m/e 214 (M+NH₄)⁺.

### Example 5C

### (± )-1-((1',2'Oxiranyl)propyl)-3,4,4-trimethyl-2,5-dioxoimidazolidine

A solution of Example 5B (3.0 g, 15.3 mmol) in dichioromethane (50 mL) was treated with m-chloroperbenzoic acid(4.4 g), stirred at ambient temperature for 2 hours, treated with saturated aqueous sodium carbonate and partitioned between ethyl acetate and brine. The organic layer was dried (Na₂SO₄) and concentrated to a solid which was purified on silica gel with 70% ethyl acetate/hexanes to provide 1.5 g (46%) of the title compound.
MS (DCI/NH₃) m/e 213 (M+1)⁺ and 230 (M+NH₄)⁺.

### Example 5D

### (±)-1-(2-Hydroxy-3-iodo-propyl)-3,4,4-trimethyl-2,5-dioxoimidazolidine

A solution of iodine (0.29 g, 1.88 mmol) in dichloromethane (20 mL) was treated with triphenylphosphine (0.3 g, 1.88 mmol), stirred for 5 minutes, treated with Example 5C (0.2 g, 0.94 mmol) in a single portion, stirred at ambient temperature for 30 minutes, treated with water and partitioned between ethyl acetate and brine. The organic layer was dried (Na₂SO₄) and concentrated to provide a yellow solid which was purified on silica gel with 75% ethyl acetate/hexanes to provide 0.26 g (80%) of the title compound.
MS (DCI/NH₃) m/e 342 (M+H)⁺ and 358 (M+NH₄)⁺.

### Example 5E

### 1-(3-Iodo-propan-2-onyl)-3,4,4-trimethyl-2,5-dioxoimidazolidine

Example 5D was processed according to the procedure in Example 2C. Purification the crude product on silica gel with 60% ethyl acetate/hexanes provided 0.3 g (96%) of the title compound.
MS (DCI/NH₃) m/e 339 (M+H)⁺ and 356 (M+NH₄)+

### Example 5F

### (±)-1-(3-[(4'-Cyano-[1,1'-biphenyl]-4-yl)oxy]-propan-2-on-1-yl)-3,4,4-trimethyl-2,5-dioxoimidazolidine

A solution of 4'-hydroxy-4-biphenylcarbonitrile (0.38 g, 1.9 mmol) in THF (50 mL) was treated with potassium carbonate (0.5 g) then Example 5E (0.44 g, 1.30 mmol), refluxed for 7 hours, cooled, treated with 10% aqueous HCl and partitioned between ethyl acetate and brine. The organic layer was dried (Na₂SO₄) and concentrated to provide a yellow solid which was purified on silica gel with 75% ethyl acetate/hexanes to provide 0.52 g (99%) of the title compound.
MS (DCI/NH₃) m/e 423 (M+NH₄)⁺.

### Example 5G

### (±)-1-(3-[(4'-Cyano-[1,1'-biphenyl]-4-yl)oxy]-propan-2-oximino-1-yl)-3,4,4,-trimethyl-2,5-dioxoimidazolidine

Example 5F was processed according to the procedure in Example 2D. The crude product was purified on silica gel with 75% ethyl acetate/hexanes to provide 0.68 g (1.60 mmol; 100%) of the title compound.
MS (DCI/NH₃) m/e 439 (M+NH₄)⁺.

### Example 5H

### (±)-N-[1-[[(4'-cyano-[1,1'-biphenyl]-4-yl)oxy]methyl]-3-(3,4,4-trimethyl-2,5-dioxo-1-imidazolidinyl)propyl]-N-hydroxyformamide

Example 5G was processed according to the procedures in Examples 2E and 2F. Purification of the crude product on silica gel with 75% ethyl acetate/hexanes provided 0.408 g (56%) of the title compound.
mp 68-70 °C;
¹H NMR (300 MHz, CDCl₃) δ 9.99 (s; 0.5H), 9.46 (s; 0.5H), 8.35 (s; 0.5H), 7.92 (s; 0.5H), 7.92 (d; 2H; J=5.6Hz), 7.85 (d; 2H; J=5.6Hz), 7.70 (d; 2H; J-5.6 Hz), 7.05 (d; 2H; J=5.6Hz), 4.52 (m; 0.5H), 4.18-3.95 (m; 3.5H), 3.46 (m; 2H), 2.82 (s; 1.5H), 2.79 (s; 1.5H), 2.02-1.72 (m; 1H), 1.32 (s; 6H);
MS (DCI/NH₃) m/e 468 (M+NH₄)⁺;
Anal. calcd for C₂₄H₂₆N₄O₅: C, 63.93; H, 5.77; N, 12.43. Found: C, 63.38; H, 5.99; N, 11.97.

### Example 6

### (±)-N-[1-[[[3'-(cyanomethyl)-[1,1'-biphenyl]-4-yl]oxy]methyl]pentyl]-N-hydroxyformamide

### Example 6A

### 4-((t-Butyldimethyl)silyloxy)phenyl boronic acid

A solution of(4-bromophenoxy)trimethylsilane (69 g, 20.9 mmol) in THF (60 mL) was treated with n-butyllithium at -78 °C, stirred for 15 minutes, treated with triisopropyl borate, stirred at -78 °C for 10 minutes, warmed to ambient temperature, stirred for another 30 minutes, treated with water and partitioned between ethyl acetate and brine. The organic layer was dried (MgSO₄) and concentrated to provide 4.79 g (91%) of the title compound.

### Example 6B

### 4'-Hydroxy-3-biphenylcarbonitrilemethane

A mixture of Example 6A (4.8 g. 19.0 mmol), 3-bromophenyl acetonitrile (3.1 g, 16.0 mmol), cesium carbonate (7.8 g. 24.0 mmol) and tetrakis(triphenylphosphine)palladium(0) (0.55 g, 0.48 mmol) was treated via syringe with DMF (30 mL) under positive nitrogen pressure, stirred at 100 °C for 10 h, treated with water and partitioned between ethyl acetate and brine. The organic layer was dried (MgSO₄) and concentrated to provide a brown oil which was purified on silica gel with 50% ethyl acetate/hexanes to provide 3.3 g (82%) of the title compound.
MS (DCI/NH₃) m/e 227 (M+NH₄)⁺.

### Example 6C

### Ethyl 2-(4-(3'-carbonitrilemethylphenyl)phenoxy)acetate

A solution of 6B (0.5 g, 2.4 mmol) in THF (20 mL) was treated with potassium carbonate (0.5 g) and ethyl bromoacetate (0.6 g, 3.6 mmol), refluxed for 3 hours, cooled, treated with 10% aqueous HCl and partitioned between ethyl acetate and brine. The organic layer was dried (Na₂SO₄) and concentrated to provide a yellow solid which was purified on silica gel with 50% ethyl acetate/hexanes to provide 0.48 g (68%) of the title compound.
¹H NMR (300 MHz, CDCl₃) δ 7.52 (m; 4H), 7.40 (m; 1H), 7.36 (m; 1H), 7.0 (m; 2H), 4.65 (s; 2H), 4.30 (q; 2H; J=4.8 Hz), 3.80 (s; 2H), 1.32 (t; 3H; J=4.8Hz).

### Example 6D

### 2-(4-(3'-Carbonitrilemethylphenyl)phenoxy)acetic acid

A solution of 6C (0.47 g, 1.6 mmol ) in 1,4-dioxane (20 mL) and water (10 mL) was treated with lithium hydroxide (0.5 g), stirred at ambient temperature for 30 minutes, treated with 10% aqueous HCl and partitioned between ethyl acetate and brine. The organic layer was dried (Na₂SO₄) and concentrated to provide a yellow solid which was purified on silica gel with 50% ethyl acetate/hexanes to provide 0.37 g (83%) of the title compound.
¹H NMR (300 MHz, CDCl₃) δ 7.60 (m; 4H), 7.46 (m; 1H), 7.32 (m; 1H), 7.02 (m: 2H), 4.72 (s; 2H), 4.08 (s; 2H).

### Example 6E

### N,O-dimethyl-2-(4-(3'-carbonitrilemethylphenyl)phenoxy)acetyl hydroxylamine

A solution of 6D (0.35 g, 1.3 mmol), triethylamine (0.5mL) and bis(2-oxo-3-oxazolidinyl)-phosphinic chloride (0.78 g, 2.6 mmol) in dichloromethane (20 mL) was treated with N.O-dimethyl-hydroxylamine hydrochloride (0.25 g, 2.6 mmol), stirred at ambient temperature for 2 hours, treated with water and partitioned between ethyl acetate and brine. The organic layer was dried (Na₂SO₄) and concentrated to provide a yellow solid which was purified on silica gel with 50% ethyl acetate/hexanes to provide 0.28 g (69%) of the title compound.
¹H NMR (300 MHz, CDCl₃) δ 7.59 (m; 4H), 7.46 (m, 1H), 7.32 (m; 1H), 7.02 (m; 2H), 4.96 (s; 2H), 4.08 (s; 2H), 3.78 (s; 3H), 3.15 (s; 3H).

### Example 6F

### 1-(4-(3'-Carbonitrilemethylphenyl)phenoxy)-2-hexanone

A solution of 6E (0.27 g, 0.85 mmol) in THE (10 mL) was treated with n-butylmagnsium bromide (1 mL, 2.0 mmol) at -78 °C, stirred at -78 °C for 1 h, treated with water and partitioned between ethyl acetate and brine. The organic layer was dried (Na₂SO₄) and concentrated to provide a yellow solid which was purified on silica gel with 25% ethyl acetate/hexanes to provide 0.15 g (59%) of the title compound.
¹H NMR (300 MHz, CDCl₃) δ 7.52 (m; 4H), 7.42 (m; 1H), 7.28 (m; 1H), 6.98 (m; 2H), 4.60 (s; 2H), 3.82 (s; 2H), 2.62 (t; 2H; J=5.5 Hz), 1.64 (m; 2H); 1.38 (m, 2H), 0.92 (t; 3H: J=4.8Hz).

### Example 6G

### (±)-N-[1-[[[3'-(cyanomethyl)-[1,1'-biphenyl]-4-yl]oxy]methyl]pentyl]-N-hydroxyformamide

Example 6F (0.15 g, 0.50 mmol) was processed according to the procedures described in Examples 2D-F (inclusive). Purification of the crude final product on silica gel with 40% ethyl acetate/hexanes provided 0.07 g (41%) of the title compound.
mp 99-101 °C;
MS (DCI/NH₃) m/e 352 (M+NH₄)⁺.
¹H NMR (300 MHz, CDCl₃) δ 8.58 (brs; 0.5H), 8.04 (brs; 0.5H), 8.0 (s; 1H), 7.48 (m; 4H), 7.42 (m; 1H), 7.26 (m; 1H), 6.98 (m; 2H), 4.05 (t; 1H; J=5.6 Hz), 3.8-4.0 (m; 2H), 3.80(s; 2H), 1.92 (m; 1H0, 1.60 (m; 2H), 1.38 (m: 3H), 0.98 (t; 3H; J=4.8 Hz).
Anal. calcd for C₂₁H₂₄N₂O₃: C, 71.50; H, 6.81; N. 7.94. Found: C, 71.44; H, 6.90; N, 7.80.

### Example 7

### (±)-N-[1-[[(4-cyano-[1,1'-biphenyl]-4-yl)oxy]methyl]-3-methylbutyl]-N-hydroxyformamide

4-hydroxy-4-biphenylcarbonitrile (1.0 g, 5.12 mmol) was processed according to the procedures described in Examples 6C-G (inclusive), but substituting isobutylmagnesium bromide for the n-butylmagnesium bromide used in Example 6F. Purification of the crude final product on silica gel with 30% ethyl acetate/hexanes provided 0.036 g of the title compound.
mp 112-113 °C;
MS (DCI/NH₃) m/e 356 (M+NH₄)⁺.
¹H NMR (300 MHz, CDCl₃) δ 7.95 (s; 1H), 7.70 (d; 2H; J=5.6 Hz), 7.62 (d; 2H; J=5.8), 7.52 (d; 2H; J=5.8 Hz), 6.98 (d; 2H; J=5.8Hz), 4.25 (m; 1H), 3.92-4.05 (m; 2H), 1.95 (m; 1H), 1.75 (m; 1H), 1.35(m; 1H), 1.00 (d; 3H; I=4.8 Hz), 0.98 (d; 3H; J=4.8 Hz).
Anal. calcd for C₂₀H₂₂N₂O₃: C, 70.92; H, 6.50; N, 8.27. Found: C, 70.91; H, 6.68; N, 8.13.

### Example 8

### (± )-N-[1-[[(4'-cyano-[1,1'-biphenyl]-4-yl)oxy]methyl]-2-methylbutyl]-N-hydroxyformamide

The title compound was prepared following the sequence of steps described in Example 7 but substituting sec-butylmagnesium chloride for isobutylmagnesium bromide. Purification of the crude final product on silica gel with 30% ethyl acetate/hexanes provided 0.10 g of the title compound.
mp 96-98 °C;
MS (DCI/NH₃) m/e 356 (M+NH₄)⁺.
¹H NMR (300 MHz, CDCl₃) δ 7.95 (s; 1H), 7.70 (d; 2H; J=5.6 Hz), 7.62 (d; 2H: J=5.8), 7.52 (d; 2H; J=5.8 Hz), 6.98 (d; 2H; J=5.8Hz), 4.32 (m; 1H), 4.15 (m; 2H), 3.65 (m; 1H), 1.98 (m; 1H), 1.62 (m; 1H), 1.02 (m; 3H), 0.98 (m; 3H;).
Anal, calcd for 0.8 H₂O+C₂₀H₂₂N₂O₃; C, 68.03; H, 6.69; N, 7.90. Found: C, 68.60; H, 6.58; N, 7.23.

### Example 9

### (±)-N-[1-[[(4'-cyano-[1,1'-biphenyl]-4-yl)oxy]methyl]pentyl]-N-hydroxyformamide

The title compound was prepared following the sequence of steps described in Example 7 but substituting n-butylmagnesium bromide for isobutylmagnesium bromide. Purification of the crude final product on silica gel with 30% ethyl acetate/hexanes provided 0.210 g of the title compound.
mp 105-108 °C;
MS (DCI/NH₃) m/e 356 (M+NH₄)⁺.
¹H NMR (300 MHz. CDCl₃) δ 7.95 (s; 1H), 7.70 (d; 2H; J=5.6 Hz), 7.62 (d; 2H; J=5.8), 7.52 (d; 2H; J=5.8 Hz), 6.98 (d; 2H; J=5.8Hz), 4.25 (m; 1H), 3.99-3.82 (m; 2H), 1.92 (m; 1H), 1.60 (m; 2H), 1.40 (m; 3H), 0.98 (t; 3H; J=4.3Hz).
Anal. calcd for 0.5C₆H₆+C₂₀H₂₂N₂O₃: C, 73.13; H, 6.62; N, 7.42. Found: C, 73.18; H. 6.65: N, 7.39.

### Example 10

### (±)-N-[1-[[(4'-cyano-[1,1'-biphenyl]-4-yl)oxy]methyl]-2-(4-methylphenyl)ethyl]-N-hydroxyformamide

The title compound was prepared following the sequence of steps described in Example 7 but substituting 4-methylbenzylmagnesium bromide for isobutylmagnesium bromide. Purification of the crude final product on silica gel with 30% ethyl acetate/hexanes provided 0.24 g of the title compound.
mp 173-175 °C;
MS (DCI/NH₃) m/e 404 (M+NH₄)⁺.
¹H NMR (300 MHz, CDCl₃) δ 7.70 (d; 2H; J=5.6 Hz), 7.68 (s; 1H), 7.62 (d; 2H; J=5.8), 7.52 (d; 2H: J=5.8 Hz), 7.12 (s; 4H), 6.98 (d; 2H; J=5.8Hz), 4.35 (m; 1H), 4.12-3.98 (m; 2H), 3.15 (m; 1H), 2.94 (m; 1H), 1.35 (s; 3H).
Anal. calcd for C₂₄H₂₂N₂O₃: C, 74.52; H, 5.69; N, 7.24. Found; C, 73.95 ; H, 5.79; N, 7.06.

### Example 11

### (±)-N-[2-[(4'-cyano-[1,1'-biphenyl]-4-yl)oxy]-1-(4-fluorophenyl)ethyl)-N-hydroxyformamide

The title compound was prepared following the sequence of steps described in Example 7, but substituting 4-fluorophenylmagnesium bromide for isobutylmagnesium bromide. Purification of the crude final product on silica gel with 30% ethyl acetate/hexanes provided 0.285 g of the title compound.
mp 194-196 °C;
MS (DCI/NH₃) m/e 394 (M+NH₄)⁺.
¹H NMR (300 MHz, CDCl₃) δ 9.70 ( brs; 1H), 8.42 (s, 0.5H), 8.28 (s; 0.5H), 7.86 (m; 4H), 7.72 (d; 2H; J=5.6 Hz), 7.55 (m; 2H), 7.25 (m; 2H), 7.12 (d; 2H; J=5.8 Hz), 5.72 (brs; 0.5H), 5.35 (brs; 0.5H), 4.60 (m; 1H), 4.36 (m; 1H).
Anal. calcd for C₂₂H₁₇N₂O₃F: C, 70.14; H, 4.45; N, 7.44. Found: C, 70.19 ; H, 4.25; N, 7.30.

### Example 12

### (±)-N-[1-[[(4'-cyano-[1,1'-biphenyl]-4-yl)oxy]methyl]-2-(4-fluorophenyl)ethyl]-N-hydroxyformamide

The title compound was prepared following the sequence of steps described in Example 7 but substituting 4-fluorobenzylmagnesium bromide for isobutylmagnesium bromide. Purification of the crude final product on silica gel with 30% ethyl acetate/hexanes provided 0.22 g of the title compound.
MS (DCI/NH₃) m/e 408 (M+NH₄)⁺.
¹H NMR (300 MHz, CDCl₃) δ 8.35 (brs; 1H), 7.65 (m; 5H), 7.52 (d; 2H; J=5.6 Hz), 7.20 (m; 2H), 6.98 (m; 4H), 4.35 (m; 1H), 4.15-3.98 (m; 2H), 3.18 (dd; 1H; J=6.0, 9.0 Hz), 2.95 (dd; 1H; J=3.0, 9.0 Hz).
Anal. calcd for C₂₃H₁₉N₂O₃F: C, 70.69; H, 4.87; N, 7.17. Found: C, 70.38 ; H, 4.96; N, 6.98.

### Example 13

### (±)-N-[1-[[(4'-cyano-[1,1'-biphenyl]-4-yl)oxy]methyl]ethyl]-N-hydroxyformamide

### Example 13A

### (4-(4'-Carbonitrilephenyl)phenoxy)propan-2-one

A solution of 4-(4'-carbonitrilephenyl)phenol (4.86 g, 24.9 mmol) in DMF (100 mL) was treated with potassium carbonate (13.8 g, 99.6 mmol), heated at 50 °C for 10 minutes, treated in a single portion with chloroacetone (2.48 mL, 30 mmol), stirred for 4 hours at ambient temperature and partitioned between 3:1 ether:hexanes and saturated aqueous sodium carbonate. The organic layer was dried (MgSO₄) and concentrated under vacuum to 1/3 of its original volume to cause precipitation of product from solution. The solution was treated with more ether and stored at -20 °C for 17 hours. The title compound (2.01 g, 32%) was collected by filtration and dried under vacuum.
MS (DCI/NH₃) m/e 251 (M)⁺, 269 (M+NH₄)⁺ and 286 (M+NH₄+NH₃)⁺.

### Example 13B

### (±)-N-[1-[[(4'-cyano-[1,1'-biphenyl]-4-yl]oxy]methyl]ethyl]-N-hydroxyformamide

The title compound was obtained following the procedures in Examples 2D-F (inclusive) but substituting Example 13A (2.00 g, 7.96 mmol) for Example 2C. Purification of the crude final product on silica gel with 5% methanol/dichloromethane provided 325 mg of the title compound.
mp 141-144 °C;
¹H NMR (300 MHz, d₆-DMSO) δ 9.88 and 9.45 (br s; 1H), 8.02 and 8.33 (s; 1H), 7.90 (AB; 2H; J=7.5 Hz), 7.84 (AB; 2H; J=7.5 Hz), 7.61 (d; 2H; J=9 Hz), 7.06 (d; 2H; J=9 Hz), 4.67 (m; 0.32H), 3.92-4.25 (m; 2.68H), 1.23 and 1.18 (d; 3H; J=6 Hz);
MS (DCI/NH₃) m/e 314 (M+NH₄)⁺.
Anal. calcd for C₁₇H₁₆N₂O₃: C, 68.90; H, 5,44; N, 9.45. Found: C, 68.61; H, 5.55; N, 9.21.

### Example 14

### N-[2-[(4'-cyano-[1,1'-biphenyl]-4-yl)oxy]ethyl]-N-hydroxyacetamide

### Example 14A

### 2-(4-(4'-Carbonitrilephenyl)phenoxy)-bromoethane

A solution of 4-(4'-carbonitrilephenyl)phenol (3.00 g, 24.8 mmol) in DMF (20 mL) was treated with potassium carbonate (8.24 g, 99.4 mmol) and 1.2-dibromoethane (6.42 mL, 124 mmol), heated at 50 °C for 19 hours and partitioned between 3:1 ether:hexanes and water. The organic layer was separated and the aqueous layer was extracted with 3:1 ether:hexanes. The combined organic layers were dried (MgSO₄) and concentrated. Purification of the crude product on silica gel with 50% dichloromethane/hexanes) provided a white solid which was recrystallized from ether/pentane to provide 1.25 g (17%) of the title compound as colorless needles.
MS (DCI/NH₃) m/e 301/303 (M)⁺, 319/321 (M+NH₄)⁺ and 336/338 (M+NH₄+NH₃)⁺.

### Example 14B

### N,O-bis(t-butyloxycarbonyl)-2-[(4-(4'-carbonitrilephenyl)phenoxy)ethyl-N-hydroxylamine

A solution of N,O-bis(t-butyloxycarbonyl)-N-hydroxylamine (443 mg, 1.9 mmol) in DMF (15 mL) was treated with a 60 % oil dispersion of sodium hydride (76 mg, 1.9 mmol), stirred at ambient temperature for 15 minutes, treated with Example 13A (0.54 g, 1.79 mmol), stirred for 4 hours at ambient temperature and partitioned between 1:1 ether:hexanes and saturated aqueous ammonium chloride. The organic layer was separated, and the aqueous layer was extracted with 1:1 ether:hexanes. The combined organic layers were dried (MgSO₄) and concentrated. Purification on silica gel with 10% ethyl acetate/hexanes provided 0.65 g (80%) of the title compound as colorless viscous oil.
MS (DCI/NH₃) m/e 472 (M+NH₄)⁺.

### Example 14C

### 2-[(4-(4'-Carbonitrilephenyl)phenoxy)ethyl-N-hydroxylamine hydrochloride

Example 14B (0.64 g, 1.41 mmol) was treated with 4N hydrochloric acid in dioxane (10 mL) and stirred at ambient temperature for 2.5 hours, during which time a colorless precipitate formed. The precipitate was collected by filtration, washed with dioxane, and dried to afford the HCl salt of the title compound as a colorless solid (0.22 g, 56%).

### Example 14D

### N,O-bis(acetyl)-2-[(4-(4'-carbonitrilephenyl)phenoxy)ethyl-N-hydroxylamine

A solution of Example 14C (27 mg, 0.093 mmol) in THF at 0 °C was treated with triethylamine (32 µL, 0.23 mmol), stirred for 1 hour at 0 °C, treated dropwise with acetyl chloride (16 µL), stirred for 1 hour at 0 °C and 18 hours at ambient temperature and partitioned between 1N aqueous hydrochloric acid and ether. The organic layer was separated, and the aqueous layer was extracted with ethyl acetate. The combined organic layers were dried (MgSO₄) and concentrated. Purification of the residue on silica gel with 2% acetone/dichloromethane provided 29 mg (83%) of the title compound.
MS (DCI/NH₃) m/e (M+NH₄)⁺.

### Example 14D

### N-[2-[(4'-cyano-[1,1'-biphenyl]-4-yl)oxy]ethyl]-N-hydroxyacetamide

A solution of Example 14D (29 mg, 0.077 mmol) in THE (5 mL) and ethanol (2 mL) was cooled to 0 °C, treated with aqueous lithium hydroxide (0.31 mL of 1.0 N lithium hydroxide), stirred at 0 °C for 10 minutes and at ambient temperature for 1.5 hours and partitioned between water and ethyl acetate. The organic layer was separated and the aqueous layer was extracted with ether. The combined organic layers were washed with 1N aqueous hydrochloric acid, dried (MgSO₄) and concentrated to a semi-solid which was purified by trituration with ethyl acetate to provide 19.7 mg (86%) of the title compound as a colorless solid.
mp 174-175°C;
¹H NMR (300 MHz, d₆-DMSO) δ 9.90 (br s; 1H), 7.88 (AB; 2H; J=7.5 Hz), 7.84 (AB; 2H; J=7.5 Hz), 7.71 (d; 2H; J=8.5 Hz), 7.07 (d; 2H; J=8.5 Hz), 4.20 (t; 2H; J=7.5,7.5 Hz), 3.89 (t; 2H; J=7.5,7.5 Hz), 2.02 (s; 3H);
MS (DCI/NH₃) m/e 297 (M+H)⁺ and 314 (M+NH₄)⁺.
Anal. calcd for C₁₇H₁₆N₂O₃(0.25H₂O): C, 67.87; H, 5.52; N, 9.31. Found: C, 67.65; H, 5.55; N, 9.12.

### Example 15

### N-[2-[(4'-cyano-[1,1'-biphenyl]-4-yl)oxylethyl]-N-hydroxyformamide

A solution of Example 14C (78 mg, 0.27 mmol) in THF (2.0 mL) was treated with triethylamine (75 µL, 0.54 mmol) and then dropwise with formicacetyl anhydride (41 mg, 0.30 mmol) in THF (0.5 mL), stirred for 2 hours at ambient temperature and partitioned between ethyl acetate and 1N aqueous hydrochloric acid. The organic layer was separated and the aqueous layer was extracted with ethyl acetate. The combined organic layers were dried (MgSO₄) and concentrated. Purification by chromatography on silica gel with 0.2% acetic acid/ethyl acetate and subsequent recrystallization from cold methanol provided 14.7 mg (19%) of the title compound.
mp 142-145 °C;
¹H NMR (300 MHz, d₆-DMSO) δ 10.17 and 9.74 (br s; 1H), 8.34 and 7.98 (br s; 1H), 7.88 (AB; 2H; J=7.5 Hz), 7.84 (AB; 2H; J=7.5 Hz), 7.73 (d; 2H; J=8.5 Hz), 7.07 (d; 2H; J=8.5 Hz), 4.14-4.26 (m; 2H), 3.77-3.88 (m; 2H);
MS (DCI/NH₃) m/e 300 (M+NH₄)⁺.
Anal. calcd for C₁₆H₁₄N₂O₃(0.125H₂O): C, 67.54; H, 5.05; N, 9.84. Found: C, 67.59; H, 5.32; N, 9.53.

### Example 16

### N-[1-[4-[(2E-phenylethenyl)phenoxy]methyl]-2-(3,4,4-trimethyl-2,5-dioxo-1-imidazolidinyl)ethyl]-N-hydroxyformamide

### Example 16A

### 3-(3,4,4-trimethyl-2,5-dioxoimidazolidin-1-yl)-2-methoxymethyloxy-prop-1-ene

A mixture of 1,5,5 trimethylhydantoin (7.0 g, 49.2 mmol), powdered potassium carbonate (8.16g, 59 mmol) and 2-methoxymethyloxy-allyl chloride (7.65 g, 56 mmol) in dry DMF (100 mL) was heated to 100° C with stirring for 1.5 h. The reaction mixture was cooled . fliltered and the filtrate was concentrated, then partitioned between ethyl acetate and water. The organic extract was washed twice with water, brine, dried and concentrated, then purified via silica gel chromatography eluting with 50% ethyl acetate: hexane to give 10.58 g (89%) of the title compound. MS (DCI/NH₃) m/e 243 (M+H)⁺ and 260 (M+NH₄)⁺.

### Example 16B

### 1-bromo-3-(3,4,4-trimethyl-2,5-dioxoimidazolidin-1-yl)propan-2-one

A 0° C solution of example 16A (10.58 g, 43.7 mmol) in acetone (200 mL) was treated sequentially with a solution of potassium carbonate (0.58g, 4.2 mmol) in water (60 mL) and N-bromo succinimide (8.56g, 48 mmol)and the resulting mixture was stirred with the ice bath in place. An additional 2 portions of 1.5 g of N-bromo succinimide were added after 1 and 2 h respectively. The ice bath was then removed and the reaction was allowed to stir for an additional 10 min, then was concentrated and extracted twice with ethyl acetate. The combined extracts were washed with aq. 0.5 M NaHSO₃. 1M NaHCO3, water, brine, dried and concentrated. The residue was purified via silica gel chromatography eluting with 50% ethyl acetate: hexane to give 7.39 g (61%) of the title compound.
MS (DCI/NH₃) m/e 277/279 (M+H)⁺ and 294/296 (M+NH₄)⁺.

### Example 16C

### 1-(4'-bromophenyloxy)-3-(3,4,4-trimethyl-2,5-dioxoimidazolidin-1-yl)-2-propanone

To a suspension of 4-bromophenol (3.99g, 23.0mmol) and cesium carbonate (7.45g, 22.9mmol) in DMF (150mL) was added a solution of bromoketone 16B (3g, 11.5mmol) in DMF (5mL), drop-wise over 30 minutes. The suspension held at RT for 16h, diluted with ethyl acetate (500mL) and the organics washed with water, brine and dried over magnesium sulfate and concentrated *in vacuo*. Flash chromatography (hexane/ethyl acetate 1:1) gave 2.55g of **16C** as a white solid.

### Example 16D

To a warm (100°) solution of **16C** (0.5g, 1.35mmol) and tributyl(phenylethynyl)tin (0.64g, 1.62mmol, Aldrich) in toluene (25mL) was added a catalytic amount of Pd(PPh₃)₄. The reaction was brought to reflux and held for 7h. The resulting black solution was diluted with ethyl acetate (125mL) and the organics washed with 1M NaOH, brine, dried over magnesium sulfate and concentrated *in vacuo*. Flash chromatography (gradient elution; hexane/ethyl acetate 4:1 to 1:1) gave 0.24g of the desired compound after trituration with diethyl ether.

### Example 16E

### N-[1-[4-[(2E-phenylethenyl)phenoxy]methyl]-2-(3,4,4-trimethyl-2,5-dioxo-1-imidazolidinyl)ethyl]-N-hydroxyformamide

The title compound was prepared in the same manner as example 2D,E,F substituting 16D for 2C.
¹H NMR (300 MHz, d₆-DMSO) δ 9,90 (s, 0.5H), 9.58 (s, 0.5H), 8.31 (s, 0.5H), 7.9 (s, 0.5H), 7.57-7.54 (m, 6H), 7.38-7.33 (m, 2 H), 7.26-708 (m, 6H), 6.94-6.90 (m, 2H), 4.80-4.60 (m, 0.511), 4.55-4.40 (m, 0.5H), 4.16-4.04 (m, 4H), 3.76-3.73 (m, 2H), 3.61-3.57 (2, 2H), 2.79 (s, 6H). 1.28 (s, 12H).
MS (ESI) m/e M-H (436), M+H (438),
Anal. Calcd for: C₂₄H₂₇N₃O₅•1.0H₂O: C, 63.28; H. 6.41; N, 9.22, Found: C, 63.06; H, 5.97; N, 8.94.

### Example 17

### N-[1-[[4-(2-furanyl)phenoxy]methyl]-2-(3,4,4-trimethyl-2,5-dioxo-1-imidazolidinyl)ethyl]-N-hydroxyformamide

Prepared in the same manner as example 16 substituting 2-(tributylstannyl) furan for tributyl(phenylethynyl)tin in example16D.
¹H NMR (300 MHz, d₆-DMSO) δ 9.86 (s, 0.5H), 9.53 (s, 0.5H), 8.30 (s, 0.5H), 7.91 (s, 0.5 H), 7.67-7.61 (m, 6H), 6.96-6.94 (m, 4H); 6.79-6.74 (d, 2H, J=3 4 Hz), 6.55-6.54 (m, 2H), 4.80-4.60 (m, 0.5H), 4.45-4.30 (m, 0.5H), 4.17-3.98 (m, 6H), 3.76-3.70 (m, 2H), 3.62-3.56 (m, 2H), 2.79 (s, 6H), 1.28 (s, 12H).
MS (ESI) m/e M+H (402), M-H (400), M+NH4 (419).
Anal. Calcd for: C₂₀H₂₃N₃O₆: C, 59.84; H, 5.77; N, 10.46. Found: C, 59.83; H, 5.90; N, 10.12.

### Example 18

### N-[1-[[(4'-butoxy[1,1'-biphenyl]-4-yl)oxy]methyl]-2-(3,4,4-trimethyl-2,5-dioxo-1-imidazolidinyl)ethyl]-N-hydroxyformamide

### Example 18A

### 1-(4'-butyloxy-[1,1'-biphenyl]-4-yl)oxy)-3-(3,4,4-trimethyl-2,5-dioxoimidazolidin-1-yl)-2-propanone

To a warm (75°) solution of example 16C (0.1g, 0.27mmol) and 4-butoxyphenylboronic acid (0.08g, 0.41mmol) in DME (2mL) was added a 1M Na₂CO₃ solution (0.4mL) followed by a catalytic amount of PdCl₂(dppf). The reaction was held at 100°for 2h, diluted with ethyl acetate (25mL), washed with sat'd ammonium chloride, water, brine, dried over magnesium sulfate and concentrated *in vacuo*. Flash chromatography (20% ethyl acetate in methylene chloride) gave 0.105g of 5 as a white solid.

### Example 18B

### N-[1-[[(4'-butoxy[1,1'-biphenyl]-4-yl)oxy]methyl]-2-(3,4,4-trimethyl-2,5-dioxo-1-imidazolidinyl)ethyl]-N-hydroxyformamide

Prepared in the same manner as example 2D.E.F substituting 18A for 2C. ¹H NMR (300 MHz, d₆-DMSO) δ 9.87 (s, 0.5H), 9.55 (s, 0.5H), 8.31 (s, 0.5H), 7.91 (s, 0.5H), 7.55-7.50 (m, 8H), 6.98-6.95 (m, 8H), 4.80-4.60 (m, 0.5H), 4.50-4.35 (m, 0.5H), 4.16-4.06 (m, 4H), 4.01-3.96 (t, 4H, J=7.0, 5.9 Hz), 3.76-3.70 (m, 2H), 3.62-3.59 (m, 2H), 2.80 (s, 6H), 1.72-1.65 (m, 4H), 1.48-1 40 (m, 4H), 1.29 (s, 12H), 0.96-0.91 (t, 6H, J=7.1, 7.5 Hz).
MS (ESI) m/e M+H (484), M+NH4 (506).
Anal. Calcd for: C₂₆H₃₃N₃O₆: C, 64.57; H, 6.87; N, 8.68. Found: C, 64.70; H, 7.04; N, 8.50.

### Example 19

### N-[1-[[(4'-fluoro[1,1'-biphenyl]-4-yl)oxy]methyl]-2-(3,4,4-trimethyl-2,5-dioxo-1-imidazolidinyl)ethyl)-N-hydroxyformamide

### Example 19A

### 3-(3,4,4-trimethyl-2,5-dioxo-1-imidazolidinyl)-propan-[1,2]oxirane

The title compound was prepared following the procedures described for example 5A and 5C, but usinh allyl alcohol in place of 3-buten-1-ol.

### Example 19B

### N-[1-[[(4'-fluoro[1,1'-biphenyl]-4-yl)oxy]methyl]-2-(3,4,4-trimethyl-2,5-dioxo-1-imidazolidinyl)ethyl]-N-hydroxyformamide

Prepared according to the sequence of reaction described in examples 5D through 5H, substituting 19A for 5C in example 5D and 4-(4'-fluorophenyl)-phenol for 4'-hydroxy-4-biphenyl carbonitrile in example 5F.
¹H NMR (300 MHz, d₆-DMSO) δ 9.86 (S, 0.5H), 9.54 (S, 0.5H), 8.31 (S, 0.5H), 7.91 (S, 0.5H), 7.67-7.57 (M, 6H), 7.27-7.22 (M, 3H), 7.01-6.97 (M, 3H), 4.96-4.70 (M, 0.5H), 4.50-4.40 (M, 0.5H), 4.18-4.08 (M, 3H), 3.77-3.73 (M, 2H), 2.79 (S, 6H), 1.28 (S, 12H). MS (ESI) m/e 430 (M+H), 428 (M-H).
Anal. Calcd for: C₂₂H₂₄N₃O₅F•0.5H₂O: C, 60.26; H, 5.74; N, 9.58. Found: C, 60.48; H, 5.66; N, 8.72.

### Example 20

### N-[1-[(3,4,4-trimethyl-2,5-dioxo-1-imidazolidinyl)methyl]-2-[[4'-(trifluoromethyl)[1,1'-biphenyl]-4-yl]oxy]ethyl]-N-hydroxyformamide

Prepared according to the sequence of reaction described in examples 5D through 5H, substituting 19A for 5C in example 5D and substituting 4-(4'-Trifluoromethylphenyl)phenol for 4'-hydroxy-4-biphenyl carbonitrile in example 5F.
¹H NMR (300 MHz, d₆-DMSO) δ 9.80 (S, 0.5H), 9.58 (S, 0.5H), 8.32 (S, 0.5H), 7.92 (S, 0.5H). 7.87-7.84 (D, 4H, J=8.1 Hz), 7.79-7.76 (D, 4H, J=8.8 Hz), 7.72-7.69 (d, 4H, J=8.4 Hz), 7.06-7.02 (m, 4H), 4.80-4.60 (m, 0.5H), 4.45-4.40 (m, 0.5H), 4.20-4.12 (m, 4H), 3.78-3.74 (m, 2H), 3.63-3.62 (m, 2H), 2.08 (s, 6H), 1.30 (s, 12H).
MS (ESI) m/e M-H (478), M+H (480).
Anal. Calcd for: C₂₃H₂₄N₃O₅F₃•0.5H₂O: C, 56.55; H, 5.15; N, 8.60. Found: C, 56.52; H, 5.07; N, 8.43.

### Example 21

### N-[1-[[(4'-methoxy[1,1'-biphenyl]-4-yl)oxy]methyl]-2-(3,4,4-trimethyl-2,5-dioxo-1-imidazolidinyl)ethyl]-N-hydroxyformamide

Prepared according to the sequence of reaction described in examples 5D through 5H, substituting 19A for 5C in example 5D and substituting 4-(4'-methoxyphenyl)phenol for 4'-hydroxy-4-biphenyl carbonitrile in example 5F.
¹H NMR (300 MHz, d₆-DMSO) δ 9.89 (s, 0.5H), 9.57 (s, 0.5H), 8.31 (s, 0.5H), 7.91 (s, 0.5H), 7.56-7.53 (d, 8H, J=8.8 Hz), 7.01-6.94 (m, 8H), 4.80-4.60 (m, 0.5H), 4.44-4.35 (m, 0.5H), 4.17-3.95 (m, 4H), 3.74-3.71 (m, 2H), 3.63-3.58 (m, 2H), 2.79 (s, 6H), 1.28 (s, 12H).
MS (ESI) m/e M+H (442).
Anal. Calcd for: C₂₃H₂₆N₃O₆•0.25H₂O: C, 62.08; H, 6.00; N, 9.44. Found: C, 62.25; H, 6.30; N, 8.94.

### Example 22

### N-[1-[[(4'-methyl[1,1'-biphenyl]-4-yl)oxy]methyl]-2-(3,4,4-trimethyl-2,5-dioxo-1-imidazolidinyl)ethyl]-N-hydroxyformamide

Prepared according to the sequence of reaction described in examples 5D through 5H, substituting 19A for 5C in example 5D and substituting 4-(4'-methylphenyl)phenol for 4'-hydroxy-4-biphenyl carbonitrile in example 5F.
¹H NMR (300 MHz, d₆-DMSO) δ 9.89 (s, 0.5H), 9.57 (s, 0.5H). 8.31 (s, 0.5H), 7.92 (s, 0.5H). 7.59-7.56 (d, 4H, J=8.8 Hz), 7.52-7.49 (d, 4H, J=8.1 Hz), 7.24-7.22 (d, 4H, J=7.7 Hz), 7.00-6.97 (m, 4H), 4.80-4.60 (m, 0.5H), 4.40-4.35 (m, 0.5H), 4.40-4.1 (m, 4H), 3.80-3.55 (m, 2H), 3.60-3.50 (m, 2H), 2.79 (s, 6H), 2.32 (s, 6H), 1.28 (s, 12H). MS (ESI) m/e 424 (M-H), 426 (M+H).
Anal. Calcd for: C₂₃H₂₇N₃O₅•0.25H₂O: C. 64.24; H, 6.44, N, 9.77. Found: C, 64.30; H, 6.55; N, 9.36.

### Example 23

### N-[1-[[(4'-butoxy[1,1'-biphenyl]-4-yl)oxy]methyl]-2-(4,4-dimethy-2,5-dioxo-1-imidazolidinyl)ethyl]-N-hydroxyformamide

### Example 23A

### 1-bromo-3-(4,4-dimethyl-2,5-dioxoimidazolidin-1-yl)propan-2-one

The title compound was prepared following the procedure desribed in examples 16A and 16 B, except substituting 5.5 dimethylhydantoin for 1.5.5 trimethylhydantoin in example 16A.

### Example 23B

### N-[1-[[(4'-butoxy[1,1'-biphenyl]-4-yl)oxy]methyl]-2-(4,4-dimethy-2,5-dioxo-1-imidazolidinyl)ethyl]-N-hydroxyformamide

Prepared according to the procedures of example 16C and 16E, substituting example 23A for 16B and 4-(4'-Butyloxyphenyl)phenol for 4-bromophenol in example 16C.
¹H NMR (300 MHz, d₆-DMSO) δ 9.86 (s, 0.5H), 9.55 (s, 0.5H), 8.36 (s, 0.5H), 8.34 (s, 0.5H), 8.32 (s, 0.5H), 7.94 (s, 0.5H), 7.55-7.51 (m, 8H), 6.99-6.96 (m, 8H), 4.85-4.80 (m, 0.5H), 4.40-4.36 (m, 0.5H), 4.20-4.06 (mm, 2H), 4.01-3.97 (m, 4H), 3.78-3.71 (m, 2H), 3.60-3.51 (m, 2H), 1.73-1.66 (m, 6H), 1.48-1.38 (m, 4H), 1.25 (s, 12H), 0.96-0.86 (m, 6H).
MS (ESI) m/e M-H (468).
Anal. Calcd for: C₂₅H₃₀N₃O_{6:} C, 63.95; H, 6.65; N, 8.94. Found: C, 63.89; H, 6.91; N, 8.63.

### Example 24

### N-[1-[(4,4-dimethy-2,5-dioxo-1-imidazolidinyl)methyl]-2-[(4'-ethoxy[1,1'-biphenyl]-4-yl)oxy]ethyl]-N-hydroxyformamide

Prepared according to the procedures of example 23B, except substituting 4-(4'-ethoxyphenyl)phenol for 4-(4'-Butyloxyphenyl)phenol.
¹H NMR (300 MHz, d₆-DMSO) δ 9.84 (s, 0.5H), 9.52 (s, 0.5H), 8.37 (s, 0.5H), 8.32 (s, 2H), 7.92 (s. 0.5H), 7.55-7.52 (m, 8H), 6.98-6.95 (d, 4H, J=8.8 Hz), 4.90-4.80 (m, 0.5H), 4.45-4.30 (m, 0.5H), 4.19-3.98 (m, 8H), 3.74-3.67 (m, 2H), 3.59-3.53 (m, 2H), 1.36-1.25 (m, 18H).
MS (ESI) m/e M-H (440), M+H (442).
Anal. Calcd for: C₂₃H₂₇N₃O₆•0.5H₂O: C, 61.32; H. 6.26, N, 9.32. Found: C, 61.12; H. 6.35; N, 9.32.

### Example 25

### N-[1-[[4-(1,3-benzodioxol-5-yl)phenoxy]methyl]-2-(4,4-dimethy-2,5-dioxo-1-imidazolidinyl)ethyl]-N-hydroxyformamide

Prepared according to the procedures of example 23B, except substituting 4-(3',4'-methylenedioxyphenyl)-phenol for 4-(4'-Butyloxyphenyl)phenol.
¹H NMR (300 MHz, d₆-DMSO) δ 9.84 (s. 0.5H), 9.52 (s, 0.5H), 8.37-8.31 (m, 3H), 7.91 (s, 0.5H), 7.55-7.52 (d, 4H, J=8.5 Hz), 7.19 (s, 2H), 7.09-7.06 (m, 2H), 6.97-6.93 (d, 6H, J=10.2 Hz), 6.03 (s, 4H), 4.70-4.60 (m, 0.5H), 4.45-4.30 (m, 0.5H), 4.16-3.96 (s, 6H), 3.73-3.57 (m, 5H), 1.27 (s, 12H).
MS TEST) m/e M+H (442), M-H (440).
Anal. Calcd for: C₂₂H₂₃N₃O₇·0.50H₂O: C, 58.66; H, 5.37; N, 9.32. Found: C, 58.70; H, 5.80; N, 8.79.

### Example 26

### N-[1-[[(4'-butoxy[1,1'-biphenyl]-4-yl)oxy]methyl]-2-(3-methy-2,5-dioxo-1-imidazolidinyl)ethyl]-N-hydroxyformamide

### Example 26A

### 1-bromo-3-(3-methyl-2,5-dioxoimidazolidin-1-yl)propan-2-one

The title compound was prepared following the procedure desribed in examples 16A and 16 B, except substituting 1-methylhydantoin for 1,5,5 trimethylhydantoin in example 16A.

### Example 26B

### N-[1-[[(4'-butoxy[1,1'-biphenyl]-4-yl)oxy]methyl]-2-(3-methy-2,5-dioxo-1-imidazolidinyl)ethyl]-N-hydroxyformamide

Prepared according to the procedures of example 16C and 16E, substituting example 26A for 16B and 4-(4'-Butyloxyphenyl)phenol for 4-bromophenol in example 16C.
¹H NMR (300 MHz, d₆-DMSO) δ 9.97 (s, 0.5H), 9.60 (s, 0.5H), 8.34 (s, 0.5H), 7.97 (s, 0.5H), 7.55-7.50 (m, 8H), 6.99-6.91 (m, 8H), 4.86-4.82 (m, 0.5H), 4.33-4.31 (m, 0.5H), 4.18-4.12 (m, 2H), 3.99-3.94 (m, 4H), 2.85 (s, 6H), 1.82-1.68 (m, 4H), 1.50-1.38 (m, 6H), 0.96-0.91 (m, 6H).
MS (ESI) m/e M-H (454).
Anal. Calcd for: C₂₄H₂₉N₃O₆•0.25C₄H₅0: C, 63.51; H, 6.44; N, 8.88. Found: C, 63.59; H, 6.46; N, 8.68.

### Example 27

### N-[1-[[4-(3-thienyl)phenoxy]methyl]-2-(3,4,4-trimethyl-2,5-dioxo-1-imidazolidinyl)ethyl]-N-hydroxyformamide

Prepared according to the procedures of example 16C and 16E, substituting 4-(4'-(3-thienyl)phenyl)phenol for 4-bromophenol in example 16C.
¹H NMR (300 MHz, d₆-DMSO) δ 9.90 (s, 0.5H), 9.57 (s, 0.5H), 8.31 (s, 0.5H), 7.91 (s, 0.5H), 7.75-7.74 (m, 2 H), 7.67-7.60 (m, 6H). 7.52-7.49 (m, 2H), 6.96-6.92 (m, 4H), 4.80-4.6 (m, 0.5H), 4.50-4.4 (m, 0.5H), 4.19-3.98 (m, 6H), 3.81-3.70 (m, 2H), 3.61-3.56 (m, 2H), 2.79 (s, 6H), 1.29 (s, 12H).
Anal. Calcd for: C₂₀H₂₃N₃O₅S: C, 57.54; H, 5.55; N, 10.06, Found: C, 57.72; H, 5.84; N, 9.76.

### Example 28

### N-[1-[[([1,1'-biphenyl]-4-yl)oxy]methyl]-2-(3,4,4-trimethyl-2,5-dioxo-1-imidazolidinyl)ethyl]-N-hydroxyformamide

Prepared according to the procedures of example 16C and 16E, substituting 4-phenyl-phenol for 4-bromophenol in example 16C.
¹H NMR (300 MHz, d₆-DMSO) δ 9.90 (S, 0.5H), 9.57 (S, 0.5H), 8.30 (S, 0.5H), 7.92 (S, 0.5H), 7.62-7.60 (D, 8H, J=8.1 Hz), 7.45-7.40 (t, 4H, J=5.8, 7.8 Hz), 7.33-7.28 (t, 2H, J=7.1, 6.9 Hz), 7.02-6.97 (m, 4H), 4.80-4.60 (m, 0.5H), 4.45-4.40 (m, 0.5H), 4.18-4.01 (m, 4H), 3.77-3.70 (m, 2H), 3.63-3.6 (m, 2H), 2.80 (s, 6H), 1.28 (s, 12H).
MS (ESI) m/e M-H (410), M+H (412).
Anal. Calcd for: C₂₂H₂₅N₃O₅•0.25H₂O: C, 63.25; H, 6.17; N, 10.10. Found: C, 63.94; H, 6.41; N, 9.60.

### Example 29

### N-[1-[[(3'-chloro-4'-fluoro[1,1'-biphenyl]-4-yl)oxy]methyl]-2-(3,4,4-trimethyl-2,5-dioxo-1-imidazolidinyl)ethyl]-N-hydroxyformamide

Prepared according to the procedures of example 16C and 16E, substituting 4-(4'-fluoro-3'-chloro-phenyl)phenol for 4-bromophenol in example 16C.
¹H NMR (300 MHz, d₆-DMSO) δ 9.90 (s, 0.5H), 9.57 (s, 0.5H), 8.31 (s, 0.5H), 7.92 (s, 0.5H), 7.84-7.82 (m, 2H), 7.65-7.61 (m, 6H), 7.49-7.43 (t, 2H, J=9.2,8.8 Hz), 7.02-6.96 (m, 4H), 4.80-4.60 (m, 0.5H), 4.43-4.40 (m, 0.5H), 4.21-4.06 (m, 4H), 3.82-3.70 (m, 2H), 3.62-3.59 (m, 2H), 2.79 (s, 6H), 1.28 (s, 12H).
MS (ESI) m/e M-H (462), M+H (464).
Anal. Calcd for: C₂₂H₂₃N₃O₅ClF•0.25H₂O: C, 56.41; H, 5.05; N, 8.97. Found: C, 56.78; H, 5.24; N, 8.55.

### Example 30

### N-[1-[[(2'-methyl[1,1'-biphenyl]-4-yl)oxy]methyl]-2-(3,4,4-trimethy-2,5-dioxo-1-imidazolidinyl)ethyl]-N-hydroxyformamide

Prepared according to the procedures of example 16C and 16E, substituting 4-(3'-methyl-phenyl)phenol for 4-bromophenol in example 16C.
¹H NMR (300 MHz, d₆-DMSO) δ 9.90 (s, 0.5H), 9.57 (s, 0.5H), 8.32 (s, 0.5H), 7.93 (s, 0.5H), 7.28-7.14 (mm, 12H), 6.99-6.64 (m, 4H), 4.90-4.80 (m, 0.5H), 4.42-4.40 (m, 0.5H), 4.22-4.04 (m, 6H), 3.82-3.74 (m, 2H), 3.62-3.58 (m. 2H), 2.80 (s, 6H), 2.20 (s. 6H), 1.29 (s, 12H).
MS (ESI) m/e M+H (426), M-H (424).
Anal. Calcd for: C₂₃H₂₇N₃O₅•0.25H₂0: C, 64.24; H, 6.44; N, 9.77. Found: C, 64.50; H, 6.69; N, 9.31.

### Example 31

### N-[1-[[(4'-cyanol[1,1'-biphenyl]-4-yl)oxy]methyl]-2-(2,5-dioxo-1-imidazolidinyl)ethyl]-N-hydroxyformamide

### Example 31A

### 3-(4-(4'-Carbonitrilephenyl)phenoxy)-1-bromopropan-2-one

The title compound was prepared according to the procedure described in examples 16A nd 16B, but substituting 4-(4'-cyanophenyl)-phenol for 1,5,5-trimethyl hydantoin in example 16A.

### Example 31B

### N-[1-[[(4'-cyanol[1,1'-biphenyl]-4-yl)oxy]methyl]-2-(2,5-dioxo-1-imidazolidinyl)ethyl]-N-hydroxyformamide

Prepared according to the procedures of example 16C and 16E, substituting example 31A for 16B and hydantoin for 4-bromophenol in example 16C.
¹H NMR (300 MHz, DMSO-d₆) d 3.6-3.8 (m, 2H), 3.92 (d, 2H, J=8.4 Hz), 4.10-4.25 (m, 2H), 4.3-4.4 (m, 0.5H), 4.8-4.9 (m, 0.5H), 7.0-7.1 (m, 2H), 7.74 (d, 2H, J=9.0 Hz), 7.84 (d, 2H, J=8.4 Hz), 7.89 (d, 2H, J=8.4 Hz), 7.98 (s, 0.5H), 8.1-8.2 (m, 1H), 8.35 (s, 0.5H), 9.57 (br s, 0.5H), 9.53 (br s, 0.5H). MS (ESI+) 395 (M+H). Anal. Calcd for C₂₀H₁₈N₄O₅•0.2H₂O•0.4EtOAc: C. 59.88; H, 5.03; N, 12.93. Found: C, 59.80; H, 4.81; N, 12.74.

### Example 32

### N-[1-[[(4'-cyanol[1,1'-biphenyl]-4-yl)oxy]methyl]-2-(1,1-dioxido-3-oxo-1,2-benzisothiazol-2(3H)-yl)ethyl]-N-hydroxyformamide

Prepared according to the procedures of example 16C and 16E, substituting example 31A for 16B and saccharin for 4-bromophenol in example 16C.
¹H NMR (300 MHz, DMSO-d₆) d 3.9-4.2 (m, 2H), 4.2-4.3 (m, 2H), 4.45-4.55 (m, 0.5H), 5.0-5.1 (m, 0.5H), 7.0-7.1 (m, 2H), 7.74 (d, 2H, J=8.4 Hz), 7.85 (d, 2H, J=8.7 Hz), 7.88 (d, 2H, J=8.4 Hz), 8.0-8.2 (m. 3.5H). 8.3-8.4 (m, 1.5H), 9.78 (s, 0.5H), 10.14(s, 0,5H). MS (ESI-) 476 (M-H). Anal. Calcd for C₂₄H₁₉N₃O₆S•1.1H₂O: C, 57.97; H. 4.30; N, 8.45. Found: C, 58.01; H, 3.96; N, 8.16.

### Example 33

### N-[1-[(4,4-dimethy-2,5-dioxo-1-imidazolidinyl)methyl]-2-[[4'-(trifluoromethoxy)[1,1'-biphenyl]-4-yl]oxy]ethyl]-N-hydroxyformamide

Prepared according to the procedures of example 23B, except substituting 4-(4'-trifluoromethoxy phenyl)-phenol for 4-(4'-Butyloxyphenyl)-phenol
¹H NMR (300 MHz, DMSO-d₆) d 1.27 (s, 6H), 3.5-3.8 (m, 2H), 4.0-4.3 (m, 2H), 4.4-4.5 (m, 0.5H), 4.8-4.9 (m, 0.5H), 7.0-7.2 (m, 2H), 7.42 (d, 2H, J=7,8 Hz), 7.64 (d, 2H, J=8.4 Hz), 7.75 (d, 2H, J=8.7 Hz), 7.93 (s, 0.5H), 8.33 (s, 0.5H), 8.35 (s, 0.5H), 8.40 (s, 0.5H), 9.56 (s, 0.5H), 9.87 (s, 0.5H). MS (ESI+) 482 (M+H). Anal. Calcd for C₂₂H₂₁N₃O₆F₃: C, 54.88; H, 4.60; N, 8.72. Found: C, 55.22; H, 4.87; N, 8.36.

### Example 34

### N-[1-[[4-(4-phenyl-1-piperidinyl)phenoxy]methyl]-2-(3,4,4-trimethyl-2,5-dioxo-1-imidazolidinyl)ethyl]-N-hydroxyformamide

Prepared according to the procedures of example 16C and 16E, substituting the 4-phenyl-N-phenyl piperidine (prepared as in Wamer-Lambert patent WO 97/19068), for 4-bromophenol in example 16C.
¹H NMR (300 MHz, DMSO-d₆) d 1.27 (s, 3H), 1.28 (s, 3H), 1.7-1.9 (m, 4H), 2.55-2.75 (m, 3H), 2.78 (s, 1.5H), 2.79 (s, 1.5H), 3.5-3.8 (m, 4H), 3.9-4.1 (m, 2H), 4.3-4.4 (m, 0.5H), 4.7-4.8 (m, 0.5H), 6.81 (d, 2H, J=8.7Hz), 6.93 (d, 2H, J=9.0 Hz), 7.15-7.25 (m, 1H), 7.25-7.35 (m, 4H), 7.89 (s, 0.5H), 8.30 (s, 0.5H), 9.54 (s, 0.5H), 9.86 (s, 0.5H). MS (ESI+) 495 (M+H). Anal. Calcd for C₂₇H₃₄N₄O₅: C, 65.56; H, 6.92; N, 11.32. Found: C, 65.35; H, 7.24; N, 10.93.

### Example 35

### N-[1-[(4,4-dimethy-2,5-dioxo-1-imidazolidinyl)methyl]-2-[[4'-(trifluoromethyl)[1,1'-biphenyl]-4-yl]oxy]ethyl]-N-hydroxyformamide

Prepared according to the procedures of example 23B. except substituting 4-(4'-trifluoromethylphenyl)phenol for 4-(4'-Butyloxyphenyl)phenol
¹H NMR (300 MHz, DMSO-d₆) d 1.28 (s, 6H), 3.5-3.8 (m, 2H), 4.1-4.3 (m, 2H), 4.4-4.5 (m, 0.5H), 4.8-4.9 (m, 0.5H), 7.0-7.2 (m, 2H), 7.72 (d, 2H, J=8.4 Hz), 7.78 (d, 2H. J=8.4 Hz), 7.86 (d, 2H, J=8.4 Hz), 7.93 (s, 0.5H), 8.33 (s. 0.5H), 8.35 (s, 0.5H), 8.40 (s, 0.5H), 9.56 (s, 0.5H), 9.87 (s, 0.5H). MS (ESI+) 466 (M+H). Anal. Calcd for C₂₂H₂₂N₃O₅F₃•0.6H₂O: C. 55.49; H, 4.91; N, 8.82. Found: C, 55.55; H, 4.66; N, 8.77.

### Example 36

### N-[1-[[(3'-cyano[1,1'-biphenyl]-4-yl)oxy]methyl]-2-[methyl[(4-methylphenyl)sulfonyl]amino]ethyl]-N-hydroxyformamide

Prepared according to the procedures of example 16C and 16E, substituting example 31A for 16B and N-methyl-(p-tolyl)sulfonamide for 4-bromophenol in example 16C.
¹H NMR (300 MHz, DMSO-d₆) d 2.41 (s, 3H), 2.70 (s, 1.5H), 2.73 (s, 1.5H), 3.05-3.35 (m, 2H), 4.0-4.2 (m, 2H), 4.3-4.4 (m, 0.5H), 4.8-4.9 (m, 0.5H), 7.06 (d, 2H, J=8.7 Hz), 7.46 (d, 2H, J=8.1 Hz), 7.65-7.8 (m, 4H), 7.85 (d, 2H, J=8.7 Hz), 7.89 (d, 2H, J=8.7 Hz), 8.04 (s, 0.5H), 8.40 (s, 0.5H), 9.71 (s, 0.5H), 10.0 (s, 0.5H). MS (ESI+) 480 (M+H). Anal. Calcd for C₂₅H₂₅N₃O₅S: C, 62.61; H, 5.25; N, 8.76. Found: C, 62.52; H, 5.27; N, 7.98.

### Example 37

### N-[1-[[(4'-cyano[1,1'-biphenyl]-4-yl)oxy]methyl]-2-[4,4-dimethy-2,5-dioxo-3-(3-pyridinylmethyl)-1-imidazolidinyl]ethyl]-N-hydroxyformamide

### Example 37A

### 3-(3-pyridinylmethyl))-2,5-dioxo-4,4-dimethylimidazolidine

The title compound was prepared following the prosedures described in examples 68A, 68B and 69B, except substituting 3-picolyl chloride for methyl iodide in example 68B.

### Example 37B

### N-[1-[[(4'-cyano[1,1'-biphenyl]-4-yl)oxy]methyl]-2-[4,4-dimethy-2,5-dioxo-3-(3-pyridinylmethyl)-1-imidazolidinyl]ethyl]-N-hydroxyformamide

The title compound was prepared according to the procedures of example 16C and 16E, substituting example 31A for 16B and 37A for 4-bromophenol in example 16C.
¹H NMR (300 MHz, DMSO-d₆) d 1.25 (s, 6H), 3.6-3.7 (m, 1H), 3.8-3.9 (m, 1H), 4.1-4.3 (m, 2H), 4.4-4.5 (m, 0.5H), 4.56 (s, 2H), 4.85-4.95 (m, 0.5H), 7.0-7.1 (m, 2H), 7.35 (dd, 1H, J=8.1,4.8 Hz), 7.7-7.8 (m, 3H), 7.86 (d, 2H, J=8.4 Hz), 7.90 (d, 2H, J=8.4 Hz), 7.96 (s, 0.5H), 8.34 (s, 0.5H), 8.45-8.50 (narrow m, 1H), 8.60 (s, 1H), 9.64 (s, 0.5H), 9.97 (s, 0.5H). MS (ESI (+)) 514(M+H). Anal. Calcd for C₂₈H₂₇N₅O₅•1.7H₂O: C, 61.80; H, 5.63; N, 12.87. Found C, 61.77; H. 5.08; N, 12.48.

### Example 38

### N-[2-[(4'-cyano[1,1'-biphenyl]-4-yl)oxy]-1-methylpropyl]-N-hydroxyformamide

The title compound was prepared according to the procedures of example 16C and 16E, substituting 3-bromo-2-butanone for 16B and 4-(4'-cyanophenyl)phenol for 4-bromophenol in example 16C.
¹H NMR (300 MHz, DMSO-d₆) d 1.1-1.3 (m, 6H), 3.8-4.0 (m, 1H), 4.3-4.7 (m, 1H), 7.0-7.1 (m, 2H), 7.6-7.7 (m, 2H), 7.8-7.9 (m, 4H), 8.02 (s, 0.5H), 8.28 (s, 0.25H), 8.33 (s, 0.25H), 9.43 (s, 0.25H), 9.60 (s, 0.25H), 9.85 (s, 0.25H), 9.95 (s, 0.25H). MS (ESI+) 311 (M+H). Anal. Calcd for C₁₈H₁₈N₂O₃•0.2H2O: C, 68.86; H, 5.91; N, 8.92. Found: C, 68.73; H, 5.79; N, 8.58.

### Example 39

### N-[1-[[(3'-cyano[1,1'-biphenyl]-4-yl]oxy]methyl]-2-(3,4,4-trimethyl-2,5-dioxo-1-imidazolidinyl)ethyl]-N-hydroxyformamide

### Example 39A

### 3'-cyano-4-hydroxy biphenyl

A 250 ml flask was charged with 2.21g (2.7 mmol) [1,1'-Bis(diphenylphosphino) ferrocene] dichloropalladium (II) • CH2Cl2 and 6.26g (2.73 mmol) 3-iodobenzonitrile, 6.0g (3.95 mmol) 4-methoxyphenylboronic acid and 12.45g (8.20 mmol) cesium fluoride added as solids followed by addition Of 180 ml 1,2-dimethoxyethane. The flask was flushed with N2 and the suspension heated to reflux which was maintained for 3 hours. the cooled reaction mixture was filtered through a pad of 300g flash silica gel and the pad was washed with 1L ethyl acetate. The ethyl acetate was concentrated and the residue purified by flash chromatography eluting with 10% hexanes/ 90% ethyl acetate to give 3.3g of the desired product(58% yield). This material was dissolved in 50 ml anhydrous CH2Cl2 and the solution cooled in a dry ice- acetone bath and a solution of boron tribromide ( 40 ml, 4 mmol) was added dropwise under inert atmosphere. The reaction solution was then stirred at room temperature overnight. The reaction solution was cooled in an ice bath and 5 ml of H2O added dropwise followed by the addition of 20 ml 1N HCl. The mixture was stirred for 1 hour and the resulting suspension was filtered and the filtrate transferred to a separatory funnel and the organic layer separated off and set aside, the filtered solid was washed with H2O and ethyl acetate and filtered and the filtrate transferred to the separatory funnel and the organic layer combined with the previous organic layer, dried over Na2SO4, filtered and the filtrate concentrated to a 3.05g of a white solid ( 99% yield).

### Example 39B

### N-[1-[[(3'-cyano[1,1'-biphenyl]-4-yl)oxy]methyl]-2-(3,4,4-trimethyl-2,5-dioxo-1-imidazolidinyl)ethyl]-N-hydroxyformamide

Prepared according to the procedures of example 16C and 16E, substituting 3'-cyano-4-hydroxy biphenyl for 4-bromophenol in example 16C.
mp:130-132
1H NMR (DMSO-d6) d 9.86 (s, 1/2H), 9.48-9.63 (c, 1/2H), 8.33 (s, 1/2H), 8.09 (s, 1H), 7.97 (d, 1H, J=4.5 Hz), 7.93 (s, 1/2H), 7.75 (d, 1H, J=4.5 Hz), 7.70 (d, 2H, J=6.0 Hz), 7.63 (t, 1H, J=4.5 Hz), 7.00-7.07 (c, 2H), 4.83-4.90 (c, 1/2H), 4.60-4.67 (c, 1/2H) ESI(+): 409(M-27),437(M+H).454(M+NH4),459(M+Na)
Anal. Calcd for: C23H24N4O5•0.25C4H8O2: C, 62.87; H, 5.71; N, 12.21. Found: C, 62.68; H, 5.55; N, 12.27.

### Example 40

### N-[1-[[[4'-(methylthio)[1,1'-biphenyl]-4-yl]oxy]methyl]-2-(3,4,4-trimethyl-2,5-dioxo-1-imidazolidinyl)ethyl]-N-hydroxyformamide

Prepared according to the procedures of example 16C and 16E, substituting 4'-thiomethyl-4-hydroxy biphenyl for 4-bromophenol in example 16C.
mp172-174.
1H NMR (DMSO-d6) d 9.48-9.96 (BS. 1H), 8.34 (S, 1/2H), 7.94 (S, 1/2H), 7.54-7.63 (C, 4H), 7.29-7.34 (C, 2H), 6.97-7.03 (C, 2H), 4.82-4.92 (C, 1/2H), 4.39-4.47 (C, 1/2H), 4.07-4.25 (C, 2H), 3.73-3.85 (C, 1H), 3.59-3.68 (C, 1H), 2.80 (S, 1.5H), 2.79 (S, 1.5H)
MS (ESI(-)) 456 ((M-H)), 913 ((2M-H)) Theory:458.175 Found:458.1747
Anal. Calcd for: C23H27N3O5S C, 60.37; H, 5.95; N, 9.19;S;7.01 Found: C,60.29; H, 5.82; N, 9.08;S;6.98.

### Example 41

### N-[1-[4-[[4-(trifluoromethyl)phenoxy]phenoxy]methyl]-2-(3,4,4-trimethyl-2,5-dioxo-1-imidazolidinyl)ethyl]-N-hydroxyformamide

Prepared according to the procedures of example 16C and 16E, substituting 4-(4'-trifluoromethylphenoxy)phenol for 4-bromophenol in example 16C.
mp121-123
1H NMR (DMSO-d6) d 9.46-9.97 (C, 1H), 8.33 (S, 1/2H), 7.94 (S, 1/2H), 7.71 (S, 1H), 7.69 (S, 1H), 7.04-7.14 (C, 4H), 6.97-7.03 (C, 2H), 4.81-4.91 (C, 1/2H), 4.39-4.47 (C, 1/2H), 4.14-4.22 (C, 1H), 4.04-4.13 (C, 1H), 2.81 (S, 1.5H), 2.80 (S, 1.5H), 1.30 (S, 1.5H)
MS CESI(-)) 494 (M-H), 530 (M+Cl), 989 (2M-H), 1011 (2M+Na2H) Theory:496.169, Found:496.1696
Anal. Calcd for: C23H24F3N3O6 Theory:C, 55.75; H, 4.88; N, 8.48;F,11.50. Found: C,55.68; H, 4.92; N, 8.40;F,11.24.

### Example 42

### N-[1-[[[4'-(trifluoromethoxy)[1,1'-biphenyl]-4-yl]oxy]methyl]-2-(3,4,4-trimethy-2,5-dioxo-1-imidazolidinyl)ethyl]-2-N-hydroxyformamide

Prepared according to the procedures of example 16C and 16E, substituting 4'-trifluoromethoxy-4-hydroxy biphenyl for 4-bromophenol in example 16C.
mp 129.3-130
1HNMR, 400Mz (DMSO-d6): d 9.46-9.84(c,1H); 8.26(s,1/2H); 7.87(s,1/2H); 7.67(s,1H); 7.65(s,1H); 7.57(s,1H); 7.55(s,1H); 7.34(s,1H); 7.32(s,1H); 6.94-6.97(c,2H); 4.78-4.82(c,1/2H); 4.34-4.38(c,1/2H); 4.02-4.17(c,2H); 3.67-3.77(c,1H); 3.53-3.60(c,1H); 2.73(s,1.5H); 2.72(s,1.5H); 1.22(s.3H); 1.21(s,3H).
MS (ESI(-)): 494(M-H), 530(M+Cl), 989(2M-H), 1011(2M+Na-2H).
Theory: 496.1695 Found: 496.1680
Anal. Calcd. for C23H24F3N3O6 Theory: C,55.75; H,4.88; N,8.48; F,11.50. Found: C,55.69; H.4.94; N,8.23; F,11.71.

### Example 43

### N-[1-[[[4'-(methylsulfonyl)[1,1'-biphenyl]-4-yl]oxy]methyl]-2-(3,4,4-trimethy-2,5-dioxo-1-imidazolidinyl)ethyl]-N-hydroxyformamide

Prepared according to the procedures of example 48A,48B and 48C, substituting 16B for 23A for 4-bromophenol in example 48A.
mp 174-175
1HNMR, 400MHz (DMSO-d6): d 9.47-9.98(c,1H); 8.35(s,1/2H); 7.92-8.00(c,4.5H); 7.77(s,1H); 7.75(s,1H); 7.07-7.10(c,2H); 4.85-4.94(c,1/2H); 4.42-4.50(1/2H); 4.13-4.30(c,2H); 3.76-3.86(c,1H); 3.63-3.69(c,1H); 3.39(s,3H); 2.83(s,1.5H); 2.82(s,1.5H); 1.32(s,3H); 1.31(s,3H).
MS [ESI(-)]: 488(M-H), 977(2M-H), 999(2M+Na-2H)
[ESI(+)]: 490(M+H), 507(M+NH4), 512(M+Na)
Anal. calcd. for C23H28.5N3O7.75S: Theory: C,54.91; H.5.71; N,8.35;S,6.37 Found: C,54.85; H,5.76; N,8.00; S,6.31.

### Example 44

### N-[1-[[[3'-(cyanomethyll)-4'-methoxyl[1,1'-biphenyl]-4-yl]oxy]methyl]-2-(3,4,4-trimethy-2,5-dioxo-1-imidazolidinyl)ethyl]-N-hydroxyformamide

Prepared according to the procedures of example 16C and 16E, substituting 4-(3'-cyanomethyl-4'-methoxyphenyl)phenol for 4-bromophenol in example 16C.
¹H NMR (300 MHz, DMSO-d6) δ ; 1.275, 1.290 (6H), 2.788, 2.800 (3H), 3.566-3.641 (m, 1H), 3.708-3.821 (m, 1H), 4.047-4.214 (m, 2H), 4.399-4.416 (m, 0.5H), 4.846 (m, 0.5H), 6.973-7.013 (2H), 7.110-7.140 (1H), 7.543-7.608 (m, 4H), 7.291 (s, 0.5H), 8.319 (s, 0.5H), 9.576 (s, 0.5H), 9.904 (s, 0.5H).
MS (ESI) m/e 481 (M+H)⁺, 498 (M+NH₄)⁺, 479 (M-H)⁻.
Anal. calcd for C₂₅H₂₈N₄O₆-0.5MeOH: C, 61.68; H, 6.08; N, 11.28, Found: C, 62.07; H, 6.21; N, 10.91.

### Example 45

### N-[1-[[[3'-(cyanomethyl)[1,1'-biphenyl]-4-yl]oxy]methyl]-3-(4,4-dimethyl-2,5-dioxo-1-imidazolidinyl)propyl]]-N-hydroxyformamide

The title compouns was prepared according to the procedures of example 5, except avoiding the methylation step in example 5B and substituting 4-(3'-cyanomethylphenyl)phenol for 4'-hydroxy -4-biphenylcarbonitrile in example 5F.
¹H NMR (300 MHz, DMSO-d6) δ ; 1.27 (s, 6H), 1.70-2.00 (m, 2H), 3.37-3.47 (m, 2H), 3.96-4.08 (s+m, 5H), 7.00-7.03 (d, 2H, 8.4Hz), 7.28--7.31 (d, 1H, 8.7Hz), 7.426-7.477 (t, 1H, 7.5Hz), 7.56-7.61 (m, 4H), 7.915 (s, 0.73H), 8.28-8.34 (1.27H), 9.55 (s, 0.75H), 9.96 (s, 0.25H).
MS (ESI) m/e 451 (M+H)⁺, 468 (M+NH₄)⁺, 449 (M-H)⁻.
Anal. calcd for C₂₅H₂₈N₄O₆·MeOH: C, 62.22; H, 6.26; N, 11.61, Found: C, 62.25; H, 5.95; N, 11.57.

### Example 46

### N-[1-[[(4'-butoxy[1,1'-biphenyl]-4-yl)sulfonyl]methyl]-2-(4,4-dimethy-2,5-dioxo-1-imidazolidinyl)ethyl]-N-hydroxyformamide

### Example 46A

### 1-(4-bromophenylthio)-3-(4,4-dimethyl-2,5-dioxoimidazolidin-1-yl)-2-propanone

A solution of 4-bromothiophenol (2.15 g, 11.4 mmol) in DMF (50 ml) at ambient temperature was treated with cesium carbonate ( 5.57 g, 17.1 mmol) for 20 minutes, treated in a single portion with example 23A (2.5 g, 9.5 mmol), stirred for 1 hours at ambient temperature and diluted with water, extracted with ethyl acetate, the combined extracts were washed with water and brine, dried (Na₂SO₄), concentrated and purified on silica gel with 20 t0 35 to 50% ethyl acetate/hexane to provide 3.17 g (90%) of the titled compound as a white solid.
MS (APCI) m/e 371, 373 (M+H)⁺, 388, 390 (M+NH₄)⁺, 369, 371 (M-H), 405, 407 (M+Cl)⁻.

### Example 46B

### 1-[(4'-butoxy[1,1'-biphenyl]-4-yl)thio]-3-(4,4-dimethyl-2,5-dioxoimidazolidin-1-yl)-2-propanone

A solution of example 46A (700 mg, 1.89 mmol) in DME (20 ml) at ambient temperature was treated with 4-n-butoxybenzeneboronic acid (549 mg, 2.83 mmol), tetrakis-(triphenylphosphine)-palladium (218 mg, 0.189 mmol) and 1M sodium carbonate (3.54 mL, 3.54 mmol), the reaction vessel was sealed and heated at 90°C for 6 hours, diluted with ethyl acetate, washed with sequentially saturated ammonium chloride solution, water and brine, dried (Na₂SO₄) concentrated and purified on silica gel with 30 to 50% ethyl acetate/dichloromethane to provide 650 mg (78%) of the title compound as a yellow solid.
MS (APCI) m/e 441 (M+H)⁺, 458 (M+NH₄)⁺, 439 (M-H), 475 (M+Cl)⁻.

### Example 46C

### N-[1-[[(4'-butoxy[1,1'-biphenyl]-4-yl)thio]methyl]-2-(4,4-dimethy-2,5-dioxo-1-imidazolidinyl)ethyl]-N-hydroxyformamide

The title compound was prepared from 46B following the procedures described in example 2D, 2E, 2F.
¹H NMR (300 MHz, DMSO-d6) δ ; 0.919-0.967 (t, 3H, J=7.2Hz), 1.225-1.237(s+s, 6H), 1.389-1.512(m, 2H), 1.666-1.760(m, 2H), 3.110-3.192(m, 2H), 3.528-3.735 (m, 2H), 3.987-4.030(t, 2H, J=6.3Hz), 4.030(m, 0.5H), 4.750(m, 0.5H),6.991-7.020 (d, 2H, J=9 Hz), 7.383-7.417 (dd, 2H, J=1.8, 8.4 Hz), 7.561-7.601 (4H), (1.5H), 9.56 (s, 7.767(s, 0.5H), 8.299(s, 1H), 8.337(s, 0.5H), 9.457(br s, 0.5H), 9.695(br s, 0.5H).
MS (ESI) m/e 484 (M-H)⁻.
High resolution MS(FAB) Calc. m/z for m·⁺485.1984, observed m/z 485.1980.

### Example 46D

### N-[1-[[(4'-butoxy[1,1'-biphenyl]-4-yl)sulfonyl]methyl]-2-(4,4-dimethy-2,5-dioxo-1-imidazolidinyl)ethyl]-N-hydroxyformamide

A solution of example 46C (127 mg, 0.262 mmol) in methanol (2 mL), and PH 7 buffer (1 mL) at 0°C was treated with oxone (402 mg, 0.655 mmol) for 30 minutes then ambient temperature for 1 hour, neutralized with saturated sodium bicarbonate, extracted with dichloromethane, combined extracts were washed with brine, dried (Na₂SO₄) and concentrated. The crude mixture was purified on silica gel with 50% ethyl acetate/hexane then 10% methanol/dichloromethane to provide 82 mg (60%) of the title compound as an white solid.
¹H NMR (300 MHz, DMSO-d6) δ ; 0.92-0.97 (t, 3H, 7.5Hz), 1.20, 1.22 (s+s, 6H), 1.42-1.52 (m, 2H), 1.68-1.77 (m, 2H), 3.41-3.72 (m, 3.5H), 4.02-4.06 (t, 2H, 6.6Hz), 4.52 (m, 0.5H), 4.89 (m, 0.5H), 7.05-7.08 (d, 2H, 8.4Hz), 7.70--7.74 (2H),7.91 (s, 3.5H), 8.10 (s, 0.5H), 8.32-8.35 (d, 1H, 9.6Hz), 9.48 (s, 0.5H), 9.62 (s, 0.5H).
MS (ESI) m/e 518 (M+H)⁺, 535 (M+NH4)⁺, 516 (M-H)-, 552 (M+Cl)⁻.
Anal. calcd for C₂₅H₂₈N₄O₆·0.25H₂O: C, 57.51; H, 6.08; N, 8.04. Found: C, 57.78; H, 6.18; N, 7.84.

### Example 47

### N-[1-[[(4'-cyano[1,1'-biphenyl]-4-yl)oxy]methyl]-3-(4,4-dimethy]-2,5-dioxo-1-imidazolidinyl)propyl]-N-hydroxyformamide

### Example 47A

### 1-bromo-4-(3,4,4-trimethyl-2,5-dioxoimidazolidin-1-yl)butan-2-one

To a suspension of CuBr2 (1.91g, 8.5 mmol) and lithium bromide (1.48g,17 mmol) in THF (10 mL) was added a solution of 1.06g(5.3 mmol)of 1-((1',2'Oxiranyl)propyl-4,4-dimethyl-2,5-dioxoimidazolidine(prepared from example 5A following the procedure of example5C) in 15 mL of THF. The reaction mixture was stirred for 2 h at room temperature, then partitioned between ethyl acetate and ph7 buffer.The organic extract was washed with brine, dried and concentrated. The residue was filtered through a plug of silice eluting with ethyl acetate, and the filtrate was concentrated toi give a white solid, whic was dissolved in acetone (25 mL), cooled to 0° C, then treated with 2.5 mL of 8M Jones reagent and stirred at room temperature for 3h. The reaction was quenched with 2 mL isopropanol, then partitioned between ethyl acetate and water.The organic extract was washed with brine, dried and concentrated. The residue was filtered through a plug of silice eluting with ethyl acetate, and the filtrate was concentrated to give the title compound.

### Example 47B

### N-[1-[[(4'-cyano[1,1'-biphenyl]-4-yl)oxy]methyl-3-(4,4-dimethyl-2,5-dioxo-1-imidazolidinyl)propyl]-N-hydroxyformamide

The title compound was prepared according to the procedures of example 16C and 16E, exept substituting 47A for 16B and 4'-hydroxy-4-biphenylcarbonitrile for 4-bromophenol in example 16C.
mp 202-204°C
¹H NMR (300 MHz, DMSO-d6) δ ; 1.272 (6H), 1.70-2.00 (m, 2H), 3.38-3.46 (t, 2H, J=6 Hz), 3.92-4.18 (m, 2.5H), 4.46-4.57 (m, 0.5H), 7.03-7.06 (d, 2H, J=8.7 Hz), 7.695-7.724 (d, 2H, J=8.7 Hz), 7.82-7.92 (m, 6.5H), 8.26-8.35 (1.5H), 9.75(s), 9.96(s) (1H)
MS (ESI) m/e 437 (M+H)⁺, 454 (M+NH₄)⁺, 459 (M-H)⁻.
Anal. calcd for C₂₅H₂₈N₄O₆·0.25H₂O: C, 62.64; H, 5.60; N, 12.70. Found: C, 62.55; H, 5.47; N, 12.65

### Example 48

### N-[1-[[[4'-(methylsulfonyll)[1,1'-biphenyl]-4-yl]oxy]methyl]-2-(4,4-dimethyl-2,5-dioxo-1-imidazolidinyl)ethyl]-N-hydroxyformamide

### Example 48A

### 1-(4'-(thiomethyl)[1,1'-biphenyl]-4-yl)oxy)-3-(4,4-dimethyl-2,5-dioxoimidazolidin-1-yl)-2-propanone

A solution of 4'-hydroxy-4-biphenylmethylsulfide (1.18 g, 5.47 mmol) in DMF (25 ml) at ambient temperature was treated with cesium carbonate (2.23g, 6.84 mmol) for 20 minutes, treated in a single portion with 23A (1.2 g, 4.56 mmol), stirred for 2 hours at ambient temperature and diluted with water, extracted with ethyl acetate, the combined extracts were washed with water and brine, dried (Na₂SO₄), concentrated and purified on silica gel with 50 to 80% ethyl acetate/hexane to provide 1.0 g (55%) of the title compound as a white solid.
MS (APCI) m/e 399 (M+H)⁺, 416 (M+NH₄)⁺, 397 (M-H)⁻, 433 (M+Cl)⁻.

### Example 48B

### N-[1-[[[4'-(thiomethyl)[1,1'-biphenyl]-4-yl]oxy]methyl]-2-(4,4-dimethyl-2,5-dioxo-1-imidazolidinyl)ethyl]-N-hydroxyformamide

The title compound was prepared from 48A following the procedures described in example 2D, 2E, 2F.
MS (ESI) m/e 444 (M+H)⁺, 461 (M+NH₄)⁺, 466 (M+Na)⁺, 442 (M-H)⁻.

### Example 48C

### N-[1-[[[4'-(methylsulfonyll)[1,1'-biphenyl]-4-yl]oxy]methyl]-2-(4,4-dimethyl-2,5-dioxo-1-imidazolidinyl)ethyl]-N-hydroxyformamide

A solution of example 48B (440 mg, 0.993 mmol) in methanol (100 mL) and water (50 mL) at 0°C was treated with oxone (1.27 g, 2.06 mmol) and sodium bicarbonate (174 mg, 2.06 mmol) for 1 hour then ambient temperature for 1.5 hour, diluted with water, extracted with dichloromethane, combined extracts were washed with brine, dried (Na₂SO₄) and concentrated. The crude mixture was purified on silica gel with 80% ethyl acetate/hexane then 10% methanol/dichloromethane then recrystallized from dichloromethane/hexane to provide 375 mg (79%) of the title compound as an off-white solid.
¹H NMR (300 MHz, DMSO-d6) δ ; 1.26-1.27 (s+s, 6H), 3.24 (s, 3H), 3.53-3.80 (m, 2H), 4.08-4.24 (m, 2H), 4.37-4.48 (m, 0.5H), 4.80-4.92 (m, 0.5H), 7.04-7.08 (dd, 2H, J=3, 8.4 Hz), 7.72-7.75 (d, 2H, J=8.7 Hz), 7.89-8.00 (4.5H), 8.33-8.40 (1.5H), 9.56 (s, 0.5H), 9.88 (s, 0.5H).
MS (ESI) m/e 476 (M+H)⁺, 493(M+NH₄)⁺, 474 (M-H)⁻, 510 (M+Cl)⁻.
Anal. calcd for C₂₂H₂₅N₃O₇·0.25H₂O:

### Example 49

### N-[1-[[(3'-cyano[1,1'-biphenyl]-4-yl)oxy]methyl]-2-(2,5-dioxo-1-pyrrolidinyl)ethyl]-N-hydroxyformamide

### Example 49A

### 1-(4'-Cyano-[1,1'-biphenyl]-4-yl)oxy)-3-(2,5-dioxopyrrolidin-1-yl)-2-propanone

The title compound was prepared as in Example 3C, except using potassium succinimide (0.10 g, 0.95 mmol) in place of potassium phthalimide. Purification by trituration with ethyl acetate provided 0.19g (68%) of the title compound as a white solid.
MS (APCI) m/e 383 (M+Cl)⁺.

### Example 49B

### ()-[1-(4'-Cyano-[1,1'-biphenyl]-4-yl)oxy)-3-(2,5-dioxopyrrolidin-1-yl)-prop-2-yl]hydroxylamine

The title compound was prepared from 49B using the procedure described in Example 2D and 2E.

### Example 49C

### N-[1-[[(3'-cyano[1,1'-biphenyl]-4-yl)oxy]methyl]-2-(2,5-dioxo-1-pyrrolidinyl)ethyl]-N-hydroxyformamide

The title compound was prepared from 49B using the procedure described in Example mp 128°C
¹H NMR (300MHz, d6-DMSO) d 10.01 (s, 0.5H), 9.63 (s, 0.5H), 8.34 (s, 0.5H), 7.98 (s, 0.5H), 7.90-7.82 (m, 4H), 7.73 (d, 2H, J=8.8 Hz), 7.06-6.89 (m, 2H), 4.90-4.78 (m, 0.5H), 4.37-4.24 (m, 0.5H), 4.22-4.04 (m, 2H), 3.74-3.60 (m, 2H), 2.65-2.61 (m, 4H).
MS (ESI) m/e 394 (M+H)⁺, 411(M+NH₄)⁺, 392 (M-1)⁺.
Anal. Calcd for: C₂₁H₁₉N₃O₅•H₂O: C, 61.30; H, 5.14; N, 10.21. Found: C, 61.20; H, 5.03; N, 10.03.

### Example 50

### N-[1-[[(4'-cyano[1,1'-biphenyl]-4-yl)oxy]methyl]-2-(4,4-dimethyl-2,6-dioxo-1-piperidinyl)ethyl]-N-hydroxyformamide

The title compound was prepared as in Example 49, except using potassium-3,3-dimethylglutarimide (0.16 g, 1.1 mmol) in place of potassium succinimide.
mp121°C
¹H NMR (d6-DMSO) d 9.88-9.78 (s, 0.5H), 9.60-9.52 (s, 0.5H), 8.31 (s, 0.5H), 7.95 (s, 0.5H), 7.90-7.82 (m, 4H), 7.73 (d, 2H, J=8.9 Hz), 7.02 (d, 2H, J=8.8 Hz), 4.88-4.77 (s, 1H), 4.30-3.78 (m, 4H), 2.56 (s, 4H), 0.98 (s, 6H).
MS (ESI) 436 (M+H)⁺, 458 (M+Na)⁺, 434 (M-H)⁺.

### Examples 51 and 52

### N-[1S-[[(4'-cyano[1,1'-biphenyl]-4-yl)oxy]methyl]-2-(2,5-dioxo-1-pyrrolidinyl)ethyl]-N-hydroxyformamide

### N-[1R-[[(4'-cyano[1,1'-biphenyl]-4-yl)oxy]methyl]-2-(2,5-dioxo-1-pyrrolidinyl)ethyl]-N-hydroxyformamide

### Example 51A and 52A

A solution of Example 49B (0.2 g, 0.55 mmol), D-Mannose diacetonide (0.13 g, 0.50 mmol), and acetic acid (0.03 mL, 0.50 mmol) in CHCl₃ (5 mL) were heated at reflux for 16h, cooled, and partitioned between CH₂Cl₂ and saturated aqueous sodium bicarbonate. The organic layer was washed sequentially with water and brine, dried (MgSO₄) and concentrated. Purification by HPLC provided the two enantiomers 51A (31%) and 52A (16%).

### Example 51B

A solution of 51A in MeOH (1 mL) and HCl(conc) (0.5 mL) was stirred at ambient temperature for 15 min, treated with saturated aqueous sodium bicarbonate, and partitioned between ethyl acetate and water. The organic layer was dried (MgSO₄) and concentrated to provide 0.014 g (79%) of the corresponding hydroxyl amine. which was then formylated as in Example 2F.

### Example 52B

The title compound was prepared according to Example 51B but using Example 52A in place of Example 51A.

### Example 53

### N-[1-[[(4'-cyano[1,1'-biphenyl]-4-yl)oxy]methyl]-2-(3-ethyl-3-methyl-2,5-dioxo-1-pyrrolidinyl)ethyl]-N-hydroxyformamide

The title compound was prepared as in Example 49. except using potassium-3-methyl-3-ethyl succinimide (0.22 g, 1.53 mmol) in place of potassium succinimide.
¹H NMR (d6-DMSO) d 9.99-9.94 (br, 0.5H), 9.64-9.58 (br, 0.5H), 8.31 (d, 0.5H, J=1.8 Hz), 7.93 (d, 0.5H, J=2.9 Hz), 7.87 (q, 4H, J=4.1 Hz), 7.74 (d, 2H, J=8.9 Hz), 7.04 (dd, 2H, J=8.8, 2.6 Hz), 4.93-4.81 (m, 0.5H), 4.44-4.33 (m, 0.5H), 4.24-4.05 (m, 2H), 3.85-3.71 (m, 1H), 3.62-3.53 (m, 1H), 2.69-2.38 (m, 2H), 1.64-1.46 (m, 2H), 1.18 (d, 3H, J=4.4 Hz), 0.85-0.75 (m, 3H).
MS (ESI) 436 (M +H)⁺, 434 (M - H)⁺, 458 (M + Na)⁺, 453 (M +NH4)⁺. Anal. Calcd for: C₂₄H₂₅N₃O₅: C, 66.19; H, 5.78; N, 9.64. Found: C, 66.07; H, 5.85; N, 9.37.

### Example 54

### N-[4-[4-[[(4-chlorophenoxy)phenyl]sulfonyl]methyl]tetrahydro-2H-pyran-4-yl]-N-hydroxyformamide

### Example 54A

The title compound was prepared as in Example 2D but using 5,6-dihydro-2H-pyran-2-one (4.3g, 43 mmol) in place of 1-(4-(4'-carbonitrilephenyl)phenoxy)-3-thiophenoxypropan-2-one and O-Benzyl hydroxylamine in place of hydroxylamine to provide the corresponding oxime. Purification on silica gel with 1 % methanol/dichloromethane provided 8.5g (96%) of the title compound as a clear liquid.
MS (ESI) 207 (M +H)⁺

### Example 54B

### N-[4-[4-[[(4-chlorophenoxy)phenyl]sulfonyl]methyl]tetrahydro-2H-pyran-4-yl]-N-benzyloxy amine

To a solution of phenoxyphenyl-4-chloro-4'-methylsulfone (0.76 g, 2.7 mmol)(preparation desribed in *J.Med. Chem.* **29**, 427-433, 1986) at -78°C was added n-BuLi (1.1 mL, 2.7 mmol). After stirring at -78°C for 15 minures, BF₃•OEt₂ was added, followed by Example 54A. After 1h, the reaction mixture was partitioned between with water and ethyl acetate, dried (MgSO₄) and concentrated. Recrystallization with ethyl acetate provided 0.41 g (35%)of the desired compound as a white solid.
MS (ESI) 488 (M+H)⁺, 510 (M+Na)⁺.

### Example 54C

### N-[4-[4-[[(4-chlorophenoxy)phenyl]sulfonyl]methyl]tetrahydro-2H-pyran-4-yl]-N-benzyloxyformamide

A solution of 54B (0.05 g, 0.10 mmol) in CH₂Cl₂ (2 mL) was treated with formic-p-methoxyphenyl anhydride, stirred at ambient temperature for 16h, treated with H2O, and partitioned between ethyl acetate and brine. The organice layer was dried (MgSO₄) and concentrated. Purification on silica gel with 10% ethyl acetate/dichloromethane provided 0.017 g (32%) of the desired compound as a white solid
MS (ESI) 516 (M+H)⁺, 533 (M+NH₄)⁺, 538 (M+Na)⁺.

### Example 54D

### N-[4-[4-[[(4-chlorophenoxy)phenyl]sulfonyl]methyl]tetrahydro-2H-pyran-4-yl]-N-hydroxyformamide

A solution of 54C (0.017 g, 0.033 mmol) and Pd black (0.006 g) in dioxane (2 mL) and acetic acid (2 mL) was stirred under H₂ for 20 min, treated with NaHCO₃, partitioned between ethyl acetate and water. The organic layer was dried (MgSO₄) and concentrated. Purification on silica gel with 2% MeOH/dichloromethane provided 0.002 g (14%) of the desired compound.
¹H NMR (d6-DMSO) d 9.50-9.45 (br, 1H), 8.19 (s, 1H), 7.90-7.86 (m, 2H), 7.53-7.50 (m, 2H), 7.22-7.18 (m, 4H), 3.70-3.58 (m, 4H), 3.55-3.44 (m, 2H), 2.22-2.07 (m, 2H), 2.07-1.91 (m, 2H).
MS (ESI) 424 (M - H)⁺, 426 (M + H)⁺, 448 (M + Na)⁺.

### Example 55

### N-[1-[[(4'-cyano[1,1'-biphenyl]-4-yl)oxy]methyl]-2-[[(2-methoxycarbonyl)phenyl]-thio]ethyl]-N-hydroxyformamide

The title compound was prepared following the procedure from Example 2B,C, D, E, F but using methyl thiosalicylate (600 mg, 2.39 mmol) in place of thiophenol in example 2B. Mixture of two rotamers: ¹H NMR (300 MHz, d₆-DMSO) d 10.11 (s, 1H), 9.73 (s, 1H), 8.41 (s, 1H), 7.95 (s, 1H), 7.90-7.83 (m, 10 H). 7.75-7.71 (m, 4H), 7.59-7.55 (m, 4H), 7.31-7.26 (m, 2H), 7.09-7.05 (m, 4H), 4.75 (m, 1H), 4.2804.24 (m, 4H0, 4.18 (m, 1H), 3.83 (s, 3H), 3.82 (s, 3H), 3.31-3.18 (m, 4H);
MS (ESI) m/e 463 (M+1)⁺.
Anal. calcd for C₂₅H₂₂N₂O₅S: C, 64.92; H, 4.79; N, 6.06. Found: C, 64.69; H, 4.63; N, 5.92.

### Example 56

### N-[1-[[(4'-cyano[1,1'-biphenyl]-4-yl)oxy]methyl]-5-[(4-methyl-2-oxo-2H-1-benzopyran-6-yl)oxy]pentyl]-N-hydroxyformamide

### Example 56A

### 6-(4-methyl-2-oxo-2H-1-benzopyran-6-yl)oxy]-hex-1-ene

The title compound was prepared following the procedure from Example 5A but using 6-hydroxy-4-methylcoumarin (500 mg, 2.84 mmol) in place of 5,5-dimethylhydantoin and 5-hexen-1-ol in place of 3-buten-1-ol. Purification on silica gel with 20% ethyl acetate/hexanes provided 560 mg (76%) of the title compound.
¹H NMR (300 MHz, d₆-DMSO) d 7.34 (dd, 1H), 7.24-7.20 (m, 2H), 6.40 (d, 1H), 5.90-5.77 (m, 1H), 5.04 (dq, 1H), 4.98 (dq, 1H), 4.06 (t, 2H), 2.43 (d, 3H), 2.10 (q, 2H), 1.75 (dt, 2H), 1.53 (dt, 2H).

### Example 56B

### N-[1-[[(4'-cyano[1,1'-biphenyl]-4-yl)oxy]methyl]-5-[(4-methyl-2-oxo-2H-1-benzopyran-6-yl)oxy]pentyl]-N-hydroxyformamide

The title compound was prepared following the procedures from Example 5C, 1B, 2C, 2D,2E and 2F but using 56A (500 mg, 1.94 mmol) in place of 5B in example 56B. Purification on silica gel with 50% ethyl acetate/hexanes provided 400 mg (75%) of the title compound.
Mixture of two rotamers: ¹H NMR (300 MHZ, d₆-DMSO) d 9.89 (s. 1H), 9.51 (s, 1H), 8.42 (s, 1H), 8.03 (s, 1H), 7.86 (m, 8H), 7.73-7.70 (m, 4H), 7.34 (d, 2H), 7.24-7.21 (m, 4H), 7.08-7.04 (m, 4H), 6.40 (s, 2H), 4.60 (s, 1H), 4.18-3.99 (m, 9H), 2.43 (s, 6H). 1.86-1.54 (m, 12H);
MS (ESI) m/e 513 (M+1)⁺.
Anal. calcd for C₃₀H₂₈N₂O₆: C, 70.30; H, 5.51; N, 5.47. Found: C, 70.52; H, 5.85; N, 5.20.

### Example 57

### N-[1-[[(4'-cyano[1,1'-biphenyl]-4-yl)oxy]methyl]-4-[(4-methyl-2-oxo-2H-1-benzopyran-6-yl)oxy]butyl]-N-hydroxyformamide

The title compound was prepared following the procedure from Example 56 but using 4-penten-1-ol in place of 5-hexen-1-ol in example 56A.
Mixture of two rotamers: ¹H NMR (300 MHZ, d₆-DMSO) d 9.94 (s, 1H), 9.55 (s, 1H0, 8.44 (s, 1H), 8.06 (s, 1H), 7.86 (m, 8H), 7.73-7.70 (m, 4H), 7.35 (d, 2H), 7.26-7.22 (m, 4H), 7.08-7/05 (m, 4H), 6.40 (s, 2H), 4.65 (m, 1H), 4.17-4.04 (m, 9H), 2.44 (s, 6H), 1.77 (m, 8H);
MS (ESI) m/e 499 (M+1)^{+.}
Anal. calcd for C₂₉H₂₆N₂O₆•0.75H₂O: C, 68.03; H, 5.41; N, 5.47. Found: C, 68.21; H, 5.25; N, 5.28.

### Example 58

### N-[1-[[(4'-cyano[1,1'-biphenyl]-4-yl)oxy]methyl]-4-[(4-methyl-2-oxo-2H-1-benzopyran-7-yl)oxy]butyl]-N-hydroxyformamide

The title compound was prepared following the procedure from Example 56 but using 7-hydroxy-4-methylcoumarin (500 mg, 2.8 mmol) in place of 6-hydroxy-4-methylcoumarin and4-penten-1-ol in place of 5-hexen-1-ol in example 56A.
Mixture of two rotamers: ¹H NMR (300 MHZ, d₆-DMSO) d 9.95 (s, 1H), 9.55 (s, 1H), 9.44 (s, 1H), 8.05 (s, 1H), 7.90-7.82 (m, 8H), 7.73-7.67 (m, 6H), 7.08-7.04 (m, 4H), 7.01-6.95 (m, 4H), 6.21 (s, 2H), 4.64 (m, 1H), 4.20-4.01 (m, 9H), 2.40 (s, 6H), 1.80-1.74 (m, 8H);
MS (ESI) m/e 499 (M+1)⁺.
Anal. calcd for C₂₉H₂₆N₂O₆: C, 69.87; H, 5.26; N, 5.62. Found: C, 69.51; H, 5.33; N, 5.40.

### Example 59

### N-[1-[[(4'-cyano[1,1'-biphenyl]-4-yl)oxy]methyl]-5-[(4-methyl-2-oxo-2H-1-benzopyran-7-yl)oxy]pentyl]-N-hydroxyformamide

The title compound was prepared following the procedure from Example 56 but using 7-hydroxy-4-methylcoumarin (500 mg, 2.8 mmol) in place of 6-hydroxy-4-methylcoumarin in example 56A.
Mixture of two rotamers: ¹H NMR (300 MHZ, d₆-DMSO) d 9.89 (s, 1H0, 9.50 (s, 1H0, 8.42 (s, 1H), 8.03 (s, 1H), 7.90-7.82 (m, 8H), 7.73-7.66 (m, 6H), 7.08-7.03 (m. 4H), 6.98-6.94 (m, 2H0, 6.21 (s, 2H), 4.60 (m, 1H), 4.15-3.98 (m, 9H), 2.40 (s, 6H), 1.84-1.40 (m, 12H);
MS (ESI) m/e 513 (M+1)⁺.
Anal. calcd for C₃₀H₂₈N₂O₆: C, 70.30; H. 5.51; N, 5.47. Found: C, 70.35; H, 5.52; N, 5.17.

### Example 60

### N-[1-[[(4'-cyano[1,1'-biphenyl]-4-yl)oxy]methyl]-2-(5,5-dimethy-2,4-dioxo-3-oxazolidinyl)ethyl]-N-hydroxyformamide

The title compound was prepared as in Example 49, except using 5,5-dimethyloxazolidinine-2,4-dione (300 mg, 0.8 mmol) in place of succinimide.
Mixture of two rotamers: ¹H NMR (300 MHZ, d₆-DMSO) d 10.08 (s, 1H), 9.70 (s, 1H), 8.35 (s, 1H), 7.98 (s, 1H), 7.90-7.83 (m, 8H), 7.74 (d, 4H), 7.06 (d, 4H), 4.90 (m, 1H), 4.47 (m, 1H), 4.24-4.16 (m, 4H), 3.85 (d, 1H), 3.80 (d, 1H), 3.69-3.65 (m, 1H), 3.64-3.61 (m, 1H), 1.49 (s, 6H), 1.48 (s, 6H); MS (ESI) m/e 441 (M+18)⁺.

### Example 61

### N-[1-[[(4'-cyano[1,1'-biphenyl]-4-yl)sulfonyl]methyl]-2-(3,4,4-trimethy-2,5-dioxo-1-imidazolidinyl)ethyl]-N-hydroxyformamide

### Example 61A

### 1-[(4'-cyano[1,1'-biphenyl]-4-yl)thio]-3-(3,4,4-trimethyl-2,5-dioxoimidazolidin-1-yl)-2-propanone

A solution of 4'-thiol-4-biphenylcarbonitrile (150 mg, 0.71 mmol) in 6 mL of DMF at -5 °C was treated with potassium carbonate (89 mg, 0.645 mmol) and 1-bromo-3-(3,4,4-trimethyl-2,5-dioxo-1-imidazolidin-1-yl)-2-propanone (179 mg, 0.645 mmol), stirred 1h at -5 °C. quenched with saturated NH₄Cl, extracted with ethyl acetate, washed with brine, dried over Na₂SO₄, filtered, and concentrated to a solid. Purification on silica gel with 1:1 ethyl acetate/hexanes provided 200 mg (75%) of the title compound.
¹H NMR (300 MHZ, d₆-DMSO) d 7.94-7.87 (m, 4H), 7.72 (d, 2H), 7.43 (d, 2H), 4.55 (s. 2H), 4.27 (s, 2H). 2.80 (s. 3H), 1.32 (s, 6H).

### Example 61B

### N-[1-[[(4'-cyano[1,1'-biphenyl]-4-yl)thio]methyl]-2-(3,4,4-trimethy-2,5-dioxo-1-imidazolidinyl)ethyl]-N-hydroxyformamide

The title compound was prepared from 61A following the procedures from Example 2D, 2E and 2F.
Mixture of two rotamers: ¹H NMR (300 MHZ, d₆-DMSO) d 9.76 (s, 1H), 9.51 (s, 1H), 8.29 (s, 1H), 7.93-7.87 (m, 8H), 7.75-7.72 (m, 5H), 7.50-7.44 (m, 4H), 4.60 (m, 1H), 4.10 (m, 1H), 3.80-3.60 (m, 4H), 3.25-3.15 (m, 4H), 2.77 (s, 6H), 1.25 (s, 12H).

### Example 61C

### N-[1-[[(4'-cyano[1,1-biphenyl]-4-yl)sulfonyl]methyl]-2-(3,4,4-trimethyl-2,5-dioxo-1-imidazolidinyl)ethyl]-N-hydroxyformamide

A solution of 61B (81 mg, 0.18 mmol) in 4:1 THF/H₂O at 0 °C was treated with OXONE (140 mg) and NaHCO₃ (33 mg), stirred at 0 °C for 30 min then 23 °C for 1 h, quenched with H₂O, extracted with ethyl acetate, washed with brine, dried over Na₂SO₄, filtered, and concentrated to a white solid. Purification on silica gel with 2% methanol/dichloromethane provided 43 mg (49%) of the title compound.
Mixture of two rotamers: ¹H NMR (300 MHZ, d₆-DMSO) d 9.71 (s, 1H), 9.54 (s, 1H), 8.09 (s, 1H), 8.07-7.96 (m, 16H), 7.74 (s, 1H), 4.90 (m, 1H), 4.54 (m, 1H), 3.74-3.60 (m, 4H), 3.55-3.44 (m, 4H), 2.74 (s, 3H), 2.74 (s, 3H), 1.24-1.22 (m, 12H);
MS (ESI) m/e 485 (M+1)⁺.
Anal. calcd for C₂₃H₂₄N₄O₆S: C, 57.01; H, 4.99; N, 11.56, Found: C, 56.86; H, 5.21; N, 11.28.

### Example 62

### N-[1-[[(4'-cyano[1,1'-biphenyl]-4-yl)oxy]methyl]-2-(3-methyl-2,5-dioxo-1-imidazolidinyl)ethyl]-N-hydroxyformamide

The title compound was prepared as in Example 49, except using 1-methylhydantoin in place of succinimide.
Mixture of two rotamers: ¹H NMR (300 MHZ, d₆-DMSO) d 9.97 (s, 1H), 9.61 (s, 1H), 8.35 (s, 1H), 7.98 (s, 1H), 7.90-7.83 (m, 8H), 7.73 (d, 4H), 7.03 (d, 2H), 7.01 (d, 2H), 4.88-4.84 (m, 1H), 4.39-4.35 (m, 1H), 4.22-4.08 (m, 4H), 3.97 (s, 2H), 3.94 (s, 2H), 3.75-3.57 (m, 4H), 2.86 (s, 3H), 2.85 (s, 3H);
MS (ESI) m/e 409 (M+1)⁺
Anal. calcd for C₂₁H₂₀N₄O₅: C, 61.76; H, 4.94; N, 13.72. Found: C, 61.47; H. 5.00; N, 13.39.

### Example 63

### N-[1-[[(4'-cyano[1,1'-biphenyl]-4-yl)sulfonyl]methyl]-2-(4,4-dimethy-2,5-dioxo-1-imidazolidinyl)ethyl]-N-hydroxyformamide

The title compound was prepared following the procedure from Example 61 but using 1-bromo-3-(4,4-dimethyl-2,5-dioxo-1-imidazolidin-1-yl)-2-propanone in place of 1-bromo-3-(3,4,4-trimethyl-2,5-dioxo-1-imidazolidin-1-yl)-2-propanone in example 61A. Mixture of two rotamers: ¹H NMR (300 MHZ, d₆-DMSO) d 9.66 (s, 1H0, 9.51 (s, 1H), 8.38 (s, 1H), 8.34 (s, 1H), 8.10 (s, 1H), 8.07-7.96 (s, 16H), 7,74 (s, 1H), 4.94-4.86 (m, 1H), 4.58-4.50 (m, 1H), 3.80-3.37 (m. 8H), 1.23-1.20 (m, 12H);
MS (ESI) m/e 488 (M+18)⁺.
Anal. calcd for C₂₂H₂₂N₄O₆S: C. 56.16; H, 4.71; N, 11.91. Found: C, 56.12; H, 5.00; N, 11.59.

### Example 64

### ()-N-[1-[[(4'-chloro-[1,1'-biphenyl]-4-yl)oxy]methyl]-2-(3,4,4-trimethyl-2,5-dioxo-1-imidazolidinyl)ethyl]-N-hydroxyformamide

### Example 64A

### 1-[4"-chloro-1',1"-biphenyl]-4'-yl)oxy]-3-(3,4,4-trimethyl-2,5-dioxoimidazolidin-1yl)-propan-2-one

A solution of Example 16B (0.81 g, 2.93 mmol), 4-chloro-4'-hydroxybiphenyl (0.50 g, 2.44 mmol), and potassium carbonate (0.35 g, 2.57 mmol) in dry DMF (50 mL) was stirred at ambient temperature for 1.5 hour and partitioned between ethyl acetate and water. The aqueous layer was drawn off and extracted with ethyl acetate (1x). The combined organic extracts were diluted with an equal volume of hexanes and washed sequentially with water (3x) and brine (2x), dried (Na₂SO₄) and concentrated to provide 0.89 g of a waxy clumpy solid which was purified by purified on silica gel with 40% ethyl acetate/dichloromethane to provide 0.46 g (47%) of the title compound as a colorless solid. mp 165-166°C;
MS (DCI/NH₃) m/e 379 (M+NH₄)⁺.

### Example 64B

### ()-N-[1-[[(4'-chloro-[1,1'-biphenyl]-4-yl)oxy]methyl]-2-(3,4,4-trimethyl-2,5-dioxo-1-imidazolidinyl)ethyl]-N-hydroxyformamide

The ketone from Example 64B was sequentially converted to the corresponding oximes, hydroxylamine, and the final compound as described in Examples 2D, 2E, and 2F The title compound was purified on silica gel with 2.5% methanol/dichlormethane to provide the title compound as a colorless solid which was recrystallized from ethyl acetate/hexanes.
mp 124-125 ∞C;
MS (DCI/NH₃) m/e 446 (M+H)⁺ and 463 (M+NH₄)⁺.
¹H NMR (300 MHz, CDCl₃) d 9.90 (s; 0.5H), 9.58 (s; 0.5H), 8.32 (s; 0.5H), 7.92 (s; 0.5H), 7.65 (d; 2H; J=9 Hz), 7.61 (d; 2H; J=9 Hz), 7,47 (d; 2H; J=9 Hz), 6.99 (d; 1H; J=9 Hz), 6.97 (d; 1H; J=9 Hz), 4.86 (m; 0.5H), 4.42 (m; 0.5H), 4.08-4.23 (m; 2H), 3.82-3.70 (m; 1H), 3.55-3.65 (m; 1H), 2.80 (s; 1.5H), 2.78 (s; 1.5H), 1.30 (s; 3H), 1.28 (s; 3H);
Anal. calcd for C₂₂H₂₄N₃O₅Cl: C, 59.26; H. 5.42; N. 9.42. Found: C, 59.54; H, 5.61; N, 9.13.

### Example 65

### (+)-N-[1-[[(3'-cyanomethyl-[1,1'-biphenyl]-4-y)oxy]methyl]-2-(3,5,5-trimethyl-2,4-dioxo-1-imidazolidinyl)propyl]-N-hydroxyformamide

### Example 65A

### 1-(3-[(3'-cyanomethyl-[1,1'-biphenyl]-4-yl)oxy]-propan-2-on-1-yl)-3,4,4-trimethyl-2,5-dioxoimidazolidine

The title compound was prepared as in Example 5F but using 4'-hydroxy-3-biphenylcarbonitrilemethane (0.95 g, 2.80 mmol) in place of 4'-hydroxy-4-biphenylcarbonitrile. Purification on silica gel with 100% ethyl acetate provided 0.78 g of title compound.
MS (DCI/NH₃) m/e 437 (M+NH₄)⁺.

### Example 65B

### (+)-N-[1-[[(3'-cyanomethyl-[1,1'-biphenyl]-4-y)oxy]methyl]-2-(3,4,4-trimethyl-2,5-dioxo-1-imidazolidinyl)propyl]-N-hydroxyformamide

Example 65A (0.78 g, 1.87 mmol) was processed sequentially according to the precedures in Example 2D, 2E, and 2F without purification of the intermediates.
Purification on silica gel with 100% ethyl acetate provided 500 mg (1.08 mmol) of the title compound.
¹H NMR (300 MHz, DMSO) 9.99 (s: 0.5H), 9.58 (s: 0.5H), 8.36 (s: 0.5H), 7.92 (s:0.5H), d 7.60 (m; 4H), 7.46 (t; 1H J=8 Hz), 7.30 (d; 1H; J=8 Hz), 7.02 (d; 2H; J=8 Hz), 4.50 (m; 0.5H), 4.18 (m; 0.5H), 4.12 (s; 2H), 4.10 (m; 2H), 3.45 (m; 2H), 2.80 (s; 3H), 1.92 (m; 1H), 1.80 (m; 1H), 1.30 (s: 6H).
MS (DCI/NH₃) m/e 482 (M+NH₄)⁺.
Anal. calcd for C₂₅H₂₈N₄O₅: C, 64.66; H, 6.03; N, 12.07, Found

### Example 66

### (+)-N-[1-[[(4'-cyano-[1,1'-biphenyl]-4-y)oxy]methyl]-2-isopropylthioethyl]-N-hydroxyformamide

### Example 66A

### (+)-1-[(4'-cyano-[1,1'-biphenyl]-4-y)oxy]-3-isopropylthio-2-propanol

A solution of isopropylthiol (0.48 g, 6.4 mmol) in THF (20mL) was treated with K2CO3 (0.5 g, 3.6 mmol ). After 30 minutes, 3-[(4'-cyano-[1,1'-biphenyl]-4-y)oxy] -(1,2) oxirane (0.8 g, 3.19 mmol ) was added in a single portion. The resulting solution was stirred at 70 oC for 3 hours, quenched by adding excess aqueous sodium bicarbonate solution and partitioned between ethyl acetate and brine. The organic layer was dried (Na₂SO₄), the product was purified on silica gel with 50% ethyl acetate/hexanes to provide 0.9 g (2.75 mmol, 86% ) of the title compound.
MS (DCI/NH3) m/e 345 (M+NH₄)⁺ and 362 (M+NH₄+NH₃)⁺.

### Example 66B

### (+)-N-[1-[[(4'-cyano-[1,1'-biphenyl]-4-y)oxy]methyl]-2-isopropylthioethyl]-N-hydroxyformamide

Example 66A was processed according to the procedures in Example 2C,2D, 2E, and 2F providing the title compound as a light orange foam.
¹H NMR (300 MHz, DMSO) 9.99 (s: 0.5H), 9.60 (s: 0.5H), 8.42 (s: 0.5H), 8.04 (s:0.5H), d 7.85 (m; 4H), 7.75 (d; 2H J=9 Hz). 7.05 (d;2H; J=9 Hz), 4.63 (m; 1H), 4.17 (m; 3H), 3.0 (m; 1H), 2.79 (m; 1H), 1.22 (dd; 6H; J=7.50 Hz);
MS (DCI/NH₃) m/e 388 (M+NH₄)⁺.
Anal. calcd for C₂₀H₂₂N₂O₃S: C, 64.86; H, 5.94; N, 7.57. Found:

### Example 67

### (+)-N-[1-[[(3'-cyanomethyl-[1,1'-biphenyl]-4-y)oxy]methyl]-2-(3,4,4-trimethyl-2,5-dioxo-1-imidazolidinyl)ethyl]-N-hydroxyformamide

### Example 67A

### (+)-1-[4-(3'-cyanoemethylphenyl)phenoxy]-3-(3,4,4-trimethyl-2,5-dioxo-1-imidazolidinyl)-2-propanol

The title compound was prepared as in Example 4A but using 4'-hydroxy-3-biphenylcarbonitrilemethane (170 mg, 0.64 mmol) and 3,5,5-trimethylhydantoin (37 mg, 0.96 mmol) in place of 4'-hydroxy-4-biphenylcarbonitrile and 5,5-dimethylhydantoin Purification on silica gel with 100% ethyl acetate provided 130 g of title compound.
MS (DCI/NH₃) m/e 415 (M+NH₄)⁺.

### Example 67B

### (+)-N-[1-[[(3'-cyanomethyl-[1,1'-biphenyl]-4-y)oxy]methyl]-2-(3,4,4-trimethyl-2,5-dioxo-1-imidazolidinyl)ethyl]-N-hydroxyformamide

Example 67A was processed according to the procedures in Example 2C,2D, 2E, and 2F providing the title compound.
¹H NMR (300 MHz, DMSO) 9.86 (s: 0.5H), 9.58 (s: 0.5H), 8.34 (s: 0.5H), 7.92 (s:0.5H), d 7.60 (m; 4H), 7.46 (t; 1H J=8 Hz), 7.30 (d; 1H; J=8 Hz), 7.02 (d; 2H; J=8 Hz), 4.85 (m; 0.5H), 4.42 (m; 0.5H), 4.10 (m; 2H), 4.12 (s; 2H), 3.68 (m; 1H), 3.62 (m; 1H), 2.80 (s; 3H), 1.30 (s: 6H).
MS (DCI/NH₃) m/e 468 (M+NH₄)⁺.
Anal. calcd for C₂₄H₂₆N₄O₅: C, 62.85; H, 5.84; N, 11.85. Found

### Example 68

### (+)-N-[1-[[(4'-cyano-[1,1'-biphenyl]-4-yl)oxy]methyl]-2-(3-ethyl-4,4-dimethyl-2,5-dioxo-1-imidazolidinyl)ethyl]-N-hydroxyformamide

### Example 68A

### 4,4-dimethyl-2,5-dioxo-N-(4-methoxoybenzyl)imidazolidine

A solution of 4,4-dimethyl-2,5-dioxoimidazolidine (17.0 g, 133 mmol), 4-methoxybenzyl chloride (30.0 g, 192 mmol), and potassium carbonate (27.5 g, 200 mmol) in dry DMF (600 mL) was heated at 80 ∞C under nitrogen for 3h. The volume was reduced under high vacuum to about 1/4 of the original volume and the resulting solution was partitioned between ethyl acetate and water. The aqueous layer was drawn off and extracted with ethyl acetate (2x). The combined organic extracts were washed sequentially with water (2x) and brine (2x). dried (Na₂SO₄) and concentrated under vacuum to provide 32.97 g of a solid. Pure title compound was obtained by recrystallization from ethyl acetate and hexanes to provide 24.5 g (74%) of a colorless solid.
mp 109-111 °C;
MS (DCI/NH₃) m/e 249 (M+H)⁺ and 266 (M+NH₄)⁺.

### Example 68B

### 3-Ethyl-1-(4'-methoxybenzyl)-2,5-dioxo-4,4-dimethylimidazolidine

A solution of 4,4-dimethyl-2,5-dioxo-N-(4-methoxoybenzyl)imidazolidine (3.5 g, 14.1 mmol) in THF (100 ml) was treated with sodium hydride (0.5 g, 21.2 mmol), stirred for 10 minute, treated with iodoethane (3.3 g, 21.2 mmol), stirred at 50 oC for 3 hours. then treated with HCl solution (10%) and partitioned between ethyl acetate and brine. The organic layer was dried and concentrated to provide 3.8g (13,8 mmol, 98%) of title compound as white solid.
MS (DCI/NH₃) m/e 294 (M+NH₄).

### Example 68C

### 3-Ethyl-2,5-dioxo-4,4-dimethylimidazolidine

A solution of 3-ethyl-1-(4'-methoxybenzyl)-2,5-dioxo-4,4-dimethylimidazolidine (3.86 g, 14.0 mmol) in methoxybenzene (100 ml) was treated with alumiun trichloride (5.5 g, 42 mmol), stirred at 75oC for 30 minute, then poured the reaction into HCl solution (10%) and partitioned between ethyl acetate and brine. The organic layer was dried and concentrated. Purification by recrystalization with ethyl acetate provided 2.1 g (13.5 mmol, 96%) of title compound as white solid.
MS (DCI/NH₃) m/e 174 (M+NH₄)⁺.

### Example 68D

### 1-[(4'-cyano-[1,1'-biphenyl]-4-y)oxy]-3-(3-ethyl-5,5-dimethyl-2,4-dioxo-1-imidazolidinyl)-2-propanone

A solution of 3-ethyl-2,5-dioxo-4,4-dimethylimidazolidine (0.7 g, 4,5 mmol) in DMF (100 ml) was treated with potassium carbonate (0.6 g, 4.5 mmol) and 1-[(4'-cyano-[1,1'-biphenyl]-4-y)oxy]-3-bromo-2-propynone (1.0 g, 3.0 mmol), stirred at 25°C for 20 hours, then the reaction was poured into aqueous HCl solution (10%) and partitioned between ethyl acetate and brine. The organic layer was dried and concentrated. Purification on silica gel with 50% ethyl acetate provided 0.8 g (1.97 mmol, 66%) of title compound as white solid.
MS (DCI/NH₃) m/e 424 (M+NH₄)⁺.

### Example 68E

### (+)-N-[1-[[(4'-cyano-[1,1'-biphenyl]-4-yl)oxy]methyl]-2-(3-ethyl-4,4-dimethyl-2,5-dioxo-1-imidazolidinyl)ethyl]-N-hydroxyformamide

Example 68B (0.72 g, 1.77 mmol) was processed sequentially according to the precedures in Example 2D, 2E, and 2F without purification of the intermediates.
Purification on silica gel with 60% ethyl acetate/hexanes provided 158 mg (0.35 mmol) of the title compound.
¹H NMR (300 MHz, DMSO) 9.85 (s: 0.5H), 9.54 (s: 0.5H), 8.32 (s: 0.5H), 7.94 (s;0.5H), d 7.86 (m; 4H), 7.72 (d; 2H J=9 Hz), 7.08 (d;2H; J=9 Hz), 4.85 (m; 0.5H), 4.42 (m; 0.5H), 4.18 (m; 2H), 3.78 (m; 1H), 3.62 (m; 1H), 1.32 (s; 6H), 1.12 (m; 3H), MS (DCI/NH₃) m/e 468 (M+NH₄)⁺.
Anal. calcd for C₂₄H₂₆N₄O₅: C, 64.00; H, 5.78; N, 12.44. Found

### Example 69

### (+)-N-[1-[[(4'-cyano-[1,1'-biphenyl]-4-yl)oxy]methyl]-2-(3-benzyl-4,4-dimethyl-2,5-dioxo-1-imidazolidinyl)ethyl]-N-hydroxyformamide

### Example 69A

### 3-benzyl-1-(4'-methoxybenzyl)-2,5-dioxo-4,4-dimethylimidazolidine

The title compound was prepared as in Example 68B but using benzyl iodine (3.9 g, 18 mmol) in place of iodoethane. Purification on silica gel with 50% ethyl acetate provided 4.0 g of title compound.
MS (DCI/NH₃) m/e 356 (M+NH₄)⁺.

### Example 69B

### 3-benzyl-2,5-dioxo-4,4-dimethylimidazolidine

A solution of 3-benzyl-1-(4'-methoxybenzyl)-2,5-dioxo-4,4-dimethylimidazolidine (3.9 g, 11.54 mmol) in acetonitrile (100 ml) was treated with a solution of ammonium cerium nitrate (31 g, 57.7 mmol) in 65 ml of water, stirred at 25 oC for 15 minute, then diluted the reaction with ethyl acetate and partitioned between ethyl acetate and brine. The organic layer was dried and concentrated. Purification by recrystalization with ethyl acetate/hexane provided 1.58 g (7.25 mmol, 63%) of title compound as white solid.
MS (DCI/NH₃) m/e 236 (M+NH₄)⁺.

### Example 69C

### 1-[(4'-cyano-[1,1'-biphenyl]-4-y)oxy]-3-(3-ethyl-4,4-dimethyl-2,5-dioxo-1-imidazolidinyl)-2-propanone

The title compound was prepared as in Example 68D but using 3-benzyl-2,5-dioxo-4,4-dimethylimidazolidine (0.5 g, 2.28 mmol) in place of using 3-ethyl-2,5-dioxo-4,4-dimethylimidazolidine. Purification on silica gel with 30% ethyl acetate/cloroform provided 634 mg of title compound.
MS (DCI/NH₃) m/e 485 (M+NH4)⁺.

### Example 69D

### (+)-N-[1-[[(4'-cyano-[1,1'-biphenyl]-4-yl)oxy]methyl]-2-(3-ethyl-4,4-dimethyl-2,5-dioxo-1-imidazolidinyl)ethyl]-N-hydroxyformamide

Example 69C (0.62 g, 1.33 mmol) was processed sequentially according to the precedures in Example 2D, 2E, and 2F without purification of the intermediates.
Purification on silica gel with 70% ethyl acetate/hexanes provided 230 mg (0.45 mmol) of the title compound.
¹H NMR (300 MHz, DMSO) 9.95 (s; 0.5H). 9.64 (s: 0.5H), 8.35 (s; 0.5H), 7.94 (s; 0.5H), d 7.86 (m; 4H), 7.75 (d; 2H J=9 Hz), 7.30 (d;2H; J=9 Hz), 7.25 (m; 3H), 7.06 (m; 2H), 4.90 (m; 0.5H), 4.52 (s; 2H), 4.50 (m; 0.5H), 4.18 (m; 2H), 3.82 (m; 1H), 3.62 (m; 1H), 1.22 (s: 6H).
MS (DCI/NH₃) m/e 530 (M+NH₄)⁺.

### Example 70

### (+)-N-[1-[[(4'-cyano-[1,1'-biphenyl]-4-y)oxy]methyl]-2,4-dioxo-1-imidazolidinyl)ethyl]-N-hydroxyformamide

### Example 70A

### 1-[(4'-cyano-[1,1'-biphenyl]-4-y)oxy]-3-(3,5,5-trimethyl-2,4-dioxo-1-imidazolidinyl)-2-propanol

A solution of 5,5-dimethylhydantoin (2.0 g, 15.6 mmol) in DMF (20 ml) was treated with sodium tert-butoxide (1.5 g, 15.6 mmol), stirred for 10 minute, treated with iodomethane (2.2 g, 15.6 mmol), stirred at 40 oC for 3 hours. The resulting solution was treated with sodium tert-butoxide (1.5 g, 15.6 mmol) followed by 3-[(4'-cyano-[1,1'-biphenyl]-4-y)oxy]-(1,2) oxirane (1.15 g, 4.58 mmol), stirred at 100 oC for 20 minute, treated with HCl solution (10%) and partitioned between ethyl acetate and brine. The organic layer was dried and concentrated to provide 1.35g ( 75%) of title compound as white solid.
MS (DCI/NH₃) m/e 411 (M+NH₄).

### Example 70B

### 1-[(4'-cyano-[1,1'-biphenyl]-4-y)oxy]-3-(3,5,5-trimethyl-2,4-dioxo-1-imidazolidinyl)-2-propanone

Example 70A (1.35 g g, 3.4 mmol) was processed according to the procedures in Example 2C. Purification on silica gel with 30% ethyl acetate provided 1.2 g (3.1 mmol, 90%) of the title compound.
MS (DCI/NH₃) m/e 409 (M+NH₄)⁺.

### Example 70C

### (+)-N-[1-[[(4'-cyano-[1,1'-biphenyl]-4-y)oxy]methyl]-2-(3,5,5-trimethyl-2,4-dioxo-1-imidazolidinyl)ethyl]-N-hydroxyformamide

Example A-264890.0-B (1.2 g, 3.07 mmol) was processed sequentially according to the precedures in Example 2D, 2E, and 2F without purification of the intermediates.
Purification on silica gel with 30% ethyl acetate/hexanes provided 380 mg (2.29 mmol) of the title compound.
¹H NMR (300 MHz, DMSO) 9.90 (s: 0.5H), 9.68 (s: 0.5H), 8.38 (s: 0.5H), 7.98 (s:0.5H), d 7.88 (m; 4H), 7.72 (d; 2H J=9 Hz), 7.08 (d;2H: J=9 Hz), 4.92 (m; 0.5H), 4.42 (m; 0.5H), 4.20 (m; 2H), 3.50 (m; 2H), 2.88 (s; 3H), 1.32 (s; 6H).
MS (DCI/NH₃) m/e 454 (M+NH₄)⁺.
Anal. calcd for C₂₃H₂₄N₄O₅: C, 63.30; H, 5.55; N, 12.69. Found

### Example 71

### N-[1-[[(4'-methoxy[1,1'-biphenyl]-4-yl)sulfonyl]methyl]ethyl]-N-hydroxyformamide

### Example 71A

### 4'-methoxy-4-thiomethyl biphenyl

A solution of 4-bromothioanisole (6.15 g, 29.4 mmol) in DMF (60 mL) was treated sequentially with palladium (II) acetate (0.34 g, 1.5 mmol) and tri-o-tolylphosphine (0.94 g, 3.0 mmol) then 4-methoxyphenylboronic acid (5.06 g, 32.3 mmol) and cesium carbonate (19.2 g, 58.8 mmol). The mixture was stirred at 75°C for 8 hours, then RT for 15 hours. The resulting suspension was partitioned between water and ether/hexane, 2:1. The organic layer was dried with Mg2SO4 and concentrated to provide crude product as a yellow solid. Recrystallization in ether at -20°C afforded 2.61 g (39%) of the title compound.
MS (ESI+) m/e 231 (M+H).

### Example 71B

### 4-(4'-methoxyphenyl)-phenyl methyl sulfone

A solution of Example 71A (2.61g, 11.3 mmol) in chloroform (100 mL) was treated with m-chloroperbenzoic acid (6.52 g, 22.7 mmol), stirred at 0°C for 3 hours and then warmed to 10°C over 1 hour. The mixture was partitioned between dilute sodium bicarbonate aqueous solution and chloroform, dried (Mg2SO4), and concentrated to provide crude product as a white solid. Recrystallized in dichloromethane and ether to afford 1.89 g (64%) of the title compound.
MS (ESI+) m/e 263 (M+H) and 280 (M+NH4).

### Example 71C

### N-[1-[[(4'-methoxy[1,1'-biphenyl]-4-yl)sulfonyl]methyl]ethyl]-N-benzyloxy amine

A suspension of Example 71B (0.26 g, 1.0 mmol) in THF (40 mL) cooled to -78°C under argon atmosphere was treated with nBuLi (0.40 mL of a 2.5 M solution in hexane, 1.0 mmol) and stirred for 3 hours. The resulting suspension was treated with BF3•Et2O (0.127 mL, 1.0 mmol) then the O-Benzyloxime of acetaldehyde(0.15 g, 1.0 mmol) J. Med. Chem. 1997, vol.40, number 13, pages 1955-1968 (Stewart. et. al.). [J. Med. Chem. 1997, vol.40, 1955-1968 (Stewart, et. al.).] in THF (10 mL). Stirred 1 hour at -78°C, then 1 hour at RT. Partitioned mixture between ether and pH 7 phosphate buffer. The organic extracts were washed with brine , dried (Mg2SO4), and concentrated to afford crude product as a white powder which was purified on silica gel with dichloromethane/methanol to provide 0.15 g (36%) of the title compound.
MS (ESI+) m/e 412 (M+H).

### Example 71D

### N-[1-[[(4'-methoxy[1,1'-biphenyl]-4-yl)sulfonyl]methyl]ethyl]-N-nenzyloxyformamide

A solution of Example 71c (0.11 g, 0.27 mmol) in THF (50 mL) cooled to 0°C under argon atmosphere was treated with formic acetic anhydride (0.24 g, 2.7 mmol), stirred for 5 minutes at 0°C then RT for 16 hours. Partitioned between 1 N Hcl and ethyl acetate. Washed organic extracts with brine, dried (Mg2SO4), and concentrated to provide crude oil product which was purified on silica gel with dichloromethane/methanol to afford 114 mg (96%) of the title compound.
MS (ESI+) m/e 440 (M+H) and 457 (M+NH4).

### Example 71E

### N-[1-[[(4'-methoxy[1,1'-biphenyl]-4-yl)sulfonyl]methyl]ethyl]-N-hydroxyformamide

A solution of Example 71D (114 mg, 0.26 mmol) in THF (20 mL) was treated with 10% palladium on carbon (35 mg, catalytic amount) and hydrogen gas at atmospheric pressure and stirred at RT for 18 hours. Filtered the suspension through Celite pad and concentrated to provide crude product as a white solid which was purified by trituration in ethyl acetate to afford 66 mg (73%) of the title compound.
mp 197-198°C;
1H NMR (DMSO-d6) ∂ 1.16 (d, 1.5H, J=6.6 Hz), 1.22 (d, 1.5H, J=6.6 Hz), 3.43-3.70 (m, 2H), 3.82 (s, 3H), 4.28-4.41 (m, 0.5H), 4.62-4.76 (m, 0.5H), 7.08 (d, 2H, J=8.7 Hz), 7.74 (d, 2H, J=8.7 Hz), 7.87-7.96 (m, 4.5H), 8.08 (s, 0.5H), 9.47 (s, 0.5H), 9.89 (s, 0.5H);
MS (ESI+) m/e 350 (M+H), 367 (M+NH4);
Anal. Calcd for: C17H19NO5S•H2O C, 55.57; 11.576, N, 3.81. Found: C, 55.32; H, 5.20; N, 3.67.

### Example 72

### N-[1-[[(4'-chloro[1,1'-biphenyl]-4-yl)sulfonyl]methyl]ethyl]-N-hydroxyformamide

The title compound was synthesized according to the procedures described in Example 71 except using 4-chlorophenylboronic acid in place of 4-methoxyphenylboronic acid in Example 71A. Purification of the crude final product by recrystallization in ethyl acetate afforded 36 mg of title compound.
mp 178-180°C;
1H NMR (DMSO-d6) ∂ 1.16 (d, 1.5H, J=6.6 Hz), 1.22 (d, 1.5H, J=6.6 Hz), 3.50 (dd, 1H, J=4.8,14.7 Hz), 3.57-3.73 (m, 1H), 4.28-4.41 (m, 0.5H). 4.61-4.77 (m, 0.5H), 7.59 (d, 2H, J=8.4 Hz), 7.81 (d, 2H, J=8.4 Hz), 7.91 (s, 0.5H), 7.92-8.00 (m, 4H). 8.07 (s, 0.5H), 9.45 (s, 0.5H), 9.86 (s, 0.5H);
MS (ESI+) m/e 354 (M+H), 376 (M+Na);
Anal. Calcd for: C16H16NO4SCl C, 54.31; H, 4.55; N, 3.95. Found: C, 54.46; H, 4.43; N, 3.85.

### Example 73

### N-[1-[[[4-(1,3-benzodioxol-5-yl)phenyl]sulfonyl]methyl]ethyl]-N-hydroxyformamide

The title compound was synthesized according to the procedures described in Example 71 except using 3.4-methylenedioxybenzeneboronic acid in place of 4-methoxyphenylboronic acid in Example 71A. mp 200-201°C;
1H NMR (DMSO-d6) ∂ 1.16 (d, 1.5H, J=6.6 Hz), 1.22 (d, 1.5H, J=6.6 Hz), 3.44-3.70 (m, 2H), 4.28-4.40 (m, 0.5H), 4.61-4.76 (m, 0.5H), 6.11 (s, 2H), 7.06 (d, 1H, J=7.8 Hz), 7.29 (d, 1H, J=8.4 Hz), 7.39 (s, 1H), 7.86-7.94 (m, 4.5H), 8.08 (s. 0.5H), 9.48 (s, 0.5H), 9.90 (s, 0.5H);
MS (ESI+) 364 (M+H), 381 (M+NH4);
Anal. Calcd for: C17H17NO6S C. 56.19; H, 4.71; N, 3.85. Found: C, 55.97; H, 4.62; N, 3.81.

### Example 74

### N-[1-[[[4-(4-chlorophenoxy)phenyl]sulfonyl]methyl]ethyl]-N-hydroxyformamide

### Example 74A

### 4-chlorophenoxyphenyl methyl sulfone

A solution of 4-chlorophenol (5.54 g, 43 mmol) in DMSO (75 mL) was treated sequentially with potassium t-butoxide (5.15 g, 46 mmol) then with a solution of 4-fluorophenyl methyl sulfone (5.00 g, 29 mmol) in DMSO (25 mL), heated at 120°C for 2 hours, cooled to RT, then partitioned between dichloromethane and 1 N sodium hydroxide, dried (Mg2SO4), and concentrated to give crude product as a white solid. Recrystallization from ethyl acetate and hexane afforded 5.44 g (66%) of the title compound.
MS (ESI+) m/e 300 (M+NH4).

### Example 74B

### N-[1-[[[4-(4-chlorophenoxy)phenyl]sulfonyl]methyl]ethyl]-N-hydroxyformamide

The title compound was prepared from Example 74A according to the procedures described in Example 71C-71E. Purification of the crude final compound by recrystallization in ethyl acetate afforded 388 mg of the title compound.
mp 144-145°C;
1H NMR (DMSO-d6) ∂ 1.15 (d, 1.5H, J=6.6 Hz), 1.21 (d, 1.5H, J=6.6 Hz), 3.39-3.68 (m, 2H), 4.25-4.37 (m, 0.5H), 4.60-4.70 (m, 0.5H), 7.16-7.23 (m, 4H), 7.53 (d, 2H, J=8.9 Hz), 7.83-7.92 (m, 2.5H), 8.06 (s, 0.5H), 9.45 (s, 0.5H), 9.87 (s, 0.5H);
MS (ESI+) 370 (M+H), 387 (M+NH4);
Anal. Calcd for: C16H16NO5SCl C, 51.96; H, 4.36; N, 3.78. Found: C, 52.22; H, 4.37; N, 3.80.

### Example 75

### N-[1-[[(4'-methoxy[1,1'-biphenyl]-4-yl)sulfonyl]methyl]propyl]-N-hydroxyformamide

### Example 75A

### 1-(4'-methoxy[1,1'-biphenyl]-4-yl)sulfonyl]-2-butanol

A solution of Example 71B (0.70 g, 2.67 mmol) in THF (200 mL) cooled to -78°C under argon was treated with nBuLi (1.17 mL of 2.5 M solution in hexane, 2.93 mmol), stirred 4 hours at -78°C, then treated with propionaldehyde (0.40 mL, 5.34 mmol) dropwise. Allowed reaction mixture to warm to RT over 1.5 hour, quenched with saturated aqueous NH4Cl solution (50 mL), partitioned between ether and water, dried (Mg2SO4). and concentrated to afford 0.90 g of crude product which was purified on silica gel with dichloromethane/methanol to provide 0.78 g (91%) of the title compound.
MS (ESI+) m/e 321 (M+H), 338 (M+NH4).

### Example 75B

### 1-(4'-methoxy[1,1'-biphenyl]-4-yl)sulfonyl]-1-butene

A solution of Example 75A (0.45 g, 1.40 mmol) in dichloromethane (40 mL) cooled to 0°C was treated sequentially with triethylamine (0.29 mL, 2.11 mmol) and methanesulfonyl chloride (0.12 mL, 1.55 mmol) dropwise. Stirred at RT for 3 hours then treated with 1,8-diazabicyclo[5.4.0]undec-7-ene (0.21 mL, 1.40 mmol), refluxed for 2 hours, cooled to RT, and partitioned between dilute sodium bicarbonate solution and dichloromethane. The organic extract was washed with 1 N Hcl, then brine, dried (Mg2SO4), and concentrated to afford white solid crude product. Recrystallization in ether at -20°C provided 0.34 g (80%) of title compound.
MS (ESI+) m/e 303 (M+H), 320 (M+NH4).

### Example 75C

### N-[1-[[(4'-methoxy[1,1'-biphenyl]-4-yl)sulfonyl]methyl]propyl]-N-hydroxy amine

A solution of Example 75B (0.34 g, 1.12 mmol) in THF (40 mL) was treated with hydroxylamine hydrochloride (0.39 g, 5.62 mmol) and potassium carbonate (0.78 g, 5.62 mmol), refluxed for 5 hours, cooled to RT, partitioned between ether and water, dried (Mg2SO4), and concentrated to give crude product as a clear, colorless oil. Recrystallization from ethyl acetate and ether provided 0.25 g (67%) of the title compound.
MS (ESI+) m/e 336 (M+H).

### Example 75D

### N-[1-[[(4'-methoxy[1,1'-biphenyl]-4-yl)sulfonyl]methyl]propyl]-N-hydroxyformamide

A solution of Example 75C (0.24 g, 0.72 mmol) in THF (30mL) cooled to 0°C was treated with formic acetic anhydride (64 mg, 0.72 mmol), stirred for 2 hours, partitioned between water and dichloromethane, dried (Mg2SO4), and concentrated to afford 0.25 g of crude product which was recrystallized in ethyl acetate to provide 106 mg (41%) of the title compound.
mp 199-200°C;
1H NMR (DMSO-d6) ∂ 0.69-0.80 (m, 3H). 1.40-1.69 (m, 2H), 3.41-3.69 (m, 2H), 3.82 (s, 3H), 3.96-4.07 (m, 0.5H), 4.43-4.54 (m, 0.5H), 7.08 (d, 2H, J=9.0 Hz), 7.71-7.78 (m, 2H), 7.87-7.96 (m, 4.5H), 8.17 (s. 0.5H), 9.49 (s, 0.5H), 9.84 (s, 0.5H);
MS (ESI+) 364 (M+H), 381 (M+NH4);
Anal. Calcd for: C18H21NO5S C, 59.48; H, 5.82; N, 3.85. Found: C, 59.67; H, 5.77; N, 3.80.

### Example 76

### N-[1-[1,1-dimethyl-2-[(4'-(trifluoromethyl)[1,1'-biphenyl]-4-yl]sulfonyl]ethyl]-N-hydroxyformamide

### Example 76A

### 4-(4'-trifluoromethylphenyl)-phenyl methyl sulfone

The title compound was prepared according to the procedure given in Example 73A substituting 4-trifluoromethylphenylboronic acid for 3,4-methylenedioxybenzeneboronic acid. Purification by recrystallization in ethyl acetate and ether afforded 3.70 g (72%) of the title compound.
MS (ESI+) m/e 318 (M+NH4).

### Example 76B

### 1-(4'-trifluoromethyl[1,1'-biphenyl]-4-yl)sulfonyl]-2-methyl-2-propanol

The title compound was prepared according to the procedure described in Example 75A substituting Example 76A for Example 71B and substituting acetone for propionaldehyde. Purification of crude product by recrystallization in ethyl acetate, ether, and pentane provided 1.40 g (73%) of the title compound.
MS (ESI+) m/e 376 (M+NH4).

### Example 76C

### N-[1-[1,1-dimethyl-2-[(4'-(trifluoromethyl)[1,1'-biphenyl]-4-yl]sulfonyl]ethyl]-N-hydroxyformamide

The title compound was prepared from Example 76B according to the procedures described in Examples 75B, 75C and 75D. Purification of the final product by recrystallization in ethyl acetate and ether provided 17 mg of the title compound.
mp 167-169°C;
1H NMR (DMSO-d6) ∂ 1.52 (s, 6H), 3.71 (s, 2H), 7.83-8.03 (m, 8.5H), 8.17 (s, 0.5H), 9.43 (s, 0.5H), 10.0 (s, 0.5H);
MS (ESI+) 402 (M+H), 419 (M+NH4), 424 (M+Na);
Anal. Calcd for: C18H18NO4F3SC, 53.86; H, 4.52; N, 3.48. Found: C, 53.58; H, 4.48; N, 3.19.

### Example 77

### N-[1-[(phenylmethoxy)methyl]-2-[[4'-(trifluoromethyl)[1,1'-biphenyl]-4-yl]sulfonyl]ethyl]-N-hydroxyformamide

The title compound was synthesized according to the procedures described in Examples 75A-75D except substituting Example 76A for Example 71B and substituting benzyloxyacetaldehyde for propionaldehyde in Example 75A. Purification of the crude final product by recrystallization in ethyl acetate afforded 0.53 g of title compound.
mp 172°C;
1H NMR (DMSO-d6) ∂ 3.37-3.61 (m, 3H). 3.61-3.72 (m, 1H), 4.28-4.50 (m, 2.5H), 4.81-4.93 (m, 0.5H), 7.20-7.35 (m, 5H), 7.85-8.06 (m, 8.5H), 8.18 (s, 0.5H), 9.57 (s, 0.5H), 9.96 (s, 0.5H);
MS (ESI+) 494 (M+H), 511 (M+NH4);
Anal. Calcd for: C24H22NO5F3S C, 58.41; H, 4.49; N, 2.83, Found: C, 58.43; H, 4.54; N, 2.77.

### Example 78

### N-[1-(hydroxymethyl)-2-[[4'-(trifluoromethyl)[1,1'-biphenyl]-4-yl]sulfonyl]ethyl]-N-hydroxyformamide

A solution of Example 78 (35 mg, 0.07 mmol) in THE (3 mL) and methanol (5 mL) was treated with palladium on carbon. 10% (30 mg, 0.03 mmol) and hydrogen gas at atmospheric pressure, stirred at RT for 16 hours, filtered through Celite, and concentrated to afford crude product. Purification by recrystallizations in ethyl acetate, ether, and hexane provided 20 mg (70%) of the title compound.
mp 159-161°C;
1H NMR (DMSO-d6) ∂ 3.25-3.68 (m, 4H), 3.98-4.10 (m, 0.5H), 4.54-4.66 (m, 0.5H), 4.97-5.09 (m, 1H), 7.81-8.07 (m, 8.5H), 8.14 (s, 0.5H), 9.44 (s, 0.5H), 9.85 (s, 0.5H);
MS (ESI+) 404 (M+H), 421 (M+NH4), 426 (M+Na);
Anal. Calcd for: C17H16NO5F3S C, 50.61; H, 3.99; N, 3.47. Found: C, 50.57; H, 3.93; N, 3.37.

### Example 79

### N-[1-[(4,4-dimethy-2,5-dioxo-1-imidazolidinyl)methyl]-2-[[4'-(trifluoromethyl)[1,1'-biphenyl]-4-yl]thio]ethyl]-N-hydroxyformamide

### Example 79A

### 1-[(4'-trifluoromethyl[1,1'-biphenyl]-4-yl)thio-3-(4,4-dimethyl-2,5-dioxoimidazolidin-1-yl)-2-propanone

The mixture of example46A (698mg, 1.88 mmol), tetrakis (217mg, 0.19 mmol), 4-triflorophenylboronic acid (714 mg, 3.76 mmol) and NaOH (1M, 3.76 mL, 3.76 mmol) in DME (20 mL) was refluxed under Ar for 4 hour. The mixture was evaporated to a small volume, and partitioned between CH2Cl2/brine. The CH2Cl2 layer was collected, dried (Na2SO2), filtered and evaporated to dryness. Purification of the crude final product on silica gel with 20%-40% ethyl acetate/CH2Cl2 provided 0.820 g of the title compound. MS (DCI/NH₃) m/e 454 (M+NH₄)⁺. 437 (M+H)+.

### Example 79B

### N-[1-[(4,4-dimethy-2,5-dioxo-1-imidazolidinyl)methyl]-2-[[4'-trifluoromethyl)[1,1'-biphenyl]-4-yl]thio]ethyl]-N-hydroxyformamide

The title compound was obtained following the procedures in Examples 2D-F (inclusive) but substituting Example 79A (0.82 g. 1.88 mmol for Example 2C.
Purification of the crude final product on silica gel with 5% methanol/dichloromethane provided 434 mg of the title compound.
mp 172-174 °C;
¹H NMR (300 MHz, d₆-DMSO) d 9.75 and 9.52 (br s, 1H), 8.37 and 8.33 (s, 1H), 8.31 and 7.77 (s, 1H), 7.90 (d; 2H, J = 8.4 Hz), 7.81 (d, 2H: J = 8.4 Hz), 7.71 (m, 2H), 7.47 (m 2H), 4.60 and 4.09 (m, 1H), 3.52-3.77 (m, 2H), 3.08-3.46 (m, 2H), 1.28 and 1.25 and 1.23 (s, 6H).
MS (DCI/NH₃) m/e499 (M+NH₄)⁺, 482 (M+H)+.
Anal. calcd for C₂₂H₂₂F₃N₃O₄S • 0.5 CH₃OH: C, 54.31; H. 4.86; N, 8.44. Found: C, 54.43; H, 4.82; N, 8.08.

### Example 80

### N-[1-[(4,4-dimethy-2,5-dioxo-1-imidazolidinyl)methyl]-2-[[4'-(trifluoromethyl)[1,1'-biphenyl]-4-yl]sulfonyl]ethyl]-N-hydroxyformamide

Example 79 was converted to example 80 following the procedure described in example 46D.
mp 180-182 °C;
¹H NMR (300 MHz, d6-DMSO) d 9.66 and 9.51 (br s, 1H), 8.39 and 8.35 (s, 1H), 8.10 and 7.73 (s, 1H), 7.79-8.02 (m, 6H), 7.89 (d, 2H, J = 8.4 Hz), 4.91 and 4.55 (m, 1H), 3.45-3.80 (m, 4H), 1.24 and 1.23 and 1.21 (s, 6H).
MS (DCI/NH₃) m/e531 (M+NH₄)⁺, 514 (M+H)+.
Anal. calcd for C₂₂H₂₂F₃N₃O₆S • 0.75 H₂O: C, 50.14; H. 4.49; N, 7.97. Found: C, 50.27; H, 4.49; N, 7.97.

### Example 81

### N-[1-[(2,5-dioxo-1-imidazolidinyl)methyl]-2-[[4'-(trifluoromethoxy)[1,1'-biphenyl]-4-yl]oxy]ethyl]-N-hydroxyformamide

### Example 81A

### 1-bromo-3-(2,5-dioxoimidazolidin-1-yl)propan-2-one

The title compound was prepared following the procedures in examples 16A and 16 B, but substituting hydantoin for 1,5,5-trimethylhydantoin.

### Example 81B

### N-[1-[(2,5-dioxo-1-imidazolidinyl)methyl]-2-[[4'-(trifluoromethoxy)[1,1'-biphenyl]-4-yl]oxy]ethyl]-N-hydroxyformamide

The title compound was prepared following the sequence described in described in examples 16C and 16E, but substituting 81A for 16B and 4-(4'-trifluoromethoxy-phenyl)-phenol for 4-bromophenol.
¹H NMR (DMSO-d₆) d 9.92 (S, 0.5H), 9.60 (BS, 0.5H), 8.31 (S. 0.5H), 8.16 (S, 0.5H), 8.14 (S, 0.5H). 7.92 (S, 0.5H), 7.76-7.72 (M, 4H), 7.64-7.62 (D, 4H. J=8.4 Hz), 7.42-7.40 (D, 4H, J=8.6 Hz), 7.02-6.98 (M, 4H), 4.84-4.82 (M. 0.5H), 4.38-4.35 (M, 0.5H), 4.19-4.04 (MM, 4H).
Anal. Calcd for: C₂₀H₁₈N₃O₆F₃: C, 52.98; H, 4.00; N, 9.13, Found: C, 53.01; H, 4.03; N, 9.13.

### Example 82

### N-[1-[[[4'-(trifluoromethyl)[1,1'-biphenyl]-4-yl]sulfonyl]methyl]-2-(3,4,4-trimethyl-2,5-dioxo-1-imidazolidinyl)ethyl]-N-hydroxyformamide

The title compound was prepared following the procedures described in example 46A,46B,46C and 46D, except substituting example 16B for 23A in example 46A and 4-trifluoromethylbenzeneboronic acid for 4-butyloxybenzeneboronic acid in example 46B.
¹H NMR (d6-DMSO) 9.70 (s, 0.5H), 9.54 (s, 0.5H), 8.10 (s, 0.5H), 8.05-7.97 (m, 6H). 7.89 (d, 2H, J=7.8 Hz), 7.75 (s, 0.5H), 4.97-4.86 (m, 0.5H), 4.60-4.48 (m, 0.5H), 3.80-.344 (m, 4H), 2.75 (s, 3H), 1.24 (s, 3H), 1.22 (s, 3H), MS (ESI) 528 (M+H), 545 (M+NH4), 526 (M-H). Anal. Calcd for: C₂₃H₂₄N₃O₆SF₃ C, 52.36; H, 4.58; N, 7.96. Found: C, 52.05; H, 4.70; N, 7.63.

### Example 83

### N-[1-[[(4'-butyl[1,1'-biphenyl]-4-yl)oxy]methyl]-2-(3-methy-2,5-dioxo-1-imidazolidinyl)ethyl]-N-hydroxyformamide

The title compound was prepared following the procedures described in example 16C and 16E, except substituting example 26A for 16B and 4-(4'-butylphenyl)phenol for 4-bromophenol.
¹H NMR (d6-DMSO) 10.00-9.94 (br, 0.5H), 9.64-9.58 (br, 0.5H), 8.34 (s, 0.5H), 7.98 (s. 0.5H), 7.58 (d, 2H, J=8.8 Hz), 7.52 (d, 2H, J=8.6 Hz), 7.24 (d, 2H, J=8.5 Hz), 7.00-6.92 (m, 2H), 4.92-4.79 (m, 0.5H), 4.41-4.30 (m, 0.5H), 4.20-4.03 (m, 2H), 3.95 (d, 2H. J=7.8 Hz), 3.75-3.57 (m, 2H), 2.86 (s, 1.5H), 2.85 (s. 1.5H), 2.60 (t, 2H, J=7.4 Hz), 1.63-1.51 (m, 2H), 1.39-1.25 (m, 2H), 0.91 (t, 3H. J=7.4 Hz). MS (ESI) 440 (M+H), 457 (M+NH₄), 438 (M-H). Anal. Calcd for: C₂₄H₂₉N₃O₅·0.25 H₂O C, 64.92; H. 6.69; N. 9.46, Found: C, 64.76; H. 6.62; N, 9.29.

### Example 84

### N-[1-[(3-methy-2,5-dioxo-1-imidazolidinyl)methyl]-2-[[4'-(trifluoromethoxy)[1,1'-biphenyl]-4-yl]oxy]ethyl]-N-hydroxyformamide

The title compound was prepared following the procedures described in example 16C and 16E, except substituting example 26A for 16B and 4-(4'-trifluoromethoxy)phenol for 4-bromophenol.
¹H NMR (d6-DMSO) 10.02-9.92 (br, 0.5H), 9.64-9.58 (br, 0.5H), 8.35 (s, 0.5H), 7.98 (s, 0.5H), 7.74 (d, 2H, J=8.9 Hz), 7.64 (d. 2H. J=8.8 Hz), 7.41 (d, 2H, J=8.1 Hz), 7.03-6.97 (m, 2H), 4.91-4.82 (m, 0.5H), 4.41-4.31 (m, 0.5H), 4.21-4.07 (m, 2H), 3.96 (d, 2H, J=7.7 Hz), 3.72-3.57 (m, 2H), 2.86 (s, 1.5H), 2.85 (s, 1.5H). MS (ESI) 468 (M+H), 485 (M+NH₄), 466 (M-H). Anal. Calcd for: C₂₁H₂₀N₃O₆F₃ C, 53.96; H, 4.31; N, 8.99. Found: C, 53.85; H, 4.40; N, 8.85.

### Example 85

### N-[4-[4-[(4'-chloro[1,1'-biphenyl]-4-yl)sulfonyl]methyl]tetrahydro-2H-pyran-4-yl]-N-hydroxyformamide

### Example 85A

### N-[4-[4-[(4'-chloro[1,1'-biphenyl]-4-yl)sulfonyl]methyl]tetrahvdro-2H-pyran-4-yl]-N-benzyloxy amine

The title compound was prepared following the procedure described in example 54A, except using 4'-chloro-40methylsulfone-biphenyl in place of phenoxyphenyl-4-chloro-4'-methylsulfone.

### Example 85B

### N-[4-[4-[(4'-chloro[1,1'-biphenyl]-4-yl)sulfonyl]methyl]]tetrahydro-2H-pyran-4-yl]-N-hydroxy amine

A solution of 85A (0.436g, 0.92 mmol) was treated with (CF3CO2)3B (4.6 mL, 1M solution in THF, 4.6 mmol), then stirred overnight at room temperature. The solution was concentrated, partitioned between ethyl acetate and aq. Na2CO3 and the organic extrace was dried (MgSO4), concentrated thhen purified via column chromatography to give the title compound in 51% yield.

### Example 85C

### N-[4-[4-[(4'-chloro[1,1'-biphenyl]-4-yl)sulfonyl]methyl]tetrahydro-2H-pyran-4-yl]-N-hydroxyformamide

Example 85B was converted to the title compound using the formylation procedure of example 2F.
¹H NMR (d6-DMSO) 9.52-9.48 (br, 1H), 8.23 (s, 1H), 7.97 (s, 4H), 7.81 (d, 2H, J=8.4 Hz), 7.59 (d, 2H, J=8.5 Hz), 3.72 (s, 2H), 3.69-346 (m, 4H), 2.35-1.94 (m, 4H). MS (ESI) 410 (M+H), 427 (M+NH₄), 432 (M+Na), 408 (M-H).

### Example 86

### N-[1-[[[4-(4-chlorophenoxy)phenyl]sulfonyl]methyl]-2-(4,4-dimethyl-2,5-dioxo-1-imidazolidinyl)ethyl]-N-hydroxyformamide

The title compound was prepared following the procedures described in examples 46A, 46C and 46D, except using 4-(4'-chlorophenoxy)thiophenolinstead of 4-bromothiophenol.
¹H NMR (d6-DMSO) 9.67 (s, 0.5H), 9.50 (s, 0.5H), 8.36 (d, 1H, J=13.2 Hz), 8.10 (s, 0.5H), 7.90 (dd, 2H, J=8.8, 3.0 Hz), 7.68 (s, 0.5H), 7.53 (d, 2H, J=8.8 Hz), 7.20 (d, 4H. J=8.8 Hz), 4.89-4.77 (m, 0.5H), 4.52-4.40 (m, 0.5H), 3.68-3.38 (m, 4H), 1.25-1.21 (m, 6H). MS (ESI) 496 (M+H), 513 (M+NH4), 494 (M-H). Anal. Calcd for: C₂₁H₂₂N₃O₇SCl C, 50.85; H, 4.47; N, 8.47. Found: C, 50.53; H. 4.58; N, 8.25.

### Example 87

### N-[1-[[[4-(4-chlorophenoxy)phenyl]sulfonyl]methyl]-2-(3,4,4-trimethyl-2,5-dioxo-1-imidazolidinyl)ethyl]-N-hydroxyformamide

The title compound was prepared following the procedures described in examples 46A, 46C and 46D, except using 16B in place of 23A and 4-(4'-chlorophenoxy)thiophenol instead of 4-bromothiophenol.
¹H NMR (d6-DMSO) 9.78-9.71 (m. 0.5H), 9.58-9.49 (m, 0.5H), 8.09 (s, 0.5H), 7.89 (dd. 2H, J=5.8, 2.9 Hz), 7.68 (s, 0.5H), 7.53 (d, 2H, J=9.2 Hz), 7.20 (d, 4H, J=8.8 Hz), 4.88-4.78 (m. 0.5H), 4.50-4.38 (m, 0.5H), 3.72-3.40 (m, 4H), 2.76 (s, 1.5H), 2.76 (s, 1.5H), 1.26-1.22 (m, 6H). MS (ESI) 510 (M+H), 527 (M+NH4), 508 (M-H). Anal. Calcd for: C₂₂H₂₄N₃O₇SCl C. 51.81; H, 4.74; N, 8.23. Found: C. 51.61; H, 4.90; N, 7.96.

### Example 88

### N-[1-[[(4-butyl[1,1'-biphenyl]-4-yl)sulfonyl]methyl]-2-(3,4,4-trimethyl-2,5-dioxo-1-imidazolidinyl)ethyl]-N-hydroxyformamide

The title compound was prepared following the procedures described in example 46A,46B,46C and 46D, except substituting example 16B for 23A in example 46A and 4-nbutylbenzeneboronic acid for 4-butyloxybenzeneborome acid in example 46B.
¹H NMR (d6-DMSO) 9.70 (s, 0.5H), 9.54 (s, 0.5H), 8.10 (s, 0.5H), 7.94 (d, 4H, J=1.0 Hz), 7.73 (s, 0.5H), 7.69 (dd, 2H, J=8.1, 2.0 Hz), 7.35 (d, 2H, J=8.4 Hz). 4.96-4.86 (m, 0.5H), 4.60-4.48 (m. 0.5H), 3.77-3.42 (m, 4H), 2.75 (s, 3H), 2.64 (t, 2H. J=7.4 Hz), 1.64-1.54 (m, 2H), 1.40-1.27 (m. 2H), 1.24 (s, 3H), 1.22 (s, 3H), 0.91 (t, 3H, J=7.5 Hz), MS (ESI) 516 (M+H), 533 (M+NH₄), 538 (M+Cl), 514 (M-H). Anal. Calcd for: C₂₆H₃₃N₃O₆S C, 60.56; H. 6.45; N. 8.14. Found. C, 60.32; H. 6.44; N, 8.09.

### Example 89

### N-[1-[[(4'-butyl[1,1'-biphenyl]-4-yl)oxy]methy]-2-(4,4-dimethy-2,5-dioxo-1-imidazolidinyl)ethyl]-N-hydroxyformamide

The title compound was prepared according to the procedures of example 23B, except substituting4-(4'-butylphenyl)-phenol in place of 4-(4'-ethoxyphenyl)-phenol.
1H NMR (DMSO-d6) d 0.93 (t, 3H, J=8 Hz). 1.28 (s, 6H), 1.30-1.39 (m, 2H), 1.50-1.65 (m, 2H), 2.60 (t, 2H, J=7 Hz), 3.51-3.64 (m, 1H), 3.67-3.80 (m, 1H), 4.03-4.24 (m, 2H), 4.35-4.48 (m, 0.5H), 4.78-4.92 (m, 0.5H), 6.99 (dd, 2H, J=3,9 Hz), 7,25 (d, 2H, J=9 Hz). 7.53 (d, 2H, J=9 Hz), 7.58 (d. 2H, J=9 Hz), 7.94 (s, 0.5H), 8.34 (d, 1H, J=6 Hz), 8.39 (s, 0.5H), 9.55 (s, 0.5H), 9.87 (s, 0.5H). MS (ESI-) 452 (M-H). Anal. Calcd for: C25H31N305 C, 66.20; H, 6.88; N, 9.26. Found: C, 65.99; H, 6.71; N, 9.19.

### Example 90

### N-[1-[[[3'-(cyanomethyl)[1,1'-biphenyl]-4-yl]oxy]methyl]-2-(4,4-dimethy-2,5-dioxo-1-imidazolidinyl)ethyl]-N-hydroxyformamide

The title compound was prepared according to the procedures of example 23B, except substituting 4-(3'-cyanomethylphenyl)-phenol in place of 4-(4'-ethoxyphenyl)-phenol.
1H NMR (DMSO-d6) d 2.80 (s, 6H), 3.52-3.83 (m, 2H), 4.10 (s, 2H), 4.12-4.25 (m, 2H), 4.38(4.46, mH, J=0.5 Hz), 4.80-4.90 (m, 0.5H), 7.03 (dd, 2H, J=3,9 Hz), 7.30 (d, 1H, J=10 Hz), 7.48 (t, 1H, J=10 Hz), 7.57-7.66 (m, 4H). 7.93 (s, 0.5H), 8.34 (d, 1H, J=6 Hz), 8.39 (s, 0.5H), 9.55 (s, 0.5H), 9.88 (s, 0.5W). MS (ESI-) 435 (M-H). Anal. Calcd for: C23H24N4O5•0.25CH3CO2C2H5 C, 62.87; H. 5.71: N, 12.21. Found: C. 62.85; H, 5.80; N, 12.16.

### Example 91

### N-[1-[4-(2-thienyl)phenoxy]methyl]-2-[1-(3,4,4-trimethy]-2,5-dioxo-1-imidazolidinyl)ethyl]-N-hydroxyformamide

The title compound was preapred following the procedures of examples 16C and 16E, except substituting 4-(4'-(2-thienyl)phenyl)phenol for 4-bromophenol in example 16C.
1H NMR (DMSO-d6) d 1.29 (s, 6H). 2.80 (s, 3H), 3.54-3.66 (m, 1H), 3.69-3.84 (m, 1H), 4.04-4.22 (m, 2H), 4.33-4.47 (m, 0.5H), 4.77-4.90 (m, 0.5H), 6.96 (dd, 2H, J=3.9 Hz), 7.08-7.13 (m, 1H), 7.38 (d, 1H, J=3 Hz), 7.46 (d, 1H, J=4 Hz), 7.59 (d, 2H, J=9 Hz), 7.92 (s. 0.5H), 8.31 (s. 0.5H), 9.54 (s, 0.5H), 9.86 (s, 0.5H), MS (ESI-) 416 (M-H). Anal. Calcd for: C20H23N3O5S·0.25H2O C. 56.92; H. 5.61; N, 9.95. Found: C. 56.65; H. 5.48: N, 9.77.

### Example 92

### N-[1-[[(3-nitro[1,1'-biphenyl]-4-yl)oxy]methyl]-2-(3,4,4-trimethyl-2,5-dioxo-1-imidazolidinyl)ethyl]-N-hydroxyformamide

The title compound was preapred following the procedures of examples 16C and 16E. except substituting 4-phenyl-2-nitrophenol for 4-bromophenol in example 16C.
1H NMR (DMSO-d6) d 1.28 (s, 6H), 2.80 (s, 3H), 3.54-3.65 (m, 1H), 3.70-3.88 (m, 1H), 4.22-4.39 (m. 2H). 4.40-4.50 (m, 0.5H), 4.80-4.91 (m, 0.5H), 7.34-7.41 (m. 2H), 7.43-7.52 (m, 2H), 7.71 (d, 2H, J=8 Hz), 7.87 (s, 0.5H), 8.0 (d, 1H, J=9 Hz), 8.16 (dd, 1H, J=3,6 Hz), 8.29 (s, 0.5H), 9.55 (s, 0.5H), 9.80 (s. 0.5H). MS (ESI-) 455 (M-H). Anal. Calcd for: C22H24N407 C, 57.88; H, 5.29; N, 12.27, Found: C, 57.62; H. 5.44; N, 11.95.

### Example 93

### N-[1-[[(4'-methyl[1,1'-biphenyl]-4-yl)oxy]methyl]-2-[[3-(methylsulfonyl)amino]phenyl]ethyl]-N-hydroxyformamide

### Example 93A

### 3-(methylsulfonyl)amino-1-bromo-benzene

The m-bromo aniline was dissolved in 40 ml of pyridine and cooled to 0 C followed by addition of MsCl dropwise via syringe. After 10 mm. the solution was warmed to room temperature and stirred for 4h. Upon concentration *in vacuo* the residue was partitioned between 350 ml of H2O and 500 ml of CH2Cl2 in a separatory funnel. The organics were separated and washed with 100 ml of 3N HCl, 200 ml of sat'd NaHCO3 and dried over MgSO4. Upon filtration and concentration in vacuo an off-white solid was obtained. This product was recrystallized from CH2Cl2/Hexanes to afford 6.7g (90%) of 93A as white needles.

### Example 93B

### 3-(methylsulfonyl)amino-1-(prop-2-enyl)-benzene

Using a glass sealed vessel the sulphonamide 93A (3.0g, 12.1mmol) was suspended in 10 ml of toluene followed by addition of the allyltributyl tin reagent and bubbled with argon for 5 min. To the above suspension was added 280 mg (2 mol %) of Pd(PPh3)4 and the vessel sealed and heated at 120 C for 17h. After 15 min a homogeneous solution was obtained which turned dark brown after 30 mm After cooling, the catalyst was filtered off washing with CH2Cl2/MeOH. Concentration of the filtrate followed by purification on silica gel eluting with 10% Ethyl Acetate/Hexanes then 20% Ethyl Acetate/Hexanes afforded 93B, 0.99g (38%) as a colorless oil which solidified upon standing.

### Example 93C

### N-[1-[[(4'-methyl[1,1'-biphenyl]-4-yl)oxy]methyl]-2-[[3-(methylsulfonyl)amino]phenyl]ethyl]-N-hydroxyformamide

The title compound was preapred from example 93B, first by epoxidizing as desribed in example 5C, then opening the epoxide with 4'-hydroxy-4-biphenylcarbonitrile as in example 5F, then following the sequence of reactions described in examples 2C through 2F.
1H NMR (DMSO-d6) d 2.32 (s, 3H), 2.88 (d, 2H, J=6 Hz), 2.96 (s, 1H), 2.98 (s, 3H), 4.03-4.11 (m, 1H), 4.15-4.27 (m, 1,5H), 4.72-4.82 (bs, 0.5H), 6.97-7.10 (m, 4H), 7.05 (s, 1H), 7.20-7.30 (m, 3H), 7.50 (d, 2H, J=9 Hz), 7.57 (d, 2H, J=9 Hz), 7.73 (s, 0.5H), 8.25 (s, 0.5H), 9.18 (s, 0.5H), 10.01 (s, 0.5H). MS (ESI-) 453 (M-H). Anal. Calcd for: C24H26N2O5S C, 63.41; H. 5.76; N. 6.16. Found: C, 63.16; H, 6.12; N, 5.76.

### Example 94

### N-[1-[[[3-(diethylamino)carbonyl]phenyl]methyl]-2-[(4'-methyl[1,1'-biphenyl]-4-yl)oxy]ethyl]-N-hydroxyformamide

### Example 94A

### 3-bromo-1-(N,N-diethylcarboxamide)-benzene

Diethylamine (10.0ml, 97mmol) was dissolved in 60ml of dry ethyl ether and cooled to 0° C. Benzoyl chloride (3.67ml, 28mmol) was dissolved in 10 ml of dry ethyl ether and was slowly added dropwise via syringe to to the above solution. A white slurry developed upon addition and stirring was continued for 10 min at 0° C then warmed to room temperature for 1h. The mixture was poured into a separatory funnel containing 500ml of ethyl ether and 75ml of 10% NaOH. The organics were separated and washed a second time with 75 ml of 10% NaOH followed by 75 ml of 10% HCl then 200 ml of water. A final wash with 100 ml of brine followed by drying over MgSO4. filtering and then concentration *in vacuo*, afforded the 94A as a colorless liquid, 5.9g (83%) which was used without further purification.

### Example 94B

### N-[1-[[[3-(diethylamino)carbonyl]phenyl]methyl]-2-[(4'-methyl[1,1'-biphenyl]-4-yl)oxy]ethyl]-N-hydroxyformamide

The title compound was prepared following the procedure described in examples 93B and 93C, except substituting 94A for 93A.
1H NMR (DMSO-d6) d 0.98-1.20 (bd, 6H), 2.32 (s, 3H), 2.93 (d, 2H, J=6 Hz), 3.11-3.48 (bd, 4H), 4.0-4.13 (m, 1H), 4.16-4.30 (m, 1.5H), 4.74-4.86 (bs, 0.5H), 6.98 (d, 2H, J=9 Hz), 7.13-7.40 (m, 6H), 7.50 (d, 2H, J=8 Hz), 7.56 (d, 2H, J=9 Hz), 7.73 (s, 0.5H), 8.23 (s, 0.5H), 9.63 (s, 0.5H), 10.02 (s, 0.5H). MS (ESI+) 461 (M+H). Anal. Calcd for: C28H32N2O4•1.5H2O C. 68.97; H, 7.23; N, 5.74, Found: C, 68.96; H, 7.09; N, 5.42.

### Example 95

### N-[1-[[(4'-cyano[1,1'-biphenyl]-4-yl)oxy]methyl]-2-[(4'-cyano[1,1'-biphenyl]-4-yl)oxy]ethyl]-N-hydroxyformamide

The title compound was prepared using the procedures of examples 5F,5G and 5H, except using example 3B in place of 5E.
1H NMR (DMSO-d6) d 4.18-4.36 (m, 4H), 4.43-4.57 (bs, 0.5H), 4.97-5.03 (bs, 0.5H), 7.10 (d, 4H, J=9 Hz), 7.77 (d, 4H, J=9 Hz), 7.81-7.95 (m, 8H), 8.13 (s, 0.5H), 8.42 (s, 0.5H), 9.75 (s, 0.5H), 10.15 (s, 0.5H). MS (DCI/NH3) M+H (490), M+18 (507). Anal. Calcd for: C30H23N304•0.25H2O C. 72.93; H, 4.79; N, 8.50. Found: C, 72.80; H, 4.74; N, 8.26.

### Example 96

### N-[1-[[(4)-cyano[1,1'-biphenyl]-4-yl)oxy]methyl]-2-(4,4-dimethy-2,5-dioxo-1-imidazolidinyl)ethyl]-N-hydroxyformamide

The title compound was prepared according to the procedures of example 23B. except substituting 4-(4'-cyanophenyl)-phenol in place of 4-(4-ethoxyphenyl)-phenol
1H NMR (DMSO-d6) d 1.27 (s, 6H), 3.50-3.66 (m, 1H), 3.67-3.82 (m, 1H), 4.08-4.28 (m, 2H), 4.38-4.50 (m, 0.5H), 4.80-4.93 (m, 0.5H), 7.06 (dd, 2H, J=3,9 Hz), 7.73 (d, 2H, J=9 Hz), 7.82-7.93 (m, 4H), 7.94 (s, 0.5H), 8.35 (d, 1H, J=6 Hz), 8.40 (s, 0.5H), 9.56 (s, 0.5H), 9.87 (s, 0.5H). MS (ESI-) 421 (M-H). Anal. Calcd for: C22H22N4O5•0.25H2O C, 61.89; H, 5.31; N, 13.12. Found: C, 61.82; H, 5.34; N, 12,82.

### Example 97

### N-[1-[(4,4-dimethyl-2,5-dioxo-1-imidazolidinyl)methyl]-2-[[4'-(2-methoxyethoxy)[1,1'-biphenyl]-4-yl]oxy]ethyl]-N-hydroxyformamide

The title compound was prepared according to the procedures of example 23B. except substituting4-(4'-(2-methoxyethoxy)-phenyl)-phenol in place of 4-(4'-ethoxyphenyl)-phenol.
1H NMR (DMSO-d6) d 1.26 (s, 6H), 3.33 (s, 3H), 3.50-3.64 (m, 1H), 3.66-3.69 (m, 2H), 3.70-3.81 (m, 1H). 4.03-4.22 (m, 4H), 4.35-4.48 (m, 0.5H), 4.78-4.90 (m, 0.5H), 6.98 (dd, 4H. J=3,9 Hz), 7.55 (dd, 4H. J=3,6 Hz), 7.91 (s, 0.5H), 8.33 (d, 1H, J=7 Hz). 8.40 (s, 0.5H), 9.55 (s, 0.5H), 9.86 (s, 0.5H). MS (ESI-) 470 (M-1). Anal. Calcd for: C24H29N3O7 C. 61.13; H, 6.19; N, 8.91. Found: C, 60.86; H, 6.41; N, 8.65.

### Example 98

### N-[1-[(4,4-dimethy-2,5-dioxo-1-imidazolidinyl)methyl]-2-[(4'-propoxy[1,1'-biphenyl]-4-yl)oxy]ethyl]-N-hydroxyformamide

The title compound was prepared according to the procedures of example 23B, except substituting 4-(4'-propyloxyphenyl)-phenol in place of 4-(4'-ethoxyphenyl)-phenol.
mp. 158-160°C
Calc. for C₂₄H₂₉N₃O₆: C, 63.28; H, 6.42; N, 9.22. Found: C, 63.25; H, 6.48; N, 9.29, Mass Spec. (ESI): +456 (m+1), +473 (m+18), -454 (m-1), -490 (m+35)
¹H NMR (DMSO-d6): 0.90 (3H, t, J=6 Hz), 1.17 (2.4H, s), 1.20(3.6H, s), 1.65 (2H, sextuplet, J=6 Hz), 3.46-3.55 (1H, m), 3.59-3.74 (1H, m), 3.86 (2H, t, J=6 Hz), 3.96-4.06 (1H, m), 4.06-4.14 (1H, m), 4.28-4.38 (0.6H, m), 4.72-4.81 (0.4H, m), 6.88 (4H, d, J=4.8 Hz), 7.42, (2H, d, J=4.8 Hz), 7.44 (2H, d, J=4.8 Hz), 7.83 (0.4H, s), 8.24 (1H, s), 8.28 (0.6W, s), 9.43 (0.6H, s), 9.74(0.4H, s).
¹³C NMR (DMSO-d6): 10.4, 22.0, 24.4, 24.5, 36.0, 36.4, 52.1, 56.1, 57.8, 57.9, 64.7, 65.0, 69.0, 114.8, 115.0, 127.2, 132.0, 132.8, 132.9, 155.0, 155.2, 157.0, 157.1, 157.9, 158.2, 163.1, 177.2, 177.3.

### Example 99

### N-[1-[(4,4-dimethy-2,5-dioxo-1-imidazolidinyl)methyl]-2-[(4'-pentyloxy[1,1'-biphenyl]-4-yl)oxy]ethyl]-N-hydroxyformamide

The title compound was prepared according to the procedures of example 23B, except substituting4-(4'-pentylyloxyphenyl)-phenol in place of 4-(4'-ethoxyphenyl)-phenol. ¹H NMR (300 MHz, DMSO-d₆) d 0.90 (t, 3H, J=6.9 Hz), 1.27 (s, 6H), 1.3-1.5 (m, 4H), 1.7-1.8 (m, 2H), 3.5-3.8 (m, 2H), 3.98 (t, 2H, J=6.9 Hz), 4.0-4.2 (m, 2H), 4.35-4.45 (m, 0.5H), 4.8-4.9 (m, 0.5H), 6.9-7.0 (m, 4H), 7.5-7.6 (m, 4H), 7.92 (s, 0.5H), 8.3-8.4 (m. 1.5H), 9.53 (s, 0.5H), 9.84 (s, 0.5H), MS (ESI) 484 (M+H), 501 (M+NH₄). Anal. calcd for C₂₆H₃₃N₃O₆: C, 64.57: H. 6.87; N, 8.68. Found: C. 64.27; H. 6.85; N, 8.60.

### Example 100

### N-[1-[[[3'-(cyanomethyl)[1,1'-biphenyl]-4-yl]sulfonyl]methyl]-3-(4,4-dimethy-2,5-dioxo-1-imidazolidinyl)propyl]-N-hydroxyformamide

The title compound was made according to the procedures of example 61, but using example23A in place of example 16B and 4'-thiol-3-cyanomethyl biphenyl in place of 4'-thiol-4-biphenylcarbonitrile in example 61A.
¹H NMR (300 MHz, d₆-DMSO) δ 9.98 (br, 0.5H), 9.63 (br, 0.5H), 8.31 (s, 0.5H), 8.23 (s, 0.5H), 8.12 (s, 0.5H), 7.99-7.92 (m, 4H), 7.82 (s, 0.5H), 7.74 (m, 2H), 7.57 (t, 1H), 7.46 (d, 1H), 4.52 (m, 0.5H), 4.14 (s, 2H), 4.00 (m, 0.5H), 3.69-3.57 (m, 2H), 3.42-3.28 (m, 2H), 2.02-1.88 (m, 1H), 1.78-1.64 (m, 1H), 1.21 (s, 6H);
MS (ESI) m/e 499 (M+1)⁺.
Anal. calcd for C₂₄H₂₆N₄O₆S: C. 57.82; H, 5.26; N, 11.24. Found: C, 57.56; H. 5.41; N, 10.89.

### Example 101

### N-[1-[[[4'-trifluoromethoxy)[1,1'-biphenyl]-4-yl]sulfonyl]methyl]-2-(3,4,4-trimethy-2,5-dioxo-1-imidazolidinyl)ethyl]-N-hydroxyformamide

The title compound was prepared following the procedures of example 46, except substituting example 16B for 23A in example 46A and 4-trifluoromethoxybenzeneboronic acid for 4-butoxybenzeneboronic acid in example 46B.
¹H NMR (300 MHz, d₆-DMSO) δ 9.72 (br, 0.5H), 9.56 (br, 0.5H), 8.10 (s, 0.5H), 7.99 (m, 4H), 7.94-7.88 (m, 2H), 7.74 (s, 0.5H), 7.53 (d. 2H), 4.91 (m, 0.5H), 4.54 (m, 0.5H), 3.75-3.44 (m, 4H), 2.75 (s. 3H), 1.24-1.22 (m. 6H);
MS (ESI) m/e 544 (M+1)⁺.
Anal. calcd for C₂₃H₂₄F₃N₃O₇S: C, 50.83; H, 4.45; N, 7.73. Found: C, 51.17; H, 4.77; N. 7.29.

### Example 102

### N-[1-[[(4'-cyano[1,1'-biphenyl]-4-yl)sulfonyl]methyl]-2-(3-methy-2,5-dioxo-1-imidazolidinyl)ethyl]-N-hydroxyformamide

The title compound was made according to the procedures of example 61, but using example 26A in place of example 16B in example 61A.
¹H NMR (300 MHz, d₆-DMSO) δ 9.83 (s, 0.5H), 9.58 (s, 0.5H), 8.09 (s, o.5H), 8.04-8.00 (m, 8H), 7.80 (s, 0.5H), 4.94-4.85 (m, 0.5H), 4.52-.4.43 (m, 0.5H), 3.91-3.88 (m, 2H), 3.78-3.44 (m, 4H), 2.80 (s, 1.5H), 2.79 (s, 1.5H);
MS (ESI) m/e 457 (M+1)⁺.
Anal. calcd for C₂₁H₂₀N₄O₆S: C, 55.26; H, 4.42; N, 12.27. Found: C. 54.99; H. 4.38; N, 12.07.

### Example 103

### N-[1-[[[3'-(cyanomethyl)[1,1'-biphenyl]-4-yl]sulfonyl]methyl]-2-(3,4,4-trimethyl-2,5-dioxo-1-imidazolidinyl)ethyl]-N-hydroxyformamide

The title compound was prepared following the procedures of example 46, except substituting example 16B for 23A in example 46A and 3-cyanomethylbenzeneboronic acid for 4-butoxybenzeneboronic acid in example 46B.
The title compound was made in the usual way from the α-bromo ketone nd 4-bromothiophenol.
¹H NMR (300 MHz, d₆-DMSO) δ 9.71 (s, 0.5H), 9.56 (s, 0.5H), 8.10 (s, 0.5H), 8.03-7.94 (m, 4H), 7.75 (m, 2.5H), 7.57 (t, 2H), 7.46 (d, 2H), 4.96-4.88 (m, 0.5H), 4.59-4.49 (m, 0.5H), 4.14 (s, 2H), 3.69-3.48 (m, 4H), 2.75 (s, 3H), 1.24 (s, 3H), 1.22 (s, 3H);
MS (ESI) m/e 499 (M+1)⁺.
Anal. calcd for C₂₄H₂₆N₄O₆S: C, 57.82; H, 5.26; N, 11.24. Found: C, 57.73; H, 5.36; N, 10.95.

### Example 104

### N-[1-[[(4'-cyano[1,1'-biphenyl]-4-yl)oxyl]methyl]-2-(1,6-dihydro-3-methyl-6-oxo-1-pyridazinyl)ethyl]-N-hydroxyformamide

The title compound was prepared following the procedures of example 3C and 3D, except substituting the potassium salt 6-methyl-3(2H)-pyridazinone (generated in situ with potassium carbonate) for potassium phthalimide in example 3C.
¹H NMR (300 MHz, CD₃OD) δ 8.31 (s, 0.5H), 7.91 (s, 0.5H), 7.76 (s, 4H), 7.67.64 (d, 2H), 7.38 (dd, 1H), 7.08 (d, 2H), 6.94 (dd, 1H), 5.20-5.11 (m, 0.5H), 4.64-4.52 (m, 2H), 4.42-4.32 (m, 2H), 4.27-4.19 (m 0.5H), 2.35 (s, 1.5H), 2.34 (s, 1.5H);
MS (ESI) m/e 405 (M+1)⁺.
Anal. calcd for C₂₂H₂₀N₄O₄: C, 65.34; H, 4.98; N, 13.85 Found: C, 64.85 H, 5.36; N, 13.44.

### Example 105

### (±)-N-[1-[[(4'-cyano[1,1'-biphenyl]-4-yl)sulfonyl]methyl]-2-(4,4-dimethyl-2,5-dioxo-1-imidazolidinyl)ethyl]-N-hydroxyformamide;

The title compound was made according to the procedures of example 61, but using example 47A in place of example 16B in example 61A.
¹H NMR (300 MHz, d₆-DMSO) δ 9.98 (s, 0.5HO, 9.62 (s, 0.5H), 8.31 (s, 0.5H), 8.22 (s, 0.5H), 8.12 (s, 0.5H), 8.05-7.96 (m, 8H), 7.82 (s, 0.5H), 4.55-4.46 (m, 0.5H), 4.07-3.97 (m, 0.5H), 3.70-3.56 (m, 2H), 3.32-3.24 (m, 2H), 2.02-1.88 (m, 0.5H), 1.76-1.64 (m, 0.5H), 1.21-1.18 (m, 6H):
MS (ESI) m/e 485 (M+1)⁺.

### Example 106

### (±)-N-[1-[[[4-(4-fluorophenoxy)phenyl]sulfonyl]methyl]-2-(4,4-dimethyl-2,5-dioxo-1-imidazolidinyl)ethyl]-N-hydroxyformamide

The title compound was made according to the procedures of example 61, but using example 26A in place of example 16B and 4-(4'-fluoroohenoxy)-benzene thiol in place of 4'thiol-4-biphenyl carbonitrile in example 61A.
¹H NMR (300 MHz, d₆-DMSO) δ 9.66 (s, 0.5H), 9.50 (s, 0.5H), 8.39 (s, 0.5H), 8.35 (s, 0.5H), 8.10 (s, 0.5H), 7.88 (dd, 2H), 7.68 (s, 0.5H), 7.36-7.30 (m, 2H), 7.26-7.20 (m, 2H), 7.15 (d, 2H), 4.88-4.80 (m, 0.5H), 4.51-4.41 (m, 0.5H), 3.70-3.39 (m, 4H), 1.24-1.22 (m, 6H);
MS (ESI) m/e 480 (M+1)⁺.
Anal. calcd for C₂₁H₂₂FN₃O₇S: C, 52.60; H, 4.62; N, 8.76. Found: C, 52.79; H. 4.57; N. 8.68.

### Example 107

### N-[1-[[4-(4-pyridinyl)phenoxy]methyl]-2-(3,4,4-trimethy-2,5-dioxo-1-imidazolidinyl)ethyl]-N-hydroxyformamide

The title compound was prepared following the procedures of examples 16C and 16E, except substituting4-(4-pyridinyl)-phenol in place of 4-bromophenol in example 16C.
mp: 217°-218°C
1H NMR (DMSO-d6): d 9.53-9.97 (c, 1H), 8.55-8.60 (c, 2H), 8.32 (s, 1/2H), 7.92 (s, 1/2H), 7.77 (s, 1H), 7.75 (s, 1H), 7.65 (s, 1H), 7.64 (s, 1H), 7.01-7.08 (c, 2H), 4.82-4.89 (c, 1/2H), 4.39-4.46 (c, 1/2H), 4.18-4.25 (c, 1H), 4.08-4.17 (c, 1H), 3.71-3.83 (c, 1H), 3.57-3.66(c, 1H), 2.78(s, 1.5H), 2.77(s, 1.5H), 1.27(s, 3H), 1.26(s, 3H).
MS (ESI(+)) 413 (M+H), 435 (M+Na), 847 (2M+Na)
Anal, Calcd for: C21H24N4O5•0.5H2O C, 59.84; H, 5.98; N, 13.29, Found: C. 60.18; H. 6.05; N, 13.10.

### Example 108

### (S)-N-[1-[(4,4-dimethy-2,5-dioxo-1-imidazolidinyl)methyl]-2-[[4'-(trifluoromethoxy)[1,1'-biphenyl]-4-yl]oxy]ethyl]-N-hydroxyformamide

### Example 108A

### (R) 1-(4-(4'-(trifluoromethoxyphenyl)phenoxy)-3-benzyloxy-2-propanol

A solution of 4-(4'-trifluoromethoxyphenyl)-phenol (1.854g, 7.3 mmol) and (S)-2-(benzyloxymethyl)-oxirane (1.0g, 6.1 mmol) in DMF (15 mL) was treated with potassium carbonate (1.007 g, 7.3 mmol), then stirred at 80° C overnight. The reaction mixture was allowed to cool to r.t., poured into water (100 mL) and extracted twice with ethyl acetate (200 mLx 2). The combined organics were washed with sat. aq. NH4Cl, water , brine, dried (Na₂SO₄) and concentrated. Purification via flash silica chromatography eluting with 20 to 25% ethyl acetate: hexane afforded 2.03 g (80% yield) of 108A as a white solid.

### Example 108B

A solution of example 108A (1.505 g, 3.6 mmol), di-Boc hydroxylamine (1.007 g, 4.3 mmol), triphenyl phosphine (1.23 g, 4.7 mmol) in THF (15 mL) was treated with diethylazodicarboxylate (0.735 mL, 4.7 mmol) at room temperature, stirred for 1h, then concentrated. The crude was purified by column chromatography eluting with 10% ethyl acetate: hexane to give 1.16 g (50%) of 108B.

### Example 108C

A solution of example 108B (248 mg, 0.4 mmol) in THF (3mL) was hydrogenated (H2 balloon) overnight in the presence of 23 mg of 10%pd on carbon. The reaction mixture was filtered , concentrated and purified via silca gel column chromatography eluting with 25% ethyl acetate: hexane to afford 180 mg (85%) of the title compound.

### Example 108D

A solution of example 108C (228 mg, 0.42 mmol), 5,5-dimethyl hydantoin (94 mg, 0.73 mmol) and triphenyl phospine (165 mg, 0.63 mmol) in THF (4 mL) was treated with diethylazodicarboxylate (0.1 mL, 0.63 mmol) added drowise via syringe. The resulting light yellow solution was stirred at rt for 45 mn, concentarted and purigies via silca gel column chromatography eluting with 25% ethyl acetate: hexane to afford 193 mg (71 %)of 108D.

### Example 108E

### (S)-N-[1-[(4,4-dimethyl-2,5-dioxo-1-imidazolidinyl)methyl]-2-[[4'-(trifluoromethoxy)[1,1'-biphenyl]-4-yl]oxy]ethyl]-N-hydroxy amine

A solution of example 108D (191 mg, 0.29 mmol) in methylene chloride (3 mL) was treated with TFA (1.5 mL), added dropwise via syringe. The reaction was stirred at rt for 40 min then concentrated and the residue was partitioned between ethyl acetate and aq. NaHCO3. the organic extract was washed with brine, dried (Na₂SO₄) and concentrated to afford 113 mg (86%) of 108E as a white solid.

### Example 108F

### (S)-N-[1-[(4,4-dimethy-2,5-dioxo-1-imidazolidinyl]methyl]-2-[[4'(trifluoromethoxy)[1,1'-biphenyl]-4-yl]oxy]ethyl]-N-hydroxyformamide

The title compound was prepared from 108E following the procedure of example 2F.
MS (ESI) m/e 482 (M+H)⁺.

### Example 109

### (R)-N-[1-[(4,4-dimethy-2,5-dioxo-1-imidazolidinyl)methyl]-2-[[4'-(trifluoromethoxy)[1,1'-biphenyl]-4-yl]oxy]ethyl]-N-hydroxyformamide

The title compound was prepared following the procedures of example 108, except using (R)-2-(benzyloxymethyl)-oxirane in place of (S)-2-(benzyloxymethyl)-oxirane.
¹H NMR (300 MHz, d₆-DMSO) δ 9.86 (s, 0.5H), 9.55 (s, 0.5H), 8.39 (s, 0.5H), 8.35 (s, 0.5H), 8.33 (s, 0.5H), 7.93 (s, 0.5H), 7.76-7.73 (m, 2H), 7.64 (d, 2H), 7.42 (d. 2H), 7.02 (dd, 2H), 4.91-4.80 (m, 0.5H), 4.47-4.38 (m, 0.5H), 4.23-4.06 (m, 2H), 3.79-3.50 (m, 2H), 1.27 (s, 1.5H), 1.26 (s, 1.5H);
MS (ESI) m/e 482 (M+1)⁺.

### Example 110

### N-[1-[[[4'-(trifluoromethoxy))[1,1'-biphenyl]-4-yl]oxy]methyl]-3-(4,4-dimethyl-2,5-dioxo-1-imidazolidinyl)propyl]-N-hydroxyformamide

The title compound was prepared according to the procedures of example 5, except avoiding the methylation step in example 5B and substituting 4-(4'-trifluoromethoxyphenyl)phenol for 4'-hydroxy-4-biphenylcarbonitrile in example 5F.
mp: 197.1-197.9°C.
¹H NMR (300 MHz. DMSO-d6) δ ; 1.27 (s, 6H), 1.70-2.00 (m, 2H), 3.35-3.46 (2H), 3.97-4.16 (m, 2.75H), 4.51 (br s, 0.25H), 7.00-7.03 (d, 2H, J=9 Hz), 7.39-7.42 (d, 2H, J=9 Hz), 7.60-7.63 (d, 2H, J=9 Hz), 7.72-7.75 (d, 2H, J=9 Hz), 8.25-8.35 (2H), 9.55 (s, 0.75H), 9.95 (br s, 0.25H),
MS (ESI) m/e 496 (M+H)⁺, 518 (m+Na)+, 494 (m-H)-. 530 (m+Cl)-.
Anal. calcd for C23H24F3N3O6: C, 55.75; H. 4.88: N, 8.48. Found: C, 55.72; H, 5.07: N, 8.59.

### Example 111

### N-[1-[4-[(4-pyridinylthio)phenoxy]methyl]-2-(4,4-dimethyl-2,5-dioxo-1-imidazolidinyl)ethyl]-N-hydroxyformamide

The title compound was prepared following the procedures of example 23B, except substituting (prepared by the addition of 4-hydroxythiophenol to 4-chloropyridine) for 4-(4'-butyloxyphenyl)-phenol.
¹H NMR (300 MHz, DMSO-d6) δ : 1.258-1.272 (s+s, 6H), 3.492-3.793 (m, 2H), 4.082-4.248 (m, 2H), 4.437 (m, 0.5H), 3.861 (m, 0.5H), 5.759 (s, 1H), 6.927-6.948 (dd, 2H, J=1.5, 4.8 Hz). 7.063-7.102 (dd, 2H, J=3, 8.7 Hz), 7.529-7.557 (d. 2H, J=8.4 Hz), 7.939 (s, 0.5H), 8.323-8.343 (dd, 2H, J=1.2, 4.8 Hz), 8.391 (s, 0.5H). 9.555 (s, 0.5H), 9.866 (s, 0.5H).
MS (ESI) m/e 431 (M+H)⁺, 453 (m+Na)+, 429 (m-H)-, 465 (m+Cl)-.

### Example 112

### N-[1-[[[(4-chlorophenoxy)phenyl]sulfonyl]methyl]-3-(4,4-dimethyl-2,5-dioxo-1-imidazolidinyl)propyl]-N-hydroxyformamide

The title compound was prepared according to the procedures of example 61, except substituting 4-(4'-chlorophenoxy)benzene thiol for 4'-thiol-4-biphenylcarbonitrile and example 47A for example 16B in example 61A.
¹H NMR (d6-DMSO) 9.94 (s, 0.5H). 9.58 (s, 0.5H), 8.23 (d, 0.5H, J=9.5 Hz), 8.11 (s. 0.5H), 8.05 (d, 0.5H, J=9.2 Hz), 7.89-7.83 (m, 2H), 7.76 (s, 0.5H), 7.54-7.50 (m, 2H). 7.22-7.16 (m, 4H), 4.52-4.41 (m, 0.5H), 4.10-3.92 (m, 0.5H), 3.66-3.37 (m, 2H), 3.31-3.24 (m, 3H), 1.96-1.84 (m, 1H), 1.74-1.62 (m, 1H), 1.28-1.21 (m, 6H). MS (ESI) 508 (M-H), 510 (M+H), 532 (M+Na).

### Example 113

### N-[1-[[(4'-cyano[1,1"-biphenyl]-4-yl)oxyl]methyl]-2-(1,6-dihydro-6-oxo-1-pyridazinyl)ethyl]-N-hydroxyformamide

The title compound was prepared following the procedures of example 104, except using pyridazinone in place of 6-methyl-3(2H)-pyridazinone
¹H NMR (d6-DMSO) 9.99 (s, 0.5H), 9.64 (s, 0.5H), 8.28 (s, 0.5H), 7.96-7.83 (m, 5.5H), 7.75-7.71 (m, 2.0H), 7.47-7.41 (m, 1H), 7.07-6.95 (m, 3H), 5.11-5.00 (m, 0.5H), 4.62-4.12 (m. 4.5H).
MS (ESI) 391 (M+H), 413 (M+Na), 389 (M-H).
Anal. Calcd for: C₂₁H₁₈N₄O₄•0.5H₂O C, 63.15; H, 4.79; N, 14.02, Found: C, 63.33; H, 4.66; N, 13.68.

### Example 114

### N-[1-[[[4'-(aminosulfonyl)[1,1'-biphenyl]-4-yl]oxy]methyl]-2-(3,4,4-trimethyl-2,5-dioxo-1-imidazolidinyl)ethyl]-N-hydroxyformamide

The title compound was prepared following the procedures of examples 16C and 16E. except substituting 4-(4'-sulfonamidephenyl)-phenol for 4-bromophenol.
mp 203°-205°
1H NMR (DMSO-d6): d 9.88 (bs, 1/2H), 9.54 (bs, 1/2H), 8.32 (s, 1/2H), 7.56-8.01 (c, 5 1/2H), 7.34 (s, 1H), 7.00-7.14 (c. 4H), 4.78-4.97 (c, 1/2H), 4.34-4.50 (c, 1/2H), 4.06-4.27 (c, 2H), 3.69-3.85 (c, 1H), 3.57-3.68 (c, 1H), 2.78 (s, 3H), 1.17-1.28 (c, 6H).
13C NMR (DMSO-d6): d 176.5, 176.2, 163.1, 158.1, 154.2, 154.1, 128.3, 128.2, 128.0, 127.0, 126.8, 126.2, 115.2, 64.8, 60.7, 36.7, 36.4, 24.2, 21.4
MS (ESI(+)) 491 (M+H), 508 (M+NH4), 513 (M+Na)

### Example 115

### N-[1-[[[4'-(trifluoromethoxy)[1,1'-biphenyl]-4-yl]sulfonyl]methyl]-2-(4,4-dimethyl-2,5-dioxo-1-imidazolidinyl)ethyl]-N-hydroxyformamide

The title compound was prepared following the procedures of example 46, except substituting 4-trifluoromethoxybenzeneboronic acid for 4-butyloxybenzeneboronic in example 46B.
mp 195°-197°
1H NMR, (DMSO-d6): d 9.62 (bs, 1H), 8.29-8.43 (c, 1H), 8.10 (s, 1/2H), 7.95-8.05 (c, 4H), 7.92 (d, 1H, J=3 Hz), 7.88 (d, 1H, J=3 Hz), 7.74 (s, 1/2H), 7.54 (s, 1H), 7.49 (s, 1H), 4.87-4.99 (c, 1/2H), 4.50-4.63 (c, 1/2H), 3.43-3.80 (C, 4H), 1.22 (s, 6H).
13C NMR (DMSO-d6): d 177.2, 177.1, 162.3, 157.1, 155.0, 154.8, 148.7, 144.0, 143.9, 138.0, 137.8, 137.5, 129.2, 128.6, 128.4, 127.9, 127.7, 121.5, 118.4, 57.8, 53.3, 53.0. 51.3, 47.5, 38.8, 38.1, 24.31, 24.30.
MS (ESI(+)) 530 (M+H), 547 (M+NH4), 552 (M+Na), 1076 (2M+NH4), 1081 (2M+Na)
HRMS: CALC: 530.120 FOUND:530.1193
Anal. Calcd for: C22H22F3N3O7S C, 49.90; H, 4.19; N, 7.94;F,10,76;S,6.06.
Found: C,49.58; H, 4.10; N, 7.75;F,11.04:S,5.96.

### Example 116

### N-[1-[4-[(4-pyridinyloxy)phenyl]sulfonyl]ethyl]-N-hydroxyformamide

### Example 116A

### 4-[4-(methylsulfonyl)phenoxy]pyridine

A mixture of4-methylsulfonylphenol (2.93g, 17 mmol) and 4-chloropyridine hydrochloride (2.93g, 19.5 mmol) was heated at 150° C, resullting in a gradual melt, which was stirred at 150° C for 4h, then partitioned between ethyl acetate and 1N NaOH. The organic extract was dried over MgSO4 and concentrated to 1.3 g of a yellow solid. The solid was recrystallized from ethyl acetate-ether to give 0.81g of the titlke compound as a white solid.

### Example 116B

### N-[1-[4-[(4-pyridinyloxy)phenyl]sulfonyl]]ethyl]-N-hydroxyformamide

The tiltle compound was prepared following the procedures of examples 75, except substituting example 116A for 71B and acetaldehyde for propionaldehyde.
mp 180°-181°
1H NMR, 400MHz (DMSO-d6): d 9.71 (bs, 1H), 8.54 (d, 2H, J=3 Hz), 8.05 (s, 1/2H), 7.97 (d, 2H, J=6 Hz), 7.84 (s, 1/2H), 7.49 (d, 2H, J=6 Hz), 7.03-7.13 (c, 2H), 4.63-4.73 (c, 1/2H), 4.28-4.39 (c, 1/2H), 3.59-3.78 (c, 1H),3.48 (dd, 1H, J=3,10.5 Hz), 1.20 (dd, 3H, J=4.5, 10.5 Hz).
13C NMR (DMSO-d6): d 162.62, 162.61, 161.33, 158.33, 158.31, 156.74, 151.77, 135.40, 135.13, 130.74, 130.45, 120.56, 120.34, 113.27, 56.50, 49.69, 45.02, 19.05, 17.71
MS (ESI(+)) 337 (M+H), 359 (M+Na),391 (M+Na+MeOH), 695 (2M+Na)
Anal. Calcd for: C15H16N2O5S•0.5H2O C, 52.16; H, 4.96; N, 8.11;S,9.28.
Found: C,52.32; H. 4.78; N, 7.98;S,9.45.

### Example 117

### N-[1-[[[(4-cyanophenoxy)phenyl]sulfonyl]methyl]-2-(4,4-dimethyl-2,5-dioxo-1-imidazolidinyl)ethyl]-N-hydroxyformamide

The title compound was prepared according to the procedures of example 61, except substituting 4-(4'-cyanophenoxy)benzene thiol for 4-thiol-4-biphenylcarbonitrile and example 23A for example 16B. in example 61A.
¹H NMR (300 MHz, d₆-DMSO) δ 9.70 (s, 0.5H), 9.50 (s, 0.5H), 8.39 (s, 0.5H), 8.34 (s, 0.5H), 8.10 (s, 0.5H), 7.98-7.91 (m, 4H), 7.68 (s, 0.5H), 7.37-7.27 (m, 4H), 4.88-4.77 (m, 0.5H), 4.52-4.41 (m, 0.5H), 3.78-3.39 (m, 4H), 1.24-1.22 (m, 6H);
MS (ESI) m/e 487 (M+1)⁺.
Anal. calcd for C₂₂H₂₂N₄O₇S: C. 54.31; H, 4.56. Found: C. 54.17; H, 4.79.

### Example 118

### N-[1-[[4-[[4-(trifluoromethoxy)phenoxy]phenyl]sulfonyl]methyl]-3-(4,4-dimethyl-2,5-dioxo-1-imidazolidinyl)propyl]-N-hydroxyformamide

The title compound was prepared according to the procedures of example 61, except substituting 4-(4'-trifluoromethoxyphenoxy)benzene thiol for 4'-thiol-4-biphenylcarbonitrile and example 47A for example 16B, in example 61A.
1H NMR (d6-DMSO) 9.96 (s, 0.5H), 9.60 (s, 0.5H), 8.32 (s, 0.5H), 8.23 (s. 0.5H), 8.11 (s, 0.5H), 7.93-7.86 (m, 2H), 7.75 (s, 0.5H), 7.48 (d. 05H, J=8.8 Hz). 7.25 (dd, 4H, J=22.8, 8.8 Hz), 4.53-4.42 (m, 0.5H), 4.04-3.93 (m, 0.5H), 3.65-3.46 (m, 2H), 3.34-3.22 (m, 2H), 2.02-1.62 (m, 2H), 1.26 (s, 3H), 1.23 (s, 3H).
MS (ESI) 560 (M+H), 577 (M+NH4), 582 (M+Na), 558 (M-H).
Anal. Calcd for: C23H24N308SF3C, 49.37; H, 4.32; N, 7.51. Found: C, 49.46; H, 4.23; N, 7.47.

### Example 119

### N-[1-[[4-[[4-(trifluoromethoxy)phenoxy]phenyl]sulfonyl]methyl]-2-(4,4-dimethyl-2,5-dioxo-1-imidazolidinyl)ethyl]-N-hydroxyformamide

The title compound was prepared according to the procedures of example 61, except substituting 4-(4'-trifluoromethoxyphenoxy)benzene thiol for 4'-thiol-4-biphenylcarbonitrile in example 61A.
1H NMR (d6-DMSO) 9.51 (s, 0.5H), 9.70 (s, 0.5H), 8.09 (s. 0.5H), 7.91 (dd, 2H, J=8.9, 3.1 Hz), 7.68 (s, 0.5H), 7.47 (d, 2H, J=9.2 Hz), 7.31-7.21 (m, 4H), 4.90-4.78 (m, 0.5H), 4.51-4.40 (m, 0.5H), 3.74-3.40 (m, 4H), 2.76 (d, 3H. J=1.7 Hz), 1.27-1.22 (m, 6H).
MS (ESI) 558 (M-H), 560 (M+H), 577 (M+NH4), 582 (M+Na).
Anal. Calcd for: C23H24N308SF3C, 49.37; H, 4.32; N, 7.51. Found: C, 49.41; H, 4.29; N, 7.36.

## Claims

1. A compound of formula (I), 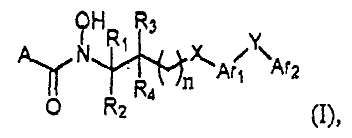 or a pharmaceutically acceptable salt or prodrug thereof, wherein
**A** is hydrogen;
**n** is zero;
**R**_{**1**} is hydrogen or alkyl of one to four carbon atoms;
**R**_{**3**} is selected from the group consisting of
(1) hydrogen and
(2) alkyl of one to four carbon atoms;
**R**_{**4**} is hydrogen;
**R**_{**2**} is selected from the group consisting of
(1) hydrogen;
(2) alkyl of one to four carbon atoms;
(3) hydroxyalkyl, wherein the alkylene group is of one to four carbon atoms;
(4) -(alkylene)-S(O)ₚ-alkyl, wherein the alkylene is of one to four carbon atoms, the alkyl is of one to four carbon atoms, and p is zero or two;
(5) phenyl;
(6) phenylalkoxyalkyl, wherein the alkylene and alkyl groups are independently of one to four carbon atoms;
(7) phenylalkyl, wherein the alkylene group is of one to four carbon atoms;
(8) phenoxyalkyl, wherein the alkylene group is of one to four carbon atoms;
(9) -(alkylene)-N(R₅)SO₂-phenyl, wherein the alkylene is of one to four carbon atoms and wherein R₅ is alkyl of one to two carbon atoms;
(10) (heterocycle)oxyalkyl, wherein the alkylene group is of one to four carbon atoms and the heterocycle is 4-methyl-2-oxo-2H-1-benzopyranyl;
(11) -(alkylene)-heterocycle, wherein the alkylene group is of one to four carbon atoms and the heterocycle is 1,6-dihydro-3-methyl-6-oxo-1-pyridazinyl;
(12) -(alkylene)-NR₆R₇; wherein the alkylene group is of one to four carbon atoms, wherein for (5)-(9), the phenyl and the phenyl parts of the phenylalkoxyalkyl, phenylalkyl, -(alkylene)-N(R₅)SO₂-phenyl, and phenoxyalkyl are optionally substituted with one or two substituents independently selected from the group consisting of
(a) alkyl of one to four carbon atoms,
(b) halo,
(c) -N(R₅)SO₂-alkyl, and
(d) phenyl, wherein the phenyl is optionally substituted with one cyano substituent; and
R₆ and R₇, taken together with the nitrogen atom to which they are attached, define
a group selected from the group consisting of
(1) phthalimidyl;
(2) succinimidyl;
(3) 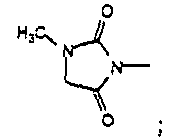
(4) 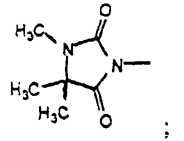
(5) 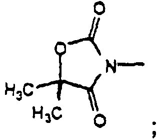
(6) 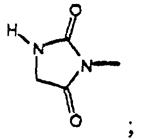
(7) 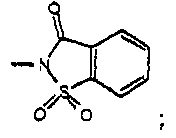
(8) 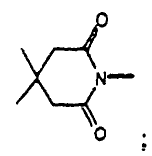 and
(9) 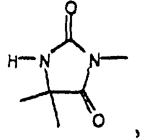
wherein option (2) is optionally substituted with one or two alkyl of one to four carbon atom substituents and option (9) is optionally substituted with one phenylmethyl substituent;
**X** is selected from the group consisting of
(1) -O- and
(2) -S(O)ₚ- wherein p is zero or two;
**Ar**_{**1**} is phenyl which is optionally substituted with one nitro substituent;
**Y** is selected from the group consisting of
(1) a covalent bond,
(2) -O-,
(3) alkenylene of two to four carbon atoms,
(4) piperidinyl, and
(5) -S(O)ₚ-; and
**Ar**_{**2**} is an aryl group selected from the group consisting of
(1) phenyl,
(2) pyridyl,
(3) furyl, and
(4) thienyl,
wherein the aryl group is optionally substituted with one, two, or three substituents independently selected from the group consisting of
(a) alkyl of one to four carbon atoms;
(b) alkoxy of one to four carbon atoms;
(c) alkoxy of one to four carbon atoms substituted with alkoxy of one to four carbon atoms;
(d) cyano;
(e) cyanoalkyl of one to four carbon atoms;
(f) halo;
(g) haloalkyl of one to four carbon atoms;
(h) thioalkoxy of one carbon atom;
(i) perfluoroalkyl of one carbon atom;
(j) perfluoroalkoxy of one carbon atom;
(k) sulfinylalkyl, wherein the alkyl part is of one to four carbon atoms;
(l) sulfonylalkyl, wherein the alkyl part is of one to four carbon atoms; and
(m) 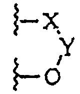
where X is -O-, and Y is -(C(R")₂)ᵥ-, where R" is hydrogen.

2. A compound according to Claim 1 wherein
**R**_{**1**} and **R**_{**3**} are independently selected from the group consisting of hydrogen and methyl;
**R**_{**4**} is hydrogen;
**X** is selected from the group consisting of
(1) -O-, and
(2) -S(O)ₚ-; and
**Ar**_{**1**} is phenyl.

3. A compound according to Claim 2, wherein R₂ is alkyl of one to four carbon atoms.

4. A compound according to Claim 3 selected from the group consisting of
(±)-N-[1-[[[3'-(cyanomethyl)-[1,1'-biphenyl]-4-yl]oxy]methyl]pentyl]-N-hydroxyformamide;
(±)-N-[1-[[(4'-cyano-[1,1'-biphenyl]-4-yl)oxy]methyl]-3-methylbutyl]-N-hydroxyformamide;
(±)-N-[1-[[(4'-cyano-[1,1'-biphenyl]-4-yl)oxy]methylbutyl]-N-hydroxyformamide;
(±)-N-[1-[[(4'-cyano-[1,1'-biphenyl]-4-yl)oxy]methyl]pentyl]-N-hydroxyformamide;
(±)-N-[1-[[(4'-cyano-[1,1'-biphenyl]4-yl)oxy]methyl]ethyl]-N-hydroxyformamide;
(±)-N-[2-[(4'-cyano[1,1'-biphenyl]-4-yl)oxy]-1-methylpropyl]-N-hydroxyformamide;
(±)-N-[1-[[(4'-methoxy[1,1'-biphenyl]-4-yl)sulfonyl]methyl]ethyl]-N-hydroxyformamide;
(±)-N-[1-[[(4'-chloro[1,1'-biphenyl]-4-yl)sulfonyl]methyl]ethyl]-N-hydroxyformamide;
(±)-N-[1-[[[4-(1,3-benzodioxol-5-yl)phenyl]sulfonyl]methyl]ethyl]-N-hydroxyformamide;
(±)-N-[1-[[[4-(4-chlorophenoxy)phenyl]sulfonyl]methyl]ethyl]-N-hydroxyformamide;
(±)-N-[1-[[(4'-methoxy[1,1'-biphenyl]-4-yl)sulfonyl]methyl]propyl]-N-hydroxyformamide;
(±)-N-[1-[1,1-dimethyl-2-[(4'-(trifluoromethyl)[1,1'-biphenyl]-4-yl]sulfonyl]ethyl]-N-hydroxyformamide; and
N-[1-[4-[(4-pyridinyloxy)phenyl]sulfonyl]]ethyl]-N-hydroxyformamide.

5. A compound according to Claim 2, wherein R₂ is hydrogen.

6. A compound according to Claim 5 which is N-[2-[(4'-cyano-[1,1'-biphenyl]-4-yl)oxy]ethyl]-N-hydroxyformamide.

7. A compound according to Claim 2, wherein R₂ is selected from the group consisting of
(1) phenyl, wherein the phenyl group is unsubstituted or substituted;
(2) phenoxyalkyl, wherein the alkylene group is of one to four carbon atoms, and wherein the phenyl group is unsubstituted or substituted;
(3) -(alkylene)-S(O)ₚ-alkyl;
(4) hydroxyalkyl, wherein the alkylene group is of one to four carbon atoms;
(5) -(alkylene)-N(R₅)SO₂-phenyl, wherein the alkylene is of one to four carbon atoms, the phenyl group is unsubstituted or substituted, and R₅ is alkyl of one to two carbon atoms;
(6) phenylalkoxyalkyl, wherein the alkylene and alkyl groups are independently of one to four carbon atoms and the phenyl group is unsubstituted or
substituted;
(7) (heterocycle)oxyalkyl, wherein the alkylene group is of one to four carbon atoms and the heterocycle is unsubstituted or substituted, and
(8) phenylalkyl, wherein the alkylene group is of one to four carbon atoms and
wherein the phenyl group is unsubstituted or substituted.

8. A compound according to Claim 7 selected from the group consisting of
(±)-N-[1-[[(4'-cyano-[[1,1'-biphenyl]-4-yl)oxy]methyl]-2-phenoxyethyl]-N-hydroxyformamide;
(±)-N-[1-[[(4'-cyano-[1,1'-biphenyl]-4-yl)oxy]methyl]-2-(4-methylphenyl)ethyl]-N-hydroxyformamide;
(±)-N-[2-[(4'-cyano-[1,1'-biphenyl]-4-yl)oxy]-1-(4-fluorophenyl)ethyl]-N-hydroxyformamide;
(±)-N-[1-[[(4'-cyano-[1,1'-biphenyl]-4-yl)oxy]methyl]-2-(4-fluorophenyl)ethyl]-N-hydroxyformamide;
(±)-N-[1-[[(3'-cyano[1,1'-biphenyl]-4-yl)oxy]methyl]-2-[methyl[(4-methylphenyl)sulfonyl]amino]ethyl]-N-hydroxyformamide;
(±)-N-[1-[[(4'-cyano[1,1'-biphenyl]-4-yl)oxy]methyl]-5-[(4-methyl-2-oxo-2H-1-benzopyran-6-yl)oxy]pentyl]-N-hydroxyformamide;
(±)-N-[1-[[(4'-cyano[1,1-biphenyl]-4-yl)oxy]methyl]-4-[(4-methyl-2-oxo-2H-1-benzopyran-6-yl)oxy]butyl]-N-hydroxyformamide;
(±)-N-[1-[[(4'-cyano[1,1'-biphenyl]-4-yl)oxy]methyl]-4-[(4-methyl-2-oxo-2H-1-benzopyran-7-yl)oxy]butyl]-N-hydroxyformamide;
(±)-N-[1-[[(4'-cyano[1,1'-biphenyl]-4-yl)oxy]methyl]-5-[(4-methyl-2-oxo-2H-1-benzopyran-7-yl)oxy]pentyl]-N-hydroxyformamide;
(±)-N-[1-[[(4'-cyano-[1,1'-biphenyl]-4-yl)oxy]methyl]-2-isopropylthioethyl]-N-hydroxyformamide;
(±)-N-[1-[(phenylmethoxy)methyl]-2-[[4'-(trifluoromethyl)[1,1'-biphenyl]-4-yl]sulfonyl]ethyl]-N-hydroxyformamide;
(±)-N-[1-(hydroxymethyl)-2-[[(4'-(trifluoromethyl)[1,1'-biphenyl]-4-yl]sulfonyl]ethyl]-N-hydroxyformamide;
(±)-N-(1-[[(4'-methyl[1,1'-biphenyl]-4-yl)oxy]methyl]-2-[[3-(methylsulfonyl)-amino]phenyl]ethyl]-N-hydroxyformamide;
(±)-N-[1-[[[3-(diethylamino)carbonyl]phenyl]methyl]-2-[(4'-methyl[1,1'-biphenyl]-4-yl)oxy]ethyl]-N-hydroxyformamide;
N-[1-[[(4'-cyano[1,1'-biphenyl]-4-yl)oxy]methyl]-2-[(4'-cyano [1,1'-biphenyl]-4-yl)oxy]ethyl]-N-hydroxyformamide;
N-[1-[[(4'-cyano[1,1'-biphenyl]-4-yl)oxy]methyl]-2-(1,6-dihydro-3-methyl-6-oxo-1-pyridazinyl)ethyl]-N-hydroxyformamide; and
N-[1-[[(4'-cyano[1,1'-biphenyl]-4-yl)oxyl]methyl]-2-(1,6-dihydro-6-oxo-1-pyridazinyl)ethyl]-N-hydroxyformamide.

9. A compound according to Claim 2 wherein R₂ is -(alkylene)-NR₆R₇.

10. A compound according to Claim 9 wherein -NR₆R₇ is

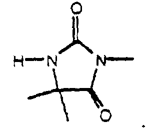

11. A compound according to Claim 10 selected from the group consisting of
(±)-N-[1-[[(4'-butoxy[1,1'-biphenyl]-4-yl)oxy]methyl]-2-(4,4-dimethyl-2,5-dioxo-1-imidazolidinyl)ethyl]-N-hydroxyformamide;
(±)-N-[1-[(4,4-dimethyl-2,5-dioxo-1-imidazolidinyl)methyl]-2-[(4'-ethoxy[1,1'-biphenyl]-4-yl)oxy]ethyl]-N-hydroxyformamide;
(±)-N-[1-[(4-(1,3-benzodioxol-5-yl)phenoxy]methyl]-2-(4,4-dimethyl-2,5-dioxo-1-imidazolidinyl)ethyl]-N-hydroxyformamide:
(±)-N-[1-[(4,4-dimethyl-2,5-dioxo-1-imidazolidinyl)methyl]-2-[[4'-(trifluoromethoxy)[1,1'-biphenyl]-4-yl]oxy]ethyl]-N-hydroxyformamide;
(±)-N-[1-[(4,4-dimethyl-2,5-dioxo-1-imidazolidinyl)methyl]-2-[(4'-(trifluoromethyl)[1,1'-biphenyl]-4-yl]oxy]ethyl]-N-hydroxyformamide;
(±)-N-[1-[[[3'-(cyanomethyl)[1,1'-biphenyl]-4-yl]oxy]methyl]-3-(4,4-dimethyl-2,5-dioxo-1-imidazolidinyl)propyl]]-N-hydroxyformamide;
(±)-N-[1-[[(4'-butoxy[1,1'-biphenyl]-4-yl)sulfonyl]methyl]-2-(4,4-dimethyl-2,5-dioxo-1-imidazolidinyl)ethyl]-N-hydroxyformamide;
(±)-N-[1-[[(4'-cyano[1,1'-biphenyl]-4-yl)oxy]methyl]-3-(4,4-dimethyl-2,5-dioxo-1-imidazolidinyl)propyl]-N-hydroxyformamide;
(±)-N-[1-[[[4'-(methylsulfonyll)[1,1'-biphenyl]-4-yl]oxy]methyl]-2-(4,4-dimethyl-2,5-dioxo-1-imidazolidinyl)ethyl]-N-hydroxyformamide;
(±)-N-[1-[[(4'-cyano[1,1'-biphenyl]-4-yl)sulfonyl]methyl]-2-(4,4-dimethyl-2,5-dioxo-1-imidazolidinyl)ethyl]-N-hydroxyformamide;
(±)-N-[1-[(4,4-dimethyl-2,5-dioxo-1-imidazolidinyl)methyl]-2-[[4'-(trifluoromethyl)[1,1'-biphenyl]-4-yl]thio]ethyl]-N-hydroxyformamide;
(±)-N-[1-[(4,4-dimethyl-2,5-dioxo-1-imidazolidinyl)methyl]-2-[[4'-(trifluoromethyl)[1,1'-biphenyl]-4-yl]sulfonyl]ethyl]-N-hydroxyformamide;
(±)-N-[1-[[[4-(4-chlorophenoxy)phenyl]sulfonyl]methyl]-2-(4,4-dimethyl-2,5-dioxo-1-imidazolidinyl)ethyl]-N-hydroxyformamide;
(±)-N-[1-[[(4'-butyl[1,1'-biphenyl]4-yl)oxy]methyl]-2-(4,4-dimethyl-2,5-dioxo-1-imidazolidinyl)ethyl]-N-hydroxyformamide;
(±)-N-[1-[[[3'-(cyanomethyl)[1,1'-biphenyl]-4-yl]oxy)methyl]-2-(4,4-dimethyl-2,5-dioxo-imidazolidinyl)ethyl]-N-hydroxyformamide;
(±)-N-[1-[[(4'-cyano[1,1'-biphenyl]4-yl)oxy]methyl]-2-(4,4-dimethyl-2,5-dioxo-1-imidazolidinyl)ethyl]-N-hydroxyformamide;
(±)-N-[1-[(4,4-dimethyl-2,5-dioxo-1-imidazolidinyl)methyl]-2-[[4'-(2-methoxyethoxy)[1,1'-biphenyl]-4-yl]oxy]ethyl]-N-hydroxyformamide;
(±)-N-[1-[(4,4-dimethyl-2,5-dioxo-1-imidazolidinyl)methyl]-2-[(4'-propoxy[1,1'-biphenyl]-4-yl)oxy]ethyl]-N-hydroxyformamide;
N-[1-[(4,4-dimethyl-2,5-dioxo-1-imidazolidinyl)methyl]-2-[(4'-pentyloxy[1,1'-biphenyl]-4-yl)oxy]ethyl]-N-hydroxyformamide;
(±)-N-[1-[[[3'-(cyanomethyl)[1,1'-biphenyl]-4-yl]sulfonyl]methyl]-3-(4,4-dimethyl-2,5-dioxo-1-imidazolidinyl)propyl]-N-hydroxyformamide;
(±)-N-(1-[[(4'-cyano[1,1'-biphenyl]-4-yl)sulfonyl]methyl]-2-(4,4-dimethyl-2,5-dioxo-1-imidazolidinyl)ethyl]-N-hydroxyformamide;
(±)-N-[1-[[[4-(4-fluorophenoxy)phenyl]sulfonyl]methyl]-2-(4,4-dimethyl-2,5-dioxo-1-imidazolidinyl)ethyl]-N-hydroxyformamide
(S)-N-[1-[(4,4-dimethyl-2,5-dioxo-1-imidazolidinyl)methyl]-2-[[4'-(trifluoromethoxy)[1,1'-biphenyl]-4-yl]oxy]ethyl]-N-hydroxyformamide;
(R)-N-[1-[(4,4-dimethyl-2,5-dioxo-1-imidazolidinyl)methyl]-2-[[4'-(trifluoromethoxy)[1,1'-biphenyl]-4-yl]oxy]ethyl]-N-hydroxyformamide;
N-[1-[[[4'-(trifluoromethoxy))[1,1'-biphenyl]-4-yl]oxy]methyl]-3-(4,4-dimethyl-2,5-dioxo-1-imidazolidinyl)propyl]-N-hydroxyformamide;
N-[1-[4-[(4-pyridinylthio)phenoxy]methyl]-2-(4,4-dimethyl-2,5-dioxo-1-imidazolidinyl)ethyl]-N-hydroxyformamide;
N-[1-[[[(4-chlorophenoxy)phenyl]sulfonyl]methyl]-3-(4,4-dimethyl-2,5-dioxo-1-imidazolidinyl)propyl]-N-hydroxyformamide;
N-[1-[[[4'-(trifluoromethoxy)[1,1'-biphenyl]-4-yl]sulfonyl]methyl]-2-(4,4-dimethyl-2,5-dioxo-1-imidazolidinyl)ethyl]-N-hydroxyformamide;
N-[1-[[[(4-cyanophenoxy]phenyl]sulfonyl]methyl]-2-(4,4-dimethyl-2,5-dioxo-1-imidazolidinyl)ethyl]-N-hydroxyformamide; and
N-[1-[[4-[[4-(trifluoromethoxy)phenoxy]phenyl]sulfonyl]methyl]-3-(4,4-dimethyl-2,5-dioxo-1-imidazolidinyl)propyl]-N-hydroxyformamide.

12. A compound according to Claim 9 selected from the group consisting of
(±)-N-[1-[[(4'-cyano-[1,1'-biphenyl]-4-yl)oxy]methyl]-2-(2,3-dihydro-1,3-dioxo-1H-isoindol-2-yl)ethyl]-N-hydroxyformamide;
(±)-N-[1-[[(4'-cyano-[1,1'-biphenyl]-4-yl)oxy]methyl]-2-(3,4,4-trimethyl-2,5-dioxoimidazolidin-1-yl)ethyl]-N-hydroxyformamide;
(±)-N-[1-[[(4'-cyano-[1,1'-biphenyl]-4-yl)oxy]methyl]-3-(3,4,4-trimethyl-2,5-dioxoimidazolidiny-1-yl)propyl]-N-hydroxyformamide;
(±)-N-[1-[4-[(2-E-phenylethenyl)phenoxy]methyl]-2-(3,4,4-trimethyl-2,5-dioxo-1-imidazolidinyl)ethyl]-N-hydroxyformamide;
(±)-N-[1-[[4-(2-furanyl)phenoxy]methyl]-2-(3,4,4-trimethyl-2,5-dioxo-1-imidazolidinyl)ethyl]-N-hydroxyformamide;
(±)-N-[1-[[(4'-butoxy[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(4'-fluoro[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[(3,4,4-trimethyl-2,5-dioxo-1-imidazolidinyl)methyl]-2-[[4'-(trifluoro methyl)[1,1'-biphenyl]-4-yl]oxy]ethyl]-N-hydroxyformamide;
(±)-N-[1-[[(4'-methoxy[1,'-biphenyl]-4-yl)oxy]methyl]-2-(3,4,4-trimethyl-2,5-dioxo-1-imidazolidinyl)ethyl]-N-hydroxyformamide;
(±)-N-[1-[[(4'-methyl[1,1'-biphenyl]-4-yl)oxy]methyl]-2-(3,4,4-trimethyl-2,5-dioxo-1-imidazolidinyl)ethyl]-N-hydroxyformamide:
(±)-N-[1-[[(4'-butoxy[1,1'-biphenyl]-4-yl)oxy]methyl]-2-(3-methy-2,5-dioxo-1-imidazolidinyl)ethyl]-N-hydroxyformamide;
(±)-N-[1-[[4-(3-thienyl)phenoxy]methyl]-2-(3,4,4-trimethyl-2,5-dioxo-1-imidazolidinyl)ethyl]-N-hydroxyformamide;
(±)-N-[1-[[([1,1'-biphenyl]-4-yl)oxy]methyl]-2-(3,4,4-trimethyl-2,5-dioxo-1-midazolidinyl)ethyl]-N-hydroxyformamide;
(±)-N-[1-[[(3'-chloro-4'-fluoro[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(2'-methyl[1,1'-biphenyl]-4-yl)oxy]methyl]-2-(3,4,4-trimethy-2,5-dioxo-1-imidazolidinyl)ethyl]-N-hydroxyformamide;
(±)-N-[1-[[(4'-cyanol[1,1'-biphenyl]-4-yl)oxy]methyl]-2-(2,5-dioxo-1-imidazolidinyl)ethyl]-N-hydroxyformamide;
(±)-N-[1-[[(4'-cyanol[1,1'-biphenyl]-4-yl)oxy]methyl]-2-(1,1-dioxido-3-oxo-1,2-benzisothiazol-2(3H)-yl)ethyl]-N-hydroxyformamide;
(±)-N-[1-[[4-(4-phenyl-1-piperidinyl)phenoxy]methyl]-2-(3,4,4-trimethyl-2,5-dioxo-1-imidazolidinyl)ethyl]-N-hydroxyformamide;
(±)-N-[1-[[(4'-cyano[1,1'-biphenyl]-4-yl)oxy]methyl]-2-[4,4-dimethyl-2,5-dioxo-3-(3-pyridinylmethyl)-1-imidazolidinyl]ethyl]-N-hydroxyformamide;
(±)-N-[1-[[(3'-cyano[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[[4'-(methylthio)[1,1'-biphenyl]-4-yl]oxy]methyl]-2-(3,4,4-trimethyl-2,5-dioxo-1-imidazolidinyl)ethyl]-N-hydroxyformamide;
(±)-N-[1-[4-[[4-(trifluoromethyl)phenoxy]phenoxy]methyl]-2-(3,4,4-trimethyl-2,5-dioxo-1-imidazolidinyl)ethyl]-N-hydroxyformamide;
(±)-N-[1-[[[4'-(trifluoromethoxy)[1,1'-biphenyl]-4-yl]oxy]methyl]-2-(3,4,4-trimethy-2,5-dioxo-1-imidazolidinyl)ethyl]-2-N-hydroxyformamide;
(±)-N-[1-[[[4'-(methylsulfonyl)[1,1'-biphenyl]-4-yl]oxy]methyl]-2-(3,4,4-trimethy-2,5-dioxo-1-imidazolidinyl)ethyl]-N-hydroxyformamide;
(±)-N-[1-[[[(3'-(cyanomethyl])-4'-methoxyl(1,1'-biphenyl]-4-yl]oxy]methyl]-2-(3,4,4-trimethy-2,5-dioxo-1-imidazolidinyl)ethyl]-N-hydroxyformamide;
(±)-N-[1-[[(3'-cyano[1,1'-biphenyl]-4-yl)oxy]methyl]-2-(2,5-dioxo-1-pyrrolidinyl)ethyl]-N-hydroxyformamide;
(±)-N-[1-[[(4'-cyano[1,1'-biphenyl]-4-yl)oxy]methyl]-2-(4,4-dimethyl-2,6-dioxo-1-piperidinyl)ethyl]-N-hydroxyformamide;
N-[1S-[[(4'-cyano[1,1'-biphenyl]-4-yl)oxy]methyl]-2-(2,5-dioxo-1-pyrrolidinyl)ethyl]-N-hydroxyformamide;
N-[1R-[[(4'-cyano[1,1'-biphenyl]-4-yl)oxy]methyl]-2-(2,5-dioxo-1-pyrrolidinyl)ethyl]-N-hydroxyformamide,
(±)-N-[1-[[(4'-cyano[1,1'-biphenyl]-4-yl)oxy]methyl]-2-(3-ethyl-3-methyl-2,5-dioxo-1-pyrrolidinyl)ethyl]-N-hydroxyformamide;
(±)-N-[1-[[(4'-cyano[1,1'-biphenyl]-4-yl)oxy]methyl]-2-(5.5-dimethyl-2,4-dioxo-3-oxazolidinyl)ethyl]-N-hydroxyformamide;
(±)-N-[1-[[(4'-cyano[1,1'-biphenyl]-4-yl)sulfonyl]methyl]-2-(3,4,4-trimethy-2,5-dioxo-1-imidazolidinyl)ethyl)-N-hydroxyformamide;
(±)-N-[1-[[(4'-cyano[1,1'-biphenyl]-4-yl)oxy]methyl]-2-(3-methyl-2,5-dioxo-1-imidazolidinyl)ethyl]-N-hydroxyformamide;
(±)-N-[1-[[(4'-chloro-[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(3'-cyanomethyl-[1,1'-biphenyl]-4-yl)oxy]methyl]-2-(3,5,5-trimethyl-2,4-dioxo-1-imidazolidinyl)propyl]-N-hydroxyformamide;
(±)-N-[1-[[(3'-cyanomethyl-[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(4'-cyano-[1,1'-biphenyl]-4-yl)oxy]methyl]-2-(3-ethyl-4,4-dimethyl-2,5-dioxo-1-imidazolidinyl)ethyl]-N-hydroxyformamide;
(±)-N-[1-[[(4'-cyano-[1,1'-biphenyl]-4-yl)oxy]methyl]-2-(3-benzyl-4,4-dimethyl-2,5-dioxo-1-imidazolidinyl)ethyl]-N-hydroxyformamide,
(±)-N-[1-[[(4'-cyano-[1,1'-biphenyl]-4-y)oxy]methyl]-2-(3,5,5-trimethyl-2,4-dioxo-1-imidazolidinyl)ethyl]-N-hydroxyformamide;
(±)-N-[1-[(2,5-dioxo-1-imidazolidinyl)methyl]-2-[[4'-(trifluoromethoxy)[1,1'-biphenyl]-4-yl]oxy]ethyl]-N-hydroxyformamide;
(±)-N-[1-[[[4'-(trifluoromethyl)[1,1'-biphenyl]-4-yl]sulfonyl]methyl]-2-(3,4,4-trimethyl-2,5-dioxo-1-imidazolidinyl)ethyl]-N-hydroxyformamide;
(±)-N-[1-[[(4'-butyl(1,1'-biphenyl]-4-yl)oxy]methyl]-2-(3-methy-2,5-dioxo-1-imidazolidinyl)ethyl]-N-hydroxyformamide;
(±)-N-[1-[(3-methy-2,5-dioxo-1-imidazolidinyl)methyl]-2-[[4'-(trifluoromethoxy)[1,1'-biphenyl]-4-yl]oxy]ethyl]-N-hydroxyformamide;
(±)-N-[1-[[[4-(4-chlorophenoxy)phenyl]sulfonyl]methyl]-2-(3,4,4-trimethyl-2,5-dioxo-1-imidazolidinyl)ethyl]-N-hydroxyformamide;
(±)-N-[1-[[(4-butyl[1,1'-biphenyl]-4-yl)sulfonyl]methyl]-2-(3,4,4-trimethyl-2,5-dioxo-1-imidazolidinyl)ethyl]-N-hydroxyformamide;
(±)-N-[1-[4-(2-thienyl)phenoxy)methyl]-2-[1-(3,4,4-trimethyl-2,5-dioxo-1-imidazolidinyl)ethyl]-N-hydroxyformamide;
(±)-N-[1-[[(3-nitro[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[[4'-(trifluoromethoxy)[1,1'-biphenyl]-4-yl]sulfonyl]methyl]-2-(3,4,4-trimethy-2,5-dioxo-1-imidazolidinyl)ethyl]-N-hydroxyformamide;
(±)-N-[1-[[(4'-cyano[1,1'-biphenyl]-4-yl)sulfonyl]methyl]-2-(3-methy-2,5-dioxo-1-imidazolidinyl)ethyl]-N-hydroxyformamide;
(±)-N-[1-[[[3'-(cyanomethyl)[1,1'-biphenyl]-4-yl]sulfonyl]methyl]-2-(3,4,4-trimethyl-2,5-dioxo-1-imidazolidinyl)ethyl]-N-hydroxyformamide;
(±)-N-[1-[[4-(4-pyridinyl)phenoxy]methyl]-2-(3,4,4-trimethy-2,5-dioxo-1-imidazolidinyl)ethyl]-N-hydroxyformamide;
N-[1-[[[4'-(aminosulfonyl)[1,1'-biphenyl]-4-yl]oxy]methyl]-2-(3,4,4-trimethyl-2,5-dioxo-1-imidazolidinyl)ethyl]-N-hydroxyformamide; and
N-[1-[[4-[[4-(trifluoromethoxy)phenoxy]phenyl]sulfonyl]methyl]-2-(4,4-dimethyl-2,5-dioxo-1-imidazolidinyl)ethyl]-N-hydroxyformamide.

13. A compound according to claim 1 wherein **R**_{**1**} and **R**_{**2,**}, taken together with the carbon atom to which they are attached, form a tetrahydropyranyl ring.

14. A compound according to claim 13 selected from the group consisting of
N-[4-[4-[[(4-chlorophenoxy)phenyl]sulfonyl]methyl]tetrahydro-2H-pyran-4-yl]-N-hydroxyformamide and
N-[4-[4-[(4'-chloro[1,1'-biphenyl]-4-yl)sulfonyl]methyl]tetrahydro-2H-pyran-4-yl]-N-hydroxyformamide.

15. A compound selected from the group consisting of
(±)-N-[1-[[(4'-cyano-[1,1'-biphenyl]-4-yl)oxy]methyl]-2-phenoxyethyl]-N-hydroxyformamide;
(±)-N-[1-[[(4'-cyano-[1,1'-biphenyl]-4-yl)oxy]methyl]-2-(2,3-dihydro-1,3-dioxo-1H-isoindol-2-yl)ethyl]-N-hydroxyformamide;
(±)-N-[1-[[(4'-cyano-[1,1'-biphenyl]-4-yl)oxy]methyl]-2-(3,4,4-trimethyl-2,5-dioxoimidazolidin-1-yl)ethyl]-N-hydroxyformamide;
(±)-N-[1-[[(4'-cyano-[1,1'-biphenyl]-4-yl)oxy]methyl]-3-(3,4,4-trimethyl-2,5-dioxoimidazolidin-1-yl)propyl]-N-hydroxyformamide;
(±)-N-[1-[[[3'-(cyanomethyl)-[1,1'-biphenyl]-4-yl]oxy]methyl]pentyl]-N-hydroxyformamide;
(±)-N-[1-[[(4'-cyano-[1,1'-biphenyl]-4-yl)oxy]methyl]-3-methylbutyl]-N-hydroxyformamide;
(±)-N-[1-[[(4'-cyano-[1,1'-biphenyl]-4-yl)oxy]methyl]-2-methylbutyl]-N-hydroxyformamide;
(±)-N-[1-[[(4'-cyano-[1,1'-biphenyl]-4-yl)oxy]methyl]pentyl]-N-hydroxyformamide;
(±)-N-[1-[[(4'-cyano-[1,1'-biphenyl]-4-yl)oxy]methyl]-2-4-methylphenyl)ethyl]-N-hydroxyformamide;
(±)-N-[2-[(4'-cyano-[1,1'-biphenyl]-4-yl)oxy]-1-(4-fluorophenyl)ethyl]-N-hydroxyformamide;
(±)-N-[1-[[(4'-cyano-[1,1'-biphenyl]-4-yl)oxy]methyl]-2-(4-fluorophenyl)ethyl]-N-hydroxyformamide;
(±)-N-[1-[[(4'-cyano-[1,1 '-biphenyl]-4-yl)oxy]methyl]ethyl]-N-hydroxyformamide;
(±)-N-[2-[(4'-cyano-[1,1'-biphenyl]-4-yl)oxy]ethyl]-N-hydroxyformamide;
(±)-N-[1-[4-[(2E-phenylethenyl)phenoxy]methyl]-2-(3,4,4-trimethyl-2,5-dioxo-1-imidazolidinyl)ethyl]-N-hydroxyformamide;
(±)-N-[1-[[4-(2-furanyl)phenoxy]methyl]-2-(3,4,4-trimethyl-2,5-dioxo-1-imidazolidinyl)ethyl]-N-hydroxyformamide;
(±)-N-[1-[[(4'-butoxy[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(4'-fluoro[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[(3,4,4-trimethyl-2,5-dioxo-1-imidazolidinyl)methyl]-2-[[4'-(trifluoromethyl)[1,1'-biphenyl]-4-yl]oxy]ethyl]-N-hydroxyformamide;
(±)-N-[1-[[(4'-methoxy[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(4'-methyl[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(4'-butoxy[1,1'-biphenyl]-4-yl)oxy]methyl]-2-(4,4-dimethyl-2,5-dioxo-1-imidazolidinyl)ethyl]-N-hydroxyformamide;
(±)-N-[1-[(4,4-dimethyl-2,5-dioxo-1-imidazolidinyl)methyl]-2-[(4'-ethoxy[1,1'-biphenyl]-4-yl)oxy]ethyl]-N-hydroxyformamide;
(±)-N-[1-[[4-(1,3-benzodioxol-5-yl)phenoxy]methyl]-2-(4,4-dimethyl-2,5-dioxo-1-imidazolidinyl)ethyl]-N-hydroxyformamide;
(±)-N-[1-[[(4'-butoxy[1,1'-biphenyl]-4-yl)oxy]methyl]-2-(3-methy-2,5-dioxo-1-imidazolidinyl)ethyl]-N-hydroxyformamide;
(±)-N-[1-[[4-(3-thienyl)phenoxy]methyl]-2-(3,4,4-trimethyl-2,5-dioxo-1-imidazolidinyl)ethyl]-N-hydroxyformamide;
(±)-N-[1-[[([1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(3'-chloro-4'-fluoro[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(2'-methyl[1,1'-biphenyl]-4-yl)oxy]methyl]-2-(3,4,4-trimethy-2,5-dioxo-1-imidazolidinyl)ethyl]-N-hydroxyformamide;
(±)-N-[1-[[(4'-cyanol[1,1'-biphenyl]4-yl)oxy]methyl]-2-(2,5-dioxo-1-imidazolidinyl)ethyl]-N-hydroxyformamide;
(±)-N-[1-[[(4'-cyanol[1,1'-biphenyl]-4-yl)oxy]methyl]-2-(1,1-dioxido-3-oxo-1,2-benzisothiazol-2(3H)-yl)ethyl]-N-hydroxyformamide;
(±)-N-[1-[(4,4-dimethyl-2,5-dioxo-1-imidazolidinyl)methyl]-2-[[4'-(trifluoromethoxy)[1,1'-biphenyl]-4-yl)oxy]ethyl]-N-hydroxyformamide;
(±)-N-[1-[[4-(4-phenyl-1-piperidinyl)phenoxy]methyl]-2-(3,4,4-trimethyl-2,5-dioxo-1-imidazolidinyl)ethyl]-N-hydroxyformamide;
(±)-N-[1-[(4,4-dimethyl-2,5-dioxo-1-imidazolidinyl)methyl]-2-[[4'-(trifluoromethyl)[1,1'-biphenyl]-4-yl]oxy]ethyl]-N-hydroxyformamide;
(±)-N-[1-[[(3'-cyano[1,1'-biphenyl]-4-yl)oxy]methyl]-2-[methyl[(4-methylphenyl)sulfonyl]amino]ethyl]-N-hydroxyformamide;
(±)-N-[1-[[(4'-cyano[1,1'-biphenyl]-4-yl)oxy]methyl]-2-[4,4-dimethyl-2,5-dioxo-3-(3-pyridinylmethyl)-1-imidazolidinyl]ethyl]-N-hydroxyformamide;
(±)-N-[2-[(4'-cyano[1,1'-biphenyl]-4-yl)oxy]-1-methylpropyl]-N-hydroxyformamide;
(±)-N-[1-[[(3'-cyano[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[[4'-(methylthio)[1,1'-biphenyl]-4-yl]oxy]methyl]-2-(3,4,4-trimethyl-2,5-dioxo-1-imidazolidinyl)ethyl]-N-hydroxyformamide;
(±)-N-[1-[4-[[4-(trifluoromethyl)phenoxy]phenoxy]methyl]-2-(3,4,4-trimethyl-2,5-dioxo-1-imidazolidinyl)ethyl]-N-hydroxyformamide;
(±)-N-[1-[[[4'-(trifluoromethoxy)[1,1'biphenyl]-4-yl]oxy]methyl]-2-(3,4,4-trimethy-2,5-dioxo-1-imidazolidinyl)ethyl]-2-N-hydroxyformamide;
(±)-N-[1-[[[4'-(methylsulfonyl)[1,1'-biphenyl]-4-yl]oxy]methyl]-2-(3,4,4-trimethy-2,5-dioxo-1-imidazolidinyl)ethyl]-N-hydroxyformamide;
(±)-N-[1-[[[3'-(cyanomethyll)-4'-methoxyl[1,1'-biphenyl]-4-yl]oxy]methyl]-2-(3,4,4-trimethy-2,5-dioxo-1-imidazolidinyl)ethyl]-N-hydroxyformamide;
(±)-N-[1-[[[3'-(cyanomethyl)[1,1-biphenyl]-4-yl]oxy]methyl]-3-(4,4-dimethyl-2,5-dioxo-1-imidazolidinyl)propyl]]-N-hydroxyformamide;
(±)-N-[1-[[(4'-butoxy[1,1'-biphenyl]-4-yl)sulfonyl]methyl]-2-(4,4-dimethyl-2,5-dioxo-1-imidazolidinyl)ethyl]-N-hydroxyformamide;
(±)-N-[1-[[(4'-cyano[1,1'-biphenyl]-4-yl)oxy]methyl]-3-(4,4-dimethyl-2,5-dioxo-1-imidazolidinyl)propyl]-N-hydroxyformamide;
(±)-N-[1-[[[4'-(methylsulfonyl)[1,1'-biphenyl]-4-yl]oxy]methyl]-2-(4,4-dimethyl-2,5-dioxo-1-imidazolidinyl)ethyl]-N-hydroxyformamide;
(±)-N-[1-[[(3'-cyano[1,1'-biphenyl)-4-yl)oxy]methyl]-2-(2,5-dioxo-1-pyrrolidinyl)ethyl]-N-hydroxyformamide;
(±)-N-[1-[[(4'-cyano[1,1'-biphenyl]-4-yl)oxy]methyl]-2-(4,4-dimethyl-2,6-dioxo-1-piperidinyl)ethyl]-N-hydroxyformamide;
(±)-N-[1S-[[(4'-cyano[1,1'-biphenyl]-4-yl)oxy]methyl]-2-(2,5-dioxo-1-pyrrolidinyl)ethyl]-N-hydroxyformamide;
(±)-N-[1R-[[(4'-cyano[1,1'-biphenyl]-4-yl)oxy]methyl]-2-(2,5-dioxo-1-pyrrolidinyl)ethyl]-N-hydroxyformamide;
(±)-N-[1-[[(4'-cyano[1,1'-biphenyl]-4-yl)oxy]methyl]-2-(3-ethyl-3-methyl-2,5-dioxo-1-pyrrolidinyl)ethyl]-N-hydroxyformamide;
N-[4-[4-[[(4-chlorophenoxy)phenyl]sulfonyl]methyl]tetrahydro-2H-pyran-4-yl]-N-hydroxyformamide;
(±)-N-[1-[[(4'-cyano[1,1'-biphenyl]-4-yl)oxy]methyl]-5-[(4-methyl-2-oxo-2H-1-benzopyran-6-yl)oxy]pentyl]-N-hydroxyformamide;
(±)-N-[1-[[(4'-cyano[1,1'-biphenyl]-4-yl)oxy]methyl]-4-[(4-methyl-2-oxo-2H-1-benzopyran-6-yl)oxy]butyl]-N-hydroxyformamide;
(±)-N-[1-[[(4'-cyano[1,1'-biphenyl]-4-yl)oxy]methyl]-4-[(4-methyl-2-oxo-2H-1-benzopyran-7-yl)oxy]butyl]-N-hydroxyformamide;
(±)-N-[1-[[(4'-cyano[1,1'-biphenyl]-4-yl)oxy]methyl]-5-[(4-methyl-2-oxo-2H-1-benzopyran-7-yl)oxy]pentyl]-N-hydroxyformamide;
(±)-N-[1-[[(4'-cyano[1,1'-biphenyl]-4-yl)oxy]methyl]-2-(5,5-dimethyl-2,4-dioxo-3-oxazolidinyl)ethyl]-N-hydroxyformamide;
(±)-N-[1-[[(4'-cyano[1,1'-biphenyl]-4-yl)sulfonyl]methyl]-2-(3,4,4-trimethy-2,5-dioxo-1-imidazolidinyl)ethyl]-N-hydroxyformamide;
(±)-N-[1-[[(4'-cyano[1,1'-biphenyl]-4-yl)oxy]methyl]-2-(3-methyl-2,5-dioxo-1-imidazolidinyl)ethyl]-N-hydroxyformamide;
(±)-N-[1-[[(4'-cyano[1,1'-biphenyl]-4-yl)sulfonyl]methyl]-2-(4,4-dimethyl-2,5-dioxo-1-imidazolidinyl)ethyl]-N-hydroxyformamide;
(±)-N-[1-[[(4'-chloro-[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(3'-cyanomethyl-[1,1'-biphenyl]-4-y)oxy]methyl]-2-(3,5,5-trimethyl-2,4-dioxo-1-imidazolidinyl)propyl]-N-hydroxyformamide;
(±)-N-[1-[[(4'-cyano-[1,1'-biphenyl]-4-y)oxy]methyl]-2-isopropylthioethyl]-N-hydroxyformamide;
(±)-N-[1-[[(3'-cyanomethyl-[1,1'-biphenyl]-4-y)oxy]methyl]-2-(3,4,4-trimethyl-2,5-dioxo-1-imidazolidinyl)ethyl]-N-hydroxyformamide;
(±)-N-[1-[[(4'-cyano-[1,1'-biphenyl]-4-yl)oxy]methyl]-2-(3-ethyl-4,4-dimethyl-2,5-dioxo-1-imidazolidinyl)ethyl]-N-hydroxyformamide;
(±)-N-[1-[[(4'-cyano-[1,1'-biphenyl]-4-yl)oxy]methyl]-2-(3-benzyl-4,4-dimethyl-2,5-dioxo-1-imidazolidinyl)ethyl]-N-hydroxyformamide;
(±)-N-[1-[[(4'-cyano-[1,1'-biphenyl]-4-y)oxy]methyl]-2-(3,5,5-trimethyl-2,4-dioxo-1-imidazolidinyl)ethyl]-N-hydroxyformamide;
(±)-N-[1-[[4'-methoxy[1,1'-biphenyl]-4-yl)sulfonyl]methyl]ethyl]-N-hydroxyformamide;
(±)-N-[1-[[(4'-chloro[1,1'-biphenyl]-4-yl)sulfonyl]methyl]ethyl]-N-hydroxyformamide;
(±)-N-[1-[[[4-(1,3-benzodioxol-5-yl)phenyl]sulfonyl]methyl]ethyl]-N-hydroxyformamide;
(±)-N-[1-[[[4-(4-chlorophenoxy)phenyl]sulfonyl]methyl]ethyl]-N-hydroxyformamide;
(±)-N-[1-[[(4'-methoxy[1,1'-biphenyl]-4-yl)sulfonyl]methyl]propyl]-N-hydroxyformamide;
(±)-N-[1-[1,1-dimethyl-2-[(4'-(trifluoromethyl)[1,1'-biphenyl]-4-yl]sulfonyl]ethyl]-N-hydroxyformamide;
(±)-N-[1-[(phenylmethoxy)methyl]-2-[[4'-(trifluoromethyl)[1;1'-biphenyl]-4-yl]sulfonyl]ethyl]-N-hydroxyformamide;
(±)-N-[1-(hydroxymethyl)-2-[[(4'-(trifluoromethyl)[1,1'-biphenyl]-4-yl]sulfonyl]ethyl]-N-hydroxyformamide;
(±)-N-[1-[(4,4-dimethyl-2,5-dioxo-1-imidazolidinyl)methyl]-2-[[4'-(trifluoromethyl)[1,1'-biphenyl]-4-yl]thio]ethyl]-N-hydroxyformamide;
(±)-N-[1-[(4,4-dimethyl-2,5-dioxo-1-imidazolidinyl)methyl]-2-[[4'-(trifluoromethyl)[1,1'-biphenyl]-4-yl]sulfonyl]ethyl]-N-hydroxyformamide;
(±)-N-[1-[(2,5-dioxo-1-imidazolidinyl)methyl]-2-[[4'-(trifluoromethoxy)[1,1'-biphenyl] -4-yl]oxy]ethyl]-N-hydroxyformamide;
(±)-N-[1-[[[4'-(trifluoromethyl)[1,1'-biphenyl]-4-yl]sulfonyl]methyl]-2-(3,4,4-trimethyl-2,5-dioxo-1-imidazolidinyl)ethyl]-N-hydroxyformamide;
(±)-N-[1-[[(4'-butyl[1,1'-biphenyl]-4-yl)oxy]methyl]-2-(3-methy-2,5-dioxo-1-imidazolidinyl)ethyl]-N-hydroxyformamide;
(±)-N-[1-[(3-methy-2,5-dioxo-1-imidazolidinyl)methyl]-2-[[4'-(trifluoromethoxy)[1,1'-biphenyl]-4-yl]oxy]ethyl]-N-hydroxyformamide;
N-[4-[4-[(4'-chloro[1,1'-biphenyl]-4-yl)sulfonyl]methyl]tetrahydro-2H-pyran-4-yl]-N-hydroxyformamide;
(±)-N-[1-[[[4-(4-chlorophenoxy)phenyl]sulfonyl]methyl]-2-(4,4-dimethyl-2,5-dioxo-1-imidazolidinyl)ethyl]-N-hydroxyformamide;
(±)-N-[1-[[[4-(4-chlorophenoxy)phenyl]sulfonyl]methyl]-2-(3,4,4-trimethyl-2,5-dioxo-1-imidazolidinyl)ethyl]-N-hydroxyformamide;
(±)-N-[1-[[(4-butyl[1,1'-biphenyl]-4-yl)sulfonyl]methyl]-2-(3,4,4-trimethyl-2,5-dioxo-1-imidazolidinyl)ethyl]-N-hydroxyformamide;
(±)-N-[1-[[(4'-butyl[1,1'-biphenyl]-4-yl)oxy]methyl]-2-(4,4-dimethyl-2,5-dioxo-1-imidazolidinyl)ethyl]-N-hydroxyformamide;
(±)-N-[1-[[[3'-(cyanomethyl)[1,1'-biphenyl]-4-yl]oxy]methyl]-2-(4,4-dimethyl-2,5-dioxo-1-imidazolidinyl)ethyl]-N-hydroxyformamide;
(±)-N-[1-[4-(2-thienyl)phenoxy]methyl]-2-[1-(3,4,4-trimethyl-2,5-dioxo-1-imidazolidinyl)ethyl]-N-hydroxyformamide;
(±)-N-[1-[[(3-nitro[1,1'-biphenyl]-4-yl)oxy]methyl]-2-(3,4,4-trimethyl-2,5-dioxo-1-imidazolidinyl)ethyl]-N-hydroxyformamide;
(±)-N-[1-[[(4'-methyl[1,1'-biphenyl]-4-yl)oxy]methyl]-2-[[3-(methylsulfonyl)-amino]phenyl]ethyl]-N-hydroxyformamide;
(±)-N-[1-[[[3-(diethylamino)carbonyl]phenyl]methyl]-2-[(4'-methyl[1,1'-biphenyl]-4-yl)oxy]ethyl]-N-hydroxyformamide;
(±)-N-[1-[[(4'-cyano[1,1'-biphenyl]-4-yl)oxy]methyl]-2-[(4'-cyano[1,1'-biphenyl]-4-yl)oxy]ethyl]-N-hydroxyformamide;
(±)-N-[1-[[(4'-cyano[1,1'-biphenyl]-4-yl)oxy]methyl]-2-(4,4-dimethyl-2,5-dioxo-1-imidazolidinyl)ethyl]-N-hydroxyformamide;
(±)-N-[1-[(4,4-dimethyl-2,5-dioxo-1-imidazolidinyl)methyl]-2-[[4'-(2-methoxyethoxy)[1,1'-biphenyl]-4-yl]oxy]ethyl]-N-hydroxyformamide;
(±)-N-[1-[(4,4-dimethyl-2,5-dioxo-1-imidazolidinyl)methyl]-2-[(4'-propoxy [1,1'-biphenyl]-4-yl)oxy]ethyl]-N-hydroxyformamide;
(±)-N-[1-[(4,4-dimethyl-2,5-dioxo-1-imidazolidinyl)methyl]-2-[(4'-pentyloxy[1,1'-biphenyl]-4-yl)oxy]ethyl]-N-hydroxyformamide;
(±)-N-[1-[[[3'-(cyanomethyl)[1,1-biphenyl]-4-yl]sulfonyl]methyl]-3-(4,4-dimethyl-2,5-dioxo-1-imidazolidinyl)propyl]-N-hydroxyformamide;
(±)-N-[1-[[[4'-(trifluoromethoxy)[1,1'-biphenyl]-4-yl]sulfonyl]methyl]-2-(3,4,4-trimethy-2,5-dioxo-1-imidazolidinyl)ethyl]-N-hydroxyformamide;
(±)-N-[1-[[(4'-cyano[1,1'-biphenyl]-4-yl)sulfonyl]methyl]-2-(3-methy-2,5-dioxo-1-imidazolidinyl)ethyl]-N-hydroxyformamide;
(±)-N-[1-[[[3'-(cyanomethyl)[1,1'-biphenyl]-4-yl]sulfonyl]methyl]-2-(3,4,4-trimethyl-2,5-dioxo-1-imidazolidinyl)ethyl]-N-hydroxyformamide;
(±)-N-[1-[[(4'-cyano[1,1"-biphenyl]-4-yl)oxyl]methyl]-2-(1,6-dihydro-3-methyl-6-oxo-1-pyridazinyl)ethyl]-N-hydroxyformamide;
(±)-N-[1-[[(4'-cyano[1,1'-biphenyl]-4-yl)sulfonyl]methyl]-2-(4,4-dimethyl-2,5-dioxo-1-imidazolidinyl)ethyl)-N-hydroxyformamide;
(±)-N-[1-[[[4-(4-fluorophenoxy)phenyl]sulfonyl]methyl]-2-(4,4-dimethyl-2,5-dioxo-1-imidazolidinyl)ethyl]-N-hydroxyformamide;
(±)-N-[1-[[4-(4-pyridinyl)phenoxy]methyl)-2-(3,4,4-trimethy-2,5-dioxo-1-imidazolidinyl)ethyl]-N-hydroxyformamide;
(S)-N-[1-[(4,4-dimethyl-2,5-dioxo-1-imidazolidinyl)methyl]-2-[[4'-(trifluoromethoxy)[1,1'-biphenyl]-4-yl]oxy]ethyl]-N-hydroxyformamide;
(R)-N-[1-[(4,4-dimethyl-2,5-dioxo-1-imidazolidinyl)methyl]-2-[[4'-(trifluoromethoxy)[1,1'-biphenyl]-4-yl]oxy]ethyl]-N-hydroxyformamide;
N-[1-[[[4'-(trifluoromethoxy))[1,1'-biphenyl]-4-yl]oxy]methyl]-3-(4,4-dimethyl-2,5-dioxo-1-imidazolidinyl)propyl]-N-hydroxyformamide;
N-[1-[4-[(4-pyridinylthio)phenoxy]methyl]-2-(4,4-dimethyl-2,5-dioxo-1-imidazolidinyl)ethyl]-N-hydroxyformamide;
N-[1-[[[(4-chlorophenoxy)phenyl]sulfonyl]methyl]-3-(4,4-dimethyl-2,5-dioxo-1-imidazolidinyl)propyl]-N-hydroxyformamide;
N-[1-[[(4'-cyano[1,1''-biphenyl]-4-yl)oxyl]methyl]-2-(1,6-dihydro-6-oxo-1-pyridazinyl)ethyl]-N-hydroxyformamide;
N-[1-[[[4'-(aminosulfonyl)[1,1'-biphenyl]-4-yl]oxy]methyl]-2-(3,4,4-trimethyl-2,5-dioxo-1-imidazolidinyl)ethyl]-N-hydroxyformamide;
N-[1-[[[4'-(trifluoromethoxy)[1,1'-biphenyl]-4-yl]sulfonyl]methyl]-2-(4,4-dimethyl-2,5-dioxo-1-imidazolidinyl)ethyl]-N-hydroxyformamide;
N-[1-[4-[(4-pyridinyloxy)phenyl]sulfonyl]]ethyl]-N-hydroxyformamide;
N-[1-[[[(4-cyanophenoxy)phenyl]sulfonyl]methyl]-2-(4,4-dimethyl-2,5-dioxo-1-imidazolidinyl)ethyl]-N-hydroxyformamide;
N-[1-[[4-[[4-(trifluoromethoxy)phenoxy]phenyl]sulfonyl]methyl]-3-(4,4-dimethyl-2,5-dioxo-1-imidazolidinyl)propyl]-N-hydroxyformamide; and
N-[1-[[4-[[4-(trifluoromethoxy)phenoxy]phenyl]sulfonyl]methyl]-2-(4,4-dimethyl-2,5-dioxo-1-imidazolidinyl)ethyl]-N-hydroxyformamide.

16. A compound according to claim 15 which is
(S)-N-[1-[(4,4-dimethyl-2,5-dioxo-1-imidazolidinyl)methyl]-2-[[4'-(trifluoromethoxy)[1,1'-biphen yl]-4-yl]oxy]ethyl]-N-hydroxyformamide.

17. Use of a compound of Claim 1 for manufacturing a medicament for inhibiting matrix metalloproteinases in a mammal in need of such treatment.

18. A composition for inhibiting matrix metalloproteinases comprising a pharmaceutical carrier and a therapeutically effective amount of a compound of Claim 1.

## Patentansprüche

1. Eine Verbindung mit der Formel (I), 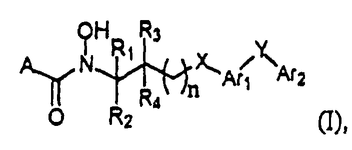 oder ein pharmazeutisch verträgliches Salz oder Prodrug davon, worin **A** Wasserstoff ist;
**n** Null ist;
**R**_{**1**} ist Wasserstoff oder Alkyl von ein bis vier Kohlenstoffatomen;
**R**_{**3**} ist gewählt aus der Gruppe bestehend aus
(1) Wasserstoff und
(2) Alkyl von ein bis vier Kohlenstoffatomen;
**R**_{**4**} ist Wasserstoff;
**R**_{**2**} ist gewählt aus der Gruppe bestehend aus
(1) Wasserstoff;
(2) Alkyl von ein bis vier Kohlenstoffatomen;
(3) Hydroxyalkyl, worin die Alkylengruppe von ein bis vier Kohlenstoffatomen ist;
(4) -(Alkylen)-S(O)ₚ-alkyl, worin das Alkylen von ein bis vier Kohlenstoffatomen ist, das Alkyl ist von ein bis vier Kohlenstoffatomen, und p ist null oder zwei;
(5) Phenyl;
(6) Phenylalkoxyalkyl, worin die Alkylen- und Alkylgruppen unabhängig von ein bis vier Kohlenstoffatomen sind;
(7) Phenylalkyl, worin die Alkylengruppe von ein bis vier Kohlenstoffatomen ist;
(8) Phenoxyalkyl, worin die Alkylengruppe von ein bis vier Kohlenstoffatomen ist;
(9) -(Alkylen)-N(R₅)SO₂-phenyl, worin das Alkylen von ein bis vier Kohlenstoffatomen ist und worin R₅ Alkyl ist von ein bis zwei Kohlenstoffatomen;
(10) (Heterozyklus)oxyalkyl, worin die Alkylengruppe von ein bis vier Kohlenstoffatomen ist und der Heterozyklus ist 4-Methyl-2-oxo-2H-1-benzopyranyl;
(11) -(Alkylen)-Heterozyklus, worin die Alkylengruppe von ein bis vier Kohlenstoffatomen ist und der Heterozyklus ist 1,6-Dihydro-3-methyl-6-oxo-1-pyridazinyl;
(12) -(Alkylen)-NR₆R₇; worin die Alkylengruppe von ein bis vier Kohlenstoffatomen ist, worin für (5)-(9) das Phenyl und die Phenylteile von dem Phenylalkoxyalkyl, Phenylalkyl, -(Alkylen)-N(R₅)SO₂-phenyl und Phenoxyalkyl sind, wahlweise substituiert mit ein oder zwei Substituenten unabhängig gewählt aus der Gruppe bestehend aus
(a) Alykl von ein bis vier Kohlenstoffatomen,
(b) Halo,
(c) -N(R₅)SO₂-Alkyl, und
(d) Phenyl, worin das Phenyl wahlweise substituiert ist mit einem Cyanosubstituenten; und
R₆ und R₇ definieren zusammengenommen mit dem Stickstoffatom, an welches sie gebunden sind
eine Gruppe gewählt aus der Gruppe bestehend aus
(1) Phthalimidyl;
(2) Succinimidyl;
(3) 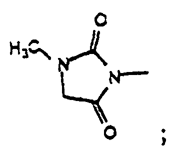
(4) 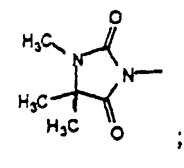
(5) 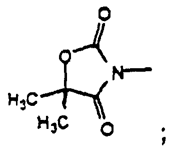
(6) 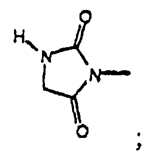
(7) 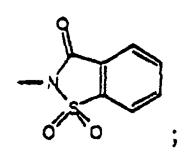
(8) 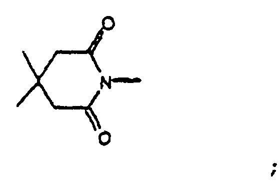 und
(9) 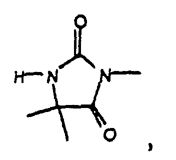
worin Option (2) wahlweise substituiert ist mit ein oder zwei Alkyl von ein bis vier Kohlenstoffatomsubstituenten und Option (9) ist wahlweise substituiert mit einem Phenylmethylsubstituenten;
**X** ist gewählt aus der Gruppe bestehend aus
(1) -O- und
(2) -S(O)ₚ-, worin p null oder zwei ist;
**Ar**₁ ist Phenyl, welches wahlweise substituiert ist mit einem Nitrosubstituenten;
**Y** ist gewählt aus der Gruppe bestehend aus
(1) einer kovalenten Bindung,
(2) -O-,
(3) Alkylen von zwei bis vier Kohlenstoffatomen,
(4) Piperidinyl, und
(5) -S(O)ₚ-, und
**Ar**_{**2**} ist eine Arylgruppe gewählt aus der Gruppe bestehend aus
(1) Phenyl,
(2) Pyridyl,
(3) Furyl, und
(4) Thienyl,
worin die Arylgruppe wahlweise substituiert ist mit ein, zwei oder drei Substituenten unabhängig gewählt aus der Gruppe bestehend aus
(a) Alkyl von ein bis vier Kohlenstoffatomen;
(b) Alkoxy von ein bis vier Kohlenstoffatomen;
(c) Alkoxy von ein bis vier Kohlenstoffatomen, substituiert mit Alkoxy von ein bis vier Kohlenstoffatomen;
(d) Cyano;
(e) Cyanoalkyl von ein bis vier Kohlenstoffatomen;
(f) Halo;
(g) Haloalkyl von ein bis vier Kohlenstoffatomen;
(h) Thioalkoxy von einem Kohlenstoffatom;
(i) Perfluoralkyl von einem Kohlenstoffatom;
(j) Perfluoralkoxy von einem Kohlenstoffatom;
(k) Sulfinylalkyl, worin der Alkylteil von ein bis vier Kohlenstoffatomen ist;
(l) Sulfonylalkyl, worin der Alkylteil von ein bis vier Kohlenstoffatomen ist; und 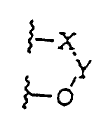
(m) worin X -O- ist, und Y ist -(C(R'')₂)ᵥ-, worin R'' Wasserstoff ist.

2. Eine Verbindung gemäß Anspruch 1, worin **R**_{**1**} und **R**_{**3**} unabhängig gewählt sind aus der Gruppe bestehend aus Wasserstoff und Methyl;
**R**_{**4**} ist Wasserstoff;
**X** ist gewählt aus der Gruppe bestehend aus
(1) -O-, und
(2) -S(O)ₚ-; und
**Ar**_{**1**} ist Phenyl.

3. Eine Verbindung gemäß Anspruch 2, worin R₂ Alkyl ist von ein bis vier Kohlenstoffatomen.

4. Eine Verbindung gemäß Anspruch 3, gewählt aus der Gruppe bestehend aus
(±)-N-[1-[[[3'-(Cyanomethyl)-[1,1'-biphenyl]-4-yl]oxy]methyl]pentyl]-N-hydroxyformamid;
(±)-N-[1-[[(4'-Cyano-[1,1'-biphenyl]-4-yl)oxy]methyl]-3-methylbutyl]-N-hydroxyformamid;
(±)-N-[1-[[(4'-Cyano-[1,1'-biphenyl]-4-yl)oxy]methyl]-2-methylbutyl]-N-hydroxyformamid;
(±)-N-[1-[[(4'-Cyano-[1,1'-biphenyl]-4-yl)oxy]methyl]pentyl]-N-hydroxyformamid;
(±)-N-[1-[[(4'-Cyano-[1,1'-biphenyl]-4-yl)oxy]methyl]ethyl]-N-hydroxyformamid;
(±)-N-[2-[(4'-Cyano[1,1'-biphenyl]-4-yl)oxy]-1-methylpropyl]-N-hydroxyformamid;
(±) -N-[1-[[(4'-Methoxy[1,1'-biphenyl]-4-yl)sulfonyl]methyl]ethyl]-N-hydroxyformamid;
(±)-N-[1-[[(4'-Chlor[1,1'-biphenyl]-4-yl)sulfonyl]methyl]ethyl]-N-hydroxyformamid;
(±)-N-[1-[[[4-(1,3-Benzodioxol-5-yl)phenyl]sulfonyl]methyl]ethyl]-N-hydroxyformamid;
(±)-N-[1-[[[4-(4-Chlorphenoxy)phenyl]sulfonyl]methyl]ethyl]-N-hydroxyformamid;
(±)-N-[1-[[(4'-Methoxy[1,1'-biphenyl]-4-yl)sulfonyl]methyl]propyl]-N-hydroxyformamid;
(±)-N-[1-[1,1-Dimethyl-2-[(4'-(trifluormethyl) [1,1'-biphenyl]-4-yl]sulfonyl]ethyl]-N-hydroxyformamid; und
N-[1-[4-[(4-Pyridinyloxy)phenyl]sulfonyl]]ethyl]-N-hydroxyformamid.

5. Eine Verbindung gemäß Anspruch 2, worin R₂ Wasserstoff ist.

6. Eine Verbindung gemäß Anspruch 5, die N-[2-[(4'-Cyano-[1,1'-biphenyl]-4-yl)oxy]ethyl]-N-hydroxyformamid ist.

7. Eine Verbindung gemäß Anspruch 2, worin R₂ gewählt ist aus der Gruppe bestehend aus
(1) Phenyl, worin die Phenylgruppe unsubstituiert oder substituiert ist;
(2) Phenoxyalkyl, worin die Alkylengruppe von ein bis vier Kohlenstoffatomen ist, und worin die Phenylgruppe unsubstituiert oder substituiert ist;
(3) -(Alkylen)-S(O)ₚ-alkyl;
(4) Hydroxyalkyl, worin die Alkylengruppe von ein bis vier Kohlenstoffatomen ist;
(5) -(Alkylen)-N(R₅)SO₂-phenyl, worin das Alkylen von ein bis vier Kohlenstoffatomen ist, die Phenylgruppe ist unsubstituiert oder substituiert und R₅ ist Alkyl von ein bis zwei Kohlenstoffatomen;
(6) Phenylalkoxyalkyl, worin die Alkylen- und Alkylgruppen unabhängig von ein bis vier Kohlenstoffatomen sind und die Phenylgruppe ist unsubstituiert oder substituiert;
(7) (Heterozyklus)oxyalkyl, worin die Alkylgruppe ein bis vier Kohlenstoffatome ist und der Heterozyklus ist unsubstituiert oder substituiert, und
(8) Phenylalkyl, worin die Alkylengruppe von ein bis vier Kohlenstoffatomen ist
und
worin die Phenylgruppe unsubstituiert oder substituiert ist.

8. Eine Verbindung gemäß Anspruch 7 gewählt aus der Gruppe bestehend aus
(±)-N-[1-[[(4'-Cyano-[1,1'-biphenyl]-4-yl)oxy]methyl]-2-phenoxyethyl]-N-hydroxyformamid;
(±)-N-[1-[[(4'-Cyano-[1,1'-biphenyl]-4-yl)oxy]methyl]-2-(4-methylphenyl)ethyl]-N-hydroxyformamid;
(±)-N-[2-[(4'-Cyano-[1,1'-biphenyl]-4-yl)oxy]-1-(4-fluorphenyl)ethyl]-N-hydroxyformamid;
(±)-N-[1-[(4'-Cyano-[1,1'-biphenyl]-4-yl)oxy]methyl]-2-(4-fluorphenyl)ethyl]-N-hydroxyformamid;
(±)-N-[1-[[(3'-Cyano-[1,1'-biphenyl]-4-yl)oxy]methyl]-2-[methyl[(4-methylphenyl)sulfonyl]amino]ethyl]-N-hydroxyformamid;
(±)-N-[1-[[(4'-Cyano-[1,1'-biphenyl]-4-yl)oxy]methyl]-5-[(4-methyl-2-oxo-2H-l-benzopyran-6-yl)oxy]pentyl]-N-hydroxyformamid;
(±)-N-[1-[[(4'-Cyano-[1,1'-biphenyl]-4-yl)oxy]methyl]-4-[(4-methyl-2-oxo-2H-1-benzopyran-6-yl)oxy]butyl]-N-hydroxyformamid;
(±)-N-[1-[[(4'-Cyano-[1,1'-biphenyl]-4-yl)oxy]methyl]-4-[(4-methyl-2-oxo-2H-l-benzopyran-7-yl)oxy]butyl]-N-hydroxyformamid;
(±) -N- [1-[[(4'-Cyano-[1,1'-biphenyl]-4-yl)oxy]methyl]-5-[(4-methyl-2-oxo-2H-1-benzopyran-7-yl)oxylpentyl]-N-hydroxyformamid;
(±)-N- [1- [[(4'-Cyano-[1,1'-biphenyl]-4-yl)oxy]methyl]-2-isopropylthioethyl]-N-hydroxyformamid;
(±)-N-[1-[(Phenylmethoxy)methyl]-2-[[4'-(trifluormethyl)[1,1'-biphenyl]-4-yl]sulfonyl]ethyl]-N-hydroxyformamid;
(±)-N-[1-(Hydroxymethyl)-2-[[(4'-(trifluormethyl)[1,1'biphenyl]-4-yl]sulfonyl]ethyl]-N-hydroxyformamid;
(±)-N-[1-[[(4'-Methyl[1,1'-biphenyl)-4-yl)oxy]methyl]-2-[[3-(methylsulfonyl)-amino]phenyl]ethyl]-N-hydroxyformamid;
(±)-N-[1-[[[3-(Diethylamino)carbonyl]phenyl]methyl]-2-[(4'methyl[1,1'-biphenyl]-4-yl)oxy]ethyl]-N-hydroxyformamid;
N-[1-[[(4'-Cyano[1,1'-biphenyl]-4-yl)oxy]methyl]-2-[(4'cyano[1,1'-biphenyl]-4-yl)oxy]ethyl]-N-hydroxyformamid;
N-[1-[[(4'-Cyano[1,1'-biphenyl]-4-yl)oxy]methyl]-2-(1,6-dihydro-3-methyl-6-oxo-1-pyridazinyl)ethyl]-N-hydroxyformamid;
und
N-[1-[[(4'-Cyano[1,1'-biphenyl]-4-yl)oxy]methyl]-2-(1,6-dihydro-6-oxo-1-pyridazinyl)ethyl]-N-hydroxyformamid.

9. Eine Verbindung gemäß Anspruch 2, worin R₂ ein -(Alkylen)-NR₆R₇ ist.

10. Eine Verbindung gemäß Anspruch 9, worin -NR₆R₇ folgendes ist

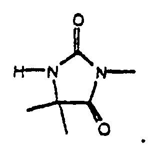

11. Eine Verbindung gemäß Anspruch 10, gewählt aus der Gruppe bestehend aus
(±)-N-[1-[[(4'-Butoxy[1,1'-biphenyl]-4-yl)oxy]methyl]-2-(4,4-dimethyl-2,5-dioxo-1-imidazolidinyl)ethyl]-N-hydroxyformamid;
(±)-N-[1-[(4,4-Dimethyl-2,5-dioxo-1-imidazolidinyl)methyl]-2-[(4'-ethoxy[1,1'-biphenyl]-4-yl)oxy]ethyl]-N-hydroxyformamid;
(±)-N-[1-[[4-(1,3-Benzodioxol-5-yl)phenoxy]methyl]-2-(4,4-dimethyl-2,5-dioxo-1-imidazolidinyl)ethyl]-N-hydroxyformamid;
(±)-N-[1-[(4,4-Dimethyl-2,5-dioxo-1-imidazolidinyl)methyl]-2-[[4'-(trifluormethoxy)[1,1'-biphenyl]-4-yl]oxy]ethyl]-N-hydroxyformamid;
(±)-N-[1-[(4,4-Dimethyl-2,5-dioxo-1-imidazolidinyl)methyl]-2-[[4'-(trifluormethyl)[1,1'-biphenyl]-4-yl]oxy]ethyl]-N-hydroxyformamid;
(±)-N-[1-[[[3'-(Cyanomethyl) [1,1'-biphenyl]-4-yl]oxy]methyl]-3-(4,4-dimethyl-2,5-dioxo-1-imidazolidinyl)propyl]]-N-hydroxyformamid;
(±)-N-[1-[[(4'-Butoxy[1,1'-biphenyl]-4-yl)sulfonyl]methyl]-2-(4,4-dimethyl-2,5-dioxo-1-imidazolidinyl)ethyl]-N-hydroxyformamid;
(±)-N-[1-[[(4'-Cyano[1,1'-biphenyl]-4-yl)oxy]methyl]-3-(4,4-dimethyl-2,5-dioxo-1-imidazolidinyl)propyl]-N-hydroxyformamid;
(±)-N-[1-[[[4'-(Methylsulfonyl)[1,1'-biphenyl]-4-yl]oxy]methyl]-2-(4,4-dimethyl-2,5-dioxo-1-imidazolidinyl)ethyl]-N-hydroxyformamid;
(±)-N-[1-[[(4'-Cyano[1,1'-biphenyl]-4-yl]sulfonyl]methyl]-2-(4,4-dimethyl-2,5-dioxo-1-imidazolidinyl)ethyl]-N-hydroxyformamid;
(±)-N-[1-[(4,4-Dimethyl-2,5-dioxo-1-imidazolidinyl)methyl]-2-[[4'-(trifluormethyl)[1,1'-biphenyl)-4-yl]thio]ethyl]-N-hydroxyformamid;
(±)-N-[1-[(4,4-Dimethyl-2,5-dioxo-1-imidazolidinyl)methyl]-2-[[4'-(trifluormethyl)[1,1'-biphenyl]-4-yl]sulfonyl]ethyl]-N-hydroxyformamid;
(±)-N-[1-[[[4-(4-Chlorphenoxy)phenyl]sulfonyl]methyl]-2-(4,4-dimethyl-2,5-dioxo-1-imidazolidinyl)ethyl]-N-hydroxyformamid;
(±)-N-[1-[[(4'-Butyl[1,1'-biphenyl]-4-yl)oxy]methyl]-2-(4,4-dimethyl-2,5-dioxo-1-imidazolidinyl)ethyl]-N-hydroxyformamid;
(±)-N-[1-[[[3'-(Cyanomethyl)[1,1'-biphenyl]-4-yl]oxy]methyl]-2-(4,4-dimethyl-2,5-dioxo-imidazolidinyl)ethyl]-N-hydroxyformamid;
(±)-N-[1-[[(4'-Cyano[1,1'-biphenyl]-4-yl)oxy]methyl]-2-(4,4-dimethyl-2,5-dioxo-imidazolidinyl)ethyl]-N-hydroxyformamid;
(±) -N- [1- [(4,4-Dimethyl-2,5-dioxo-1-imidazolidinyl)methyl]-2-[[4'-(2-methoxyethoxy) [1,1'-biphenyl]-4-yl]oxy]ethyl]-N-hydroxyformamid;
(±)-N-[1-[(4,4-Dimethyl-2,5-dioxo-1-imidazolidinyl]methyl]-2-[[4'-propoxy[1,1'-biphenyl]-4-yl)oxy]ethyl]-N-hydroxyformamid;
N-[1-[(4,4-Dimethyl-2,5-dioxo-1-imidazolidinyl)methyl]-2-[[4'pentyloxy[1,1'-biphenyl]-4-yl)oxy]ethyl]-N-hydroxyformamid;
(±)-N-[1-[[[3'-(Cyanomethyl)[1,1'-biphenyl]-4-yl]sulfonyl]methyl]-3-(4,4-dimethyl-2,5-dioxo-1-imidazolidinyl)propyl]-N-hydroxyformamid;
(±)-N-[1-[[(4'-Cyano[1,1'-biphenyl]-4-yl)sulfonyl]methyl]-2-(4,4-dimethyl-2,5-dioxo-1-imidazolidinyl)ethyl]-N-hydroxyformamid;
(±)-N-[1-[[[4-(4-Fluorphenoxy)phenyl]sulfonyl]methyl]-2-(4,4-dimethyl-2,5-dioxo-1-imidazolidinyl)ethyl]-N-hydroxyformamid;
(S)-N-[1-[(4,4-Dimethyl-2,5-dioxo-1-imidazolidinyl)methyl]-2-[[4'-(trifluormethoxy)[1,1'-biphenyl]-4-yl]oxy]ethyl]-N-hydroxyformamid;
(R)-N-[1-[(4,4-Dimethyl-2,5-dioxo-1-imidazolidinyl)methyl)-2-[[4'-(trifluormethoxy)[1,1'-biphenyl]-4-yl]oxy]ethyl]-N-hydroxyformamid;
N-[1-[[[4'-(Trifluormethoxy))[1,1'-biphenyl]-4-yl]oxy]methyl]-3-(4,4-dimethyl-2,5-dioxo-1-imidazolidinyl)propyl]-N-hydroxyformamid;
N-[1-[4-[(4'-Pyridinylthio)phenoxy]methyl]-2-(4,4-dimethyl-2,5-dioxo-1-imidazolidinyl)ethyl]-N-hydroxyformamid;
N-[1-[[[(4-Chlorphenoxy)phenyl]sulfonyl]methyl]-3-(4,4-dimethyl-2,5-dioxo-1-imidazolidinyl)propyl]-N-hydroxyformamid;
N-[1-[[[4'-(Trifluormethoxy)[1,1-biphenyl]-4-yl]sulfonyl]methyl]-2-(4,4-dimethyl-2,5-dioxo-1-imidazolidinyl)ethyl]-N-hydroxyformamid;
N-[1-[[[(4-Cyanophenoxy)phenyl]sulfonyl]methyl]-2-(4,4-dimethyl-2,5-dioxo-1-imidazolidinyl)ethyl]-N-hydroxyformamid; und
N-[1-[[4-[[4-(Trifluormethoxy)phenoxy]phenyl]sulfonyl]methyl]-3-(4,4-dimethyl-2,5-dioxo-1-imidazolidinyl)propyl]-N-hydroxyformamid.

12. Eine Verbindung gemäß Anspruch 9, gewählt aus der Gruppe bestehend aus
(±)-N-[1-[[(4'-Cyano-[1,1'-biphenyl]-4-yl)oxy]methyl]-2-(2,3-dihydro-1,3-dioxo-1H-isoindol-2-yl)ethyl]-N-hydroxyformamid;
(±)-N-[1-[[(4'-Cyano-[1,1'-biphenyl]-4-yl) oxy]methyl]-2-(3,4,4-trimethyl-2,5-dioxoimidazolidin-1-yl)ethyl]-N-hydroxyformamid;
(±)-N-[1-[[(4'-Cyano-[1,1'-biphenyl]-4-yl)oxy]methyl]-3-(3,4,4-trimethyl-2,5-dioxoimidazolidin-1-yl)propyl]-N-hydroxyformamid;
(±)-N-[1-[4-[(2-E-Phenylethenyl)phenoxy]methyl]-2-(3,4,4-trimethyl-2,5-dioxo-1-imidazolidinyl)ethyl]-N-hydroxyformamid;
(±)-N-[1-[[4-(2-Furanyl)phenoxy]methyl]-2-(3,4,4-trimethyl-2,5-dioxo-1-imidazolidinyl)ethyl]-N-hydroxyformamid;
(±)-N-[1-[[(4'-Butoxy[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(4'-Fluor[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[(3,4,4-Trimethyl-2,5-dioxo-1-imidazolidinyl)methyl]-2-[[4'-(trifluormethyl)[1,1'-biphenyl]-4-yl]oxy]ethyl]-N-hydroxyformamid;
(±)-N-[1-[[(4'-Methoxy[1'-biphenyl]-4-yl]oxy]methyl]-2-(3,4,4-trimethyl-2,5-dioxo-1-imidazolidinyl)ethyl]-N-hydroxyformamid;
(±)-N-[1-[[(4'-Methyl[1,1'-biphenyl]-4-yl]oxy]methyl]-2-(3,4,4-trimethyl-2,5-dioxo-1-imidazolidinyl)ethyl]-N-hydroxyformamid;
(±)-N-[1-[[(4'-Butoxy[1,1'-biphenyl]-4-yl]oxy]methyl]-2-(3-methyl-2,5-dioxo-1-imidazolidinyl)ethyl]-N-hydroxyformamid;
(±)-N-[1-[[4-(3-Thienyl)phenoxy]methyl]-2-(3,4,4-trimethyl-2,5-dioxo-1-imidazolidinyl)ethyl]-N-hydroxyformamid;
(±)-N-[1-[[([1,1'-Biphenyl]-4-yl)oxy]methyl]-2- (3,4,4-trimethyl-2,5-dioxo-1-imidazolidinyl)ethyl]-N-hydroxyformamid;
(±)-N-[1-[[(3'-Chlor-4'-fluor[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(2'-Methyl[1,1'-biphenyl]-4-yl)oxy]methyl]-2-(3,4,4-trimethyl-2,5-dioxo-1-imidazolidinyl)ethyl]-N-hydroxyformamid;
(+)-N-[1-[[(4'-Cyano[1,1'-biphenyl]-4-yl)oxy]methyl]-2-(2,5-dioxo-1-imidazolidinyl)ethyl]-N-hydroxyformamid;
(±)-N-[1-[[(4'-Cyano[1,1'-biphenyl]-4-yl)oxy]methyl]-2-(1,1-dioxido-3-oxo-1,2-benzisothiazol-2(3H)-yl)ethyl]-N-hydroxyformamid;
(±)-N-[1-[[4-(4-Phenyl-1-piperidinyl)phenoxy]methyl]-2-(3,4,4-trimethyl-2,5-dioxo-1-imidazolidinyl)ethyl] -N-hydroxyformamid;
(±)-N-[1-[[(4'-Cyano[1,1'-biphenyl]-4-yl)oxy]methyl]-2-[4,4-dimethyl-2,5-dioxo-3(3-pyridinylmethyl)-1-imidazolidinyl]ethyl]-N-hydroxyformamid;
(±)-N-[1-[[(3'-Cyano[1,1'-biphenyl]-4-yl)oxy]methyl]-2-[3,4,4-trimethyl-2,5-dioxo-1-imidazolidinyl)ethyl]-N-hydroxyformamid;
(±)-N-[1-[[[4'-(Methylthio)[1,1'-biphenyl]-4-yl)oxy]methyl]-2-[3,4,4-trimethyl-2,5-dioxo-1-imidazolidinyl)ethyl]-N-hydroxyformamid;
(±)-N-[1-[4-[[4-(Trifluormethyl)phenoxy]phenoxy]methyl]-2-(3,4,4-trimethyl-2,5-dioxo-1-imidazolidinyl)ethyl]-N-hydroxyformamid;
(±)-N-[1-[[[4'-(Trifluormethyl)[1,1'-biphenyl]-4-yl]oxy]methyl)-2-(3,4,4-trimethyl-2,5-dioxo-1-imidazolidinyl)ethyl]-2-N-hydroxyformamid;
(±)-N-[1-[[[4'-(Methylsulfonyl)[1,1'-biphenyl]-4-yl]oxy]methyl]-2-(3,4,4-trimethyl-2,5-dioxo-1-imidazolidinyl)ethyl] -N-hydroxyformamid;
(±)-N-[1-[[[3'-(Cyanomethyl)-4'-methoxy[1,1'-biphenyl]-4-yl]oxy]methyl]-2-(3,4,4-trimethyl-2,5-dioxo-1-imidazolidinyl)ethyl]-N-hydroxyformamid;
(±)-N-[1-[[(3'-Cyano[1,1'-biphenyl]-4-yl]oxy]methyl]-2-(2,5-dioxo-1-pyrrolidinyl)ethyl]-N-hydroxyformamid;
(±)-N-[1-[[(4'-Cyano[1,1'-biphenyl]-4-yl]oxy]methyl]-2-(4,4-dimethyl-2,6-dioxo-1-piperidinyl)ethyl]-N-hydroxyformamid;
N-[1S-[[(4'-Cyano[1,1'-biphenyl] -4-yl] oxy]methyl] -2-(2,5-dioxo-1-pyrrolidinyl)ethyl]-2-N-hydroxyformamid;
N-[1R-[[(4'-Cyano[1,1'-biphenyl]-4-yl]oxy]methyl]-2-(2,5-dioxo-1-pyrrolidinyl)ethyl]-2-N-hydroxyformamid;
(±)-N-[1-[[(4'-Cyano[1,1'-biphenyl]-4-yl]oxy]methyl]-2-(3-ethyl-3-methyl-2,5-dioxo-1-piperidinyl)ethyl]-2-N-hydroxyformamid;
(±)-N-[1-[[(4'-Cyano[1,1'-biphenyl]-4-yl)oxy]methyl]-2-(5,5-dimethyl-2,4-dioxo-3-oxazolidinyl)ethyl]-N-hydroxyformamid;
(±)-N-[1-[[(4'-Cyano[1,1'-biphenyl]-4-yl)sulfonyl]methyl]-2-(3,4,4-trimethyl-2,5-dioxo-1-imidazolidinyl)ethyl)-N-hydroxyformamid;
(±)-N-[1-[[(4'-Cyano[1,1'-biphenyl]-4-yl)oxy]methyl]-2-(3-methyl-2, 5-dioxo-1-imidazolidinyl)ethyl]-N-hydroxyformamid;
(±)-N-[1-[[(4'-Chlor-[1,1'-biphenyl]-4-yl)oxy]methyl]-2-(3,4,4-trimethyl-2,5-dioxo-1-imidazolidinyl)ethyl)-N-hydroxyformamid;
(±)-N-[1-[[(3'-Cyanomethyl-[1,1'-biphenyl]-4-yl)oxy]methyl]-2-(3,5,5-trimethyl-2,5-dioxo-1-imidazolidinyl)propyl]-N-hydroxyformamid;
(±)-N-[1-[[(3'-cyanomethyl-[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(4'-Cyano-[1,1'-biphenyl]-4-yl]oxy]methyl]-2-(3-ethyl-4,4-dimethyl-2,5-dioxo-1-imidazolidinyl)ethyl]-N-hydroxyformamid;
(±)-N-[1-[[(4'-Cyano-[1,1'-biphenyl]-4-yl)oxy]methyl]-2-(3-benzyl-4,4-dimethyl-2,5-dioxo-1-imidazolidinyl)ethyl]-N-hydroxyformamid;
(±)-N-[1-[[(4'-Cyano-[1,1'-biphenyl-4-yl)oxy]methyl]-2-(3,5,5-trimethyl-2,4-dioxo-1-imidazolidinyl)ethyl]-N-hydroxyformamid;
(±)-N-[1-[(1,5-Dioxo-1-imidazolidinyl)methyl]-2-[[4'-(trifluormethoxy)[1,1'-biphenyl]-4-yl]oxy]ethyl]-N-hydroxyformamid;
(±)-N-[1-[[[4'-(Trifluormethyl)[1,1'-biphenyl]-4-yl]sulfonyl]methyl]-2-(3,4,4-trimethyl-2,5-dioxo-1-imidazolidinyl)ethyl]-N-hydroxyformamid;
(±)-N-[1-[[(4'-Butyl[1,1'-biphenyl]-4-yl)oxy]methyl]-2-(3-methyl-2,5-dioxo-1-imidazolidinyl)ethyl]-N-hydroxyformamid;
(±)-N-[1-[(3-Methyl-2,5-dioxo-1-imidazolidinyl)methyl]-2-[[4'-(trifluormethoxy)[1,1'-biphenyl]-4-yl]oxy]ethyl]-N-hydroxyformamid;
(±) -N-[1-[[[4-(4-Chlorphenoxy)phenyl]sulfonyl]methyl]-2-(3,4,4-trimethyl-2,5-dioxo-1-imidazolidinyl)ethyl]-N-hydroxyformamid;
(±)-N-[1-[[(4-Butyl(1,1'-biphenyl]-4-yl)sulfonyl]methyl]-2-(3,4,4-trimethyl-2,5-dioxo-1-imidazolidinyl)ethyl]-N-hydroxyformamid;
(±)-N-[1-[4-(2-Thienyl)phenoxy]methyl)-2-[1-(3,4,4-trimethyl-2,5-dioxo-1-imidazolidinyl)ethyl]-N-hydroxyformamid;
(±)-N-[1-[[(3-Nitro[1,1'-biphenyl]-4-yl)oxy]methyl]-2-(3,4,4-tirmethyl-2,5-dioxo-1-imidazolidinyl)ethyl]-N-hydroxyformamid;
(±)-N-[1-[[[4'-(Trifluormethoxy)[1,1'-biphenyl]-4-yl]sulfonyl]methyl]-2-(3,4,4-trimethyl-2,5-dioxo-1-imidazolidinyl)ethyl]-N-hydroxyformamid;
(±)-N-[1-[[(4'-Cyano[1,1'-biphenyl]-4-yl)sulfonyl]methyl]-2-(3-methyl-2,5-dioxo-1-imidazolidinyl)ethyl]-N-hydroxyformamid;
(±)-N-[1-[[[3'-(Cyanomethyl)[1,1'-biphenyl]-4-yl]sulfonyl]methyl]-2-(3,4,4-trimethyl-2,5-dioxo-1-imidazolidinyl)ethyl]-N-hydroxyformamid;
(±)-N-[1-[[4-(4-Pyridinyl)phenoxy]methyl]-2-(3,4,4-trimethyl-2,5-dioxo-1-imidazolidinyl)ethyl]-N-hydroxyformamid;
N-[1-[[[4'-(Aminosulfonyl) [1,1'-biphenyl]-4-yl]oxy]methyl]-2-(3,4,4-trimethyl-2,5-dioxo-1-imidazolidinyl)ethyl]-N-hydroxyformamid; und
N-[1-[[4-[[4-(Trifluormethoxy)phenoxy]phenyl]sulfonyl]methyl]-2-(4,4-dimethyl-2,5-dioxo-1-imidazolidinyl)ethyl]-N-hydroxyformamid.

13. Eine Verbindung gemäß Anspruch 1, worin R₁ und R₂ zusammengenommen mit dem Kohlenstoffatom an welches sie gebunden sind einen Tetrahydropyranylring bilden.

14. Eine Verbindung gemäß Anspruch 13 gewählt aus der Gruppe bestehend aus
N-[4-[4-[[(4-Chlorphenoxy)phenyl]sulfonyl]methyl]tetrahydro-2Hpyran-4-yl]-N-hydroxyformamid und
N-[4-[4-[[(4'-Chlor[1,1'-biphenyl]-4-yl)sulfonyl]methyl]tetrahydro-2H-pyran-4-yl]-N-hydroxyformamid.

15. Eine Verbindung gewählt aus der Gruppe bestehend aus
(±)-N-[1-[[(4'-Cyano-[1,1'-biphenyl]-4-yl]oxy]methyl]-2-phenoxyethyl]-N-hydroxyformamid;
(±)-N-[1-[[(4'-Cyano-[1,1'-biphenyl)-4-yl)oxy]methyl]-2-(2,3-dihydro-1,3-dioxo-lH-isoindol-2-yl)ethyl]-N-hydroxyformamid;
(±)-N-[1-[[(4'-Cyano-[1,1'-biphenyl]-4-yl)oxy]methyl]-2-(3,4,4-trimethyl-2,5-dioxoimidazolidin-1-yl)ethyl]-N-hydroxyformamid;
(±)-N-[1-[[(4'-Cyano-[1,1'-biphenyl]-4-yl)oxy]methyl]-3-(3,4,4-trimethyl-2,5-dioxoimidazolidin-1-yl)propyl]-N-hydroxyformamid;
(±)-N-[1-[[[3'-(Cyanomethyl)-[1,1'-biphenyl]-4-yl]oxy]methyl]pentyl]-N-hydroxyformamid;
(±)-N-[1-[[(4'-Cyano-[1,1'-biphenyl]-4-yl)oxy]methyl]-3-methylbutyl]-N-hydroxyformamid;
(±)-N-[1-[[(4'-Cyano-[1,1'-biphenyl]-4-yl)oxy]methyl]-2-methylbutyl]-N-hydroxyformamide;
(±)-N-[1-[[(4'-Cyano-[1,1'-biphenyl]-4-yl)oxy]methyl]pentyl]-N-hydroxyformamid;
(±)-N-[1-[[(4'-Cyano-[1,1'-biphenyl]-4-yl)oxy]methyl]-2-(4-methylphenyl)ethyl]-N-hydroxyformamid;
(±)-N-[2-[(4'-Cyano-[1,1'-biphenyl]-4-yl)oxy]-1-(4-fluorphenyl)ethyl]-N-hydroxyformamid;
(±)-N-[1-[[(4'-Cyano[1,1'-biphenyl]-4-yl)oxy]methyl]-2-(4-fluorphenyl)ethyl]-N-hydroxyformamid;
(±)-N-[1-[[(4'-cyano-[1,1'-biphenyl]-4-yl)oxy]methyl]ethyl]-N-Hydroxyformamid;
(±)-N-[2-[(4'-Cyano-[1,1'-biphenyl]-4-yl)oxy]ethyl]-N-hydroxyformamid;
(±)-N-[1-[4-[(2E-Phenylethenyl)phenoxy]methyl]-2-(3,4,4-trimethyl-2,5-dioxo-1-imidazolidinyl)ethyl]-N-hydroxyformamid;
(±)-N-[1-[[4-(2-Furanyl)phenoxy]methyl]-2-(3,4,4-trimethyl-2,5-dioxo-1-imidazolidinyl)ethyl]-N-hydroxyformamid;
(±)-N-[1-[[(4'-Butoxy[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(4'-fluor[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[(3,4,4-Trimethyl-2,5-dioxo-1-imidazolidinyl)methyl]-2-[[4'-(trifluormethyl) [1,1'-biphenyl]-4-yl]oxy]ethyl]-N-hydroxyformamid;
(±)-N-[1-[[(4'-Methoxy[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(4'-Methyl[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(4'-Butoxy[1,1'-biphenyl]-4-yl)oxy]methyl]-2-(4,4-dimethyl-2,5-dioxo-1-imidazolidinyl)ethyl]-N-hydroxyformamid;
(±)-N-[1-[(4,4-Dimethyl-2,5-dioxo-1-imidazolidinyl)methyl]-2-[(4'-ethoxy[1,1'-biphenyl]-4-yl)oxy]ethyl]-N-hydroxyformamid;
(±)-N-[1-[[4-(1,3-Benzodioxol-5-yl)phenoxy]methyl]-2-(4,4-dimethyl-2,5-dioxo-imidazolidinyl)ethyl]-N-hydroxyformamid;
(±)-N-[1-[[(4'-Butoxy[1,1'-biphenyl]-4-yl)oxy]methyl]-2-(3-methyl-2,5-dioxo-1-imidazolidinyl)ethyl]-N-hydroxyformamid;
(±)-N-[1-[[4-(3-Thienyl)phenoxy]methyl]-2-(3,4,4-trimethyl-2,5-dioxo-1-imidazolidinyl)ethyl]-N-hydroxyformamid;
(±)-N-[1-[[([1,1'-Biphenyl]-4-yl)oxy]methyl] -2-(3,4,4-trimethyl-2, 5-dioxo-1-imidazolidinyl)ethyl]-N-hydroxyformamid;
(±)-N-[1-[[(3'-Chlor-4'-fluor[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(2'-Methyl[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(4'-Cyano[1,1'-biphenyl]4-yl)oxy]methyl]-2-(2,5-dioxo-imidazolidinyl)ethyl]-N-hydroxyformamid;
(±)-N-[1-[[(4'-Cyanol[1,1'-biphenyl]4-yl)oxy]methyl] -2-(1,1-dioxido-3-oxo-1,2-benzisothiazol-2(3H)-yl)ethyl]-N-hydroxyformamid;
(±)-N-[1-[4,4-Dimethyl-2,5-dioxo-1-imidazolidinyl)methyl]-2-[[4'-(trifluormethoxy)[1,1'-biphenyl]-4-yl]oxy]ethyl]-N-hydroxyformamid;
(±)-N-[1-[[4-(4-Phenyl-1-piperidinyl)phenoxy]methyl]-2-(3,4,4-trimethyl-2,5-dioxo-1-imidazolidinyl)ethyl]-N-hydroxyformamid;
(±)-N-[1-[(4,4-Dimethyl-2,5-dioxo-1-imidazolidinyl)methyl]-2-[[4'-(trifluormethyl)[1,1'-biphenyl]-4-yl]oxy]ethyl]-N-hydroxyformamid;
(±)-N-[1-[[(3'-Cyano[1,1'-biphenyl]-4-yl)oxy]methyl]-2-[methyl[(4-methylphenyl)sulfonyl]amino]ethyl]-N-hydroxyformamid;
(±)-N-[1-[[(4'-Cyano[1,1'-biphenyl]-4-yl)oxy]methyl]-2-[4, 4-dimethyl-2,5-dioxo-3-(3-pyridinylmethyl)-1-imidazolidinyl]ethyl]-N-hydroxyformamid;
(±)-N-[2-[(4'-Cyano[1,1'-biphenyl]-4-yl)oxy]-1-methylpropyl]-N-hydroxyformamid;
(±)-N-[1-[[(3'-Cyano[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[[4'-(Methylthio)[1,1'-biphenyl]-4-yl]oxy]methyl]-2-(3,4,4-trimethyl-2,5-dioxo-1-imidazolidinyl)ethyl]-N-hydroxyformamid;
(±)-N-[1-[4-[[4-(Trifluormethyl)phenoxy]phenoxy]methyl]-2-(3,4,4-trimethyl-2,5-dioxo-1-imidazolidinyl)ethyl]-N-hydroxyformamid;
(±)-N-[1-[[[4'-(Trifluormethoxy)[1,1'-biphenyl]-4-yl]oxy]methyl]-2-(3,4,4-trimethyl-2,5-dioxo-1-imidazolidinyl)ethyl]-2-N-hydroxyformamid;
(±)-N-[1-[[[4'-(Methylsulfonyl)[1,1'-biphenyl]-4-yl]oxy]methyl]-2-(3,4,4-trimethyl-2,5-dioxo-1-imidazolidinyl)ethyl]-N-hydroxyformamid;
(±)-N-[1-[[[3'-(Cyanomethyl)-4'-methoxy[1,1'-biphenyl]-4-yl]oxy]methyl]-2-(3,4,4-trimethyl-2,5-dioxo-1-imidazolidinyl)ethyl]-N-hydroxyformamid;
(±)-N-[1-[[[3'-(Cyanomethyl)[1,1'-biphenyl]-4-yl]oxy]methyl]-3- (4,4-dimethyl-2,5-dioxo-1-imidazolidinyl)propyl]]-N-hydroxyformamid;
(±)-N-[1-[[(4'-Butoxy[1,1'-biphenyl]-4-yl)sulfonyl]methyl]-2-(4,4-dimethyl-2,5-dioxo-1-imidazolidinyl)ethyl]-N-hydroxyformamid;
(±)-N-[1-[[(4'-Cyano[1,1'-biphenyl]-4-yl)oxy]methyl]-3-(4,4-dimethyl-2,5-dioxo-1-imidazolidinyl)propyl]-N-hydroxyformamid;
(±)-N-[1-[[[4'-(Methylsulfonyl)[1,1'-biphenyl]-4-yl]oxy]methyl]-2-(4,4-dimethyl-2,5-dioxo-1-imidazolidinyl)ethyl]-N-hydroxyformamid;
(±)-N-[1-[[(3'-Cyano[1,1'-biphenyl]-4-yl)oxy]methyl]-2-(2,5-dioxo-1-pyrrolidinyl)ethyl]-N-hydroxyformamid;
(±)-N-[1-[[(4'-Cyano[1,1'-biphenyl]-4-yl)oxy]methyl]-2-(4,4-dimethyl-2,6-dioxo-1-piperidinyl)ethyl]-N-hydroxyformamid;
(±)-N-[1S-[[(4'-Cyano[1,1'-biphenyl]-4-yl)oxy]methyl]-2-(2,5-dioxo-1-pyrrolidinyl)ethyl]-N-hydroxyformamid;
(±)-N-[1R-[[(4'-Cyano[1,1'-biphenyl]-4-yl)oxy]methyl]-2-(2,5-dioxo-1-pyrrolidinyl)ethyl]-N-hydroxyformamid;
(±)-N-[1-[[(4'-Cyano[1,1'-biphenyl]-4-yl)oxy]methyl]-2-(3-ethyl-3-methyl-2,5-dioxo-1-pyrrolidinyl)ethyl]-N-hydroxyformamid;
N-[4-[4-[[(4-Chlorphenoxy)phenyl]sulfonyl]methyl]tetrahydro-2Hpyran-4yl]-N-hydroxyformamid;
(±)-N-[1-[[(4'-Cyano[1,1'-biphenyl)-4-yl)oxy]methyl]-5-[(4-methyl-2-oxo-2H-1benzopyran-6-yl)oxy]pentyl]-N-hydroxyformamid;
(±)-N-[1-[[(4'-Cyano[1,1'-biphenyl]-4-yl)oxy]methyl]-4-[(4-methyl-2-oxo-2H-lbenzopyran-6-yl)oxy]butyl]-N-hydroxyformamid;
(±)-N-[1-[[(4'-Cyano[1,1'-biphenyl]-4-yl)oxy]methyl]-4-[(4-methyl-2-oxo-2H-1-benzopyran-7-yl)oxy]butyl]-N-hydroxyformamid;
(±)-N-[1-[[(4'-Cyano[1,1'-biphenyl]-4-yl)oxy]methyl]-5-[(4-methyl-2-oxo-2H-[benzopyran-7-yl)oxy]pentyl]-N-hydroxyformamid;
(±)-N-[1-[[(4'-Cyano[1,1'-biphenyl]-4-yl)oxy]methyl]-2-(5,5-dimethyl-2,4-dioxo-3-oxazolidinyl)ethyl]-N-hydroxyformamid;
(±)-N-[1-[[(4'-Cyano[1,1'-biphenyl]-4-yl)sulfonyl]methyl]-2-(3,4,4-trimethyl-2,5-dioxo-1-imidazolidinyl)ethyl]-N-hydroxyformamid;
(±)-N-[1-[[(4'-Cyano[1,1'-biphenyl]-4-yl)oxy]methyl]-2-(3-methyl-2,5-dioxo-1-imidazolidinyl)ethyl]-N-hydroxyformamid;
(±)-N-[1-[[(4'-Cyano[1,1'-biphenyl]-4-yl)sulfonyl]methyl]-2-(4,4-dimethyl-2,5-dioxo-1-imidazolidinyl)ethyl]-N-hydroxyformamid;
(±)-N-[1-[[(4'-Chlor-[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(3'-Cyanomethyl-[1,1'-biphenyl]-4-yl)oxy]methyl]-2-(3,5,5-trimethyl-2,4-dioxo-1-imidazolidinyl)propyl]-N-hydroxyformamid;
(±)-N-[1-[[(4'-Cyano-[1,1'-biphenyl]-4-yl)oxy]methyl]-2-isopropylthioethyl]-N-hydroxyformamid;
(±) -N-[1-[[(3'-Cyanomethyl-[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(4'-Cyano-[1,1'-biphenyl]-4-yl)oxy]methyl]-2-(3-ethyl-4,4-dimethyl-2,5-dioxo-1-imidazolidinyl)ethyl]-N-hydroxyformamid;
(±)-N-[1-[[(4'-Cyano-[1,1'-biphenyl]-4-yl)oxy]methyl]-2-(3-benzyl-4,4-dimethyl-2,5-dioxo-1-imidazolidinyl)ethyl] -N-hydroxyformamid;
(±)-N-[1-[[(4'-Cyano-[1,1'-biphenyl]-4-yl)oxy]methyl]-2-(3,5,5-trimethyl-2,4-dioxo-1-imidazolidinyl)ethyl]-N-hydroxyformamid;
(±)-N-[1-[[(4'-Methoxy[1,1'-biphenyl]-4-yl)sulfonyl]methyl]ethyl}-N-hydroxyformamid;
(±)-N-[1-[[(4'-Chlor[1,1'-biphenyl]-4-yl)sulfonyl]methyl]ethyl]-N-hydroxyformamid;
(±)-N-[1-[[[4-(1,3-Benzodioxol-5-yl)phenyl]sulfonyl]methyl]ethyl]-N-hydroxyformamid;
(±)-N-[1-[[[4-(4-Chlorphenoxy)phenyl]sulfonyl]methyl]ethyl]-N-hydroxyformamid;
(±)-N-[1-[[(4'-Methoxy[1,1'-biphenyl]-4-yl)sulfonyl]methyl]propyl]-N-hydroxyformamid;
(±)-N-[1-[1,1-Dimethyl-2-[(4'-(trifluormethyl)[1,1'-biphenyl]4-yl]sulfonyl]ethyl]-N-hydroxyformamid;
(±)-N-[1-[(Phenylmethoxy)methyl]-2-[[4'-(trifluormethyl)[1,1'biphenyl]-4-yl]sulfonyl]ethyl]-N-hydroxyformamid;
(±)-N-[1-(Hydroxymethyl)-2-[[(4'-(trifluormethyl) [1,1'biphenyl]-4-yl] sulfonyl]ethyl]-N-hydroxyformamid;
(±)-N-[1-[(4,4-Dimethyl-2,5-dioxo-1-imidazolidinyl)methyl]-2-[[4'(trifluormethyl)[1,1'-biphenyl]-4-yl]thio]ethyl]-N-hydroxyformamid;
(±)-N-[1-[(4,4-Dimethyl-2,5-dioxo-1-imidazolidinyl)methyl]-2-[[4'-(trifluormethyl)[1,1'-biphenyl]-4-yl]sulfonyl]ethyl]-N-hydroxyformamid;
(±)-N-[1-[(2,5-Dioxo-1-imidazolidinyl)methyl]-2-[[4'-(trifluormethoxy)[1,1'-biphenyl]-4-yl]oxy]ethyl]-N-hydroxyformamid;
(±)-N-[1-[[[4'-(Trifluormethyl) [1,1'-biphenyl]-4-yl]sulfonyl]methyl]-2-(3,4,4-trimethyl-2,5-dioxo-1-imidazolidinyl)ethyl]-N-hydroxyformamid;
(±)-N-[1-[[(4'-Butyl[1,1'-biphenyl]4-yl)oxy]methyl]-2-(3-methyl-2,5-dioxo-imidazolidinyl)ethyl]-N-hydroxyformamid;
(±)-N-[1-[(3-Methyl-2,5-dioxo-1-imidazolidinyl)methyl]-2-[[4'-(trifluormethoxy)[1,1'-biphenyl]-4-yl]oxy]ethyl]-N-hydroxyformamid;
N-[4-[4-[(4'-Chlor[1,1'-biphenyl]-4-yl)sulfonyl]methyl]tetrahydro-2H-pyran-4-yl]-N-hydroxyformamid;
(±)-N-[1-[[[4-(4-Chlorphenoxy)phenyl]sulfonyl]methyl]-2-(4,4-dimethyl-2,5dioxo-1-imidazolidinyl)ethyl]-N-hydroxyformamid;
(±)-N-[1-[[[4-(4-Chlorphenoxy)phenyl]sulfonyl]methyl]-2-(3,4,4-trimethyl-2,5-dioxo-1-imidazolidinyl)ethyl]-N-hydroxyformamid;
(±)-N-[1-[[(4-Butyl[1,1'-biphenyl]4-yl)sulfonyl]methyl]-2-(3,4,4-trimethyl-2,5-dioxo-1-imidazolidinyl)ethyl]-N-hydroxyformamid;
(±)-N-[1-[[(4'-Butyl[1,1'-biphenyl]-4-yl)oxy]methyl]-2-(4,4-dimethyl-2,5-dioxo-imidazolidinyl)ethyl]-N-hydroxyformamid;
(±)-N-[1-[[[3'-(Cyanomethyl)[1,1'-biphenyl]-4-yl]oxy]methyl]-2-(4,4-dimethyl-2,5-dioxo-1-imidazolidinyl)ethyl]-N-hydroxyformamid;
(±)-N-[1-[4-(2-Thienyl)phenoxy]methyl]-2-[1-(3,4,4-trimethyl-2,5-dioxo-1-imidazolidinyl)ethyl]-N-hydroxyformamid;
(±)-N-[1-[[(3-Nitro[1,1'-biphenyl]-4-yl)oxy]methyl]-2-(3,4,4-trimethyl-2,5-dioxo-1-imidazolidinyl)ethyl]-N-hydroxyformamid;
(±)-N-[1-[[(4'-Methyl[1,1'-biphenyl]4-yl)oxy]methyl]-2-[[3-(methylsulfonyl)-amino]phenyl]ethyl]-N-hydroxyformamid;
(±)-N-[1-[[[3-(Diethylamino)carbonyl]phenyl]methyl]-2-[(4'methyl[1,1'-biphenyl]-4-yl)oxy]ethyl]-N-hydroxyformamid;
(±)-N-[1-[[(4'-Cyano[1,1'-biphenyl]-4-yl]oxy]methyl]-2-[(4'cyano[1,1'biphenyl]-4-yl)oxy]ethyl]-N-hydroxyformamid;
(±)-N-[1-[[(4'-Cyano[1,1'-biphenyl]-4-yl)oxy]methyl]-2-(4,4-dimethyl-2,5-dioxo-1-imidazolidinyl)ethyl]-N-hydroxyformamid;
(±)-N-[1-[(4,4-Dimethyl-2,5-dioxo-1-imidazolidinyl)methyl]-2-[[4'-(2-methoxyethoxy)[1,1'-biphenyl]-4-yl]oxy]ethyl]-N-hydroxyformamid;
(±)-N-[1-[(4,4-Dimethyl-2,5-dioxo-1-imidazolidinyl)methyl]-2-[(4'-propoxy[1,1'-biphenyl]-4-yl)oxy]ethyl]-N-hydroxyformamid;
(±)-N-[1-[(4,4-Dimethyl-2,5-dioxo-1-imidazolidinyl)methyl]-2-[(4'-pentyloxy[1,1'-biphenyl]4-yl)oxy]ethyl]-N-hydroxyformamid;
(±)-N-[1-[[[3'-(Cyanomethyl)[1,1'-biphenyl]-4-yl]sulfonyl]methyl]-3-(4,4-dimethyl-2,5-dioxo-1-imidazolidinyl)propyl]-N-hydroxyformamid;
(±)-N-[1-[[[4'-(Trifluormethoxy)[1,1'-biphenyl]-4-yl]sulfonyl]methyl]-2- (3,4,4-trimethyl-2,5-dioxo-1-imidazolidinyl)ethyl]-N-hydroxyformamid;
(±)-N-[1-[[(4'-Cyano[1,1'-biphenyl]-4-yl)sulfonyl]methyl]-2-(3-methyl-2,5-dioxo-1-imidazolidinyl)ethyl]-N-hydroxyformamid;
(±)-N-[1-[[[3'-(Cyanomethyl)[1,1'-biphenyl]-4-yl]sulfonyl]methyl]-2- (3,4,4-trimethyl-2,5-dioxo-1-imidazolidinyl)ethyl]-N-hydroxyformamid;
(±)-N-[1-[[(4'-Cyano[1,1''-biphenyl]-4-yl)oxy]methyl]-2-(1,6-dihydro-3-methyl6-oxo-1-pyridazinyl)ethyl]-N-hydroxyformamid;
(±)-N-[1-[[(4'-Cyano[1,1'-biphenyl]-4-yl)sulfonyl]methyl]-2- (4,4-dimethyl-2,5-dioxo-1-imidazolidinyl) ethyl]-N-hydroxyformamid;
(±)-N-[1-[[[4-(4-Fluorphenoxy)phenyl]sulfonyl]methyl]-2-(4,4-dimethyl-2,5-dioxo-1-imidazolidinyl)ethyl]-N-hydroxyformamid;
(±)-N-[1-[[4-(4-Pyridinyl)phenoxy]methyl]-2-(3,4,4-trimethyl-2,5-dioxo-1-imidazolidinyl)ethyl]-N-hydroxyformamid;
(S)-N-[1-[(4,4-Dimethyl-2,5-dioxo-1-imidazolidinyl)methyl]-2-[[4'(trifluormethoxy)[1,1'-biphenyl]-4-yl]oxy]ethyl]-N-hydroxyformamid;
(R)-N-[1-[(4,4-Dimethyl-2,S-dioxo-1-imidazolidinyl)methyl]-2-[[4'(trifluormethoxy)[1,1'-biphenyl]-4-yl]oxy]ethyl]-N-hydroxyformamid;
N-[1-[[[4'-(Trifluormethoxy))[1,1'-biphenyl]-4-yl]oxy]methyl]-3-(4,4-dimethyl-2,5-dioxo-1-imidazolidinyl)propyl]-N-hydroxyformamid;
N-[1-[4-[(4-Pyridinylthio)phenoxy]methyl]-2-(4,4-dimethyl-2,5-dioxo-1-imidazolidinyl)ethyl]-N-hydroxyformamid;
N-[1-[[[(4-Chlorphenoxy)phenyl]sulfonyl]methyl]-3-(4,4-dimethyl-2, 5-dioxo-1-imidazolidinyl)propyl]-N-hydroxyformamid;
N-[1-[[(4'-Cyano[1,1'-biphenyl]-4-yl)oxy]methyl]-2-(1,6-dihydro-6-oxo-1-pyridazinyl)ethyl]-N-hydroxyformamid;
N-[1-[[[4'-(Aminosulfonyl)[1,1'-biphenyl]-4-yl)oxy]methyl]-2-(3,4,4-trimethyl-2,5-dioxo-1-imidazolidinyl)ethyl]-N-hydroxyformamid;
N-[1-[[[4'-(Trifluormethoxy)[1,1'-biphenyl]-4-yl]sulfonyl]methyl]-2-(4,4dimethyl-2,5-dioxo-1 -imidazolidinyl)ethyl]-N-hydroxyformamid;
N-[1-[4-[(4-Pyridinyloxy)phenyl]sulfonyl]]ethyl]-N-hydroxyformamid;
N-[1-[[[(4-Cyanophenoxy)phenyl]sulfonyl]methyl]-2-(4,4-dimethyl-2,5-dioxo-1-imidazolidinyl)ethyl]-N-hydroxyformamid;
N-[1-[[4-[[4-(Trifluormethoxy)phenoxy]phenyl]sulfonyl]methyl]-3-(4,4-dimethyl-2,5-dioxo-1-imidazolidinyl)propyl]-N-hydroxyformamid; und
N-[1-[[4-[[4-(Trifluormethoxy)phenoxy]phenyl]sulfonyl]methyl]-2-(4,4-dimethyl-2,5-dioxo-1-imidazolidinyl)ethyl]-N-hydroxyformamid.

16. Eine Verbindung gemäß Anspruch 15, die
(S)-N-[1-[(4,4-Dimethyl-2,5-dioxo-1-imidazolidinyl)methyl]-2-[[4'-(trifluormethoxy)[1,1'-biphenyl]-4-yl]oxy]ethyl]-N-hydroxyformamid ist.

17. Verwendung einer Verbindung von Anspruch 1 zur Herstellung eines Medikaments zur Hemmung von Matrixmetallproteinasen in einem Säugetier, das einer solchen Behandlung bedarf.

18. Eine Zusammensetzung zur Hemmung von Matrixmetallproteinasen, die einen pharmazeutischen Träger und eine therapeutisch effektive Menge einer Verbindung von Anspruch 1 umfaßt.

## Revendications

1. Composé de la formule (1) 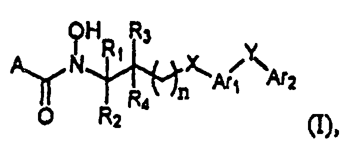 ou sel ou produit dérivé pharmaceutiquement acceptable, dans lequel
A est de l'hydrogène
R₁ est de l'hydrogène ou un alcoyle d'un à quatre atomes de carbone ;
R₃ est choisi dans le groupe composé de
(1) hydrogène et
(2) alcoyle d'un à quatre atomes de carbones ;
R₄ est de l'hydrogène
R₂ est choisi dans le groupe composé de :
(1) hydrogène
(2) alcoyle d'un à quatre atomes de carbone
(3) hydroxyalcoyle, où le groupe d'alcylène est d'un à quatre atomes de carbone
(4) -(alcylène)-S(O)ₚ-alcoyle, où l'alcylène est d'un à quatre atomes de carbone, l'acoyle est d'un à quatre atomes de carbone, et p est égal à zéro ou deux ;
(5) phényle ;
(6) phénylalcoxyalcoyle, où l'alcylène et les groupes d'alcoyle sont indépendamment d'un à quatre atomes de carbone ;
(7) phénylalcoyle, où le groupe d'alcylène est d'un à quatre atomes de carbone ;
(8) phénoxyalcoyle, où le groupe d'alcylène est d'un à quatre atomes de carbone ;
(9) -(alcylène)-N(R₅)SO₂-phényl, où l'alcylène est d'un à quatre atomes de carbone et où R₅ est un alcoyle d'un à deux atomes de carbone
(10) (hétérocycle)oxyalcoyle, où le groupe d'alcylène est d'un à quatre atomes de carbone et l'hétérocycle est de 4-méthyl-2-oxo-2H-1-benzopyranyle ;
(11) -(alcylène)-hétérocycle, où le groupe d'alcylène est d'un à quatre atomes de carbone et l'hétérocycle est 1,6-dihydro-3-méthyl-6-oxo-1-pyridazinyle ;
(12) -(alcylène)-NR₆R₇, où le groupe d'alcylène est d'un à quatre atomes de carbone, où pour (5)-(9), le phényle et les parties de phényle du phéhylalcoxyalcoyle, phénylalcoyle, -(alcylène)-N(R₅)SO₂-phényle, et le phénoxyalcoyle sont éventuellement substitués par un ou deux substituants choisis indépendamment dans le groupe composé de :
(a) alcoyle d'un à quatre atomes de carbone
(b) halo
(c) -N(R₅)SO₂-alcoyle, et
(d) phényle, où le phényle est éventuellement substitué par un substituant de cyano, et
R₆ et R₇ pris ensemble avec l'atome d'azote auquel ils sont fixés définissent un groupe choisi dans le groupe composé de :
(1) phtalimidyle
(2) succinimidyle ;
(3) 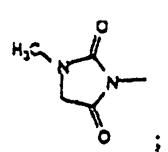
(4) 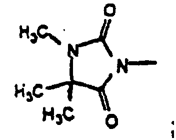
(5) 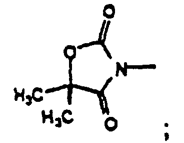
(6) 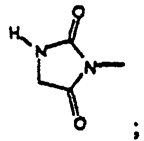
(7) 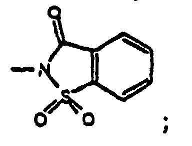
(8) 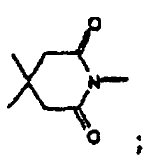 et
(9) 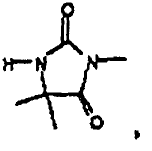
où l'option (2) est éventuellement substituée par un substituant d'un ou deux alcoyles d'un à quatre atomes de carbone et l'option (9) est éventuellement substituée par un substituant de phénylméthyle ;
X est choisi dans le groupe composé de
(1) -O- et
(2) -S(O)ₚ- où p est égal à zéro ou deux
Ar₁ est représenté par du phényle qui est éventuellement substitué par un substituant de nitro ;
Y est choisi dans le groupe composé de
(1) une liaison covalente
(2) -O-
un alcénylène de deux à quatre atomes de carbone
(4) le pipéridinyle et
(5) -S(O)ₚ- ; et
Ar₂ est un groupe d'aryle choisi dans le groupe composé de
(1) phényle
(2) pyridyle
(3) furyle et
(4) thiényle
où le groupe d'aryle est éventuellement substitué par un, deux ou trois substituants indépendamment choisis dans le groupe composé de :
(a) alcoyle d'un à quatre atomes de carbone
(b) alkoxy d'un à quatre atomes de carbone
(c) alkoxy d'un à quatre atomes de carbone substitué par un alkoxy d'un à quatre atomes de carbone ;
(d) cyano
(e) cyanoalcoyle d'un à quatre atomes de carbone
(f) halo
(g) haloalcoyle d'un à quatre atomes de carbone
(h) thioalkoxy d'un atome de carbone
(i) perfluoroalcoyle d'un atome de carbone
(j) perfluoroalkoxy d'un atome de carbone
(k) sulfinylalcoyle où la partie d'alcoyle est d'un à quatre atomes de carbone
(l) sulfonylalcoyle où la partie d'alcoyle est d'un à quatre atomes de carbone et
(m) 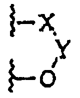 où X est -O- et Y est -(C(R'')₂)ᵥ-, où R'' est de l'hydrogène.

2. Composé selon la revendication 1 dans lequel
R₁ et R₃ sont choisis indépendamment dans le groupe composé d'hydrogène et de méthyle
R₄ est de l'hydrogène
X est choisi dans le groupe composé de
(1) -O- et
(2) -S(O)ₚ- et
Ar₁ est du phényle

3. Composé selon la revendication 2 dans lequel R₂ est un alcoyle d'un à quatre atomes de carbone.

4. Composé selon la revendication 3 choisi dans le groupe composé de :
(±)-N-[1-[[[3'-(cyanométhyl)-[1,1'-biphényl]-4-yl]oxy]méthyl]pentyl]-N-hydroxyformamide ;
(±)-N- [1- [[(4'-cyano-[1,1'-biphényl]-4-yl)oxy]méthyl]-3-méthylbutyl]-N-hydroxyformamide ;
(±)-N-[1-[[(4'-cyano-[1,1'-biphényl]-4-yl)oxy]méthyl]-2-méthylbutyl]-N-hydroxyformamide
(±)-N-[1-[[(4'-cyano-[1,1'-biphényl]-4-yl)oxy]méthyl]pentyl]-N-hydroxyformamide
(±)-N-[1-[[(4'-cyano-[1,1'-biphényl]-4-yl)oxy]méthyl]éthyl]-N-hydroxyformamide
(±)-N-[2-[(4'-cyano-[1,1'-biphényl]-4-yl)oxy]-1-méthylpropyl]-N-hydroxyformamide
(±)-N-[1-[[(4'-méthoxy-[1,1'-biphényl]-4-yl)sulfonyl]méthyl]éthyl]-N-hydroxyformamide
(±)-N-[1-[[(4'-chloro-[1,1'-biphényl]-4-yl)sulfonyl]méthyléthyl]-N-hydroxyformamide
(±)-N-[1-[[[4-(1,3-benzodioxol-5-yl)phényl] sulfonyl]méthyl]éthyl]-N-hydroxyformamide
(±)-N- [1- [[[4-4-chlorophénoxy)phényl]sulfonyl] méthyl]éthyl]-N-hydroxyformamide
(±)-N-[1-[[(4'-méthoxy[1,1'-biphényl]-4-yl) sulfonyl]méthyl]propyl]-N-hydroxyformamide
(±)-N-[1-[1,1-diméthyl-2-[(4'-(trifluorométhyl) [1,1'-biphényl]-4-yl]sulfonyl]éthyl]-N-hydroxyformamide et
N-[1-[4-[(4-pyridinyloxy)phényl]sulfonyl]]éthyl]-N-hydroxyformamide.

5. Composé selon la revendication 2, dans lequel R₂ est de l'hydrogène.

6. Composé selon la revendication 5, qui est du N-[2-[(4'-cyano-[1,1'-biphényl]-4-yl)oxy]éthyl]-N-hydroxyformamide.

7. Composé selon la revendication 2, dans lequel R₂ est choisi dans le groupe composé de
(1) phényle, où le groupe de phényle est non substitué ou substitué
(2) phénoxyalcoyle, où le groupe d'alcylène est d'un à quatre atomes de carbone et où le groupe de phényle est non substitué ou substitué
(3) -(alcylène)-S(O)ₚ-alcoyle
(4) hydroxyalcoyle, où le groupe d'alcylène est d'un à quatre atomes de carbone
(5) -(alcylène)-N(R₅)SO₂-phényle, où l'alcylène est d'un à quatre atomes de carbone, le groupe de phényle est non substitué ou substitué, et R₅ est un alcoyle d'un à deux atomes de carbone.
(6) Phénylalkoxyalcoyle, où les groupes d'alcylène et d'alcoyle sont indépendamment d'un à quatre atomes de carbone et le groupe de phényle est non substitué ou substitué ;
(7) (hétérocycle)oxyalcoyle, dans lequel le groupe d'alcylène est d'un à quatre atomes de carbone et l'hétérocycle est non substitué ou substitué et
(8) phénylalcoyle, où le groupe d'alcylène est d'un à quatre atomes de carbone et dans lequel le groupe de phényle est non substitué ou substitué.

8. Composé selon la revendication 7 choisi dans le groupe composé de
(±)-N-[1-[[(4'-cyano-[1,1'-biphényl]-4-yl)oxy] méthyl]-2-phénoxyéthyl]-N-hydroxyformamide
(±)-N-[1-[[(4'-cyano-[1,1'-biphényl]-4-yl)oxy] méthyl]-2-(4-méthylphényl)éthyl]-N-hydroxyformamide
(±)-N-[2-[(4'-cyano-[1,1'-biphényl]-4-yl)oxy]-1-(4-fluorophényl)éthyl]-N-hydroxyformamide
(=)-N-[1-[[(4'-cyano-[1,1'-biphényl]-4-yl)oxy] méthyl]-2-(4-fluorophényl)éthyl]-N-hydroxyformamide
(±)-N-[1-[[(3'-cyano[1,1'-biphényl]-4-yl)oxy] méthyl]-2-[méthyl[(4-méthylphényl)sulfonyl]amino]éthyl]-N-hydroxyformamide
(±)-N-[1-[[(4'-cyano[1,1'biphényl]-4-yl)oxy] méthyl]-5-[(4-méthyl-2-oxo-2H-1-benzopyran-6-yl]oxy]pentyl]-N-hydroxyformamide
(±)-N-[1-[[(4'-cyano[1,1'-biphényl]-4-yl)oxy]méthyl] -4-[(4-méthyl-2-oxo-2H-1-benzopyran-6-yl)oxy]butyl]-N-hydroxyformamide
(±)-N-[1-[[(4'-cyano[1,1'-biphényl]-4-yl)oxy] méthyl]-4-[(4-méthyl-2-oxo-2H-1-benzopyran-7-yl)oxy] butyl] -N-hydroxyformamide
(±)-N-[1-[[(4'-cyano[1,1'-biphényl]-4-yl)oxy] méthyl]-5-[(4-méthyl-2-oxo-2H-1-benzopyran-7-yl]oxy]pentyl]-N-hydroxyformamide
(=)-N-[1-[[(4'-cyano-[1,1'-biphényl]-4-yl)oxy] méthyl]-2-isopropylthioéthyl]-N-hydroxyformamide
(+)-N- [1-[(phénylméthoxy)méthyl]-2- [[4'-(trifluorométhyl)[1,1'-biphényl]-4-yl]sulfonyl]éthyl]-N-hydroxyformamide
(±)-N-[1-(hydroxyméthyl)-2- [[(4'-(trifluorométhyl)[1,1'-biphényl]-4-yl]sulfonyl]éthyl]-N-hydroxyformamide ;
(±)-N-[1-[[(4'-méthyl[1,1'-biphényl]-4-yl]oxy] méthyl]-2-[[3-(méthylsulfonyl)-amino)phényl]éthyl]-N-hydroxyformamide
(±)-N-[1-[[(3-(diéthylamino)carbonyl]phényl] méthyl]-2-[(4'-méthyl[1,1'-biphényl]-4-yl)oxy]éthyl]-N-hydroxyformamide
N-[1-[[(4'cyano[1,1'-biphényl]-4-yl)oxy]méthyl]-2-[(4'-cyano[1,1'-biphényl]-4-yl)oxy]éthyl]-N-hydroxyformamide
N-[1-[[(4'-cyano[1,1'-biphényl]-4-yl)oxyl]méthyl]-2-(1,6-dihydro-3-méthyl-6-oxo-1-pyridanizyl)éthyl]-N-hydroxyformamide et
N-[1-[[(4'-cyano[1,1-biphényl]-4-yl)oxyl]méthyl]-2-(1,6-dihydro-6-oxo-1-pyridazinyl)éthyl]-N-hydroxyformamide.

9. Composé selon la revendication 2 dans lequel R₂ est un -(alcylène)-NR₆R₇

10. Composé selon la revendication 9 dans lequel -NR₆R₇ est

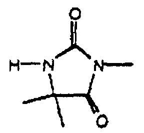

11. Composé selon la revendication 10 choisi dans le groupe composé de
(±)-N-[1-[[(4'-butoxy[1,1'-biphényl]-4-yl)oxy] méthyl]-2-(4,4-diméthyl-2,5-dioxo-1-imidazolidinyl) éthyl]-N-hydroxyformamide
(±)-N- [(4,4-diméthyl-2,5-dioxo-1-imidazolidinyl) méthyl]-2-[(4'-éthoxy[1,1'-biphényl]-4-yl)oxy]éthyl]-N-N-hydroxyformamide
(±)-N-[1-[[4-(1,3-benzodioxol-5-yl)phénoxy] méthyl]-2-(4,4-diméthyl-2,5-dioxo-1-imidazolidinyl) éthyl]-N-hydroxyformamide
(±)-N- [1- [(4,4-diméthyl-2,5-dioxo-1-imidazolidinyl)méthyl]-2-[(4'-(trifluorométhoxy)[1,1'-biphényl]-4-yl]oxy]éthyl]-N-hydroxyformamide
(±)-N-[1-[(4,4-diméthyl-2,5-dioxo-1-imidazolidinyl)méthyl]-2-[[4'-(trifluorométhyl)[1,1'-biphényl]-4-yl]oxy]éthyl]-N-hydroxyformamide
(±)-N-[1-[[[3'-(cyanométhyl)[1,1'-biphényl]-4-yl]oxy]méthyl]-3-(4,4-diméthyl-2,5-dioxo-1-imidazolidinyl)propyl]]-N-hydroxyformamide
(±)-N-[1-[[(4'-butoxy[1,1'-biphényl]-4-yl)sulfonyl]méthyl]-2-(4,4-diméthyl-2,5-dioxo-1-imidazolidinyl)éthyl]-N-hydroxyformamide
(±)-N-[1-[[(4'-cyano[1,1'-biphényl]-4-yl)oxy] méthyl]-3-(4,4-diméthyl-2,5-dioxo-1-imidazolidinyl) propyl]-N-hydroxyformamide
(±)-N-[1-[[[4'-(méthylsulfonyl)[1,1'-biphényl]-4-yl]oxy]méthyl]-2-(4,4-diméthyl-2,5-dioxo-1-imidazolidinyl)éthyl]-N-hydroxyformamide
(±)-N-[1-[[(4'-cyano[1,1'-biphényl]-4-yl) sulfonyl]méthyl]-2-(4,4-diméthyl-2,5-dioxo-1-imidazolidinyl)éthyl]-N-hydroxyformamide
(±)-N-[1-[(4,4-diméthyl-2,5-dioxo-1-imidazolidinyl)méthyl]-2-[[4'-(trifluorométhyl)[1,1'-biphényl]-4-yl]thio]éthyl]-N-hydroxyformamide
(±)-N-[1-[(4,4-diméthyl-2,5-dioxo-1-imidazolidinyl)méthyl]-2-[[4'-(trifluorométhyl)[1,1'-biphényl]-4-yl]sulfonyl]éthyl]-N-hydroxyformamide
(±)-N-[1-[[[4-(4-chlorophénoxy)phényl] sulfonyl]méthyl]-2-(4,4-diméthyl-2,5-dioxo-1-imidazolydinyl)éthyl]-N-hydroxyformamide
(±)-N-[1-[[(4'-butyl[1,1'-biphényl]-4-yl) oxy]méthyl]-2(4,4-diméthyl-2,5-dioxo-1-imidazolidinyl) éthyl]-N-hydroxyformamide
(±)-N-[1-[[[3'-(cyanométhyl)[1,1(-biphényl]-4-yl]oxy]méthyl]-2-(4,4-diméthyl-2,5-dioxo-imidazolidinyl)éthyl]-N-hydroxyformamide
(±)-N-[1-[[(4'-cyano[1,1'-biphényl]-4-yl)oxy] méthyl]-2-(4,4-diméthyl-2,5-dioxo-1-imidazolidinyl) éthyl]-N-hydroxyformamide
(±)-N-[1-[(4,4-diméthyl-2,5-dioxo-1-imidazolidinyl)méthyl]-2- [[4'-(2-méthoxyéthoxy)[1,1'-biphényl]-4-yl]oxy]éthyl]-N-hydroxyformamide
(±)-N-[1-[(4,4-diméthyl-2,5-dioxo-1-imidazolidinyl)méthyl]-2-[(4'-propoxyl[1,1'-biphényl]-4-yl)oxy] éthyl]-N-hydroxyformamide
N-[(4,4-diméthyl-2,5-dioxo-1-imidazolidinyl-méthyl]-2-[(4'-pentyloxy[1,1'-biphényl]-4-yl)oxy]éthyl]-N-hydroxyformamide
(±)-N-[1-[[[3'-(cyanométhyl)[1,1'-biphényl]-4-yl]sulfonyl]méthyl]-3-(4,4-diméthyl-2,5-dioxo-1-imidazolidinyl)propyl]-N-hydroxyformamide
(±)-N-[1-[[(4'-cyano[1,1'-biphényl]-4-yl) sulfonyl]méthyl]-2-(4,4-diméthyl-2,5-dioxo-1-imidazolidinyl)éthyl]-N-hydroxyformamide
(±)-N-[1-[[[4-(4-fluorophénoxy)phényl] sulfonyl)méthyl]-2-(4,4-diméthyl-2,5-dioxo-1-imidazolidinyl)éthyl] -N-hydroxyformamide
(S)-N-[1-[(4,4-diméthyl-2,5-dioxo-1-imidazolidinyl)méthyl]-2-[[4'-(trifluorométhoxy)[1,1'-biphényl]-4-yl]oxy]éthyl]-N-hydroxyformamide
(R)-N-[1-[(4,4-diméthyl-2,5-dioxo-1-imidazolidinyl)méthyl]-2-[[4'-(trifluorométhoxy)[1,1'-biphényl]-4-yl]oxy]éthyl]-N-hydroxyformamide
N-[1-[[[4'-(trifluorométhoxy))[1,1'-biphényl]-4-yl]oxy]méthyl]-3-(4,4-diméthyl-2,5-dioxo-1-imidazolidinyl)propyl]-N-hydroxyformamide
N-[1-[4-[(4-pyridinylthio)phénoxy]méthyl]-2-(4,4-diméthyl-2,5-dioxo-1-imidazolidinyl)éthyl]-N-hydroxyformamide
N-[1-[[[(4-chlorophénoxy)phényl]sulfonyl]méthyl]-3-(4,4-diméthyl-2,5-dioxo-1-imidazolidinyl)propyl]-N-hydroxyformamide
N-[1-[[[4'-(trifluorométhoxy)[1,1'-biphényl]-4-yl]sulfonyl]méthyl]-2-(4,4-diméthyl-2,5-dioxo-1-imidazolidinyl)éthyl]-N-hydroxyformamide
N-[1-[[[(4-cyanophénoxy)phényl]sulfonyl]méthyl]-2-(4,4-diméthyl-2,5-dioxo-1-imidazolidinyl)éthyl]-N-hydroxyformamide et
N-[1-[[4-[[4-(trifluorométhoxy)phénoxy]phényl] sulfonyl]méthyl]-3-(4,4-diméthyl-2,5-dioxo-1-imidazolidinyl)propyl]-N-hydroxyformamide.

12. Composé selon la revendication 9 choisi dans le groupe composé de
(±)-N-[1-[[(4'-cyano-[1,1'-biphényl]-4-yl)oxy] méthyl] -2-(2,3-dihydro-1,3-dioxo-1H-isoindol-2-yl)éthyl]-N-hydroxyformamide
(±)-N-[1-[[(4'-cyano-[1,1'-biphényl]-4-yl)oxy] méthyl]-2-(3,4,4-triméthyl-2,5-dioxoimidazolidine-1-yl)éthyl]-N-hydroxyformamide
(±)-N- [1- [[(4'-cyano-[1,1'-biphényl]-4-yl)oxy] méthyl]-3-(3,4,4-triméthyl-2,5-dioxoimidazolidiny-1-yl)propyl]-N-hydroxyformamide
(±)-N-[1-[4-[(2-E-phényléthènyl)phénoxy]méthyl]-2-(3,4,4-triméthyl-2,5-dioxo-1-imidazolidinyl)éthyl]-N-hydroxyformamide
(±)-N-[1-[[4-(2-furanyl)phénoxy]méthyl]-2-(3,4,4-triméthyl-2,5-dioxo-l-imidazolidinyl)éthyl]-N-hydroxyformamide
(±)-N-[1-[((4'-butoxy[1,1'-biphényl]-4-yl)oxy] méthyl]-2-(3,4,4-triméthyl-2,5-dioxo-1-imidazolidinyl)éthyl]-N-hydroxyformamide
(±)-N-[1-[[(4(-fluoro[1,1'-biphényl]-4-yl)oxy]méthyl]-2-(3,4,4-triméthyl-2,5-dioxo-1-imidazolicinyl)éthyl]-N-hydroxyformamide
(±)-N-[1-[(3,4,4-triméthyl-2,5-dioxo-1-imidazolidinyl)méthyl]-2-[[4'-(trifluorométhyl)[1,1'-biphényl]-4-yl]oxy]éthyl]-N-hydroxyformamide
(±)-N-[1-[[(4'-méthoxy[1,1'-biphényl]-4-yl)oxy] méthyl]-2-(3,4,4-triméthyl-2,5-dioxo-1-imidazolidinyl)éthyl]-N-hydroxyformamide
(±)-N-[1-[[(4'-méthyl[1,1'-biphényl]-4-yl)oxy] méthyl]-2-(3,4,4-triméthyl-2,5-dioxo-1-imidazolidinyl)éthyl]-N-hydroxyformamide
(±)-N-[1-[[(4'-butoxy[1,1-biphényl]-4-yl)oxy] méthyl]-2-(3-méthy-2,5-dioxo-1-imidazolidinyl)éthyl]-N-hydroxyformamide
(±)-N-[1-[[4-(3-thiényl)phénoxy]méthyl]-2-(3,4,4-triméthyl-2,5-dioxo-1-imidazolidinyl)éthyl]-N-hydroxyformamide
(±)-N-[1-[[([1,1'-biphényl]-4-yl)oxy)méthyl)-2-(3,4,4-triméthyl-2,5-dioxo-1-imidazolidinyl]éthyl]-N-hydroxyformamide
(±)-N-[1-[[(3'-chloro-4'-fluoro[1,1'-biphényl]-4-yl)oxy]méthyl]-2-(3,4,4-triméthyl-2,5-dioxo-1-imidazolidinyl)éthyl]-N-hydroxyformamide
(±)-N-[1-[[(2'-méthyl[1,1-biphényl]-4-yl)oxy] méthyl]-2-(3,4,4-triméthyl-2,5-dioxo-1-imidazolidinyl) éthyl]-N-hydroxyformamide
(±)-N-[1-[[(4'-cyano[1,1-biphényl]-4-yl)oxy] méthyl]-2-(2,5-dioxo-1-imidazolidinyl)éthyl]-N-hydroxyformamide
(±)-N-[1-[[(4'-cyanol[1,1'-biphényl]-4-yl)oxy] méthyl]-2-(1,1-dioxido-3-oxo-1,2-benzisothiazol-2(3H)-yl)éthyl]-N-hydroxyformamide
(±)-N-[1-[[4-(4-phényl-1-pipéridinyl) phénoxy]méthyl]-2-(3,4,4-triméthyl-2,5-dioxo-1-imidazolidinyl)éthyl]-N-hydroxyformamide
(±)-N-[1-[[(4'-cyano[1,1'-biphényl]-4-yl)oxy] méthyl]-2-[4,4-diméthyl-2,5-dioxo-3-(3-pyridinylméthyl)-1-imidazolidinyl]éthyl]-N-hydroxyformamide
(±)-N-[1-[[(3'-cyano[1,1'-biphényl]-4-yl)oxy] méthyl]-2-(3,4,4-triméthyl-2,5-dioxo-1-imidazolidinyl) éthyl]-N-hydroxyformamide
(±)-N-[1-[[[4'-(méthylthio) [1,1'-biphényl]-4-yl]oxy]méthyl]-2-(3,4,4-triméthyl-2,5-dioxo-1-imidazolidinyl)éthyl]-N-hydroxyformamide
(±)-N-[1-[4[[4-(trifluorométhyl)phénoxy] phénoxy]méthyl]-2-(3,4,4-triméthyl-2,5-dioxo-1-imidazolidinyl)éthyl] -N-hydroxyformamide
(±)-N-[1-[[[4'-(trifluorométhoxy)[1,1'-biphényl]-4-yl]oxy]méthyl]-2-(3,4,4-triméthyl-2,5-dioxo-1-imidazolidinyl)éthyl]-2-N-hydroxyformamide
(±)-N-[1-[[[4'-(méthylsulfonyl)[1,1'-biphényl]-4-yl]oxy]méthyl]-2-(3,4,4-triméthyl-2,5-dioxo-1-imidazolidinyl)éthyl] -N-hydroxyformamide
(±)-N-[1-[[[3'-(cyanométhyl)-4'-méthoxyl[1,1'-biphényl]-4-yl]oxy]méthyl]-2-(3,4,4-triméthyl-2,5-dioxo-1-imidazolidinyl)éthyl]-N-hydroxyformamide
(±)-N-[1-[[(3'-cyano[1,1'-biphényl]-4-yl)oxy]méthyl]-2-(2,5-dioxo-1-pyrrolidinyl)éthyl]-N-hydroxyformamide
(±)-N-[1-[[(4'-cyano[1,1'-biphényl]-4-yl)oxy]méthyl]-2-(4,4-diméthyl-2,6-dioxo-1-pipéridinyl) éthyl]N-hydroxyformamide
N-[1S-[[4'-cyano[1,1'-biphényl]-4-yl)oxy]méthyl]-2-(2,5-dioxo-1-pyrrolidinyl)éthyl]-N-hydroxyformamide
N-[1R-[[(4'-cyano[1,1'-biphényl]-4-yl)oxy]méthyl]-2-(2,5-dioxo-1-pyrrolidinyl)éthyl]-N-hydroxyformamide
(±)-N-[1-[[(4'-cyano[1,1'-biphényl]-4-yl)oxy] méthyl]-2-(3-éthyl-3-méthyl-2,5-dioxo-1-pyrrolidinyl) éthyl]-N-hydroxyformamide
(±)-N-[1-[(4'-cyano[1,1'-biphényl]-4-yl)oxy] méthyl]-2-(5,5-diméthyl-2,4-dioxo-3-oxazolidinyl)éthyl]-N-hydroxyformamide
(±)-N-[1-[[(4'-cyano[1,1'-biphényl]-4-yl) sulfonyl]méthyl]-2-(3,4,4-triméthy-2,5-dioxo-1-imidazolidinyl)éthyl]-N-hydroxyformamide
(±)-N-[1-[[(4'-cyano[1,1'-biphényl]-4-yl)oxy] méthyl]-2-(3-méthyl-2,5-dioxo-1-imidazolidinyl) éthyl]-N-hydroxyformamide
(±)-N-[1-[[(4'-chloro-[1,1'-biphényl]-4-yl)oxy] méthyl]-2-(3,4,4-triméthyl-2,5-dioxo-1-imidazolidinyl) éthyl]-N-hydroxyformamide
(±)-N-[1-[[(3'-cyanométhyl-[1,1'-biphényl]-4-yl)oxy]méthyl]-2-(3,5,5-triméthyl-2,4-dioxo-1-imidazolidinyl)propyl]-N-hydroxyformamide
(±)-N-[1-[[(3'-cyanométhyl-[1,1'-biphényl]-4-yl]oxy]méthyl]-2-(3,4,4-triméthyl-2,5-dioxo-1-imidazolidinyl)éthyl]-N-hydroxyformamide
(±)-N-[1-[[(4'-cyano-[1,1'-biphényl)-4-yl)oxy] méthyl]-2-(3-éthyl-4,4-diméthyl-2,5-dioxo-1-imidazolidinyl)éthyl]-N-hydroxyformamide
(±)-N-[1-[[(4'-cyano-[1,1'-biphényl]-4-yl)oxy] méthyl]-2-(3-benzyl-4,4-diméthyl-2,5-dioxo-1-imidazolidinyl)éthyl]-N-hydroxyformamide
(±)-N-[1-[[(4'-cyano-[1,1'-biphényl]-4-yl)oxy] méthyl]-2-(3,5,5-triméthyl-2,4-dioxo-1-imidazolidinyl)éthyl]-N-hydroxyformamide
(±)-N-[1-[(2,5-dioxo-1-imidazolidinyl)méthyl]-2-[[4'-(trifluorométhoxy)[1,1'-biphényl]-4-yl)oxy]éthyl]-N-hydroxyformamide
(±)-N-[1-[[[4'-(trifluorométhyl)[1,1'-biphényl]-4-yl]sulfonyl]méthyl]-2-(3,4,4-triméthyl-2,5-dioxo-1-imidazolidinyl)éthyl]-N-hydroxyformamide
(±)-N-[1-[[(4'-butyl[1,1'-biphényl]-4-yl)oxy] méthyl]-2-(3-méthy-2,5-dioxo-1-imidazolidinyl)éthyl]-N-hydroxyformamide
(±)-N-[1-[(3-méthy-2,5-dioxo-1-imidazolidinyl) méthyl]-2-[[4'-(trifluorométhoxy)[1,1'biphényl]-4-yl]oxy]éthyl]-N-hydroxyformamide
(±)-N-[1-[[[4-(4-chlorophénoxy)phényl] sulfonyl]méthyl]-2-(3,4,4-triméthyl-2,5-dioxo-1-imidazolidinyl)éthyl]-N-hydroxyformamide
(±)-N-[1-[[(4-butyl[1,1'-biphényl]-4-yl) sulfonyl]méthyl]-2-(3,4,4-triméthyl-2,5-dioxo-1-imidazolidinyl)éthyl]-N-hydroxyformamide
(±)-N-[1-[4-(2-thiényl)phénoxy]méthyl]-2-[1-(3,4,4-triméthyl-2,5-dioxo-1-imidazolidinyl)éthyl]-N-hydroxyformamide
(±)-N-[1-[[(3-nitro[1,1'-biphényl]-4-yl)oxy] méthyl]-2-(3,4,4-triméthyl-2,5-dioxo-1-imidazolidinyl) éthyl]-N-hydroxyformamide
(±)-N-[1-[[[4'-(trifluorométhoxy)[1,1'-biphényl]-4-yl]sulfonyl]méthyl]-2-(3,4,4-triméthy-2,5-dioxo-1-imidazolidinyl)éthyl]-N-hydroxyformamide
(±)-N-[1-[[(4'-cyano[1,1'-biphényl]-4-yl) sulfonyl]méthyl]-2-(3-méthy-2,5-dioxo-1-imidazolidinyl) éthyl]-N-hydroxyformamide
(+)-N-[1-[[(4'-cyano-[1,1-biphényl]-4-yl]oxy]méthyl]pentyl]-N-hydroxyformamide
(+)-N-[1-[[(4'-cyano-[1,1'-biphényl]-4-yl]oxy)méthyl]-2-(4-méthylphényl)éthyl]-N-hydroxyformamide
(±)N-[2-[(4'-cyano-[1,1'-biphényl]-4-yl)oxy]-1-(4-fluoro-phényl)éthyl)-N-hydroxyformamide
(±)-N-[1-[[(4'-cyano-[1,1'-biphényl]-4-yl)oxy]méthyl]-2-(4-fluorophényl)éthyl]-N-hydroxyformamide
(±)-N-[1-[[(4'-cyano-[1,1'-biphényl]-4-yl)oxy]méthyl]éthyl]-N-hydroxyformamide
(±)-N-[2-[(4'-cyano-[1,1'-biphényl]-4-yl)oxy]éthyl]-N-hydroxyformamide
(±)-N-[1-[4-[(2^{E}-phényléthényl)phénoxy)méthyl]-2-(3,4,4-triméthyl-2,5-dioxo-1-imidazolidinyl)éthyl]-N-hydroxyformamide
(±)-N-[1-[[4-(2-furanyl)phénoxy]méthyl]-2-(3,4,4-triméthyl-2,5-dioxo-1-imidazolidinyl)éthyl]-N-hydroxyformamide
(±)-N-[1-[[(4'-butoxy[1,1'-biphényl]-4-yl)oxy)méthyl]-2-(3,4,4-triméthyl-2,5-dioxo-1-imidazolidinyl)éthyl]-N-hydroxyformamide
(±)-N-[1-[[(4'-fluoro[1,1'-biphényl]-4-yl)oxy]méthyl]-2-(3,4,4-triméthyl-2,5-dioxo-1-imidazolidinyl)éthyl]-N-hydroxyformamide ;
(±)-N-[1-[(3,4,4-triméthyl-2,5-dioxo-1-imidazolidinyl)méthyl]-2-[[4'-(trifluorométhyl)[1,1'-biphényl]-4-yl]oxy]éthyl]-N-hydroxyformamide
(+) -N-[1-[[(4'-méthoxy[1,1'-biphényl]-4-yl]oxy]méthyl]-2-(3,4,4-triméthyl-2,5-dioxo-1-imidazolidinyl)éthyl]-N-hydroxyformamide
(±) -N-[1-[[(4'-méthy[1,1'-biphényl]-4-yl]oxy]méthyl]-2-(3,4,4-triméthyl-2,5-dioxo-1-imidazolidinyl)éthyl]-N-hydroxyformamide
(±) -N-[1-[[(4'-butonyl[1,1'-biphényl]-4-yl]oxy]méthyl]-2-(4,4-diméthyl-2,5-dioxo-1-imidazolidinyl)éthyl]-N-hydroxyformamide
(±) -N-[1-[1- [(4,4-diméthyl-2,5-dioxo-1-imidazolinyl)méthyl]-2-[(4'-éthoxy(1,1'-biphényl)-4-yl]oxy]éthyl]-N-hydroxyformamide
(±) -N-[1-[[4-(1,3-benzodioxol-5-yl)phénoxy]méthyl]-2-(4,4-diméthyl-2,5-dioxo-1-imidazolidinyl)éthyl)-N-hydroxyformamide
(±) -N-[1-[[(4'-butoxyl[1,1'-biphényl]-4-yl)oxy]méthyl]-2-(3-méthy-2,5-dioxo-1-imidazolidinyl)éthyl]-N-hydroxyformamide
(±) -N-[1-[[4-(3-thiényl)phénoxy]méthyl]-2-(3,4,4-triméthyl-2,5-dioxo-1-imidazolidinyl)éthyl)-N-hydroxyformamide
(±) -N-[1-[[([1,1'-biphényl]-4-yl)oxy]méthyl]-2-(3,4,4-triméthyl-2,5-dioxo-1-imidazolidinyl]éthyl]-N-hydroxyformamide
(±) -N-[1-[[(3'-chloro-4'-fluoro[1,1'-biphényl]-4-yl)oxy]méthyl]-2-(3,4,4-triméthyl-2,5-dioxo-1-imidazolidinyl)éthyl]-N-hydroxyformamide
(±) -N-[1-[[(2'-méthyl[1,1'-biphényl]-4-yl)oxy]méthyl]-2-(3,4,4-triméthy-2,5-dioxo-1-imidazolidinyl)éthyl]-N-hydroxyformamide
(±) -N-[1-[[(4'-cyanol[1,1'-biphényl]-4-yl)oxy]méthyl]-2-(2,5-dioxo-1-imidazolidinyl)éthyl]-N-hydroxyformamide
(±)-N-[1-[[(4'-cyano-[1,1'-biphényl]-4-yl)oxy]méthyl]-2-(1,1-dioxido-3-oxo-1,2-benzisothiazol-2 (3H)-yl)éthyl]-N-hydroxyformamide
(±)-N-[1-[(4,4-diméthyl-2,5-dioxo-1-imidazolidinyl)méthyl)-2-[[4'-(trifluorométhoxy)[1,1'-biphényl]-4-yl] oxy] éthyl]-N-hydroxyformamide
(±)-N-[1-[(4-(4-phényl-1-pipéridinyl)phénoxy]méthyl]-2-(3,4,4-triméthyl-2, 5-dioxo-1-imidazolidinyl)éthyl]-N-hydroxyformamide
(±)-N-[1-[(4,4-diméthyl-2,5-dioxo-1-imidazolidinyl)méthyl]-2-[[4'-(trifluorométhyl)[1,1'-biphényl]-4-yl]oxy]éthyl]-N-hydroxyformamide
(±)-N-[1-[[(3'-cyano[1,1'-biphényl]-4-yl)oxy]méthyl]-2-[méthyl[(4-méthylphényl)sulfonyl]amino)éthyl]-N-hydroxyformamide
(±)-N-[1-[[(4'-cyano[1,1'-biphényl)-4-yl)oxy]méthyl]-2-[4,4-diméthyl-2,5-dioxo-3-(3-pyridinylméthyl)-1-imidazolidinyl]éthyl]-N-hydroxyformamide
(+)-N-[2-[(4'-cyano[1,1'-biphényl]-4-yl)oxy]-1-méthylpropyl]-N-hydroxyformamide
(±)-N-[1-[[(3'-cyano[1,1'-biphényl]-4-yl)oxy]méthyl]-2-(3,4,4-triméthyl-2,5-dioxo-1-imidazolidinyl)éthyl]-N-hydroxyformamide
(±)-N-[1-[[[4'-(méthylthio)[1,1'-biphényl]-4-yl]oxy]méthyl]-2-(3,4,4-triméthyl-2,5-dioxo-1-imidazolidinyl)éthyl]-N-hydroxyformamide
(±)-N-[1-[4(=-[[4-(trifluorométhyl)phénoxy]méthyl]-2-(3,4,4-triméthyl-2,5-dioxo-1-imidazolidinyl)éthyl)-N-hydroxyformamide
(±)-N-[1-[[[4'-(trifuorométhoxy)[1,1'-biphényl]-4-yl]oxy]méthyl]-2-(3,4,4-triméthy-2,5-dioxo-1-imidazolidinyl)éthyl]-2-N-hydroxyformamide
(±)-N-[1-[[[4'-(méthylsulfonyl)[1,1'-biphényl]-4-yl]oxy]méthyl]-2-(3,4,4-triméthy-2,5-dioxo-1-imidazolidinyl)éthyl]-N-hydroxyformamide
(±)-N-[1-[[[3'-(cyanométhyl)-4'-méthoxyl(1,1'-biphényl]-4-yl]oxy]méthyl]-2- (3,4,4-triméthy-2,5-dioxo-1-imidazolidinyl)éthyl]-N-hydroxyformamide
(±)-N-[1-[((3'-(cyanométhyl)[1,1'-biphényl]-4-yl]oxy]méthyl]-3-(4,4-diméthyl-2,5-dioxo-1-imidazolindyl)propyl]-N-hydroxyformamide
(±)-N-[1-[[(4'-butoxy[1,1'-biphényl]-4-yl)sulfonyl]méthyl]-2-(4,4-diméthyl-2,5-dioxo-1-imidazolidinyl)éthyl]-N-hydroxyformamide
(±)-N-[1-[[(4'-cyano[1,1'-biphényl]-4-yl)oxy]méthyl]-3-(4,4-diméthyl-2,5-dioxo-1-imidazolidinyl)propyl]-N-hydroxyformamide
(±)-N-[1-[[[4'-(méthylsulfonyl)(1,1'-biphényl]-4-yl]oxy]méthyl]-2-(4,4-diméthyl-2,5-dioxo-1-imidazolidinyl)éthyl]-N-hydroxyformamide
(±)-N- [1- [ [(3'-cyano[1,1'-biphényl]-4-yl)oxy]méthyl]-2-(2,5-dioxo-1-pyrrolidinyl)éthyl]-N-hydroxyformamide
(±)-N-[1-[[(4'-cyano[1,1'-biphényl]-4-yl]oxy]méthyl]-2-(4,4-diméthyl-2,6-dioxo-1-ipéridinyl)éthyl]-N-hydroxyformamide
(±)-N-[1S-[[(4'-cyano[1,1'-biphényl]-4-yl)oxy]méthyl]-2-(2,5-dioxo-1-pyrrolidinyl)éthyl]-N-hydroxyformamide
(±)-N-[1R-[[(4'-cyano[1,1'-biphényl]-4-yl)oxy]méthyl)-2-(2,5-dioxo-1-pyrrolidinyl)éthyl]-N-hydroxyformamide
(±)-N-[1-[ [(4'-cyano[1,1'-biphényl]-4-yl)oxy]méthyl]-2-(3-éthyl-3-méthyl-2,5-dioxo-1-pyrrolidinyl)éthyl]-N-hydroxyformamide
N-[4-[4-[[(4-chlorophénoxy)phényl]sulfonyl] méthyl]tétrahydro-2H-pyran-4-yl]-N-hydroxyformamide
(±)-N-[1-[[(4'-cyano[1,1'-biphényl)-4-yl)oxy]méthyl]-2-[[(2-méthoxycarbonyl)-phényl]thio)éthyl]-N-hydroxyformamide
(±)-N-[1-[[(4'-cyano[1,1'-ciphényl]-4-yl)oxy]méthyl]-5-[(4-méthyl-2-oxo-2H-1-benzopyran-6-yl)oxy]pentyl]-N-hydroxyformamide
(±)-N-[1-[[(4'-cyano[1,1'-biphényl]-4-yl)oxy]méthyl]-4-[(4-méthyl-2-oxo-2H-1-benzopyran-6-yl)oxy]butyl]-N-hydroxyformamide
(±)-N-[1-[[(4'-cyano[1,1'-biphényl]-4-yl)oxy]méthyl]-4-[(4-méthyl-2-oxo-2H-l-benzopyran-7-yl)oxy]butyl]-N-hydroxyformamide
(±)-N-[1-[[(4'-cyano[1,1'-biphényl]-4-yl)oxy]méthyl]-5-[(4-méthyl-2-oxo-2H-1-benzopyran-7-yl)oxy]pentyl]-N-hydroxyformamide
(±)-N-[1-[[(4'-cyano[1,1'-biphényl]-4-yl)oxy]méthyl]-2-(5,5-diméthyl-2,4-dioxo-3-oxazolidinyl) éthyl] -N-hydroxyformamide
(±)-N-[1-[[(4'-cyano[1,1'-biphényl]-4-yl)sulfonyl]méthyl]-2-(3,4,4-triméthy-2,5-dioxo-1-imidazolidinyl)éthyl]-N-hydroxyformamide
(±)-N-[1-[[(4'-cyano(1,1'-biphényl]-4-yl)oxy]méthyl]-2-(3-méthyl-2,5-dioxo-1-imidazolidinyl)éthyl]-N-hydroxyformamide
(±)-N-[1-[[(4'-cyano[1,1'-biphényl]-4-yl)sulfonyl]méthyl]-2-(4,4-diméthyl-2,5-dioxo-1-imidazolidinyl)éthyl]-N-hydroxyformamide
(±)-N-[1-[[(4'-chloro-[1,1'-biphényl]-4-yl)oxy]méthyl]-2-(3,4,4-triméthyl-2,5-dioxo-1-imidazolidinyl)éthyl]-N-hydroxyformamide
(±)-N-[1-[[(3'-cyanométhyl-(1,1'-biphényl)-4-y)oxy)méthyl]-2-(3,5,5-triméthyl-2,4-dioxo-1-imidazolidinyl)propyl]-N-hydroxyformamide
(±)-N-[1-[[4'-cyano-[1,1'-biphényl]-4-y)oxy]méthyl]-2-isopropylthioéthyl]-N-hydroxyformamide
(±)-N-[1-[[(3'-cyanométhyl-[1,1'-biphényl]-4-y)oxyméthyl]-2-(3,4,4-triméthyl-2,5-dioxo-1-imidazolidinyl)éthyl]-N-hydroxyformamide
(+)-N-[1-[[(4'-cyano-[1,1'-biphényl]-4-yl)oxy)méthyl] -2-(3-éthyl-4,4-diméthyl-2,5-dioxo-1-imidazolidinyl)éthyl]-N-hydroxyformamide
(±)-N-[1-[[(4'-cyano-[1,1'-biphényl]-4-yl)oxy)méthyl]-2-(3-benzyl-4,4-diméthyl-2,5-dioxo-1-imidazolidinyl)éthyl]-N-hydroxyformamide
(±)-N-[1-[[(4'-cyano-[1,1'-biphényl]-4-y)oxy]méthyl]-2-(3,5,5-triméthyl-2,4-dioxo-1-imidazolidinyl)éthyl]-N-hydroxyformamide
(±)-N-[1-[[4'-méthoxy[1,1'-biphényl]-4-yl)sulfonyl]méthyl] thyl]-N-hydroxyformamide
(±)-N-[1-[[(4'-chloro[1,1'-biphényl]-4-yl)sulfonyl] méthyl]éthyl]-N-hydroxyformamide
(±)-N-[1-[[(4-(1,3-benzodioxol-5-yl)phényl] sulfonyl]méthyl)éthyl]-N-hydroxyformamide
(±)-N-[1-[[[4-(4-chlorophénoxy)phényl]sulfonyl) méthyl]éthyl]-N-hydroxyformamide
(±)-N-[1-[[(4'-méthoxy[1,1'-biphényl]-4-yl)sulfonyl] méthyl]propyl]-N-hydroxyformamide
(±)-N-[1-[1,1-diméthyl-2-[(4'-(trifluorométhyl)[1,1'-biphényl]-4-yl]sulfonyl]éthyl]-N-hydroxyformamide
(±)-N-[1-[(phénylméthoxy)méthyl]-2-[[4'-(trifluorométhyl)[1,1'-biphényl]-4-yl)sulfonyl]éthyl]-N-hydroxyformamide
(±)-N-[1-(hydroxyméthyl)-2-[[(4'-(trifluorméthyl)[1,1'-biphényl]-4-yl]sulfonyl]éthyl]-N-hydroxyformamide
(±)-N- [1-[(4,4-diméthyl-2,5-dioxo-1-imidazolidinylméthyl]-2-[[4'-(trifluorométhyl)[1,1'-biphényl]-4-yl]thio]éthyl]-N-hydroxyformamide
(±)-N-[1-[(4,4-diméthyl-2,5-dioxo-1-imidazolidinylméthyl]-2-[[4'-(trifluorométhyl)[1,1'-biphényl]-4-yl]sulfonyl]éthyl]-N-hydroxyformamide
(±)-N-[1-[(2,5-dioxo-1-imidazolidinyl)méthyl]-2-[ [4'-(trifluorométhoxy)[1,1'-biphényl)-4-yl]oxy]éthyl]-N-hydroxyformamide
(±)-N-[1-[[[4'-(trifluorométhyl)[1,1'-biphényl]-4-yl)sulfonyl]méthyl]-2-(3,4,4-triméthyl-2,5-dioxo-1-imidazolidinyl)éthyl]-N-hydroxyformamide
(±)-N-[1-[['4'-bityl[1,1'-biphényl]-4-yl)oxy]méthyl]-2-(3-méthy-2,5-dioxo-1-imidazolidinyl]éthyl]-N-hydroxyformamide
(±)-N-[1-[(3-méthy-2,5-dioxo-1-imidazolidinyl)méthyl]-2-[[4'-(trifluorométhoxy)[1,1'-biphényl]-4-yl]oxy]éthyl]-N-hydroxyformamide
N-[4-[4-[(4'-chloro[1,1'-biphényl]-4-yl)sulfonyl] méthyl]tétrahydro-2H-pyran-4-yl]-N-hydroformamide
(±)-N-[1-[[[4-(4-chlorophénoxy)phényl]sulfonyl]méthyl]-2-(4,4-diméthyl-2,5-dioxo-1-imidazolidinyl)éthyl]-N-hydroxyformamide
(±)-N-[1-[[[4-(4-chlorophénoxy)phényl]sulfonyl]méthyl]-2-(3,4,4-triméthyl-2,5-dioxo-1-imidazolidinyl)éthyl]-N-hydroxyformamide
(±)-N-[1-[[(4-butyl[1,1'-biphényl]-4-yl)sulfonyl)méthyl]-2-(3,4,4-triméthyl-2,5-dioxo-1-imidazolidinyl)éthyl]-N-hydroxyformamide
(±)-N-[1-[[(4'-butyl[1,1'-biphényl]-4-yl)oxy]méthyl] -2-(4,4-diméthyl-2,5-dioxo-1-imidazolidinyl)éthyl]-N-hydroxyformamide
(±)-N-[1-[[[3'-(cyanométhyl)[1,1'-biphényl]-4-yl]oxy] méthyl]-2-(4,4-diméthyl-2,5-dioxo-1-imidazolidinyl)éthyl]-N-hydroxyformamide
(±)-N-[1-[4- (2-thiényl)phénoxy]méthyl]-2-[1-(3,4,4-triméthyl-2,5-dioxo-1-imidazolidinyl]éthyl]-N-hydroxyformamide
(±)-N-[1-[[(3-nitro[1,1'-biphényl]-4-yl)oxy]méthyl]-2-(3,4,4-triméthyl-2,5-dioxo-1-imidazolidinyl)éthyl]-N-hydroxyformamide
(±)-N-[1-[[(4'-méthyl[1,1'-biphényl]-4-yl)oxy]méthyl]-2-[[3-(méthylsulfonyl)-amino]phényl]éthyl]-N-hydroxyformamide
(±)-N-[1-[[[3-(diéthylamine)carbonyl]phényl]méthyl]-2-[(4'-méthyl[1,1'-biphényl]-4-yl)oxy]éthyl]-N-hydroxyformamide
(±)-N-[1-[[(4'-cyano[1,1-biphényl]-4-yl)oxy]méthyl]-2-[(4'-cyano[1,1'-biphényl]-4-yl)oxy]éthyl]-N-hydroxyformamide
(±)-N-[1-[[(4'-cyano[1,1'-biphényl]-4-yl)oxy]méthyl]-2-(4,4-diméthyl-2,5-dioxo-1-imidazolidinyl)éthyl]-N-hydroxyformamide
(±)-N- [1-[(4,4-diméthyl-2,5-dioxo-1-imidazolidinylméthyl]-2-[[4'-(2-méthoxyéthoxy)[1,1-biphényl]-4-yl]oxy]éthyl]-N-hydroxyformamide
(±)-N-[1-[(4,4-diméthyl-2,5-dioxo-1-imidazolidinyl) méthyl]-2-[(4'-propoxy[1,1'-biphényl]-4-yl)oxy]éthyl]-N-hydroxyformamide
(±)-N-[1-[(4,4-diméthyl-2,5-dioxo-1-imidazolidinyl)méthyl]-2-[(4'-pentyloxy[1,1'-biphényl]-4-yl)oxy]éthyl]-N-hydroxyformamide
(±)-N-[1-[[[(3'-(cyanométhyl)[1,1'-biphényl]-4-yl]sulfonyl] méthyl]-3-(4,4-diméthyl-2,5-dioxo-1-imidazolidinyl)propyl]-N-hydroxyformamide
(±)-N-[1- [[[4'-(trifluorométhoxy)[1,1'-biphényl] -4-yl] sulfonyl]méthyl]-2-(3,4,4-triméthy-2,5-dioxo-1-imidazo-lidinyl)éthyl]-N-hydroxyformamide
(±)-N-[1-[[(4'-cyano[1,1'-biphényl]-4-yl)sulfonyl]méthyl]-2-(3-méthy-2,5-dioxo-1-imidazolidinyl)éthyl]-N-hydroxyformamide
(±)-N-[1-[[[3'-(cyanométhyl)[1,1'-biphényl]-4-yl] sulfonyl]méthyl]-2-(3,4,4-triméthyl-2,5-dioxo-1-imidazolinidyl)éthyl]-N-hydroxyformamide
(±)-N-[1-[[(4'-cyano[1,1''-biphényl]-4-yl)oxy]méthyl]-2-(1,6-dihydro-3-méthyl-6-oxo-pyridazinyl)éthyl]-N-hydroxyformamide
(±)-N-[1-[[(4'-cyano[1,1''-biphényl]-4-yl) sulfonyl]méthyl]-2-(4,4-diméthyl-2,5-dioxo-1-imidazolidinyl)éthyl]-N-hydroxyformamide
(±)-N-[1-[[[4-(4-fluorophénoxy)phényl]sulfonyl]méthyl]-2-(4,4-diméthyl-2,5-dioxo-1-imidazolidinyl)éthyl]-N-hydroxyformamide
(±)-N-[1-[[4- (4-pyridinyl)phénoxy]méthyl]-2- (3,4,4-triméthyl-2,5-dioxo-1-imidazolidinyl)éthyl]-N-hydroxyformamide
(S)-N-[1-[(4,4-diméthyl-2,5-dioxo-1-imidazolidinyl) méthyl]-2-[[4'-(trifluorométhoxy)[1,1'-biphényl]-4-yl]oxy]éthyl]-N-hydroxyformamide
(R)-N-[1-[(4,4-diméthyl-2,5-dioxo-1-imidazolidinyl) méthyl]-2-[[4'-(trifluorométhoxy)[1,1'-biphényl]-4-yl]-oxy]éthyl]-N-hydroxyformamide
N-[1-[[[4'-(trifluorométhoxy))[1,1'-biphényl]-4-yl]oxy]méthyl]-3-(4,4-diméthyl-2,5-dioxo-1-imidazolidinyl)propyl]-N-hydroxyformamide
N-[1-[4-[(4-pyridinylthio)phénoxyl]méthyl]-2-(4, 4-diméthyl-2,5-dioxo-1-imidazolidinyl)éthyl]-N-hydroxyformamide
N-[1-[[[(4-chlorophénoxy)phényl] sulfonyl]méthyl]-3-(4,4-diméthyl-2,5-dioxo-1-imidazolidinyl)propyl]-N-hydroxyformamide
N-[1-[[(4'-cyano[1,1'-biphényl]-4-yl)oxyl)méthyl]-2-(1,6-dihydro-6-oxo-1-pyridazinyl)éthyl]-N-hydroxyformamide
N-[1-[[[4'-(aminosulfonyl)[1,1'-biphényl]-4-yl]oxy] méthyl] -2-(3,4,4-triméthyl-2,5-dioxo-1-imidazolidinyl)éthyl]-N-hydroxyformamide
N-[1-[[[4'-(trifluorométhoxy)[1,1'-biphényl]-4-yl] sulfonyl]méthyl]-2-(4,4-diméthyl-2,5-dioxo-1-imidazolidinyl)éthyl]-N-hydroxyformamide
N-[1-[4-[(4-pyridinyloxy)phényl]sulfonyl]éthyl]-N-hydroxyformamide
N-[1-[[[(4-cyanophénoxy)phényl]sulfonyl]méthyl]-2-(4,4-diméthyl-2,5-dioxo-1-imidazolidinyl)éthyl]-N-hydroxyformamide
N-[1-[[4-[[4-(trifluorométhoxy)phénoxy]phényl] sulfonyl]méthyl]-3-(4,4-diméthyl-2,5-dioxo-1-imidazolidinyl)propyl]-N-hydroxyformamide et
(±)-N-[1-[[[3'-(cyanométhyl)[1,1'-biphényl]-4-yl]sulfonyl]méthyl]-2-(3,4,4-triméthyl-2,5-dioxo-1-imidazolidinyl)éthyl]-N-hydroxyformamide
(±)-N- [1- [ [4- (4-pyrdinyl)phénoxyl]méthyl]-2- (3,4,4-triméthy-2,5-dioxo-1-imidazolidinyl)éthyl]-N-hydroxyformamide
N-[1-[[[4'-(aminosulfonyl)[1,1'-biphényl]-4-yl] oxy]méthyl]-2-(3,4,4-triméthyl-2,5-dioxo-1-imidazolidinyl)éthyl]-N-hydroxyformamide et
N- [1-[[4-[[4-(trifluorométhoxy)phénoxyl] sulfonyl]méthyl]-2-(4,4-diméthyl-2,5-dioxo-1-imidazolidinyl)éthyl]-N-hydroxyformamide.

13. Composé selon la revendication 1 dans lequel R₁ et R₂, pris ensemble avec l'atome de carbone auquel ils sont fixés, forment un anneau de tétrahydropyranyle.

14. Composé selon la revendication 13 choisi dans le groupe composé de
N-[4-[4-[[(4-chlorophénoxy)phényl]sulfonyl] méthyl]tétrahydro-2H-pyran-4-yl]-N-hydroxyformamide et
N-[4-[4-[(4'-chloro[1,1'-biphényl]-4-yl) sulfonyl]méthyl]tétrahydro-2H-pyran-4-yl]-N-hydroxyformamide.

15. Composé choisi dans le groupe composé de
(±)-N-[1-[[(4'-cyano-[1,1'-biphényl]-4-yl)oxy] méthyl]-2-phénoxyéthyl]-N-hydroxyformamide
(±)-N-[1-[[(4'-cyano-[1,1'-biphényl]-4-yl)oxy] méthyl] -2-(2,3-dihydro-1,3-dioxo-1H-isoindol-2-yl)éthyl]-N-hydroxyformamide
(±)-N-[1-[[(4'-cyano-[1,1'-biphényl]-4-yl)oxy] méthyl]-2-(3,4,4-triméthyl-2,5-dioxoimidazolidine-1-yl)éthyl]-N-hydroxyformamide
(±)-N-[1-[[(4'-cyano-[1,1'-biphényl]-4-yl)oxy] méthyl]-3-(3,4,4-triméthyl-2,5-dioxoimidazolidine-1-yl)propyl]-N-hydroxyformamide
(±)-N-[1-[[[3'-(cyanométhyl)-[1,1'-biphényl]-4-yl]oxy] méthyl]pentyl]-N-hydroxyformamide
(±)-N-[1-[[(4'-cyano-[1,1'-biphényl]-4-yl)oxy] méthyl]-3-méthylbutyl]-N-hydroxyformamide
(±)-N-[1-[[(4'-cyano-[1,1'-biphényl]-4-yl)oxy] méthyl]-2-méthylbutyl]-N-hydroxyformamide
(±)-N-[1-[[(4'-cyano-[1,1'-biphényl]-4-yl)oxy] méthyl]pentyl]-N-hydroxyformamide
(±)-N-[1-[[(4'-cyano-[1,1-biphényl]-4-yl)oxy] méthyl] -2-(4-méthylphényl)éthyl]-N-hydroxyformamide
(±)-N-[2-[(4'-cyano-[1,1'-biphényl]-4-yl)oxy]-1-(4-fluorophényl)éthyl]-N-hydroxyformamide
(±)-N-[1-[[(4'-cyano-[1,1'-biphényl]-4-yl)oxy] méthyl]-2-(4-fluorophényl)éthyl]-N-hydroxyformamide
(±)-N-[1-[[(4'-cyano-[1,1'-biphényl]-4-yl)oxy] méthyl]éthyl]-N-hydroxyformamide
(±)-N-[2-[(4'-cyano-[1,1'-biphényl]-4-yl)oxy] éthyl]-N-hydroxyformamide
(±)-N-[1-[4- [(2^{E}-phényléthényl)phénoxy]méthyl]-2-(3,4,4-triméthyl-2,5-dioxo-1-imidazolidinyl)éthyl]-N-hydroxyformamide
(±)-N-[1-[[4-(2-furanyl)phénoxy]méthyl]-2-(3,4,4-triméthyl-2,5-dioxo-1-imidazolidinyl)éthyl]-N-hydroxyformamide
(±)-N-[1-[[(4'-butoxyl[1,1-biphényl] -4-yl)oxy] méthyl]-2-(3,4,4-triméthyl-2,5-dioxo-1-imidazolidinyl)éthyl]-N-hydroxyformamide
(±)-N-[1-[[(4'-fluoro[1,1'-biphényl]-4-yl)oxy] méthyl]-2-(3,4,4-triméthyl-2,5-dioxo-1-imidazolidinyl)éthyl]-N-hydroxyformamide
(±)-N-[1-[(3,4,4-triméthyl-2,5-dioxo-1-imidazolidinyl)méthyl]-2-[[4'-(trifluorométhyl) [1,1'-biphényl]-4-yl]oxy]éthyl]-N-hydroxyformamide
(±)-N-[1-[[(4'-méthoxy[1,1'-biphényl]-4-yl)oxy] méthyl]-2-(3,4,4-triméthyl-2,5-dioxo-1-imidazolidinyl) éthyl]-N-hydroxyformamide
(±)-N-[1-[[(4'-méthyl[1,1'-biphényl]-4-yl)oxy] méthyl]-2-(3,4,4-triméthyl-2,5-dioxo-1-imidazolidinyl) éthyl]-N-hydroxyformamide
(±)-N-[1-[[(4'-butoxy[1,1'-biphényl]-4-yl)oxy] méthyl]-2-(4,4-diméthyl-2,5-dioxo-1-imidazolidinyl) éthyl]-N-hydroxyformamide
(±)-N-[1-[(4,4-diméthyl-2,5-dioxo-1-imidazolidinyl)méthyl]-2-[(4'-éthoxy[1,1'-biphényl]-4-yl)oxy]éthyl]-N-hydroxyformamide
(±)-N-[1- [[4-(1,3-benzodioxol-5-yl]phénoxy]méthyl]-2-(4,4-diméthyl-2,5-dioxo-1-imidazolidinyl) éthyl]-N-hydroxyformamide
(±)-N- [1-[[(4'-butoxy[1,1'-biphényl]-4-yl)oxy] méthyl]-2-(3-méthyl-2,5-dioxo-1-imidazolidinyl)éthyl]-N-hydroxyformamide
(±)-N-[1-[[4-(3-thiényl)phénoxy]méthyl]-2-(3,4,4-triméthyl-2,5-dioxo-1-imidazolidinyl)éthyl]-N-hydroxyformamide
(±)-N-[1-[[([1,1'-biphényl]-4-yl)oxy]méthyl]-2-(3,4,4-triméthyl-2,5-dioxo-1-imidazolidinyl)éthyl]-N-hydroxyformamide
(±)-N-[1-[[(3'-chloro-4'-fluoro[1,1-biphényl]-4-yl)oxy]méthyl]-2-(3,4,4-triméthyl-2,5-dioxo-1-imidazolidinyl)éthyl]-N-hydroxyformamide
(±)-N-[1-[[(2'-méthyl [1,1'-biphényl]-4-yl)oxy] méthyl] -2-(3,4,4-triméthy-2,5-dioxo-1-imidazolidinyl) éthyl]-N-hydroxyformamide
(±)-N-[1-[[(4'-cyanol[1,1'-biphényl]-4-yl)oxy] méthyl]-2-(2,5-dioxo-1-imidazolidinyl)éthyl]-N-hydroxyformamide
(±)-N-[1-[[(4'-cyanol[1,1'-biphényl]-4-yl)oxy] méthyl]-2-(1,1-dioxido-3-oxo-1,2-benzisothiazol-2(3H)-yl)éthyl]-N-hydroxyformamide
(±)-N-[1-[(4,4-diméthyl-2,5-dioxo-1-imidazolidinyl)méthyl]-2-[[4'-(trifluorométhoxy)[1,1'-biphényl]-4-yl]oxy]éthyl]-N-hydroxyformamide
(±)-N-[1- [[4-(4-phényl-1-pipéridinyl) phénoxy]méthyl]-2-(3,4,4-triméthyl-2,5-dioxo-1-imidazolidinyl)éthyl]-N-hydroxyformamide
(±)-N-[1-[(4,4-diméthyl-2,5-dioxo-1-imidazolidinyl)méthyl]-2-[[4'-(trifluorométhyl)[1,1'-biphényl]-4-yl]oxy]éthyl]-N-hydroxyformamide
(±)-N-[1-[[(3'-cyano[1,1'-biphényl]-4-yl)oxy]méthyl]-2-[méthyl[(4-méthylphényl)sulfonyl] amino] éthyl]-N-hydroxyformamide
(±)-N- [1-[[(4'-cyano[1,1'-biphényl]-4-yl)oxy] méthyl]-2-[4,4-diméthyl-2,5-dioxo-3-(3-pyridinylméthyl)-1-imidazolidinyl]éthyl]-N-hydroxyformamide
(±)-N-[2-[(4'-cyano[1,1'-biphényl]-4-yl)oxy]-1-méthylpropyl]-N-hydroxyformamide
(±)-N-[1-[[(3'-cyano[1,1'-biphényl]-4-yl)oxy] méthyl]-2-(3,4,4-triméthyl-2,5-dioxo-1-imidazolidinyl) éthyl]-N-hydroxyformamide
(±)-N-[1-[[[4'-(méthylthio)[1,1'-biphényl]-4-yl]oxy]méthyl]-2-(3,4,4-triméthyl-2,5-dioxo-1-imidazolidinyl)éthyl]-N-hydroxyformamide
(±)-N-[1-[4-[[4- (trifluorométhyl) phénoxy] phénoxy]méthyl]-2-(3,4,4-triméthyl-2,5-dioxo-1-imidazolidinyl)éthyl]-N-hydroxyformamide
(±)-N-[1-[[{4'-(trifluorométhoxy)[1,1'-biphényl]-4-yl]oxy]méthyl]-2-(3,4,4-triméthy-2,5-dioxo-1-imidazolidinyl)éthyl]-2-N-hydroxyformamide
(±)-N-[1-[[[4'-(méthylsulfonyl)[1,1'-biphényl]-4-yl]oxy]méthyl]-2-(3,4,4-triméthy-2,5-dioxo-1-imidazolidinyl)éthyl]-N-hydroxyformamide
(±)-N-[1-[[[3'-(cyanométhyl)-4'-méthoxy[1,1'-biphényl]-4-yl]oxy]méthyl]-2-(3,4,4-triméthy-2,5-dioxo-1-imidazolidinyl)éthyl]-N-hydroxyformamide
(±)-N-[1-[[[3'-(cyanométhyl)[1,1'-biphényl]-4-yl] oxy]méthyl]-3-(4,4-diméthyl-2,5-dioxo-1-imidazolidinyl) propyl]]-N-hydroxyformamide
(±)-N-[1-[[(4'-butoxy[1,1'-biphényl]-4-yl) sulfonyl]méthyl]-2-(4,4-diméthyl-2,5-dioxo-1-imidazolidinyl)éthyl]-N-hydroxyformamide
(±)-N-[1-[[(4'-cyano-[1,1'-biphényl]-4-yl)oxy] méthyl]-3-(4,4-diméthyl-2,5-dioxo-1-imidazolidinyl) propyl]-N-hydroxyformamide
(±)-N-[1-[[[4'-(méthylsulfonyl)[1,1'-biphényl]-4-yl]oxyméthyl]-2-(4,4-diméthyl-2,5-dioxo-1-imidazolidinyl) éthyl]-N-hydroxyformamide
(±)-N-[1-[[(3'-cyano[1,1'-biphényl]-4-yl)oxy] méthyl]-2-(2,5-dioxo-1-pyrrolidinyl)éthyl]-N-hydroxyformamide
(±)-N-[1-[[(4'-cyano[1,1'-biphényl]-4-yl)oxy] méthyl]-2-(4,4-diméthyl-2,6-dioxo-1-pipéridinyl)éthyl]-N-hydroxyformamide
(±)-N-[1S-[[(4'-cyano[1,1'-biphényl]-4-yl)oxy] méthyl]-2-(2,5-dioxo-1-pyrrolidinyl)éthyl]-N-hydroxyformamide
(±)-N-[1R-[[(4'-cyano[1,1'-biphényl]-4-yl)oxy] méthyl]-2-(2,5-dioxo-1-pyrrolidinyl)éthyl]-N-hydroxyformamide
(±)-N-[1-[[(4'-cyano[1,1'-biphényl]-4-yl)oxy] méthyl]-2-(3-éthyl-3-méthyl-2,5-dioxo-1-pyrrolidinyl) éthyl] -N-hydroxyformamide
N-{4-[4- [[(4-chlorophénoxy)phényl]sulfonyl] méthyl]tétrahydro-2H-pyran-4-yl]-N-hydroxyformamide
(±)-N-[1-[[(4'-cyano[1,1'-biphényl]-4-yl)oxy] méthyl]-5-[(4-méthyl-2-oxo-2H-1-benzopyran-6-yl)oxy] pentyl]}-N-hydroxyformamide
(±)-N-[1-[[(4'-cyano[1,1'-biphényl]-4-yl)oxy] méthyl]-4-[(4-méthyl-2-oxo-2H-1-benzopyran-6-yl)oxy] butyl]-N-hydroxyformamide
(±)-N-[1-[[(4'-cyano(1,1'-biphényl]-4-yl)oxy] méthyl]-4- [(4-méthyl-2-oxo-2H-1-benzopyran-7-yl]oxy] butyl]-N-hydroxyformamide
(±)-N-[1-[[(4'-cyano[1,1'-biphényl]-4-yl)oxy] méthyl]-5-[(4-méthyl-2-oxo-2H-1-benzopyran-7-yl)oxy] pentyl]-N-hydroxyformamide
(±)-N-[1-[[(4'-cyano[1,1'-biphényl]-4-yl)oxy] méthyl]-2-(5,5-diméthyl-2n4-dioxo-3-oxazolidinyl)éthyl]-N-hydroxyformamide
(±)-N-[1-[[(4'-cyano[1,1'-biphényl]-4-yl) sulfonyl]méthyl]-2- (3,4,4-triméthy-2,5-dioxo-1-imidazolidinyl]éthyl]-N-hydroxyformamide
(±)-N-[1-[[(4'-cyano[1,1'-biphényl]-4-yl)oxy] méthyl]-2-(3-méthyl-2,5-dioxo-1-imidazolidinyl)éthyl]-N-hydroxyformamide
(±)-N-[1-[[(4'-cyano[1,1'-biphényl]-4-yl) sulfonyl]méthyl]-2-(4,4-diméthyl-2,5-dioxo-1-imidazolidinyl)éthyl]-N-hydroxyformamide
(±)-N-[1-[[(4'-chloro-[1,1'-biphényl]-4-yl)oxy] méthyl] -2-(3,4,4-triméthyl-2,5-dioxo-1-imidazolidinyl) éthyl]-N-hydroxyformamide
(±)-N-[1-[ [(3'-cyanométhyl-[1,1'-biphényl]-4-yl)oxy]méthyl]-2-(3,5,5-triméthyl-2,4-dioxo-1-imidazolidinyl)propyl]-N-hydroxyformamide
(±)-N-[1-[[(4'-cyano-[1,1'-biphényl]-4-yl)oxy]méthyl]-2-isopropylthioéthyl]-N-hydroxyformamide
(±)-N-[1-[[(3'-cyanométhyl-[1,1'-biphényl]-4-yl)oxy]méthyl]-2-(3,4,4-triméthyl-2,5-dioxo-1-imidazolidinyl)éthyl]-N-hydroxyformamide
(±)-N-[1-[[(4'-cyano[1,1'-biphényl]-4-yl)oxy] méthyl]-2-(3-éthyl-4,4-diméthyl-2,5-dioxo-1-imidazolidinyl)éthyl]-N-hydroxyformamide
(±)-N-[1-[[(4'-cyano-[1,1'-biphényl]-4-yl)oxy]méthyl]-2-(3-benzyl-4,4-diméthyl-2,5-dioxo-1-imidazolidinyl)éthyl]-N-hydroxyformamide
(±)-N-[1-[[(4'-cyano-[1,1'-biphényl]-4-yl]oxy]méthyl]-2-(3,5,5-triméthyl-2,4-dioxo-1-imidazolidinyl)éthyl]-N-hydroxyformamide
(±)-N-[1-[[4'-méthoxy[1,1'-biphényl]-4-yl) sulfonyl]méthyl]éthyl]-N-hydroxyformamide
(±)-N-[1-[[(4'-chloro[1,1'-biphényl]-4-yl]sulfonyl]méthyl]éthyl]-N-hydroxyformamide
(±)-N-[1-[[[4-(1,3-benzodioxol-5-yl)phényl] sulfonyl]méthyl]éthyl]-N-hydroxyformamide
(±)-N-[1-[[[4-(4-chlorophénoxy)phényl]sulfonyl] méthyl]éthyl]-N-hydroxyformamide
(±)-N-[1-[[(4'-méthoxy[1,1'-biphényl]-4-yl)sulfonyl]méthyl]propyl]-N-hydroxyformamide
(±)-N-[1-(1,1-diméthyl-2-[(4'-(trifluorométhyl) [1,1'-biphényl-4-yl]sulfonyl]éthyl]-N-hydroxyformamide
(±)-N-[1-[(phénylméthoxy)méthyl]-2-[[4'-(trifluorométhyl) [1,1'-biphényl]-4-yl]sulfonyl]éthyl]-N-hydroxyformamide
(±)-N-[1-(hydroxyméthyl)-2-[[(4'-(trifluorométhyl)[1,1'-biphényl]-4-yl]sulfonyl]éthyl]-N-hydroxyformamide
(±)-N-[1-[(4,4-diméthyl-2,5-dioxo-1-imidazolidinyl)méthyl)-2-[[4'-(trifluorométhyl)[1,1'-biphényl]-4-yl]thio]éthyl]-N-hydroxyformamide
(±)-N-[1-[(4,4-diméthyl-2,5-dioxo-1-imidazolidinyl)méthyl]-2-[[4'-(trifluorométhyl)[1,1'-biphényl]-4-yl]sulfonyl]éthyl]-N-hydroxyformamide
(±) -N-[1-[(2,5-dioxo-1-imidazolidinyl)méthyl]-2-[ [4'-(trifluorométhoxy)[1,1'-biphényl]-4-yl]oxy]éthyl]-N-hydroxyformamide
(±)-N-[1-[[[4'-(trifluorométhyl)[1,1'-biphényl]-4-yl]sulfonyl)méthyl]-2-(3,4,4-triméthyl-2,5-dioxo-1-imidazolidinyl)éthyl]-N-hydroxyformamide
(±)-N- [1-[[(4'-butyl[1,1'-biphényl]-4-yl)oxy]méthyl]-2-(3-méthy-2,5-dioxo-1-imidazolidinyl) éthyl]-N-hydroxyformamide
(±)-N-[1-[(3-méthy-2,5-dioxo-1-imidazolidinyl) méthyl]-2-[[4'-(trifluorométhoxy][1,1'-biphényl]-4-yl]oxy]éthyl]-N-hydroxyformamide
N- [4- [4- [(4'-chloro[1,1'-biphényl]-4-yl) sulfonyl]méthyl]tétrahydro-2H-pyran-4-yl]-N-hydroxyformamide
(±)-N-[1-[[[4-(4-chlorophénoxy)phényl]sulfonyl] méthyl]-2-(4,4-diméthyl-2,5-dioxo-1-imidazolidinyl) éthyl]-N-hydroxyformamide
(±)-N-[1-[[[4-(4-chlorophénoxy)phényl) sulfonyl]méthyl]-2-(3,4,4-triméthyl-2,5-dioxo-1-imidazolidinyl)éthyl]-N-hydroxyformamide
(±)-N-[1-[[(4-butyl[1,1'-biphényl]-4-yl) sulfonyl]méthyl]-2-(3,4,4-triméthyl-2,5-dioxo-1-imidazolidinyl)éthyl]-N-hydroxyformamide
(±)-N-[1-[[(4'-butyl[1,1'-biphényl]-4-yl)oxy] méthyl]-2-(4,4-diméthyl-2,5-dioxo-1-imidazolidinyl) éthyl]-N-hydroxyformamide
(±)-N-[1-[[[3'-(cyanométhyl)[1,1'-biphényl]-4-yl]oxy]méthyl]-2-(4,4-diméthyl-2,5-dioxo-1-imidazolidinyl)éthyl]-N-hydroxyformamide
(±)-N-[1-[4-(2-thiényl)phénoxy]méthyl]-2-[1-(3,4,4-triméthyl-2,5-dioxo-1-imidazolidinyl)éthyl]-N-hydroxyformamide
(±)-N-[1-[[(3-nitro[1,1'-biphényl]-4-yl)oxy] méthyl]-2-(3,4,4,-triméthyl-2,5-dioxo-1-imidazolidinyl) éthyl]-N-hydroxyformamide
(±)-N-[1-[[(4'-méthyl[1,1'-biphényl]-4-yl)oxy] méthyl]-2-[[3-(méthylsulfonyl)-amino]phényl]éthyl]-N-hydroxyformamide
(±)-N-[1-[[[3-(diéthylamino)carbonyl]phényl] méthyl]-2-[(4'-méthyl[1,1'-biphényl]-4-yl)oxy]éthyl]-N-hydroxyformamide
(±)-N-[1-[[(4'-cyano[1,1'-biphényl]-4-yl)oxy] méthyl]-2-[(4'-cyano[1,1'-biphényl]-4-yl)oxy]éthyl]-N-hydroxyformamide
(±)-N-[1-[[(4'-cyano[1,1'-biphényl]-4-yl)oxy] méthyl]-2-(4,4-diméthyl-2,5-dioxo-1-imidazolidinyl) éthyl]-N-hydroxyformamide
(±)-N-[1-[(4,4-diméthyl-2,5-dioxo-1-imidazolidinyl)méthyl]-2-[[4'-(2-méthoxyéthoxy)[1,1'-biphényl]-4-yl]oxy]éthyl]-N-hydroxyformamide
(±)-N-[1-[(4,4-diméthyl-2,5-dioxo-1-imidazolidinyl)méthyl]-2-[(4'-propoxy[1,1'-biphényl] -4-yl)oxy]éthyl]-N-hydroxyformamide
(±)-N-[1-[(4,4-diméthyl-2,5-dioxo-1-imidazolidinyl]méthyl]-2-[(4'-pentyloxy[1,1'-biphényl]-4-yl)oxy]éthyl]-N-hydroxyformamide
(±)-N-[1-[[[3'-(cyanométhyl)[1,1'-biphényl]-4-yl]sulfonyl]méthyl]-3-(4,4-diméthyl-2,5-dioxo-1-imidazolidinyl)propyl]-N-hydroxyformamide
(±)-N-[1-[[[4'-(trifluorométhoxy)[1,1'-biphényl]-4-yl]sulfonyl]méthyl]-2-(3,4,4-triméthy-2,5-dioxo-1-imidazolidinyl)éthyl]-N-hydroxyformamide
(±)-N-[1-[[(4'-cyano[1,1'-biphényl]-4-yl) sulfonyl]méthyl]-2-(3-méthy-2,5-dioxo-1-imidazolidinyl) éthyl] -N-hydroxyformamide
(±)-N-[1-[[[3'-(cyanométhyl)[1,1'-biphényl]-4-yl]sulfonyl]méthyl]-2-(3,4,4-triméthyl-2,5-dioxo-1-imidazolidinyl)éthyl]-N-hydroxyformamide
(±)-N-[1-[[(4'-cyano[1,1''-biphényl]-4-yl)oxy] méthyl]-2-(1,6-dihydro-3-méthyl-6-oxo-1-pyridazinyl) éthyl]-N-hydroxyformamide
(±)-N-[1-[[(4'-cyano[1,1'-biphényl]-4-yl)sulfonyl]méthyl]-2-(4,4-diméthyl-2,5-dioxo-1-imidazolidinyl)éthyl]-N-hydroxyformamide
(±)-N-[1-[[[4-(4-fluorophénoxy)phényl]sulfonyl] méthyl]-2-(4,4-diméthyl-2,5-dioxo-1-imidazolidinyl) éthyl]-N-hydroxyformamide
(±)-N-[1-[[4-(4-pyridinyl)phénoxy]méthyl]-2-(3,4,4-triméthy-2,5-dioxo-1-imidazolidinyl)éthyl]-N-hydroxyformamide
(S)-N-[1-[(4,4-diméthyl-2,5-dioxo-1-imidazolidinyl)méthyl]-2-[[4'-(trifluorométhoxy) [1,1'-biphényl]-4-yl]oxy]éthyl]-N-hydroxyformamide
(R)-N-[1-[(4,4-diméthyl-2,5-dioxo-1-imidazolidinyl)méthyl]-2-[[4'-(trifluorométhoxy)[1,1'-biphényl]-4-yl]oxy]éthyl]-N-hydroxyformamide
N-[1-[[[4'-(trifluorométhoxy))[1,1'-biphényl]-4-yl]oxy]méthyl]-3-(4,4-diméthyl-2,5-dioxo-1-imidazolidinyl)propyl]-N-hydroxyformamide
N- [1-[4-[(4-pyridinylthio)phénoxy]méthyl]-2- (4,4-diméthyl-2,5-dioxo-1-imidazolidinyl)éthyl]-N-hydroxyformamide
N-[1-[[[(4-chlorophénoxy)phényl]sulfonyl]méthyl]-3-(4,4-diméthyl-2,5-dioxo-1-imidazolidinyl)propyl]-N-hydroxyformamide
N-[1-[[(4'-cyano[1,1"-biphényl]-4-yl)oxyl] méthyl]-2-(1,6-dihydro-6-oxo-1-pyridazinyl)éthyl] -N-hydroxyformamide
N-[1-[[[4'-(aminosulfonyl)[1,1'-biphényl]-4-yl]oxy]méthyl]-2-(3,4,4-triméthyl-2,5-dioxo-1-imidazolidinyl)éthyl] -N-hydroxyformamide
N-[1-[[[4'-(trifluorométhoxy)[1,1'-biphényl]-4-yl]sulfonyl]méthyl]-2-(4,4-diméthyl-2,5-dioxo-1-imidazolidinyl)éthyl] -N-hydroxyformamide
N- [1-[4-[(4-pyridinyloxy)phényl]sulfonyl]]éthyl]-N-hydroxyformamide
N-[1-[[[(4-cyanophénoxy)phényl]sulfonyl]méthyl]-2-(4,4-diméthyl-2,5-dioxo-1-imidazolidinyl)éthyl]-N-hydroxyformamide
N-[1-[[4-[[4-(trifluorométhoxy)phénoxy] phényl]sulfonyl]méthyl]-3- (4,4-diméthyl-2,5-dioxo-1-imidazolidinyl)propyl]-N-hydroxyformamide ;et
N- [1-[[4-[[4-(trifluorométhoxy)phénoxy] phényl]sulfonyl]méthyl]-2- (4,4-diméthyl-2, 5-dioxo-1-imidazolidinyl)éthyl] -N-hydroxyformamide.

16. Composé selon la revendication 15 qui est du (S) -N-[1-[(4,4-diméthyl-2,5-dioxo-1-imidazolidinyl) méthyl]-2-[[4'-(trifluorométhoxy)[1,1'-biphényl]-4-yl]oxy]éthyl]-N-hydroxyformamide.

17. Utilisation d'un composé selon la revendication 1 pour la fabrication d'un médicament permettant d'inhiber les métalloprotéinases matricielles chez un mammifère nécessitant un tel traitement.

18. Composition pour inhiber les métalloprotéinases matricielles comprenant un support pharmaceutique et une quantité thérapeutiquement efficace d'un composé selon la revendication 1.
